# EUROPEAN PATENT APPLICATION

(11) **EP 4 837 953 A2**
(43) Date of publication of application: **11.11.2026**
(21) Application number: 26193204.0
(22) Date of filing: 17.10.2019
(51) Int. Cl.: C12M 1/30

(54) **PROTO-ANTIGEN-PRESENTING SYNTHETIC SURFACES, ACTIVATED T CELLS, AND USES THEREOF**

(30) Priority: 18.10.2018 US 201862747569 P
(62) Document number(s) of the earlier application(s) in accordance with Art. 76 EPC: 19874740.4 / 3 867 353
(71) Applicant: Bruker Spatial Biology, Inc., Seattle, WA 98109-5553 (US)
(72) Inventor: BEEMILLER, Peter J., Emeryville, CA 94608 (US); MASTROIANNI, Alexander J., Emeryville, CA 94608 (US); PEI, Shao Ning, Emeryville, CA 94608 (US); LOWE, Jr., Randall D., Emeryville, CA 94608 (US); MOCCIARO, Annamaria, Emeryville, CA 94608 (US); LOUTHERBACK, Kevin D., Emeryville, CA 94608 (US); BRONEVETSKY, Yelena, Emeryville, CA 94608 (US); STADLER, Guido K., Emeryville, CA 94608 (US); MCFARLAND, Andrew W., Emeryville, CA 94608 (US); CHAPMAN, Kevin T., Emeryville, CA 94608 (US); SMITH, Duane, Emeryville, CA 94608 (US); MARKS, Natalie C., Emeryville, CA 94608 (US); GOODSELL, Amanda L., Emeryville, CA 94608 (US)
(74) Representative: D Young & Co LLP

(57) **Abstract**

Proto-antigen-presenting surfaces and related kits, methods, and uses are provided. The proto-antigen-presenting surface can comprise a plurality of primary activating molecular ligands comprising a major histocompatibility complex (MHC) molecule configured to bind to a T cell receptor (TCR) of a T cell and a plurality of of co-activating molecular ligands each including a TCR co-activating molecule or an adjunct TCR activating molecule, wherein an exchange factor is bound to the MHC molecules. Exchange factors include, e.g., dipeptides such as GL, GF, GR, etc. Proto-antigen-presenting surfaces can be used to rapidly prepare antigen-presenting surfaces comprising one or more peptide antigens of interest by contacting the proto-antigen-presenting surface with one or more peptide antigens so as to displace the exchange factor. As such, the disclosure facilitates rapid evaluation of the immunogenicity of peptide antigens for activating T lymphocytes.

## Description

This application claims the benefit of priority of US Provisional Patent Application No. 62/747,569, filed October 18, 2018, which is incorporated by reference herein for all purposes.

The present application is filed with a Sequence Listing in electronic format. The Sequence Listing is provided as a file entitled "2019-10-17_01149-0016_Seq_List_ST25.txt" created on October 17, 2019, which is 2 KB in size. The information in the electronic format of the sequence listing is incorporated herein by reference in its entirety.

### INTRODUCTION AND SUMMARY

Immunotherapy offers a potentially powerful approach to treating cancers successfully. T lymphocyte activation is one aspect of preparing tumor-targeting cytotoxic T lymphocytes for use in immunotherapy. Identifying immunogenic antigen peptide sequences from tumor-associated antigens or other disease-associated antigens that can be used to activate T lymphocytes can facilitate such activation.

T lymphocytes become activated though exposure to an antigen presented by a major histocompatibility complex (MHC) together with one or more coactivating stimuli. The MHC generally binds tightly to a peptide antigen and does not fold properly without a peptide antigen, meaning that preparation of an MHC bound to a peptide antigen of interest for use in T cell activation has been non-trivial, including in situations where there are multiple possible antigens of interest that one desires to evaluate for immunogenicity. Heuristic models based on known antigens can be used to identify potential novel peptide antigens, but these models may suffer from a high false-positive rate. Accordingly, there is a need for rapid verification of the immunogenicity of peptide antigens. More generally, T cell activation may be improved by using more reproducible and better characterizable technologies.

As discussed futher herein, exchange factors, such as dipeptides (e.g., Glycine-Xaa where Xaa is Leu, Phe, Val, Arg, Met, norleucine, homoleucine, or cyclohexylalanine) can react with an MHC, which is already or subsequently becomes surface-associated, to generate a proto-antigen-presenting surface. Such proto-antigen-presenting surfaces can then serve as substrates for generating antigen-presenting surfaces through displacement of the exchange factor with a peptide antigen. The antigen-presenting surfaces can then activate T cells if the peptide antigen is immunogenic. Thus, T cell activation provides a readout of peptide antigen immunogenicity.

The presently disclosed proto-antigen-presenting surfaces and related methods and uses can provide benefits such as more rapid evaluation of peptide antigen immunogenicity because the relatively laborious process of folding the MHC need not be performed with a peptide antigen of interest and need not be performed with each individual member of a set of peptide antigens being evaluated for immunogenicity. For example, an exemplary method comprises folding an MHC with an initial peptide, which may or may not be a peptide antigen of interest or may be any of the initial peptides described herein, and preparing a proto-antigen-presenting surface by associating the MHC with a suitable surface and contacting the MHC with an exchange factor to displace the initial peptide. The contacting step may occur before or after the associating step. An antigen-presenting surface can be prepared by contacting the proto-antigen-presenting surface with one or more peptide antigens of interest (e.g., one or more pools of peptide antigens) such that the one or more peptide antigens of interest displace the exchange factor and become associated with the MHC. The resulting surface can then be used to evaluate peptide antigen immunogenicity, e.g., by determining whether or to what extent it activates T lymphocytes. Additional embodiments include kits for preparing proto-antigen-presenting surfaces or antigen-presenting surfaces comprising an exchange factor and an MHC associated with a surface; methods of using antigen-presenting surfaces prepared as described herein to activate T lymphocytes; T lymphocytes prepared according to such methods; and methods of using such T lymphocytes, e.g., to treat diseases such as cancer. Further additional embodiments are described below.

Embodiment 1 is a kit for generating an antigen-presenting surface, the kit comprising:
(a) a covalently functionalized synthetic surface;
(b) a primary activating molecule that includes a major histocompatibility complex (MHC) molecule configured to bind to a T cell receptor (TCR), and a first reactive moiety configured to react with or bind to the covalently functionalized surface;
   and
(c) at least one of: an exchange factor (e.g., provided separately from the primary activating molecule); and an exchange factor bound to the MHC molecule or an initial peptide bound to the MHC molecule, optionally wherein the initial peptide is non-immunogenic.

Embodiment 2 is the kit of embodiment 2 further comprising one or more of:
at least one co-activating molecule that includes a second reactive moiety configured to react with or bind to the covalently functionalized surface, wherein each co-activating molecule is selected from a TCR co-activating molecule and an adjunct TCR activating molecule;
a surface-blocking molecule capable of covalently binding to the covalently functionalized synthetic surface;
a buffer suitable for performing an exchange reaction; and
instructions for performing an exchange reaction wherein a peptide antigen displaces the exchange factor.

Embodiment 3 is the kit of embodiment 1 or 2 comprising: the exchange factor, wherein the exchange factor is provided separately from the primary activating molecule; and the initial peptide bound to the MHC molecule.

Embodiment 4 is a method of forming a proto-antigen-presenting surface, the method comprising:
synthesizing a plurality of major histocompatibility complex (MHC) molecules in the presence of initial peptide, thereby forming a plurality of complexes each comprising an MHC molecule and an initial peptide; or
synthesizing a plurality of major histocompatibility complex (MHC) molecules in the presence of exchange factor, thereby forming a plurality of complexes each comprising an MHC molecule and an exchange factor; or
reacting a plurality of MHC molecules with exchange factor, thereby forming a plurality of complexes each comprising an MHC molecule and an exchange factor;
wherein: (i) a plurality of primary activating molecules comprise the MHC molecules and first reactive moieties, or (ii) a plurality of primary activating molecules is prepared by adding first reactive moieties to the MHC molecules, and
the method further comprises reacting the first reactive moieties of the plurality of primary activating molecules with a first plurality of binding moieties disposed on a covalently functionalized synthetic surface, thereby forming the proto-antigen-presenting surface; optionally wherein the initial peptide is non-immunogenic.

Embodiment 5 is the method of embodiment 4 further comprising reacting the plurality of MHC molecules synthesized in the presence of the initial peptide with exchange factor, optionally in the presence of a peptide antigen.

Embodiment 6 is a method of analyzing stability of a complex comprising a major histocompatibility complex (MHC) molecule and a peptide antigen, wherein the MHC molecule is configured to bind to a T cell receptor (TCR), the method comprising:
contacting a plurality of the MHC molecules with the peptide antigen and an exchange factor, thereby forming peptide antigen-bound MHC molecules, optionally wherein an initial peptide is bound to the MHC molecules before contact with the peptide antigen and exchange factor;
wherein
(i) a plurality of primary activating molecules comprise the MHC molecules and first reactive moieties or (ii) a plurality of primary activating molecules is prepared by adding first reactive moieties to the MHC molecules, and the method further comprises reacting the first reactive moieties of the plurality of primary activating molecules with a first plurality of binding moieties disposed on a covalently functionalized synthetic surface; and
measuring total binding and/or an extent of dissociation of the peptide antigen from the MHC molecule.

Embodiment 7 is the method of embodiment 6, wherein measuring total binding and/or the extent of dissociation comprises measuring binding of an agent to the MHC molecule, wherein the agent specifically binds to (i) the initial peptide, and/or (ii) a peptide-bound conformation of the MHC molecule.

Embodiment 8 is the method of embodiment 6 or 7, wherein the agent comprises an antibody, optionally wherein the antibody is produced by clone W6/32.

Embodiment 9 is the method of any one of embodiments 6-8, wherein measuring total binding and/or the extent of dissociation comprises performing flow cytometry.

Embodiment 10 is the method of any one of embodiments 6-9, wherein the agent does not recognize a peptide-unbound conformation of the MHC molecule.

Embodiment 11 is the method of any one of embodiments 6-10, wherein the method further comprises determining one or more kinetic parameters of the peptide antigen-bound MHC molecule.

Embodiment 12 is the method of embodiment 11, wherein the one or more kinetic parameters comprise a half-life.

Embodiment 13 is the method of any one of embodiments 6-12, wherein the method results in identification of a peptide with a half-life of at least about 4 hours (e.g., at least about 6, 8, 10, 12, 14, 16, or 18 hours).

Embodiment 14 is the method of any one of embodiments 6-13, wherein the method results in identification of a peptide with a half-life in the range of about 4 to about 40 hours (e.g., about 4 to about 10 hours, about 10 to about 15 hours, about 15 to about 20 hours, about 20 to about 25 hours, about 25 to about 30 hours, about 30 to about 35, or about 35 to about 40 hours).

Embodiment 15 is the method of any one of embodiments 6-14, wherein the one or more kinetic parameters comprise an off-rate.

Embodiment 16 is a method of analyzing stability of a plurality of complexes each comprising a histocompatibility complex (MHC) molecule and a peptide antigen, comprising performing the method of any one of embodiments 6-15 with each of a plurality of different peptide antigens.

Embodiment 17 is the kit of any one of embodiments 1-3 or the method of any one of embodiments 4-16, wherein the initial peptide comprises at least 4 or 5 amino acid residues; or has a length of about 4, 5, 6, 7, 8, 9, 10, 11, 12, 13, 14, 15, 16, 17, or amino acid residues (e.g., ranging from 8 to 10 amino acid residues, or 13 to 18 amino acid residues).

Embodiment 18 is the method or kit of any one of embodiments 1-17, wherein the initial peptide comprises a lysine as the fourth or fifth amino acid residue.

Embodiment 19 is the method or kit of any one of embodiments 1-18, wherein the initial peptide comprises a label.

Embodiment 20 is the method or kit of embodiment 19, wherein the label is attached to the fourth or fifth amino acid residue (e.g., lysine).

Embodiment 21 is the method or kit of embodiment 19 or 20, wherein the label is a fluorescent label.

Embodiment 22 is the method or kit of any one of embodiments 1-21, wherein the initial peptide has a sequence comprising or consisting of a sequence from a naturally occurring (e.g., mammalian or human) polypeptide.

Embodiment 23 is the method or kit of any one of embodiments 1-22, wherein the sequence of the initial peptide consists of sequence that appears in a wild-type (e.g., mammalian or human) polypeptide.

Embodiment 24 is the method or kit of any one of embodiments 1-23, wherein the initial peptide is non-immunogenic.

Embodiment 25 is the method or kit of any one of embodiments 1-24, wherein the sequence of the initial peptide comprises or consists of sequence from a highly conserved protein (e.g., a protein with a below average mutation rate; optionally wherein the mutation rate is at least one or two standard deviations below the average amino acid mutation rate in the organism).

Embodiment 26 is the method or kit of any one of embodiments 1-25, wherein the sequence of the initial peptide comprises or consists of sequence from a cytoskeletal polypeptide, e.g., an actin or tubulin polypeptide.

Embodiment 27 is the method or kit of any one of embodiments 1-25, wherein the sequence of the initial peptide comprises or consists of any one of SEQ ID NOs: 1-4.

Embodiment 28 is the method or kit of any one of embodiments 1-25, wherein the sequence of the initial peptide comprises or consists of sequence from a ribosomal polypeptide, e.g., the RPSA, RPS2, RPL3, RPL4, RPL5, RPL6, RPL7A, or RPP0 polypeptides.

Embodiment 29 is the method or kit of any one of embodiments 1-28, wherein the initial peptide binds the MHC molecule with high affinity, a low off-rate, and/or a long half-life, optionally wherein the binding of the initial peptide to the MHC molecule has a half-life of at least about 4, 6, 8, 10, 12, 14, 16, 18, 20, 24, 28, 32, 36, or 48 hours, or the binding of the initial peptide to the MHC molecule has a half-life in the range of about 4-12, 8-16, 12-20, 20-28, 24-32, 28-36, 32-40, 36-48, or 48-72 hours.

Embodiment 30 is the method or kit of any one of the preceding embodiments, wherein the covalently functionalized synthetic surface presents a plurality of azido groups.

Embodiment 31 is the method or kit of embodiment 30, wherein the first reactive moieties are configured to react with the azido groups of the covalently functionalized synthetic surface so as to form covalent bonds.

Embodiment 32 is the method or kit of any one of embodiments 1-29, wherein the covalently functionalized synthetic surface presents a plurality of biotin-binding agents, and wherein the first reactive moieties are configured to specifically bind to the biotin-binding agent.

Embodiment 33 is the method or kit of embodiment 32, wherein the first reactive moieties comprise or consist essentially of biotin.

Embodiment 34 is the method or kit of embodiment 32 or 33, wherein the biotin-binding agent is covalently attached to the covalently functionalized synthetic surface.

Embodiment 35 is the method or kit of embodiment 32 or 33, wherein the biotin-binding agent is noncovalently attached to the covalently functionalized synthetic surface through biotin functionalities.

Embodiment 36 is the method or kit of any one of embodiments 32-35, wherein the biotin-binding agent is streptavidin.

Embodiment 37 is the method of any one of embodiments 4-36, wherein a plurality of co-activating molecular ligands, each including a TCR co-activating molecule or an adjunct TCR activating molecule, are present on the covalently functionalized synthetic surface or are added to the covalently functionalized synthetic surface by reacting a plurality of co-activating molecules, each including second reactive moiety and a TCR co-activating molecule or an adjunct TCR activating molecule, with a second plurality of binding moieties of the covalently functionalized synthetic surface configured for binding the second reactive moieties.

Embodiment 38 is the kit of any one of embodiments 30-31, or the method of embodiment 37, wherein the covalently functionalized synthetic surface presents a plurality of azido groups, and wherein the second reactive moieties are configured to react with the azido groups of the covalently functionalized synthetic surface so as to form covalent bonds.

Embodiment 39 is the method or kit of any one of embodiments 32-38, wherein the covalently functionalized synthetic surface presents a plurality of biotin-binding agents, and wherein the second reactive moieties are configured to specifically bind to the biotin-binding agent.

Embodiment 40 is a proto-antigen-presenting surface, the surface comprising:
a plurality of primary activating molecular ligands, wherein each primary activating molecular ligand includes a major histocompatibility complex (MHC) molecule configured to bind to a T cell receptor (TCR) of a T cell and wherein an exchange factor or an initial peptide is bound to the MHC molecules, optionally wherein the initial peptide is non-immunogenic; and
a plurality of co-activating molecular ligands each including a TCR co-activating molecule or an adjunct TCR activating molecule.

Embodiment 41 is the proto-antigen-presenting surface of embodiment 40, wherein each of the plurality of primary activating molecular ligands and the plurality of co-activating molecular ligands is specifically bound to the antigen presenting surface.

Embodiment 42 is the surface, kit, or method of any one of the preceding embodiments, wherein the exchange factor comprises Leu, Phe, Val, Arg, Met, Lys, Ile, homoleucine, cyclohexylalanine, or Norleucine as its C-terminal amino acid residue.

Embodiment 43 is the surface, kit, or method of any one of the preceding embodiments, wherein the exchange factor comprises a free N-terminal amine.

Embodiment 44 is the surface, kit, or method of any one of the preceding embodiments, wherein the exchange factor comprises Gly, Ala, Ser, or Cys as its penultimate C-terminal residue.

Embodiment 45 is the surface, kit, or method of embodiment 44, wherein the exchange factor comprises Gly as its penultimate C-terminal residue.

Embodiment 46 is the surface, kit, or method of any one of the preceding embodiments, wherein the exchange factor is 2, 3, 4, or 5 amino acid residues in length.

Embodiment 47 is the surface, kit, or method of embodiment 46, wherein the exchange factor is 2 amino acid residues in length.

Embodiment 48 is the surface, kit, or method of any one of the preceding embodiments, wherein the exchange factor comprises a linkage between its C-terminal and penultimate C-terminal residues which is a peptide bond, lactam, or piperazinone.

Embodiment 49 is the surface, kit, or method of embodiment 48, wherein the exchange factor comprises a peptide bond between its C-terminal and penultimate C-terminal residues.

Embodiment 50 is the surface, kit, or method of any one of the preceding embodiments, wherein the covalently functionalized synthetic surface or the proto-antigen-presenting surface further comprises at least one plurality of surface-blocking molecular ligands covalently attached to the surface.

Embodiment 51 is the surface, kit, or method of embodiment 50, wherein:
(i) each of the plurality of surface-blocking molecular ligands includes a hydrophilic moiety, an amphiphilic moiety, a zwitterionic moiety, and/or a negatively charged moiety;
(ii) each of the plurality of surface-blocking molecular ligands includes a linker and a terminal surface-blocking group, optionally wherein the linkers of the plurality of surface-blocking molecular ligands are of the same length or are of different lengths; or
(iii) each of the plurality of surface-blocking molecular ligands includes a linker and a terminal surface-blocking group, wherein the terminal surface-blocking group comprises a hydrophilic moiety, amphiphilic moiety, zwitterionic moiety, and/or negatively charged moiety, optionally wherein the linkers of the plurality of surface-blocking molecular ligands are of the same length or are of different lengths;
(iv) each of the plurality of surface-blocking molecular ligands is covalently bound to the covalently functionalized synthetic surface or the proto-antigen-presenting surface and/or
(v) the plurality of the surface-blocking molecular ligands and may include 2, 3, or 4 different surface-blocking groups and/or 2, 3, 4, or more different lengths of linkers, chosen in any combination.

Embodiment 52 is the surface, kit, or method of embodiment 50 or 51, wherein:
(i) the plurality of surface-blocking molecular ligands all have the same terminal surface-blocking group; or
(ii) the plurality of surface-blocking molecular ligands have a mixture of terminal surface-blocking groups; optionally wherein each of the plurality of surface-blocking molecular ligands includes a polyethylene glycol (PEG) moiety, a carboxylic acid moiety, or a combination thereof.

Embodiment 53 is the surface, kit, or method of embodiment 52, wherein the PEG moiety of each of the surface-blocking molecular ligands has a backbone linear chain length of about 10 atoms to about 100 atoms.

Embodiment 54 is the surface, kit, or method of embodiment 52 or 53, wherein the PEG moiety comprises a carboxylic acid moiety.

Embodiment 55 is the surface, kit, or method of embodiment 54, wherein the PEG moiety comprises (PEG)₄-COOH.

Embodiment 56 is the surface, kit, or method of any one of the preceding embodiments, wherein a plurality of biotin or biotin-binding agent functionalities is attached to the covalently functionalized synthetic surface or the proto-antigen-presenting surface via a linker.

Embodiment 57 is the surface, kit, or method of embodiment 56, wherein the linker linking the biotin or biotin-binding agent functionality has a length of about 20 Angstroms to about 100 Angstroms.

Embodiment 58 is the surface, kit, or method of embodiment 56 or 57, wherein the linker links the biotin or biotin-binding agent functionality to the covalently functionalized synthetic surface or the proto-antigen-presenting surface through a series of about 15, 20, 25, 30, 35, 40, 45, 50, 55, 60, 65, 70, 75, 80, 85, 95, 100, 200 bond lengths, or any number of bond lengths therebetween.

Embodiment 59 is the surface, kit, or method of any one of embodiments 56-58, wherein the linker of each biotin or biotin-binding agent functionality includes a polyethylene glycol (PEG) moiety.

Embodiment 60 is the surface, kit, or method of embodiment 59, wherein the PEG linker includes a (PEG)₁₃ repeating sequence, optionally wherein the covalently functionalized synthetic surface or the proto-antigen-presenting surface includes the plurality of biotin-binding agent functionalities.

Embodiment 61 is the surface, kit, or method of embodiment 59, wherein the PEG linker includes a (PEG)₄ repeating sequence, optionally wherein the covalently functionalized synthetic surface or the proto-antigen-presenting surface includes the plurality of biotin functionalities.

Embodiment 62 is the surface, kit, or method of any one of embodiments 56-61, wherein the biotin-binding agent functionalities are streptavidin moieties.

Embodiment 63 is the surface, kit, or method of embodiment 62, wherein the at least one plurality of streptavidin moieties is disposed upon the covalently functionalized synthetic surface or the proto-antigen-presenting surface in a density from about 4X 10² to about 3X 10⁴ molecules per square micron, in each portion or sub-region where it is attached.

Embodiment 64 is the surface, kit, or method of embodiment 62, wherein the at least one plurality of streptavidin moieties is disposed upon the covalently functionalized synthetic surface or the proto-antigen-presenting surface in a density from about 5X 10³ to about 3X 10⁴ molecules per square micron, in each portion or sub-region where it is attached.

Embodiment 65 is the surface, kit, or method of embodiment 62, wherein the at least one plurality of streptavidin moieties is disposed upon the covalently functionalized synthetic surface or the proto-antigen-presenting surface from about 6X 10² to about 5X 10³ molecules per square micron, about 5X 10³ to about 2X 10⁴ molecules per square micron, about 1X 10⁴ to about 2X 10⁴ molecules per square micron, or about 1.25X 10⁴ to about 1.75X 10⁴ molecules per square micron, in each portion or sub-region where it is attached.

Embodiment 66 is the surface, kit, or method of any one of embodiments 56-65, wherein the at least one plurality of biotin-binding agent or biotin moieties is disposed upon substantially all of the covalently functionalized synthetic surface or the proto-antigen-presenting surface.

Embodiment 67 is the surface, kit, or method of any one of embodiments 56-65, wherein the covalently functionalized synthetic surface or the proto-antigen-presenting surface further includes a first portion and a second portion, wherein the distribution of the at least one plurality of biotin-binding agent or biotin functionalities is located in the first portion of the covalently modified synthetic surface, and the distribution of the at least one plurality of the surface-blocking molecular ligands is located in the second portion.

Embodiment 68 is the surface, kit, or method of embodiment 67, wherein a second plurality of surface-blocking molecular ligands is disposed in the first portion of the covalently functionalized synthetic surface or the proto-antigen-presenting surface.

Embodiment 69 is the surface, kit, or method of embodiment 67 or 68, wherein the first portion of the covalently functionalized synthetic surface or the proto-antigen-presenting surface further includes a plurality of first regions, each first region including at least a subset of the plurality of the biotin-binding agent or biotin functionalities, wherein each of the plurality of first regions is separated from another of the plurality of first regions by the second region configured to substantially exclude the streptavidin or biotin functionalities.

Embodiment 70 is the surface, kit, or method of embodiment 69, wherein each of the plurality of first regions including at least the subset of the plurality of the streptavidin or biotin functionalities has an area of about 0.10 square microns to about 4.0 square microns.

Embodiment 71 is the surface, kit, or method of embodiment 69, wherein the area of each of the plurality of first regions including at least the subset of the plurality of the primary activating molecular ligands is about 4.0 square microns to about 0.8 square microns.

Embodiment 72 is the surface, kit, or method of any one of the preceding embodiments, wherein the covalently functionalized synthetic surface or the proto-antigen-presenting surface includes glass, polymer, metal, ceramic, and/or a metal oxide.

Embodiment 73 is the surface, kit, or method of any one of the preceding embodiments, wherein the covalently functionalized synthetic surface or the proto-antigen-presenting surface is a wafer, an inner surface of a tube, or an inner surface of a microfluidic device.

Embodiment 74 is the surface, kit, or method of embodiment 73, wherein the tube is a glass or polymer tube.

Embodiment 75 is the surface, kit, or method of any one of embodiments 1-71, wherein the covalently functionalized synthetic surface or the proto-antigen-presenting surface is a bead.

Embodiment 76 is the surface, kit, or method of embodiment 75, wherein the bead includes a magnetic material.

Embodiment 77 is the surface, kit, or method of embodiment 75 or 76, wherein the bead has a surface area within 10% of the surface area of a sphere of an equal volume or diameter.

Embodiment 78 is the surface, kit, or method of embodiment 73, wherein the covalently functionalized synthetic surface or the proto-antigen-presenting surface is at least one inner surface of a microfluidic device.

Embodiment 79 is the surface, kit, or method of embodiment 78, wherein the inner surface of the microfluidic device is within a chamber of the microfluidic device.

Embodiment 80 is the surface, kit, or method of any one of embodiments 69-71, wherein each of the plurality of first regions including at least a subset of the plurality of biotin-binding agent or biotin functionalities includes at least one surface within a chamber of the microfluidic device.

Embodiment 81 is the surface, kit, or method of embodiment 79 or 80, wherein the chamber is a sequestration pen.

Embodiment 82 is the surface, kit, or method of embodiment 81, wherein the microfluidic device further comprises a flow region for containing a flow of a first fluidic medium; and the sequestration pen comprises an isolation region for containing a second fluidic medium, the isolation region having a single opening, wherein the isolation region of the sequestration pen is an unswept region of the microfluidic device; and a connection region fluidically connecting the isolation region to the flow region; optionally wherein the microfluidic device comprises a microfluidic channel comprising at least a portion of the flow region.

Embodiment 83 is the surface, kit, or method of embodiment 82, wherein the microfluidic device comprises a microfluidic channel comprising at least a portion of the flow region, and the connection region comprises a proximal opening into the microfluidic channel having a width W_{con} ranging from about 20 microns to about 100 microns and a distal opening into the isolation region, and wherein a length L_{con} of the connection region from the proximal opening to the distal opening is at least 1.0 times a width W_{con} of the proximal opening of the connection region.

Embodiment 84 is the surface, kit, or method of embodiment 83, wherein the length L_{con} of the connection region from the proximal opening to the distal opening is at least 1.5 times the width W_{con} of the proximal opening of the connection region.

Embodiment 85 is the surface, kit, or method of embodiment 83, wherein the length L_{con} of the connection region from the proximal opening to the distal opening is at least 2.0 times the width W_{con} of the proximal opening of the connection region.

Embodiment 86 is the surface, kit, or method of any one of embodiments 83-85, wherein the width W_{con} of the proximal opening of the connection region ranges from about 20 microns to about 60 microns.

Embodiment 87 is the surface, kit, or method of any one of embodiments 83-86, wherein the length L_{con} of the connection region from the proximal opening to the distal opening is between about 20 microns and about 500 microns.

Embodiment 88 is the surface, kit, or method of any one of embodiments 83-87, wherein a width of the microfluidic channel at the proximal opening of the connection region is between about 50 microns and about 500 microns.

Embodiment 89 is the surface, kit, or method of any one of embodiments 83-88, wherein a height of the microfluidic channel at the proximal opening of the connection region is between 20 microns and 100 microns.

Embodiment 90 is the surface, kit, or method of any one of embodiments 82-89, wherein the volume of the isolation region ranges from about 2 x 10⁴ to about 2 x 10⁶ cubic microns.

Embodiment 91 is the surface, kit, or method of any one of embodiments 82-90, wherein the proximal opening of the connection region is parallel to a direction of the flow of the first medium in the flow region.

Embodiment 92 is the surface, kit, or method of any one of embodiments 82-91, wherein the microfluidic device comprises an enclosure comprising a base, a microfluidic circuit structure disposed on the base, and a cover which collectively define a microfluidic circuit, and the microfluidic circuit comprises the flow region, the microfluidic channel, and the sequestration pen.

Embodiment 93 is the surface, kit, or method of embodiment 92, wherein the cover is an integral part of the microfluidic circuit structure.

Embodiment 94 is the surface, kit, or method of any one of embodiments 82-93, wherein the microfluidic circuit further comprises one or more inlets through which the first medium can be input into the flow region and one or more outlets through which the first medium can be removed from the flow region.

Embodiment 95 is the surface, kit, or method of any one of embodiments 92-94, wherein barriers defining the microfluidic sequestration pen extend from a surface of the base of the microfluidic device to a surface of the cover of the microfluidic device.

Embodiment 96 is the surface, kit, or method of any one of embodiments 92-95, wherein the cover and the base are part of a dielectrophoresis (DEP) mechanism for selectively inducing DEP forces on a micro-object.

Embodiment 97 is the surface, kit, or method of any one of embodiments 92-96, wherein the microfluidic device further comprises a first electrode, an electrode activation substrate, and a second electrode, wherein the first electrode is part of a first wall of the enclosure and the electrode activation substrate and the second electrode is part of a second wall of the enclosure, wherein the electrode activation substrate comprises a photoconductive material, semiconductor integrated circuits, or phototransistors.

Embodiment 98 is the surface, kit, or method of embodiment 97, wherein the first wall of the microfluidic device is the cover, and wherein the second wall of the microfluidic device is the base.

Embodiment 99 is the surface, kit, or method of embodiment 97 or 98, wherein the electrode activation substrate comprises phototransistors.

Embodiment 100 is the surface, kit, or method of any one of embodiments 92-99, wherein the cover and/or the base is transparent to light.

Embodiment 101 is the surface, kit, or method of any one of embodiments 78-100, wherein the covalently functionalized surface or the proto-antigen-presenting surface includes a portion configured to exclude biotin-binding agent or biotin functionalities which is disposed at at least one surface of a microfluidic channel of the microfluidic device.

Embodiment 102 is the surface, kit, or method of any one of embodiments 37 or 40-101, wherein the plurality of co-activating molecular ligands comprises TCR co-activating molecules and adjunct TCR activating molecules.

Embodiment 103 is the surface, kit, or method of embodiment 102, wherein a ratio of the TCR coactivating molecules to the adjunct TCR activating molecules of the plurality of co-activating molecular ligands is about 100:1 to about 1:100.

Embodiment 104 is the surface, kit, or method of embodiment 102, wherein a ratio of the TCR coactivating molecules to the adjunct TCR activating molecules of the plurality of co-activating molecular ligands is about 100:1 to about 90:1, about 90:1 to about 80:1, about 80:1 to about 70:1, about 70:1 to about 60:1, about 60:1 to about about 50:1, about 50:1 to about 40:1, about 40:1 to about 30:1, about 30:1 to about 20:1, about 20:1 to about 10:1, about 10:1 to about 1:1, about 1:1 to about 1:10, about 1:10 to about 1:20, about 1:20 to about 1:30, about 1:30 to about 1:40, about 1:40 to about 1:50, about 1:50 to about 1:60, about 1:60 to about 1:70, about 1:70 to about 1:80, about 1:80 to about 1:90, or about 1:90 to about 1:100.

Embodiment 105 is the surface, kit, or method of embodiment 102, wherein a ratio of the TCR coactivating molecules to the adjunct TCR activating molecules of the plurality of co-activating molecular ligands is about 10:1 to about 1:20.

Embodiment 106 is the surface, kit, or method of embodiment 102, wherein a ratio of the TCR coactivating molecules to the adjunct TCR activating molecules of the plurality of co-activating molecular ligands is about 10:1 to about 1:10.

Embodiment 107 is the surface, kit, or method of any one of the preceding embodiments, wherein the MHC molecule includes an MHC protein sequence and a beta microglobulin.

Embodiment 108 is the surface, kit, or method of embodiment 107, wherein the MHC molecule comprises a human leukocyte antigen A (HLA-A) heavy chain.

Embodiment 109 is the surface, kit, or method of embodiment 108, wherein the HLA-A heavy chain is an HLA-A*01, HLA-A*02, HLA-A*03, HLA-A*11, HLA-A*23, HLA-A*24, HLA-A*25, HLA-A*26, HLA-A*29, HLA-A*30, HLA-A*31, HLA-A*32, HLA-A*33, HLA-A*34, HLA-A*43, HLA-A*66, HLA-A*68, HLA-A*69, HLA-A*74, or HLA-A*80 heavy chain.

Embodiment 110 is the surface, kit, or method of embodiment 107, wherein the MHC molecule comprises a human leukocyte antigen B (HLA-B) heavy chain.

Embodiment 111 is the surface, kit, or method of embodiment 110, wherein the HLA-B heavy chain is an HLA-B*07, HLA-B*08, HLA-B*13, HLA-B*14, HLA-B*15, HLA-B*18, HLA-B*27, HLA-B*35, HLA-B*37, HLA-B*38, HLA-B*39, HLA-B*40, HLA-B*41, HLA-B*42, HLA-B*44, HLA-B*45, HLA-B*46, HLA-B*47, HLA-B*48, HLA-B*49, HLA-B*50, HLA-B*51, HLA-B*52, HLA-B*53, HLA-B*54, HLA-B*55, HLA-B*56, HLA-B*57, HLA-B*58, HLA-B*59, HLA-B*67, HLA-B*73, HLA-B*78, HLA-B*81, HLA-B*82, or HLA-B*83 heavy chain.

Embodiment 112 is the surface, kit, or method of embodiment 107, wherein the MHC molecule comprises a human leukocyte antigen C (HLA-C) heavy chain.

Embodiment 113 is the surface, kit, or method of embodiment 112, wherein the HLA-C heavy chain is an HLA-C*01, HLA-C*02, HLA-C*03, HLA-C*04, HLA-C*05, HLA-C*06, HLA-C*07, HLA-C*08, HLA-C*12, HLA-C*14, HLA-C*15, HLA-C*16, HLA-C*17, or HLA-C*18 heavy chain.

Embodiment 114 is the surface, kit, or method of any one of embodiments 2-3 or 37-113, wherein the TCR co-activating molecule includes a protein.

Embodiment 115 is the surface, kit, or method of embodiment 114, wherein the TCR co-activating molecule further comprises a site-specific C-terminal biotin moiety.

Embodiment 116 is the surface, kit, or method of embodiment 114 or 115, wherein the TCR coactivating protein molecule includes a CD28 binding protein or a fragment thereof which retains binding ability with CD28.

Embodiment 117 is the surface, kit, or method of embodiment 116, wherein the CD28 binding protein includes a CD80 molecule or a fragment thereof, wherein the fragment retains binding ability to CD28.

Embodiment 118 is the surface, kit, or method of embodiment 114 or 115, wherein the TCR coactivating molecule includes an anti-CD28 antibody or fragment thereof, wherein the fragment retains binding activity with CD28.

Embodiment 119 is the surface, kit, or method of any one of embodiments 2-3 or 37-116, wherein the adjunct TCR activating molecule is configured to provide adhesion stimulation.

Embodiment 120 is the surface, kit, or method of any one of embodiments 2-3 or 37-119, wherein the adjunct TCR activating molecular ligand includes a CD2 binding protein or a fragment thereof, wherein the fragment retains binding ability with CD2.

Embodiment 121 is the surface, kit, or method of embodiment 120, wherein the CD2 binding protein further comprises a site-specific C-terminal biotin moiety.

Embodiment 122 is the surface, kit, or method of any one of embodiments 120 or 121, wherein the adjunct TCR activating molecular ligand includes a CD58 molecule or fragment thereof, wherein the fragment retains binding activity with CD2.

Embodiment 123 is the surface, kit, or method of any one of embodiments 120 or 121, wherein the adjunct TCR activating molecule includes an anti-CD2 antibody or a fragment thereof, wherein the fragment retains binding activity with CD2.

Embodiment 124 is the proto-antigen-presenting surface of any one of embodiments 40-123, wherein the plurality of primary activating molecular ligands is disposed upon at least a portion of the antigen-presenting surface at a density from about 4X 10² to about 3X 10⁴ molecules per square micron, in each portion or sub-region where it is attached.

Embodiment 125 is the proto-antigen-presenting surface of embodiment 124, wherein the plurality of primary activating molecular ligands is disposed upon at least a portion of the antigen-presenting surface at a density from about 4X 10² to about 2X 10³ molecules per square micron.

Embodiment 126 is the proto-antigen-presenting surface of embodiment 124, wherein the plurality of primary activating molecular ligands is disposed upon at least a portion of the antigen-presenting surface at a density from about 2X 10³ to about 5X 10³ molecules per square micron.

Embodiment 127 is the proto-antigen-presenting surface of embodiment 124, wherein the plurality of primary activating molecular ligands is disposed upon at least a portion of a surface of the antigen-presenting surface at a density from about 5X 10³ to about 2X 10⁴ molecules per square micron, about 1X 10⁴ to about 2X 10⁴ molecules per square micron, or about 1.25X 10⁴ to about 1.75X 10⁴ molecules per square micron.

Embodiment 128 is the proto-antigen-presenting surface of any one of embodiments 124-127, wherein the plurality of primary activating molecular ligands is disposed upon substantially all of the antigen-presenting surface at the stated density.

Embodiment 129 is the proto-antigen-presenting surface of any one of embodiments 40-128, wherein the plurality of co-activating molecular ligands is disposed upon at least a portion the antigen-presenting surface at a density from about 5X 10² to about 2X 10⁴ molecules per square micron or about 5X 10² to about 1.5 X 10⁴ molecules per square micron.

Embodiment 130 is the proto-antigen-presenting surface of embodiment 129, wherein the plurality of co-activating molecular ligands is disposed upon at least a portion of the antigen-presenting surface at a density from about 5X 10³ to about 2X 10⁴ molecules per square micron, about 5X 10³ to about 1.5X 10⁴ molecules per square micron, about 1X 10⁴ to about 2X 10⁴ molecules per square micron, about 1X 10⁴ to about 1.5X 10⁴ molecules per square micron, about 1.25X 10⁴ to about 1.75X 10⁴ molecules per square micron, or about 1.25X 10⁴ to about 1.5X 10⁴ molecules per square micron.

Embodiment 131 is the proto-antigen-presenting surface of any one of embodiments 40-128, wherein the plurality of co-activating molecular ligands is disposed upon at least a portion of the antigen-presenting surface at a density from about 2X 10³ to about 5X 10³ molecules per square micron.

Embodiment 132 is the proto-antigen-presenting surface of any one of embodiments 40-128, wherein the plurality of co-activating molecular ligands is disposed upon at least a portion of a surface of the antigen-presenting surface at a density from about 5X 10² to about 2X 10³ molecules per square micron.

Embodiment 133 is the proto-antigen-presenting surface of any one of embodiments 129-132, wherein the plurality of co-activating molecular ligands is disposed upon substantially all of the antigen-presenting surface at the stated density.

Embodiment 134 is the proto-antigen-presenting surface of any one of embodiments 40-133, wherein a ratio of the primary activating molecular ligands to the co-activating molecular ligands present on the antigen-presenting surface is about 1:10 to about 2:1, about 1:5 to about 2:1, about 1:2 to about 2:1, about 1:10 to about 1:1, about 1:5 to about 1:1, about 1:1 to about 2:1, or about 1:2 to about 1:1.

Embodiment 135 is the proto-antigen-presenting surface of any one of embodiments 40-134, wherein each of the plurality of primary activating molecular ligands is noncovalently bound to a binding moiety, and further wherein the binding moiety is covalently bound to the antigen-presenting surface.

Embodiment 136 is the proto-antigen-presenting surface of embodiment 135, wherein each of the plurality of primary activating molecular ligands comprises a biotin and is noncovalently bound to a biotin-binding agent, and further wherein the biotin-binding agent is covalently bound to the antigen-presenting surface.

Embodiment 137 is the proto-antigen-presenting surface of any one of embodiments 40-136, wherein each of the plurality of primary activating molecular ligands is noncovalently bound to a binding moiety, and further wherein the binding moiety is noncovalently bound to the antigen-presenting surface.

Embodiment 138 is the proto-antigen-presenting surface of embodiment 137, wherein each of the plurality of primary activating molecular ligands comprises a biotin moiety, the binding moiety comprises a biotin-binding agent, and the biotin-binding agent is noncovalently bound to a second biotin moiety covalently attached to the antigen-presenting surface.

Embodiment 139 is the proto-antigen-presenting surface of 136 or 138, wherein the biotin-binding agent is streptavidin.

Embodiment 140 is the proto-antigen-presenting surface of any one of embodiments 40-139, wherein each of the plurality of co-activating molecular ligands is non-covalently attached to a streptavidin and the streptavidin is non-covalently attached to a streptavidin binding molecule, further wherein the streptavidin binding molecule is covalently attached via a linker to the proto-antigen-presenting surface, optionally wherein the streptavidin binding molecule comprises biotin.

Embodiment 141 is the proto-antigen-presenting surface of any one of embodiments 40-140, wherein each of the plurality of co-activating molecular ligands is covalently connected to the surface via a linker.

Embodiment 142 is the proto-antigen-presenting surface of embodiment 140 or 141, wherein the linker links the streptavidin binding molecule and/or co-activating molecular ligands to the surface through a series of about 15, 20, 25, 30, 35, 40, 45, 50, 55, 60, 65, 70 , 75, 80, 85, 95, 100, 200 bond lengths, or any number of bond lengths therebetween bonds.

Embodiment 143 is the proto-antigen-presenting surface of any one of embodiments 40-140, wherein each of the plurality of co-activating molecular ligands is non-covalently attached to a streptavidin moiety; and the streptavidin moiety is covalently attached to the antigen-presenting surface.

Embodiment 144 is the proto-antigen-presenting surface of embodiment 143, wherein the streptavidin moiety is linked by a linker to the surface through a series of about 15, 20, 25, 30, 35, 40, 45, 50, 55, 60, 65, 70, 75, 80, 85, 95, 100, 200 bond lengths, or any number of bond lengths therebetween.

Embodiment 145 is the proto-antigen-presenting surface of any one of embodiments 40-144, wherein the proto-antigen-presenting surface further comprises a plurality of surface-blocking molecular ligands.

Embodiment 146 is the proto-antigen-presenting surface of embodiment 145, wherein:
(i) each of the plurality of surface-blocking molecular ligands includes a hydrophilic moiety, an amphiphilic moiety, a zwitterionic moiety, and/or a negatively charged moiety;
(ii) each of the plurality of surface-blocking molecular ligands includes a linker and a terminal surface-blocking group, optionally wherein the linkers of the plurality of surface-blocking molecular ligands are of the same length or are of different lengths; or
(iii) each of the plurality of surface-blocking molecular ligands includes a linker and a terminal surface-blocking group, wherein the terminal surface-blocking group comprises a hydrophilic moiety, amphiphilic moiety, zwitterionic moiety, and/or negatively charged moiety, optionally wherein the linkers of the plurality of surface-blocking molecular ligands are of the same length or are of different lengths.

Embodiment 147 is the proto-antigen-presenting surface of embodiment 145 or 146, wherein:
(i) the plurality of surface-blocking molecular ligands all have the same terminal surface-blocking group; or
(ii) the plurality of surface-blocking molecular ligands have a mixture of terminal surface-blocking groups; optionally wherein each of the plurality of surface-blocking molecular ligands includes a polyethylene glycol (PEG) moiety, a carboxylic acid moiety, or a combination thereof, further optionally wherein the PEG moiety of each of the surface-blocking molecular ligands has a backbone linear chain length of about 10 atoms to about 100 atoms.

Embodiment 148 is the proto-antigen-presenting surface of any one of embodiments 145-147, wherein:
(i) each of the plurality of surface-blocking molecular ligands is covalently connected to the antigen-presenting surface; and/or
(ii) the plurality of the surface-blocking molecular ligands and may include 2, 3, or 4 different surface-blocking groups and/or 2, 3, 4, or more different lengths of linkers, chosen in any combination.

Embodiment 149 is the proto-antigen-presenting surface of any one of embodiments 40-148, further including a plurality of adhesion stimulatory molecular ligands, optionally wherein each adhesive molecular ligand includes a ligand for a cell adhesion receptor comprising an ICAM protein sequence.

Embodiment 150 is the proto-antigen-presenting surface of embodiment 149, wherein the adhesion stimulatory molecular ligand is covalently connected to the antigen-presenting surface via a linker.

Embodiment 151 is the proto-antigen-presenting surface of embodiment 150, wherein the adhesion stimulatory molecular ligand is non-covalently attached to a streptavidin moiety, wherein the streptavidin moiety is covalently attached via a linker to the antigen-presenting surface.

Embodiment 152 is the proto-antigen-presenting surface of embodiment 150, wherein the adhesion stimulatory molecular ligand is non-covalently attached to a streptavidin, wherein the streptavidin is noncovalently attached to a biotin and the biotin is covalently attached via a linker to the antigen-presenting surface.

Embodiment 153 is the proto-antigen-presenting surface of any one of embodiments 40-152, wherein the ratio of the TCR co-activating molecules to the adjunct TCR activating molecules of the plurality of co-activating molecular ligands is from about 3:1 to about 1:3.

Embodiment 154 is the proto-antigen-presenting surface of any one of embodiments 40-153, wherein the ratio of the TCR co-activating molecules to the adjunct TCR activating molecules of the plurality of co-activating molecular ligands is about 1:2 to about 2:1.

Embodiment 155 is the proto-antigen-presenting surface of any one of embodiments 40-154, wherein the ratio of the TCR co-activating molecules to the adjunct TCR activating molecules of the plurality of co-activating molecular ligands is about 1:1.

Embodiment 156 is the proto-antigen-presenting surface of any one of embodiments 40-155, further including a plurality of growth-stimulatory molecular ligands, wherein each of the growth-stimulatory molecular ligands includes a growth factor receptor ligand.

Embodiment 157 is the proto-antigen-presenting surface of embodiment 156, wherein the growth factor receptor ligand includes a cytokine or fragment thereof, wherein the fragment retains receptor binding ability, optionally wherein the cytokine comprises IL-21.

Embodiment 158 is the proto-antigen-presenting surface of any one of embodiments 40-127, 129-132, or 134-157, further including a first portion and a second portion, wherein the distribution of the plurality of primary activating molecular ligands and the distribution of the plurality of co-activating molecular ligands are located in the first portion of the antigen-presenting surface, and the second portion is configured to substantially exclude the primary activating molecular ligands.

Embodiment 159 is the proto-antigen-presenting surface of embodiment 158, wherein at least one plurality of surface-blocking molecular ligands is located in the second portion of the at least one inner surface of the antigen-presenting surface.

Embodiment 160 is the proto-antigen-presenting surface of embodiment 158 or 159, wherein the first portion of the antigen-presenting surface further includes a plurality of first regions, each first region including at least a subset of the plurality of the primary activating molecular ligands, wherein each of the plurality of first regions is separated from another of the plurality of first region by the second portion configured to substantially exclude primary activating molecular ligands.

Embodiment 161 is the proto-antigen-presenting surface of embodiment 160, wherein each of the plurality of first regions including the at least a subset of the plurality of the primary activating molecular ligands further includes a subset of the plurality of the co-activating molecular ligands.

Embodiment 162 is the proto-antigen-presenting surface of embodiment 160 or 161, wherein each of the plurality of first regions including at least the subset of the plurality of the primary activating molecular ligands has an area of about 0.10 square microns to about 4.0 square microns.

Embodiment 163 is the proto-antigen-presenting surface of any one of embodiments 160-162, wherein the area of each of the plurality of first regions including at least the subset of the plurality of the primary activating molecular ligands is about 4.0 square microns to about 0.8 square microns.

Embodiment 164 is the proto-antigen-presenting surface of any one of embodiments 160-163, wherein each of the plurality of first regions further includes at least a subset of a plurality of adhesion stimulatory molecular ligands, and optionally wherein each of the adhesion stimulatory molecular ligands includes a ligand for a cell adhesion receptor comprising an ICAM protein sequence.

Embodiment 165 is the proto-antigen-presenting surface of any one of embodiments 158-164, wherein the second portion configured to substantially exclude the primary activating molecular ligands is also configured to substantially exclude co-activating molecular ligands.

Embodiment 166 is the proto-antigen-presenting surface of any one of embodiments 158-165, wherein the second portion configured to substantially exclude the primary activating molecular ligands is further configured to include a plurality of growth stimulatory molecular ligands, wherein each of the growth stimulatory molecular ligands includes a growth factor receptor ligand.

Embodiment 167 is the proto-antigen-presenting surface of any one of embodiments 158-166, wherein the second portion configured to substantially exclude the primary activating molecular ligands includes a plurality of adhesion stimulatory molecular ligands, wherein each of the adhesion stimulatory molecular ligands includes a ligand for a cell adhesion receptor including an ICAM protein sequence.

Embodiment 168 is the proto-antigen-presenting surface of any one of embodiments 158-167, which is an antigen-presenting surface of a microfluidic device and each of the plurality of first regions including at least a subset of the plurality of primary activating molecular ligands is disposed at least one surface within a chamber of the antigen-presenting microfluidic device.

Embodiment 169 is the kit or method of embodiment 68, wherein the second plurality of surface-blocking molecular ligands limits the density of functionalizing moieties of an antigen-presenting synthetic surface formed from the covalently functionalized synthetic surface.

Embodiment 170 is the method of any one of embodiments 4-5, 18-123 or 169, further comprising reacting a plurality of surface-blocking molecules with a first additional plurality of binding moieties of the covalently functionalized surface, wherein each of the binding moieties of the first additional plurality is configured for binding the surface-blocking molecule.

Embodiment 171 is the method of any one of embodiments 4-5, 18-123, or 169-170, further comprising reacting a plurality of adhesion stimulatory molecular ligands, wherein each adhesion stimulatory molecular ligand includes a ligand for a cell adhesion receptor including an ICAM protein sequence, with a second additional plurality of binding moieties of the covalently functionalized surface, wherein each of the binding moieties of the second additional plurality is configured for binding with the cell adhesion receptor ligand molecule.

Embodiment 172 is the kit of any one of embodiments 1-3, 17-123, or 169, further comprising a plurality of surface-blocking molecules, wherein the covalently functionalized surface further comprises a first additional plurality of binding moieties configured for binding the surface-blocking molecule.

Embodiment 173 is the kit of any one of embodiments 1-3, 17-123, 169, or 172, further comprising a plurality of adhesion stimulatory molecular ligands, wherein each adhesion stimulatory molecular ligand includes a ligand for a cell adhesion receptor including an ICAM protein sequence, and the covalently functionalized surface further comprises a second additional plurality of binding moieties configured for binding the cell adhesion receptor ligand molecule.

Embodiment 174 is the kit of any one of embodiments 1-3, 17-123, 169, or 172-173, further comprising a peptide antigen.

Embodiment 175 is a method of preparing an antigen-presenting surface comprising a peptide antigen, the method comprising reacting the peptide antigen with a proto-antigen-presenting surface according to any one of embodiments 40-168, wherein the exchange factor or initial peptide is substantially displaced and the peptide antigen becomes associated with the MHC molecules.

Embodiment 176 is the kit or method of embodiment 174 or 175, wherein the peptide antigen comprises a tumor-associated antigen.

Embodiment 177 is the kit or method of any one of embodiments 174-176, wherein the peptide antigen comprises a segment of amino acid sequence from a protein expressed on the surface of a tumor cell.

Embodiment 178 is the kit or method of embodiment 177, wherein the segment comprises 5, 6, 7, 8, 9, or 10 amino acid residues or is 5, 6, 7, 8, 9, or 10 amino acid residues in length.

Embodiment 179 is the kit or method of embodiment 177 or 178, wherein the protein expressed on the surface of a tumor cell is CD19, CD20, CLL-1, TRP-2, LAGE-1, HER2, EphA2, FOLR1, MAGE-A1, mesothelin, SOX2, PSM, CA125, or T antigen.

Embodiment 180 is the kit or method of any one of embodiments 174-179, wherein the peptide antigen is a neoantigenic peptide.

Embodiment 181 is the kit or method of any one of embodiments 174-180, wherein the peptide antigen is 7, 8, 9, 10, or 11 amino acids in length.

Embodiment 182 is the kit or method of embodiment 181, wherein the peptide antigen is 8, 9, or 10 amino acids in length.

Embodiment 183 is the method of any one of embodiments 175-182, further comprising contacting a T lymphocyte with the antigen-presenting surface comprising the peptide antigen.

Embodiment 184 is the method of embodiment 183, wherein a plurality of T lymphocytes are contacted with the antigen-presenting surface.

Embodiment 185 is the method of embodiment 183 or 184, wherein a sample comprising unactivated T cells is enriched for T cells prior to activation.

Embodiment 186 is the method of any one of embodiments 183-185, wherein a sample comprising unactivated T cells is enriched for CD8⁺ T cells prior to activation.

Embodiment 187 is the method of embodiment 185 or 186, wherein the sample comprising unactivated T cells is a peripheral blood sample.

Embodiment 188 is the method of any one of embodiments 185-187, wherein the sample is from a subject in need of treatment for cancer.

Embodiment 189 is the method of any one of embodiments 183-188, wherein the T lymphocyte or the plurality of T lymphocytes is CD8⁺.

Embodiment 190 is the method of any one of embodiments 183-189, wherein the T lymphocyte or the plurality of T lymphocytes are obtained from a subject in need of treating a cancer.

Embodiment 191 is the method of any one of embodiments 183-190, wherein the T lymphocyte becomes an activated T lymphocyte following contact with the antigen-presenting surface.

Embodiment 192 is the method of any one of embodiments 184-191, wherein a plurality of the T lymphocytes become activated T lymphocytes following contact with the antigen-presenting surface.

Embodiment 193 is the method of any one of embodiments 191-192, wherein the activated T lymphocyte(s) is CD28+.

Embodiment 194 is the method of any one of embodiments 191-193, wherein the activated T lymphocyte(s) is CD45RO+.

Embodiment 195 is the method of any one of embodiments 191-194, wherein the activated T lymphocyte(s) is CD127+.

Embodiment 196 is the method of any one of embodiments 191-195, wherein the activated T lymphocyte(s) is CD197+.

Embodiment 197 is the method of any one of embodiments 192-196, wherein the method produces a population of T cells, wherein at least about 1%, about 1.5%, about 2%, about 3%, about 4%, about 5%, about 6%, about 7%, about 8%, about 9%, or about 10% of the population of T cells are antigen-specific T cells.

Embodiment 198 is the method of embodiment 197, wherein 1%-2%, 2%-3%, 3%-4%, 4%-5%, 5%-6%, 6%-7%, 7%-8%, 8%-9%, 9%-10%, 10%-11%, or 11%-12% of the T cells are antigen-specific T cells wherein each of the foregoing values are modified by "about."

Embodiment 199 is the method of embodiment 197 or 198, wherein at least about 65%, about 70%, about 75%, about 80%, about 85%, about 86%, about 87%, about 88%, about 89%, about 90%, about 91%, about 92%, about 93%, about 94%, about 95%, about 96%, about 97%, or about 98% of the antigen-specific T cells are CD45RO+/CD28^{High} cells.

Embodiment 200 is the method of any one of embodiments 197-199, further comprising rapidly expanding the antigen-specific T cells to provide an expanded population of antigen-specific T cells.

Embodiment 201 is the method of any one of embodiments 192-200, further comprising separating activated T lymphocytes from unactivated T lymphocytes.

Embodiment 202 is the method of embodiment 201, wherein separating activated T cells includes detecting a plurality of surface biomarkers of the activated T cells.

Embodiment 203 is the kit, surface, or method of any one of the preceding embodiments, wherein the MHC molecule is a MHC Class I molecule.

Embodiment 204 is the kit, surface, or method of any one of embodiments 1-203, wherein the MHC molecule is a MHC Class II molecule.

Embodiment 205 is One or more activated T lymphocytes produced by the method of any one of embodiments 183-204.

Embodiment 206 is a population of T cells comprising activated T cells produced by the method of any one of embodiments 183-204.

Embodiment 207 is the cell or population of embodiment 205 or 206, wherein the activated T cells are CD45RO+.

Embodiment 208 is the cell or population of any one of embodiments 203-207, wherein the activated T cells are CD28+.

Embodiment 209 is the cell or population of any one of embodiments 203-208, wherein the activated T cells are CD28^{high}.

Embodiment 210 is the cell or population of any one of embodiments 203-209, wherein the activated T cells are CD127+.

Embodiment 211 is the cell or population of any one of embodiments 203-210, wherein the activated T cells are CD197+.

Embodiment 212 is the cell or population of any one of embodiments 203-211, wherein the activated T cells are CD8+.

Embodiment 213 is a microfluidic device comprising the cell or population of any one of embodiments 203-212.

Embodiment 214 is a pharmaceutical composition comprising the cell or population of any one of embodiments 203-212.

Embodiment 215 is a method of screening a plurality of peptide antigens for T-cell activation, the method comprising:
reacting a plurality of different peptide antigens with a plurality of proto-antigen-presenting surfaces according to any one of embodiments 40-168, thereby substantially displacing the exchange factors or initial peptides and forming a plurality of antigen-presenting surfaces;
contacting a plurality of T cells with the antigen-presenting surfaces; and
monitoring the T cells for activation, wherein activation of a T cell indicates that a peptide antigen associated with the surface with which the T cell was contacted is able to contribute to T cell activation.

Embodiment 216 is the method of embodiment 215, wherein the proto-antigen-presenting surfaces are reacted separately with the plurality of different peptide antigens, thereby generating a plurality of different antigen-presenting surfaces.

Embodiment 217 is the method of embodiment 215, wherein the proto-antigen-presenting surfaces are reacted separately with pools of members of the plurality of different peptide antigens, thereby generating a plurality of different antigen-presenting surfaces.

Embodiment 218 is the method of embodiment 217, wherein the pools of members of the plurality of different peptide antigens comprise overlapping pools.

Embodiment 219 is the method of embodiment 217, wherein the pools of members of the plurality of different peptide antigens comprise non-overlapping pools.

Embodiment 220 is the method of any one of embodiments 215-219, wherein the plurality of proto-antigen-presenting surfaces is a plurality of proto-antigen-presenting beads.

Embodiment 221 is the method of embodiment 220, wherein T cells are contacted separately with members of the plurality of different antigen-presenting beads.

Embodiment 222 is the method of embodiment 220, wherein T cells are contacted with a pool of the different antigen-presenting beads.

Embodiment 223 is the method of embodiment 220, wherein T cells are contacted with a plurality of pools of the different antigen-presenting beads.

Embodiment 224 is the method of embodiment 223, wherein the plurality of pools of the different antigen-presenting beads comprises overlapping pools.

Embodiment 225 is the method of embodiment 223, wherein the plurality of pools of the different antigen-presenting beads comprises non-overlapping pools.

Embodiment 226 is the method of any one of embodiments 220-225, wherein the T cells are in wells of a well plate when contacted with the antigen-presenting beads.

Embodiment 227 is the method of any one of embodiments 220-225, wherein the T cells are in a microfluidic device when contacted with the antigen-presenting beads.

Embodiment 228 is the method of any one of embodiments 220-225, wherein the T cells are in sequestration pens of a microfluidic device when contacted with the antigen-presenting beads.

Embodiment 229 is the method of any one of embodiments 220-228, further comprising (i) determining that T cells contacted with a pool of antigen-presenting beads underwent activation and (ii) contacting additional T cells with a member or subset of members of the pool, or with one or more additional antigen-presenting surfaces comprising the same peptide antigen or peptide antigens as a member or subset of members of the pool.

Embodiment 230 is the method of any one of embodiments 215-219, wherein the plurality of proto-antigen-presenting surfaces is a plurality of proto-antigen-presenting surfaces of a microfluidic device.

Embodiment 231 is the method of embodiment 230, wherein the plurality of proto-antigen-presenting surfaces of the microfluidic device are separated by regions of non-antigen-presenting surface.

Embodiment 232 is the method of embodiment 230 or 231, wherein the plurality of proto-antigen-presenting surfaces of a microfluidic device are in sequestration pens of the microfluidic device.

Embodiment 233 is the method of any one of embodiments 230-232, wherein individual antigen-presenting surfaces of the microfluidic device comprise pools of peptide antigens and the method further comprises (i) determining that T cells contacted with one or more of the antigen-presenting surfaces of the microfluidic device underwent activation and (ii) contacting additional T cells with one or more additional antigen-presenting surfaces comprising a member or subset of members of the peptide antigens associated with the one or more antigen-presenting surfaces of the microfluidic device.

Embodiment 234 is the method of any one of embodiments 215-219, wherein the plurality of proto-antigen-presenting surfaces is a plurality of proto-antigen-presenting surfaces in wells of one or more well plates.

Embodiment 235 is the method of embodiment 234, wherein the wells comprise non-antigen-presenting regions.

Embodiment 236 is the method of embodiment 234 or 235, wherein individual antigen-presenting surfaces of the one or more well plates comprise pools of peptide antigens and the method further comprises (i) determining that T cells contacted with one or more of the antigen-presenting surfaces one or more well plates underwent activation and (ii) contacting additional T cells with one or more additional antigen-presenting surfaces comprising a member or subset of members of the peptide antigens associated with the one or more antigen-presenting surfaces of the one or more well plates.

Embodiment 237 is the method of any one of embodiments 215-235, wherein the T cells include CD8+T cells.

Embodiment 238 is the method of any one of embodiments 215-237, wherein monitoring the T cells for activation comprises detecting a CD45RO+ activated T cell.

Embodiment 239 is the method of any one of embodiments 215-238, wherein monitoring the T cells for activation comprises detecting a CD28+ activated T cell.

Embodiment 240 is the method of any one of embodiments 215-239, wherein monitoring the T cells for activation comprises detecting a CD28^{high} activated T cell.

Embodiment 241 is the method of any one of embodiments 215-240, wherein monitoring the T cells for activation comprises detecting a CD127+ activated T cell.

Embodiment 242 is the method of any one of embodiments 215-241, wherein monitoring the T cells for activation comprises detecting a CD197+ activated T cell.

### BRIEF DESCRIPTION OF THE DRAWINGS

Figure 1A illustrates an example of a system for use with a microfluidic device and associated control equipment according to some embodiments of the disclosure.
Figures 1B and 1C illustrate a microfluidic device according to some embodiments of the disclosure.
Figures 2A and 2B illustrate sequestration pens according to some embodiments of the disclosure.
Figure 2C illustrates a detailed sequestration pen according to some embodiments of the disclosure.
Figures 2D-F illustrate sequestration pens according to some other embodiments of the disclosure.
Figure 2G illustrates a microfluidic device according to an embodiment of the disclosure.
Figure 2H illustrates a coated surface of the microfluidic device according to an embodiment of the disclosure.
Figure 3A illustrates a specific example of a system for use with a microfluidic device and associated control equipment according to some embodiments of the disclosure.
Figure 3B illustrates an imaging device according to some embodiments of the disclosure.
Figure 4 is a graphical representation of T cell activation pathways according to an embodiment of the disclosure.
Figures 5A and 5B are schematic representations of preparation of antigen-presenting surfaces according to various embodiments of the disclosure.
Figure 6 is a schematic representation of the process of preparing an antigen presenting surface according to an embodiment of the disclosure
Figures 7A and 7B are scanning electron micrograph representations of patterned antigen presenting surfaces according to some embodiments of the disclosure.
Figures 8A-8D are graphical representations of various characterization parameters for activation of T lymphocytes at 7 days of culturing, according to some embodiments of the disclosure.
Figure 9 is a graphical representation of the distribution of characteristics for activated T lymphocytes according to some embodiments of the disclosure.
Figure 10 is a graphical representation of the distribution of activated T lymphocytes after a first period of stimulation and culturing, comparing the use of antigen-presenting bead activation to dendritic cell activation, according to one embodiment of the disclosure.
Figure 11 is a graphical representation of the distribution of activated T lymphocytes after a second period of stimulation and culturing, comparing the use of antigen-presenting bead activation to dendritic cell activation, according to one embodiment of the disclosure.
Figure 12 is a graphical representation of various characterization parameters for activation of T lymphocytes at 7 and 14 days, compared to dendritic cell activation.
Figure 13 is a graphical representation of Fourier Transform Infrared spectra of a covalently functionalized polystyrene bead at selected steps of the functionalization.
Figures 14A-14D are graphical representations of various characterization parameters for activation of T cells, according to an embodiment of the disclosure.
Figures 15A-15E are graphical representations of cell product characterization according to an embodiment of the disclosure.
Figure 16 is a graphical representation of cell product characterization according to an embodiment of the disclosure.
Figure 17 is a graphical representation of cytotoxicity experiments according to one embodiment of the disclosure.
Figures 18A-18C are graphical representations of cell product characterization according to an embodiment of the disclosure.
Figures 19A-19F are graphical representations of the characterization of activation using an antigen-presenting surface according to some embodiments of the disclosure.
Figures 20A-20I are graphical representations of the characterization of activation using an antigen-presenting surface according to some embodiments of the disclosure.
Figures 21A-21F are graphical representations of characterization of activation using antigen-presenting surfaces according to some embodiments of the disclosure.
Figures 22A-22B are images of target cells taken at selected time points after being contacted with T lymphocytes and a Caspase 3 substrate in an antigen specific cytotoxicity assay according to some embodiments of the disclosure.
Figure 22C is a graphical representation of the course of an antigen specific cytotoxicity assay according to some embodiments of the disclosure.
Figures 23A-23E are graphical representations of the characterization of the cellular product obtained using an antigen-presenting surface according to some embodiments of the disclosure.
Figure 24 shows a quantitation of the peptide switching for the indicated peptides (SEQ ID NOs: 5 and 6 from left to right).
Figures 25A-B show a time course of median fluorescence intensity versus time for binding of a conformationally sensitive antibody which only recognizes pHLAs in the folded, complex conformation to pHLA beads loaded with either SLYSYFQKV (SEQ ID NO: 5) (Fig. 25A) or SLLPIMWQLY (SEQ ID NO: 6) (Fig. 25B) peptides.
Figures 26A-D show levels of surface markers for cultured cells following culture with standard pHLA or switched pHLA.
Figures 27A-C show frequencies of types of T cells following culture with standard pHLA or switched pHLA.

### DETAILED DESCRIPTION OF CERTAIN EMBODIMENTS

This specification describes exemplary embodiments and applications of the disclosure. The disclosure, however, is not limited to these exemplary embodiments and applications or to the manner in which the exemplary embodiments and applications operate or are described herein. Moreover, the figures may show simplified or partial views, and the dimensions of elements in the figures may be exaggerated or otherwise not in proportion. In addition, as the terms "on," "attached to," "connected to," "coupled to," or similar words are used herein, one element (e.g., a material, a layer, a substrate, etc.) can be "on," "attached to," "connected to," or "coupled to" another element regardless of whether the one element is directly on, attached to, connected to, or coupled to the other element or there are one or more intervening elements between the one element and the other element. Also, unless the context dictates otherwise, directions (e.g., above, below, top, bottom, side, up, down, under, over, upper, lower, horizontal, vertical, "x," "y," "z," etc.), if provided, are relative and provided solely by way of example and for ease of illustration and discussion and not by way of limitation. In addition, where reference is made to a list of elements (e.g., elements a, b, c), such reference is intended to include any one of the listed elements by itself, any combination of less than all of the listed elements, and/or a combination of all of the listed elements. The term "or" is used in an inclusive sense, i.e., equivalent to "and/or," unless the context dictates otherwise. It is noted that, as used in this specification and the appended claims, the singular forms "a," "an," and "the," and any singular use of any word, include plural referents unless expressly and unequivocally limited to one referent. As used herein, the terms "comprise," "include," and grammatical variants thereof are intended to be non-limiting, such that recitation of items in a list is not to the exclusion of other like items that can be substituted or added to the listed items. Section divisions in the specification are for ease of review only and do not limit any combination of elements discussed. In case of any contradiction or conflict between material incorporated by reference and the expressly described content provided herein, the expressly described content controls.

Where dimensions of microfluidic features are described as having a width or an area, the dimension typically is described relative to an x-axial and/or y-axial dimension, both of which lie within a plane that is parallel to the substrate and/or cover of the microfluidic device. The height of a microfluidic feature may be described relative to a z-axial direction, which is perpendicular to a plane that is parallel to the substrate and/or cover of the microfluidic device. In some instances, a cross sectional area of a microfluidic feature, such as a channel or a passageway, may be in reference to a x-axial/z-axial, a y-axial/z-axial, or an x-axial/y-axial area.

For the purposes of this specification and appended claims, unless otherwise indicated, all numbers expressing quantities, percentages, or proportions, and other numerical values used in the specification and claims, are to be understood as being modified in all instances by the term "about," to the extent they are not already so modified. "About" indicates a degree of variation that does not substantially affect the properties of the described subject matter, e.g., within 10%, 5%, 2%, or 1%. Accordingly, unless indicated to the contrary, the numerical parameters set forth in the following specification and attached claims are approximations that may vary depending upon the desired properties sought to be obtained. At the very least, and not as an attempt to limit the application of the doctrine of equivalents to the scope of the claims, each numerical parameter should at least be construed considering the number of reported significant digits and by applying ordinary rounding techniques.

### Definitions.

As used herein, "substantially" means sufficient to work for the intended purpose. The term "substantially" thus allows for minor, insignificant variations from an absolute or perfect state, dimension, measurement, result, or the like such as would be expected by a person of ordinary skill in the field but that do not appreciably affect overall performance. When used with respect to numerical values or parameters or characteristics that can be expressed as numerical values, "substantially" means within ten percent.

The term "ones" means more than one. As used herein, the term "plurality" can be 2, 3, 4, 5, 6, 7, 8, 9, 10, or more.

As used herein, "alkyl" refers to a straight or branched hydrocarbon chain radical consisting solely of carbon and hydrogen atoms, containing no unsaturation, having from one to six carbon atoms (e.g., C₁-C₆ alkyl). Whenever it appears herein, a numerical range such as "1 to 6" refers to each integer in the given range; e.g., "1 to 6 carbon atoms" means that the alkyl group may consist of 1 carbon atom, 2 carbon atoms, 3 carbon atoms, etc., up to and including 6 carbon atoms, although the present definition also covers the occurrence of the term "alkyl" where no numerical range is designated. In some embodiments, it is a C₁-C₃ alkyl group. Typical alkyl groups include, but are in no way limited to, methyl, ethyl, propyl, isopropyl, n-butyl, iso-butyl, sec-butyl isobutyl, tertiary butyl, pentyl, isopentyl, neopentyl, hexyl, and the like. The alkyl is attached to the rest of the molecule by a single bond, for example, methyl (Me), ethyl (Et), n-propyl, 1-methylethyl (isopropyl), n-butyl, n-pentyl, 1,1-dimethylethyl (t-butyl), hexyl, and the like.

Unless stated otherwise specifically in the specification, an alkyl group may be optionally substituted by one or more substituents which independently are: aryl, arylalkyl, heteroaryl, heteroarylalkyl, hydroxy, halo, cyano, trifluoromethyl, trifluoromethoxy, nitro, trimethylsilanyl, -OR', -SR', -OC(O)-R', -N(R')₂, -C(O)R', -C(O)OR', -OC(O)N(R')₂, -C(O)N(R')₂, -N(R')C(O)OR', -N(R')C(O)R', -N(R')C(O)N(R')₂, N(R')C(NR')N(R')₂, -N(R')S(O)ₜR'(where t is 1 or 2), -S(O)ₜOR'(where t is 1 or 2), -S(O)ₜN(R')2 (where t is 1 or 2), or PO₃(R')₂ where each R' is independently hydrogen, alkyl, fluoroalkyl, aryl, aralkyl, heterocycloalkyl, or heteroaryl.

As referred to herein, a fluorinated alkyl moiety is an alkyl moiety having one or more hydrogens of the alkyl moiety replaced by a fluoro substituent. A perfluorinated alkyl moiety has all hydrogens attached to the alkyl moiety replaced by fluoro substituents.

As referred to herein, a "halo" moiety is a bromo, chloro, or fluoro moiety.

As referred to herein, an "olefinic" compound is an organic molecule which contains an "alkene" moiety. An alkene moiety refers to a group consisting of at least two carbon atoms and at least one carboncarbon double bond. The non-alkene portion of the molecule may be any class of organic molecule, and in some embodiments, may include alkyl or fluorinated (including but not limited to perfluorinated) alkyl moieties, any of which may be further substituted.

As used herein, "air" refers to the composition of gases predominating in the atmosphere of the earth. The four most plentiful gases are nitrogen (typically present at a concentration of about 78% by volume, e.g., in a range from about 70-80%), oxygen (typically present at about 20.95% by volume at sea level, e.g. in a range from about 10% to about 25%), argon (typically present at about 1.0% by volume, e.g. in a range from about 0.1% to about 3%), and carbon dioxide (typically present at about 0.04%, e.g., in a range from about 0.01% to about 0.07%). Air may have other trace gases such as methane, nitrous oxide or ozone, trace pollutants and organic materials such as pollen, diesel particulates and the like. Air may include water vapor (typically present at about 0.25%, or may be present in a range from about 10ppm to about 5% by volume). Air may be provided for use in culturing experiments as a filtered, controlled composition and may be conditioned as described herein.

As used herein, the term "plurality" can be 2, 3, 4, 5, 6, 7, 8, 9, 10, or more.

As used herein, the term "disposed" encompasses within its meaning "located."

As used herein, a "microfluidic device" or "microfluidic apparatus" is a device that includes one or more discrete microfluidic circuits configured to hold a fluid, each microfluidic circuit comprised of fluidically interconnected circuit elements, including but not limited to region(s), flow path(s), channel(s), chamber(s), and/or pen(s), and at least one port configured to allow the fluid (and, optionally, micro-objects suspended in the fluid) to flow into and/or out of the microfluidic device. Typically, a microfluidic circuit of a microfluidic device will include a flow region, which may include a microfluidic channel, and at least one chamber, and will hold a volume of fluid of less than about 1 mL, e.g., less than about 750, 500, 250, 200, 150, 100, 75, 50, 25, 20, 15, 10, 9, 8, 7, 6, 5, 4, 3, or 2 µL. In certain embodiments, the microfluidic circuit holds about 1-2, 1-3, 1-4, 1-5, 2-5, 2-8, 2-10, 2-12, 2-15, 2-20, 5-20, 5-30, 5-40, 5-50, 10-50, 10-75, 10-100, 20-100, 20-150, 20-200, 50-200, 50-250, or 50-300 µL. The microfluidic circuit may be configured to have a first end fluidically connected with a first port (e.g., an inlet) in the microfluidic device and a second end fluidically connected with a second port (e.g., an outlet) in the microfluidic device.

As used herein, a "nanofluidic device" or "nanofluidic apparatus" is a type of microfluidic device having a microfluidic circuit that contains at least one circuit element configured to hold a volume of fluid of less than about 1 µL, e.g., less than about 750, 500, 250, 200, 150, 100, 75, 50, 25, 20, 15, 10, 9, 8, 7, 6, 5, 4, 3, 2, 1 nL or less. A nanofluidic device may comprise a plurality of circuit elements (e.g., at least 2, 3, 4, 5, 6, 7, 8, 9, 10, 15, 20, 25, 50, 75, 100, 150, 200, 250, 300, 400, 500, 600, 700, 800, 900, 1000, 1500, 2000, 2500, 3000, 3500, 4000, 4500, 5000, 6000, 7000, 8000, 9000, 10,000, or more). In certain embodiments, one or more (e.g., all) of the at least one circuit elements is configured to hold a volume of fluid of about 100 pL to 1 nL, 100 pL to 2 nL, 100 pL to 5 nL, 250 pL to 2 nL, 250 pL to 5 nL, 250 pL to 10 nL, 500 pL to 5 nL, 500 pL to 10 nL, 500 pL to 15 nL, 750 pL to 10 nL, 750 pL to 15 nL, 750 pL to 20 nL, 1 to 10 nL, 1 to 15 nL, 1 to 20 nL, 1 to 25 nL, or 1 to 50 nL. In other embodiments, one or more (e.g., all) of the at least one circuit elements are configured to hold a volume of fluid of about 20 nL to 200nL, 100 to 200 nL, 100 to 300 nL, 100 to 400 nL, 100 to 500 nL, 200 to 300 nL, 200 to 400 nL, 200 to 500 nL, 200 to 600 nL, 200 to 700 nL, 250 to 400 nL, 250 to 500 nL, 250 to 600 nL, or 250 to 750 nL.

A microfluidic device or a nanofluidic device may be referred to herein as a "microfluidic chip" or a "chip"; or "nanofluidic chip" or "chip".

A "microfluidic channel" or "flow channel" as used herein refers to flow region of a microfluidic device having a length that is significantly longer than both the horizontal and vertical dimensions. For example, the flow channel can be at least 5 times the length of either the horizontal or vertical dimension, e.g., at least 10 times the length, at least 25 times the length, at least 100 times the length, at least 200 times the length, at least 500 times the length, at least 1,000 times the length, at least 5,000 times the length, or longer. In some embodiments, the length of a flow channel is about 100,000 microns to about 500,000 microns, including any value therebetween. In some embodiments, the horizontal dimension is about 100 microns to about 1000 microns (e.g., about 150 to about 500 microns) and the vertical dimension is about 25 microns to about 200 microns, (e.g., from about 40 to about 150 microns). It is noted that a flow channel may have a variety of different spatial configurations in a microfluidic device, and thus is not restricted to a perfectly linear element. For example, a flow channel may be, or include one or more sections having, the following configurations: curve, bend, spiral, incline, decline, fork (e.g., multiple different flow paths), and any combination thereof. In addition, a flow channel may have different cross-sectional areas along its path, widening and constricting to provide a desired fluid flow therein. The flow channel may include valves, and the valves may be of any type known in the art of microfluidics. Examples of microfluidic channels that include valves are disclosed in U.S. Patents 6,408,878 and 9,227,200, each of which is herein incorporated by reference in its entirety.

As used herein, the term "obstruction" refers generally to a bump or similar type of structure that is sufficiently large so as to partially (but not completely) impede movement of target micro-objects between two different regions or circuit elements in a microfluidic device. The two different regions/circuit elements can be, for example, a microfluidic sequestration pen and a microfluidic channel, or a connection region and an isolation region of a microfluidic sequestration pen.

As used herein, the term "constriction" refers generally to a narrowing of a width of a circuit element (or an interface between two circuit elements) in a microfluidic device. The constriction can be located, for example, at the interface between a microfluidic sequestration pen and a microfluidic channel, or at the interface between an isolation region and a connection region of a microfluidic sequestration pen.

As used herein, the term "transparent" refers to a material which allows visible light to pass through without substantially altering the light as is passes through.

As used herein, the term "micro-object" refers generally to any microscopic object that may be isolated and/or manipulated in accordance with the present diclosure. Non-limiting examples of micro-objects include: inanimate micro-objects such as microparticles; microbeads (e.g., polystyrene beads, Luminex^{™} beads, or the like); magnetic beads; microrods; microwires; quantum dots, and the like; biological micro-objects such as cells; biological organelles; vesicles, or complexes; synthetic vesicles; liposomes (e.g., synthetic or derived from membrane preparations); lipid nanorafts, and the like; or a combination of inanimate micro-objects and biological micro-objects (e.g., microbeads attached to cells, liposome-coated micro-beads, liposome-coated magnetic beads, or the like). Beads may include moieties/molecules covalently or non-covalently attached, such as fluorescent labels, proteins, carbohydrates, antigens, small molecule signaling moieties, or other chemical/biological species capable of use in an assay. Lipid nanorafts have been described, for example, in Ritchie et al. (2009) "Reconstitution of Membrane Proteins in Phospholipid Bilayer Nanodiscs," Methods Enzymol., 464:211-231.

As used herein, the term "cell" is used interchangeably with the term "biological cell." Non-limiting examples of biological cells include eukaryotic cells, plant cells, animal cells, such as mammalian cells, reptilian cells, avian cells, fish cells, or the like, prokaryotic cells, bacterial cells, fungal cells, protozoan cells, or the like, cells dissociated from a tissue, such as muscle, cartilage, fat, skin, liver, lung, neural tissue, and the like, immunological cells, such as T cells, B cells, natural killer cells, macrophages, and the like, embryos (e.g., zygotes), oocytes, ova, sperm cells, hybridomas, cultured cells, cells from a cell line, cancer cells, infected cells, transfected and/or transformed cells, reporter cells, and the like. A mammalian cell can be, for example, from a human, a mouse, a rat, a horse, a goat, a sheep, a cow, a primate, or the like.

A colony of biological cells is "clonal" if all of the living cells in the colony that are capable of reproducing are daughter cells derived from a single parent cell. In certain embodiments, all the daughter cells in a clonal colony are derived from the single parent cell by no more than 10 divisions. In other embodiments, all the daughter cells in a clonal colony are derived from the single parent cell by no more than 14 divisions. In other embodiments, all the daughter cells in a clonal colony are derived from the single parent cell by no more than 17 divisions. In other embodiments, all the daughter cells in a clonal colony are derived from the single parent cell by no more than 20 divisions. The term "clonal cells" refers to cells of the same clonal colony.

As used herein, a "colony" of biological cells refers to 2 or more cells (e.g. about 2 to about 20, about 4 to about 40, about 6 to about 60, about 8 to about 80, about 10 to about 100, about 20 to about 200, about 40 to about 400, about 60 to about 600, about 80 to about 800, about 100 to about 1000, or greater than 1000 cells).

As used herein, the term "maintaining (a) cell(s)" refers to providing an environment comprising both fluidic and gaseous components and, optionally a surface, that provides the conditions necessary to keep the cells viable and/or expanding.

As used herein, the term "expanding" when referring to cells, refers to increasing in cell number.

As referred to herein, "gas permeable" means that the material or structure is permeable to at least one of oxygen, carbon dioxide, or nitrogen. In some embodiments, the gas permeable material or structure is permeable to more than one of oxygen, carbon dioxide and nitrogen and may further be permeable to all three of these gases.

A "component" of a fluidic medium is any chemical or biochemical molecule present in the medium, including solvent molecules, ions, small molecules, antibiotics, nucleotides and nucleosides, nucleic acids, amino acids, peptides, proteins, sugars, carbohydrates, lipids, fatty acids, cholesterol, metabolites, or the like.

As used herein in reference to a fluidic medium, "diffuse" and "diffusion" refer to thermodynamic movement of a component of the fluidic medium down a concentration gradient.

The phrase "flow of a medium" means bulk movement of a fluidic medium primarily due to any mechanism other than diffusion. For example, flow of a medium can involve movement of the fluidic medium from one point to another point due to a pressure differential between the points. Such flow can include a continuous, pulsed, periodic, random, intermittent, or reciprocating flow of the liquid, or any combination thereof. When one fluidic medium flows into another fluidic medium, turbulence and mixing of the media can result.

The phrase "substantially no flow" refers to a rate of flow of a fluidic medium that, when averaged over time, is less than the rate of diffusion of components of a material (e.g., an analyte of interest) into or within the fluidic medium. The rate of diffusion of components of such a material can depend on, for example, temperature, the size of the components, and the strength of interactions between the components and the fluidic medium.

As used herein in reference to different regions within a microfluidic device, the phrase "fluidically connected" means that, when the different regions are substantially filled with fluid, such as fluidic media, the fluid in each of the regions is connected so as to form a single body of fluid. This does not mean that the fluids (or fluidic media) in the different regions are necessarily identical in composition. Rather, the fluids in different fluidically connected regions of a microfluidic device can have different compositions (e.g., different concentrations of solutes, such as proteins, carbohydrates, ions, or other molecules) which are in flux as solutes move down their respective concentration gradients and/or fluids flow through the device.

As used herein, a "flow path" refers to one or more fluidically connected circuit elements (e.g. channel(s), region(s), chamber(s) and the like) that define, and are subject to, the trajectory of a flow of medium. A flow path is thus an example of a swept region of a microfluidic device. Other circuit elements (e.g., unswept regions) may be fluidically connected with the circuit elements that comprise the flow path without being subject to the flow of medium in the flow path.

As used herein, "isolating a micro-object" confines a micro-object to a defined area within the microfluidic device.

A microfluidic (or nanofluidic) device can comprise "swept" regions and "unswept" regions. As used herein, a "swept" region is comprised of one or more fluidically interconnected circuit elements of a microfluidic circuit, each of which experiences a flow of medium when fluid is flowing through the microfluidic circuit. The circuit elements of a swept region can include, for example, regions, channels, and all or parts of chambers. As used herein, an "unswept" region is comprised of one or more fluidically interconnected circuit element of a microfluidic circuit, each of which experiences substantially no flux of fluid when fluid is flowing through the microfluidic circuit. An unswept region can be fluidically connected to a swept region, provided the fluidic connections are structured to enable diffusion but substantially no flow of media between the swept region and the unswept region. The microfluidic device can thus be structured to substantially isolate an unswept region from a flow of medium in a swept region, while enabling substantially only diffusive fluidic communication between the swept region and the unswept region. For example, a flow channel of a micro-fluidic device is an example of a swept region while an isolation region (described in further detail below) of a microfluidic device is an example of an unswept region.

As used herein, a "non-sweeping" rate of fluidic medium flow means a rate of flow in a flow region, such as a microfluidic channel, which is sufficient to permit components of a second fluidic medium in an isolation region of the sequestration pen to diffuse into the first fluidic medium in the flow region and/or components of the first fluidic medium to diffuse into the second fluidic medium in the isolation region; and further wherein the first medium does not substantially flow into the isolation region.

As used herein, "synthetic surface" refers to an interface between a support structure and a gaseous/liquid medium, where the synthetic surface is prepared by non-biological processes. The synthetic surface may have biologically derived materials connected to it, e.g., primary and co-activating molecules as described herein, to provide an antigen-presenting synthetic surface, provided that the synthetic surface is not expressed by a biological organism. Typically, the support structure is solid, such as the non-surface exposed portions of a bead, a wafer, or a substrate, cover or circuit material of a microfluidic device and does not enclose a biological nucleus or organelle.

As used herein, "co-activating" refers to a binding interaction between a biological macromolecule, fragment thereof, or synthetic or modified version thereof and a T cell, other than the primary T cell receptor/antigen:MHC binding interaction, that enhances a productive immune response to produce activation of the T cell. Co-activating interactions are antigen-nonspecific interactions, e.g., between a T-cell surface protein able to engage in intracellular signaling such as CD28, CD2, ICOS, etc., and an agonist thereof. "Coactivation" and "co-activating" as used herein is equivalent to the terms co-stimulation and co-stimulating, respectively.

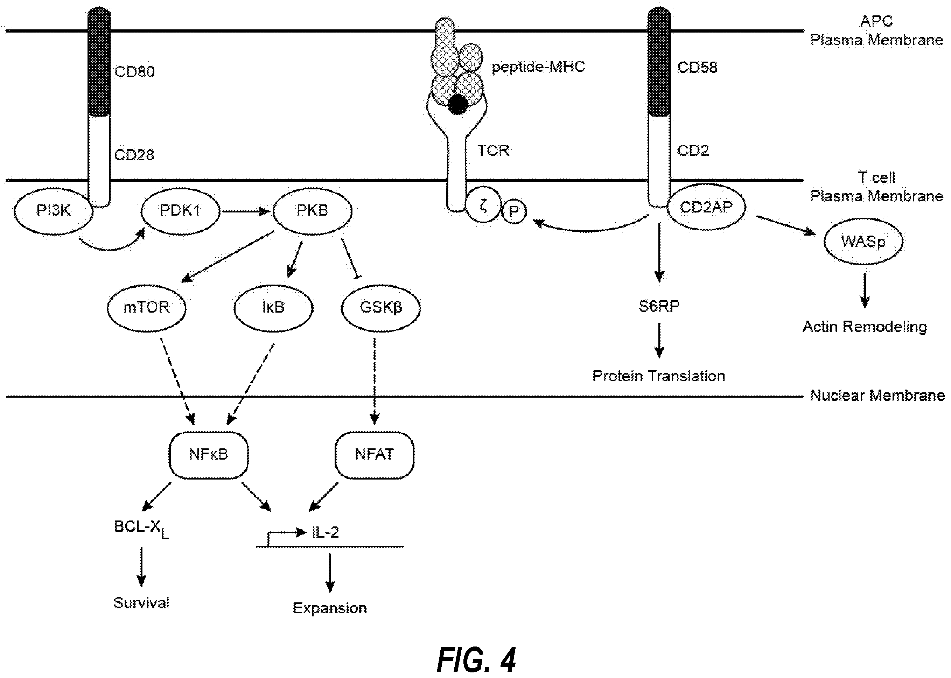

As used herein, a "TCR co-activating molecule" is a biological macromolecule, fragment thereof, or synthetic or modified version thereof that binds to one or more co-receptors on a T Cell that activate distal signaling molecules which amplify and/or complete the response instigated by antigen specific binding of the TCR. In one example, signaling molecules such as transcription factors Nuclear Factor kappa B (NF kB) and Nuclear factor of activated T cells (NFAT) are activated by the TCR co-activating molecule. The TCR coactivating molecule can be, for example, an agonist of the CD28 receptor, which signals through the phosphoinositide 3 kinase (PI3K)/Akt pathway. See FIG. 4.

As used herein, "CD28high" refers to a phenotype of high CD28 surface expression in a T cell. Those skilled in the art are familiar with the CD28high phenotype and appropriate ways of identifying CD28high T cells. Unless otherwise indicated, CD28high T cells include T cells that meet any of the following criteria. In some embodiments, a CD28high T cell is a T cell that expresses higher levels of CD28 than a resting CD8+T cell. A CD28high T cell may also express higher levels of CD28 than an irrelevant non-antigen specific T cell. In some embodiments, CD28high T cells are a population in which the level of surface CD28 which can be measured by FACS is equal to or greater than the level of surface CD28 present on circulating memory T cells which can be measured by FACS. In some embodiments, a CD28high T cell has a level of surface CD28 equal to or greater than the level of surface CD28 present on circulating memory T cells from the same sample or individual. Expression of surface CD28 can be determined by FACS and the mean (e.g., geometric mean) or median level of surface CD28 present on circulating memory T cells can be used for determining whether a given T cell is CD28high. In some embodiments, a CD28high T cell is a T cell that expresses CD28 at a significantly higher level than expression typical of naïve CD8 T cells from the same sample or individual, e.g., higher than 75%, 80%, 85%, 87.5%, 90%, 92.5%, or 95% of the naïve T cells. Naïve CD8 T cells can be identified and characterized by known methods, e.g., flow cytometrically, as CD8+ cells expressing detectable CD28 and minimal or no CD45RO.

As used herein, a "TCR adjunct activating molecule" stimulates classes of signaling molecules which amplify the antigen-specific TCR interaction and are distinct from the TCR co-activating molecules. For example, TCR proximal signaling by phosphorylation of the TCR proximal signaling complex is one route by which TCR adjunct activating molecules can act. The TCR adjunct activating molecule may be, for example, an agonist of the CD2 receptor. See FIG. 4.

As used herein, an "activated T cell" is a T cell that has experienced antigen (or a descendant thereof) and is capable of mounting an antigen-specific response to that antigen. Activated T cells are generally positive for at least one of CD28, CD45RO, CD127, and CD197.

As used herein, a "biotin functionality" refers to a moiety of a larger molecule or ligand wherein the moiety comprises a covalently bound form of biotin (and may further comprise a linker). In general, a molecule that is biotinylated comprises a biotin functionality.

As used herein, a "molecular ligand" refers to a surface-associated form of a molecule. The surfaceassociation may be a covalent or noncovalent association.

As used herein, an "exchange factor" refers to a compound of the general formula A-B, wherein A comprises one or more amino acid residues and B comprises a C-terminal amino acid residue, wherein the side chain of the C-terminal amino acid residue comprises at least three non-hydrogen atoms (e.g., carbon, nitrogen, oxygen, and/or sulfur). A and B may be but are not necessarily linked by a peptide bond formed between the carboxyl of the first amino acid residue and the amine of the second amino acid residue. The amino acid residues may be but are not necessarily members of the set of 20 canonical naturally occurring amino acids. For example, nonstandard amino acids such as homoleucine, norleucine, cyclohexylalanine, and the like are encompassed. Modified amino acid residues, e.g., wherein the residues comprise an alternative linkage such as a lactam or piperazinone in place of a simple peptide bond are also encompassed, as are peptide-like compounds as described in US2014/0370524. Exchange factors can bind in the antigen-binding pocket of a major histocompatibility complex but have sufficiently low affinity to be displaced by peptide antigens suitable for binding to and presentation by the MHC. When present in excess relative to a peptide antigen already bound to the MHC, exchange factors can displace the already bound peptide and then be displaced in turn by a new peptide antigen, thereby catalyzing a peptide exchange reaction.

As used herein, a "peptide antigen" (also sometimes referred to as an antigenic peptide) refers to a peptide that can bind in the antigen-binding pocket (also known as the antigen-binding groove or peptidebinding groove) of a major histocompatibility complex (MHC). In some embodiments, a peptide antigen is able to contribute to activation of a T lymphocyte, such as a cytotoxic T lymphocyte (e.g., which can be a naïve T cell, a central memory T cell, or the like), when the peptide antigen is bound in the antigen-binding pocket of a major histocompatibility complex (MHC), e.g., a class I MHC. In some embodiments, the peptide antigen is a candidate peptide that may or may not be able to contribute to activation of a T lymphocyte, such as a cytotoxic T lymphocyte, when the peptide antigen is bound in the antigen-binding pocket of a major histocompatibility complex (MHC), e.g., a class I MHC.

As used herein, an "initial peptide" refers to a peptide that can bind to an MHC molecule and then undergo displacement from the MHC molecule in an exchange reaction in the presence of an exchange factor and an incoming peptide antigen.

As used herein, a peptide is "non-immunogenic" when it is not capable of generating an adaptive immune response in the in the organism from which it originated, which may be a mammal, such as a human. Non-immunogenic peptides include peptides against which the organism's immune system has been tolerized.

As used herein, a "non-antigen-presenting surface" refers to a surface or region of a larger surface substantially free of primary activating molecular ligands.

### Overview.

Immunotherapy for cancer is a promising development, but requires specifically activated T lymphocytes which are compatible with the subject of the therapy. However, current approaches for activating T lymphocytes present several disadvantageous aspects. These include the need to identify suitable peptide antigens for use in activation using laborious approaches involving generating dendritic cells or otherwise preparing MHCs comprising candidate peptide antigens so that one can evaluate their immunogenicity. Dendritic cells must be obtained from donor sources, limiting throughput. Dendritic cells must be matured for each sequence of T lymphocyte activation, which requires a lead time of about 7 days. Irradiation of dendritic cells is also required, which limits where such processing can be performed. On the other hand, synthetic antigen-presentation approaches have used folding reactions to prepare MHCs comprising candidate peptide antigens, which require considerable time and effort.

Replacing the use of autologous antigen presenting dendritic cells and folding reactions with proto-antigen presenting synthetic surfaces for generating MHCs complexed with peptide antigens for evaluating immunogenicity and activating T lymphocytes may afford more rapid or cost-effective results or may enable greater reliability in stimulating and expanding T lymphocytes for a therapeutically relevant population by facilitating identification of more immunogenic peptide antigens. Proto-antigen presenting synthetic surfaces may be engineered for antigen-specific activation of T lymphocytes upon reaction with a peptide antigen, providing more controllable, characterizable, reproducible and/or more rapid development of populations of activated T lymphocytes having desirable phenotypes for treatment of cancer. Antigen-presenting synthetic surfaces generated from such proto-antigen presenting synthetic surfaces can also allow for more control and selectivity over T cell activation, including more precise targeting of desired T cell phenotypes following activation, e.g., enrichment of particular forms of memory T cells. Furthermore, proto-antigen-presenting synthetic surfaces can also exploit economies of scale and/or provide reproducibility to a greater degree than using autologous antigen presenting dendritic cells or folding reactions to prepare MHCs comprising a peptide antigen. As such, this technology can make cellular therapies available to patients in need thereof in greater numbers and/or in less time. Providing T cells useful for cellular therapies more rapidly can be especially important for patients with advanced disease. The structure of such proto-antigen-presenting synthetic surfaces and their methods of preparation and use are described herein. In some embodiments, the proto-antigen-presenting synthetic surfaces comprise primary activating ligands in combination with TCR coactivating molecules and/or adjunct TCR activating molecules, which serve to activate T cells together with the MHC upon formation of a complex with a peptide antigen. In some embodiments, the proto-antigen-presenting synthetic surfaces and their methods of preparation and use provide one or more of the foregoing advantages, or at least provide the public with a useful choice.

### Proto-antigen-presenting synthetic surfaces.

A proto-antigen-presenting synthetic surface is provided herein for activating a T lymphocyte (T cell) comprising a plurality of primary activating molecular ligands, wherein each primary activating molecular ligand includes a major histocompatibility complex (MHC) molecule configured to bind to a T cell receptor (TCR) of a T cell and wherein an exchange factor or an initial peptide is bound to the MHC molecules, optionally wherein the initial peptide is non-immunogenic. The exchange factor or initial peptide may have any of the features described herein for exchange factors or initial peptides, respectively. In some embodiments, the exchange factor or initial peptide is bound in the antigen-binding pocket of the MHC. In some embodiments, each of the plurality of primary activating molecular ligands and the plurality of co-activating molecular ligands is specifically bound to the antigen presenting synthetic surface. Each primary activating molecular ligand can comprise a major histocompatibility complex (MHC) molecule configured to bind to a T cell receptor of the T cell. In some embodiments, the MHC molecule is an MHC Class I molecule. In some other embodiments, the MHC molecule is an MHC Class II molecule. In some embodiments, the plurality of co-activating molecular ligands comprises a plurality of T cell receptor (TCR) co-activating molecules and a plurality of adjunct TCR activating molecules. In some embodiments, the T cell receptor (TCR) co-activating molecules and the adjunct TCR activating molecules are present in a ratio of about 1:100 to about 100:1, e.g., about 20:1 to about 1:20, or about 10:1 to about 1:20. In some embodiments, one or more of the plurality of co-activating molecular ligands is a TCR co-activating molecule which can activate signaling molecules such as transcription factors Nuclear Factor kappa B (NF kB) and Nuclear factor of activated T cells (NFAT). In some embodiments, the TCR co-activating molecule is an agonist of the CD28 receptor, which signals through the phosphoinositide 3 kinase (PI3K)/Akt pathway. In some embodiments, one or more of the plurality of co-activating molecular ligands is a TCR adjunct activating molecule which activates TCR proximal signaling, e.g., by phosphorylation of the TCR proximal signaling complex. The TCR adjunct activating molecule may be, for example, an agonist of the CD2 receptor. Exemplary pathways that can be activated through the CD28 and CD2 receptors (and additional details) are shown in FIG. 4. The proto-antigen-presenting synthetic surface may be a proto-antigen-presenting bead, proto-antigen-presenting wafer, a proto-antigen-presenting inner surface of a tube (e.g., glass or polymer tube), or a proto-antigen-presenting inner surface of a microfluidic device. The proto-antigen-presenting microfluidic device may be any microfluidic device as described herein, and may have any combination of features described herein.

In various embodiments, the proto-antigen-presenting synthetic surface is configured to generate an antigen-presenting synthetic surface that can activate a T lymphocyte in vitro. The primary activating molecular ligand may comprise a MHC molecule having an amino acid sequence and may be connected covalently to the proto-antigen-presenting synthetic surface via a C-terminal connection. The MHC molecule may present a N-terminal portion of the MHC molecule oriented away from the surface, thereby facilitating specific binding of the MHC molecule with the TCR of a T lymphocyte disposed upon the surface. The MHC molecule may include a MHC peptide. Clusters of at least four of the MHC molecules may be disposed at locations upon the proto-antigen-presenting synthetic surface such that when the surface is exposed to an aqueous environment, an MHC tetramer may be formed.

In some embodiments, each of the plurality of primary activating molecular ligands may be covalently connected to the antigen presenting synthetic surface via a linker. In some embodiments, an MHC molecule of a primary activating molecular ligand may be connected to the proto-antigen-presenting synthetic surface through a covalent linkage. Covalent linkages can be formed, for example, using Click chemistry and an appropriate Click reagent pair. Likewise, other ligands described herein, such as co-activating molecular ligands (comprising TCR co-activating molecules and/or adjunct TCR activating molecules), growth stimulatory molecular ligands, and additional stimulatory molecular ligands may be covalently connected to the surface of the antigen presenting synthetic surface via a linker, and the linkage can be formed using Click chemistry and an appropriate Click reagent pair.

In other embodiments, the MHC molecule may be connected to the antigen presenting synthetic surface noncovalently through a coupling group (CG), such as a biotin/streptavidin binding pair interaction. In some embodiments, one member of the coupling group is covalently associated with the surface (e.g., streptavidin). Further examples of coupling groups include, but are not limited to biotin/avidin, biotin/NeutrAvidin, and digoxygenin/anti-digoxygenin. Streptavidin, avidin, and NeutrAvidin represent examples of biotin-binding agents. Likewise, other ligands described herein, such as co-activating molecular ligands (comprising TCR co-activating molecules and/or adjunct TCR activating molecules), growth stimulatory molecular ligands, and additional stimulatory molecular ligands may be noncovalently coupled to the antigen presenting synthetic surface, and the coupling group may include biotin or digoxygenin.

In some embodiments, one member of the CG binding pair may itself be covalently bound to the surface, e.g., through one or more linkers. The covalent linkange to the surface can be through a series of about 15, 20, 25, 30, 35, 40, 45, 50, 55, 60, 65, 70 , 75, 80, 85, 95, 100, 200 bond lengths, or any number of bond lengths therebetwteen. In some embodiments, the member of the CG binding pair covalently bound to the surface is bound through a Click reagent pair. This may also be true for CG binding pair members involved in associating other ligands described herein (such as co-activating molecular ligands, TCR co-activating molecules, adjunct TCR activating molecules, growth stimulatory molecules, and additional stimulatory molecules) with the surface. Further, since some binding pair members such as streptavidin have multiple binding sites (e.g., four in streptavidin), a primary activating molecular ligand may be coupled to the antigen presenting synthetic surface by a biotin/streptavidin/biotin linkage. Again, this may also be true for CG binding pair members involved in associating other ligands described herein (such as co-activating molecular ligands, TCR co-activating molecules, adjunct TCR activating molecules, growth stimulatory molecules, and additional stimulatory molecules) with the surface.

In some embodiments, a first member of the CG binding pair is covalently associated with the primary activating molecular ligand and a second member of the CG binding pair is non-covalently associated with the surface. For example, the first member of the CG binding pair can be a biotin covalently associated with the primary activating molecular ligand; and the second member of the CG binding pair can be a streptavidin non-covalently associated with the surface (e.g., through an additional biotin, wherein the additional biotin is covalently associated with the surface). In some embodiments, the biotin covalently associated with the surface is linked to the surface through a series of about 15, 20, 25, 30, 35, 40, 45, 50, 55, 60, 65, 70 , 75, 80, 85, 95, 100, 200 bond lengths, or any number of bond lengths therebetwteen. For example, the biotin covalently associated with the surface may be linked to the surface through a series of one or more linkers having a total length as described. Again, this may also be true for CG binding pair members involved in associating other ligands described herein (such as co-activating molecular ligands, TCR coactivating molecules, adjunct TCR activating molecules, growth stimulatory molecules, and additional stimulatory molecules) with the surface. Noncovalently associating the second member of the CG binding pair, such as streptavidin, with the surface may facilitate loading ligands such as primary activating molecular ligands, co-activating molecular ligands, TCR co-activating molecules, and adjunct TCR activating molecules at greater densities than if the second member of the CG binding pair is covalently associated with the surface.

The primary activating molecular ligand (e.g., comprising a MHC molecule) further includes an exchange factor as described herein, e.g., in the section concerning exchange factors. Any suitable exchange factor may be used.

The antigen presenting synthetic surface includes a plurality of co-activating molecular ligands each comprising a TCR co-activating molecule or an adjunct TCR activating molecule. In some embodiments, the plurality of co-activating molecular ligands include a plurality of TCR co-activating molecules. In some embodiments, the plurality of co-activating molecular ligands include a plurality of adjunct TCE activating molecules. In other embodiments, the plurality of co-activating molecular ligands may include TCR coactivating molecules and adjunct TCR activating molecules. The TCR co-activating molecules and the adjunct TCR activating molecules can be present in a ratio of one to the other such as 100:1 to 1:100, 10:1 to 1:20, 5:1 to 1:5, 3:1 to 1:3, 2:1 to 1:2, or the like, wherein each of the foregoing values can be modified by "about." In some embodiments, the plurality of co-activating molecular ligands may include TCR co-activating molecules and adjunct TCR activating molecules in a ratio ranging from about 3:1 to about 1:3.

The TCR co-activating molecule or adjunct TCR activating molecule may include a protein, e.g., an antibody or a fragment thereof. In some embodiments, the TCR co-activating molecule may be a CD28 binding molecule (e.g., including a CD80 molecule) or a fragment thereof which retains binding ability to CD28. In some embodiments, the TCR co-activating molecule may be a CD28 binding molecule (e.g., including a CD80 molecule) or a fragment thereof which specifically binds to CD28. In some embodiments, the TCR coactivating molecule may be a CD28 binding molecule (e.g., including a CD80 molecule) or a CD28-binding fragment thereof. In some embodiments, the TCR co-activating molecule may include an anti-CD28 antibody or a fragment thereof (e.g., a CD28-binding fragment).

In some embodiments, each of the plurality of co-activating molecular ligands may be covalently connected to the proto-antigen-presenting synthetic surface via a linker. In other embodiments, each of the plurality of co-activating molecular ligands may be noncovalently bound to a linker covalently bound to the proto-antigen-presenting synthetic surface. The TCR co-activating molecule or adjunct TCR activating molecule may be connected to the covalently modified surface noncovalently through a CG, such as a biotin/streptavidin binding pair interaction. For example, the TCR co-activating molecule or adjunct TCR activating molecule may further comprise a site-specific C-terminal biotin moiety that interacts with a streptavidin, which may be associated covalently or noncovalently with the surface as described herein. A site-specific C-terminal biotin moiety can be added to a TCR co-activating molecule or adjunct TCR activating molecule using known methods, e.g., using a biotin ligase such as the BirA enzyme. See, e.g., Fairhead et al., Methods Mol Biol 1266:171-184, 2015. Further examples of coupling groups include biotin/avidin, biotin/NeutrAvidin, and digoxygenin/anti-digoxygenin. In some embodiments, one of the CG binding pair may itself be covalently bound to the surface, e.g., through a linker, as described above. See the examples for exemplary TCR co-activating molecules or adjunct TCR activating molecules.

In some other embodiments, the co-activating molecular ligands of the proto-antigen-presenting synthetic surface may include a plurality of adjunct TCR activating molecules, e.g., in addition to or instead of a TCR co-activating molecule as described herein. In some further embodiments, there may be additional coactivating molecular ligands. In some embodiments, the adjunct TCR activating molecules or additional coactivating molecular ligands comprise one or more of a CD2 agonist, a CD27 agonist, or a CD137 agonist. For example, the adjunct TCR activating molecule may be a CD2 binding protein or a fragment thereof, where the fragment retains binding ability with CD2. In some embodiments, the adjunct TCR activating molecule may be CD58 or a fragment thereof which retains binding ability with CD2. The adjunct TCR activating molecule may be a CD2 binding protein (e.g., CD58) or a fragment thereof, where the fragment specifically binds CD2. The adjunct TCR activating molecule may be a CD2 binding protein (e.g., CD58) or a CD2-binding fragment thereof. The adjunct TCR activating molecules or additional co-activating molecular ligands may each be an antibody to CD2, CD27, or CD137, or there may be any combination of such antibodies. The adjunct TCR activating molecules or additional co-activating molecular ligands may alternatively each be a fragment of an antibody to CD2, CD27, or CD137, or any combination thereof. Varlilumab (CDX-1127) is an exemplary anti-CD27 antibody. Utomilumab (PF-05082566) is an exemplary anti-CD137 antibody. CD70 or an extracellular portion thereof may also be used as a CD27 agonist. TNFSF9, also known as CD137L, or an extracellular portion thereof may also be used as a CD137 agonist. In some embodiments, the adjunct TCR activating molecules comprise an agonist of CD2, such as an anti-CD2 antibody. In some embodiments, each of the adjunct TCR activating molecules may be covalently connected to the surface via a linker. In other embodiments, each of the adjunct TCR activating molecules may be noncovalently bound to a linker covalently bound to the surface, e.g., through a CG, such as a biotin/streptavidin binding pair interaction. For example, the adjunct TCR activating molecules may comprise a site-specific C-terminal biotin moiety as discussed above that interacts with a streptavidin, which may be associated covalently or noncovalently with the surface as described herein. Further examples of coupling groups include biotin/avidin, biotin/NeutrAvidin, and digoxygenin/anti-digoxygenin. In some embodiments, one of the CG binding pair may itself be covalently bound to the surface, e.g., through a linker.

The proto-antigen-presenting synthetic surface may further include at least one growth stimulatory molecular ligand. The growth stimulatory molecular ligand may be a protein or peptide. The growth stimulatory protein or peptide may be a cytokine or fragment thereof. The growth stimulatory protein or peptide may be a growth factor receptor ligand. The growth stimulatory molecular ligand may comprise IL-21 or a fragment thereof. In some embodiments, the growth stimulatory molecular ligand may be connected to the proto-antigen-presenting synthetic surface via a covalent linker. In other embodiments, the growth stimulatory molecular ligand may be connected to the proto-antigen-presenting synthetic surface through a CG, such as a biotin/streptavidin binding pair interaction. Further examples of coupling groups include biotin/avidin, biotin/NeutrAvidin, and digoxygenin/anti-digoxygenin. In some embodiments, one of the CG binding pair may itself be covalently bound to the surface, e.g., through a linker. In other embodiments, the growth stimulatory molecular ligand may be attached to a surface either covalently or via a biotin/streptavidin binding interaction, where the surface is not the same surface as the proto-antigen-presenting synthetic surface having MHC molecules connected thereto. For example, the surface to which the growth stimulatory molecular ligand is attached can be a second surface of a microfluidic device also comprising a first, proto-antigen-presenting synthetic surface.

In yet other embodiments, there may be additional growth stimulatory molecular ligands, which may be one or more cytokines, or fragments thereof. In some embodiments, additional stimulatory molecular ligands including, but not limited to IL-2 or IL-7 may be connected to a the proto-antigen-presenting synthetic surface or to another surface that is not the proto-antigen-presenting synthetic surface, as discussed above with respect to growth stimulatory molecular ligands.

In some embodiments, the proto-antigen-presenting synthetic surface comprises an adhesion stimulatory molecular ligand, which is a ligand for a cell adhesion receptor including an ICAM protein sequence.

The additional stimulatory molecular ligands and/or adhesion stimulatory molecular ligands may be covalently connected to a surface or may be noncovalently connected to a surface through a CG, such as a biotin/streptavidin binding pair interaction. Further examples of coupling groups include biotin/avidin, biotin/NeutrAvidin, and digoxygenin/anti-digoxygenin. In some embodiments, one of the CG binding pair may itself be covalently bound to the surface, e.g., through a linker via a biotin/streptavidin binding interaction.

In some embodiments, the proto-antigen-presenting synthetic surface comprises a plurality of surface-blocking molecular ligands, which may include a linker and a terminal surface-blocking group. The linker can include a linear chain of 6 or more atoms (e.g., 7, 8, 9, 10, 11, 12, 13, 14, 15, 20, 25, 30, 35, 40, 45, 50, 75, 100, or more atoms) covalently linked together. Optionally, the linker has a linear structure. The terminal surface-blocking group may be a hydrophilic moiety, an amphiphilic moiety, a zwitterionic moiety, or a negatively charged moiety. In some embodiments, the terminal blocking group comprises a terminal hydroxyl group. In some embodiments, the terminal blocking group comprises a terminal carboxyl group. In some embodiments, the terminal blocking group comprises a terminal zwitterionic group. The plurality of surface-blocking molecular ligands may have all the same terminal surface-blocking group or may have a mixture of terminal surface-blocking groups. Without being bound by theory, the terminal surface-blocking group as well as a hydrophilic linker of the surface-blocking molecular ligand may interact with water molecules in the aqueous media surrounding the proto-antigen-presenting synthetic surface to create a more hydrophilic surface overall.This enhanced hydrophilic nature may render the contact between the proto-antigen-presenting synthetic surface and a cell more compatible and more similar to natural intercellular interactions and/or cellextracellular fluidic environment in-vivo. The linker can comprise, for example, a polymer. The polymer may include a polymer including alkylene ether moieties. A wide variety of alkylene ether containing polymers may be suitable for use on the surfaces described herein. One class of alkylene ether containing polymers is polyethylene glycol (PEG M_{w} <100,000Da), which are known in the art to be biocompatible. In some embodiments, a PEG may have an M_{w} of about 88Da, 100Da, 132Da, 176Da, 200Da, 220Da, 264Da, 308Da, 352Da, 396Da, 440Da, 500Da, 600Da, 700Da, 800Da, 900Da, 1000Da, 1500Da, 2000Da, 5000Da, 10,000Da or 20,000Da, or may have a M_{w} that falls within a range defined by any two of the foregoing values. In some embodiments, the PEG polymer has a polyethylene moiety repeat of about 3, 4, 5, 10, 15, 25 units, or any value therebetween. In some embodiments, the PEG is a carboxyl substituted PEG moiety. In some embodiments, the PEG is a hydroxyl substituted PEG moiety. In some embodiments, each of the plurality of surface-blocking molecular ligands may have a linker having the same length as the linkers of the other ligands of the plurality. In other embodiments, the linkers of the plurality of surface-blocking molecular ligands may have varied lengths. In some embodiments, the surface-blocking group and the length of the linker may be same for each of the plurality of surface-blocking molecular ligands. Alternatively, the surface blocking group and the length of the linker may vary within the plurality of the surface-blocking molecular ligands and may include 2, 3, or 4 different surface-blocking groups and/or 2, 3, 4, or more different lengths, chosen in any combination. In general, the surface-blocking molecular ligands have a length and/or structure that is sufficiently short so as not to sterically hinder the binding and/or function of the primary activating molecular ligands and the co-activating molecular ligands. For example, in some embodiments, the length of the surface-blocking molecular ligands is equal to or less than the length of the other linkers bound to the surface (e.g., linkers that connect coupling groups, primary activating molecular ligands, co-stimulating molecular ligands, or other ligands). In some embodiments, the length of the surface-blocking molecular ligands is about 1 or more angstroms (e.g., about 2, 3, 4, 5, 10, 15, 20, 25, 30, 35, 40, 45, 50, 75, 100, or more angstroms) less than the length of the other linkers bound to the surface (e.g., linkers that connect coupling groups, primary activating molecular ligands, co-stimulating molecular ligands, or other ligands). In some embodiments, the length of the surface-blocking molecular ligands is about 1 to about 100 angstroms (e.g., about 2 to about 75, about 3 to about 50, about 4 to about 40, or about 5 to about 30 angstroms) less than the length of the other linkers bound to the surface. When the surface-blocking molecular ligands have a length that is the same or somewhat less than the length of the other linkers bound to the surface, the resulting surface effectively presents the ligands attached to the other linkers in a manner that is readily available for coupling and/or interacting with cells. With respect to proto-antigen-presenting beads, including a surface-blocking molecular ligand such as a hydrophilic polymer, e.g., a PEG or PEO polymer and/or ligands comprising terminal hydroxyl or carboxyl groups, may beneficially reduce aggregation of the beads through hydrophobic interactions. The surface-blocking molecular ligands can be attached to the surface after the primary and other (e.g., coactivating, adjunct, etc.) ligands discussed above or may be introduced before any of the activating or coactivating species are attached to the surface, as set forth in any embodiments disclosed herein.

The proto-antigen-presenting synthetic surface may comprise glass, metal, a polymer, or a metal oxide. In some embodiments, the proto-antigen-presenting synthetic surface is a surface of a wafer having any kind of configuration, a surface of a bead, at least one inner surface of a fluidic circuit containing device (e.g., microfluidic device) configured to contain a plurality of cells, or an inner surface of a tube (e.g., glass or polymer tube). In some embodiments, the wafer having a proto-antigen-presenting synthetic surface configured to activate T lymphocytes may be sized to fit within a well of a standard 48, 96 or 384 wellplate. In various embodiments, beads having a proto-antigen-presenting synthetic surface configured to activate T lymphocytes may be disposed for use within a wellplate or within a fluidic circuit containing device. In some embodiments, the density of the plurality of primary activating molecular ligands on the proto-antigen-presenting synthetic surface (or in each portion or sub-region where it is attached) may be from about 50 to about 500 molecules per square micron; about 4X 10² to about 2X 10³ molecules per square micron; about 1x10³ to about 2X10⁴ molecules per square micron; about 5 x10³ to about 3x10⁴ molecules per square micron; about 4X 10² to about 3X 10⁴ molecules per square micron; about 4X 10² to about 2X 10³ molecules per square micron; about 2X 10³ to about 5X 10³ molecules per square micron; about 5X 10³ to about 2X 10⁴ molecules per square micron; about 1X 10⁴ to about 2X 10⁴ molecules per square micron; or about 1.25X 10⁴ to about 1.75X 10⁴ molecules per square micron.

In some embodiments, the density of the plurality of co-activating molecular ligands on the proto-antigen-presenting synthetic surface (or in each portion or sub-region where it is attached) is from about 20 to about 250 molecules per square micron; about 2X10² to about 1X 10³ molecules per square micron; about 500 to about 5x10³ molecules per square micron; about 1x10³ to about 1x10⁴ molecules per square micron; about 5X 10² to about 2X 10⁴ molecules per square micron; about 5X 10² to about 1.5 X 10⁴ molecules per square micron; about 5X 10³ to about 2X 10⁴ molecules per square micron, about 5X 10³ to about 1.5X 10⁴ molecules per square micron, about 1X 10⁴ to about 2X 10⁴ per square micron, about 1X 10⁴ to about 1.5X 10⁴ per square micron, about 1.25X 10⁴ to about 1.75X 10⁴, or about 1.25X 10⁴ to about 1.5X 10⁴ per square micron.

Without wishing to be bound by any particular theory, certain experiments have indicated that it may be advantageous to provide and use beads for T cell activation that have relatively defined surface-area to volume ratios. Such beads may present the relevant ligands in a more accessible way so that they interact more efficiently with T cells during activation. Such beads may provide a desired degree of T cell activation with fewer ligands needed than beads with higher surface-area to volume ratios and/or may provide a higher degree of T cell activation or more T cells with desired features (e.g., antigen specificity and/or marker phenotypes described herein) than beads with higher surface-area to volume ratios. An ideally spherical solid has the lowest possible surface-area to volume ratio. Accordingly, in some embodiments, the bead surface-area is within 10% of the surface-area of a sphere of an equal size (volume or diameter), and is referred to herein as "substantially spherical." For example, for a bead with a 2.8 µm diameter (1.4 µm radius), the corresponding ideal sphere would have a surface area of 4πr²=24.63 µm². A substantially spherical 2.8 µm diameter bead with a surface-area within 10% of the surface-area of an ideal sphere of an equal volume or diameterwould therefore have a surface-area less than or equal to 27.093 µm². It is noted that certain commercially available beads are reported as having higher surface areas; for example, Dynabeads M-270 Epoxy are described in their product literature as having a specific surface area of 2-5 m²/g and a 2.8 µm diameter, and the literature also indicates that 1 mg of beads is 6-7 x 10⁷ beads. Multiplying the specific surface area by 1 mg/6-7 x 10⁷ beads gives a surface area per bead of 28 to 83 µm² per bead, which is more than 10% greater than the surface-area of an ideal sphere with a 2.8 µm diameter. Polymer beads having a surface area more than 10% greater than the surface-area of an ideal sphere are referred to herein as a "convoluted bead." In some embodiments, a polymer bead may be either substantially spherical or convoluted. In some embodiments, the polymer bead is not convoluted, but is substantially spherical.

**Unpatterned surface.** In various embodiments, the proto-antigen-presenting synthetic surface may be a unpatterned surface having a plurality of primary activating molecular ligands distributed evenly thereon. The primary activating molecular ligands can comprise MHC molecules, each of which may include a tumor associated antigen. The unpatterned surface may further include a plurality of co-activating molecular ligands (e.g., TCR co-activating molecules and/or adjunct TCR activating molecules) distributed evenly thereon . The co-activating molecular ligands may be as described above for proto-antigen-presenting surfaces, in any combination. The density of the primary activating molecular ligands and the co-activating molecular ligands may the same ranges as described above for proto-antigen-presenting surfaces. The unpatterned proto-antigen-presenting synthetic surface may further include additional growth stimulatory, adhesive, and/or surface-blocking molecular ligands, as described above for proto-antigen-presenting surfaces, each of which (if present) can be evenly distributed on the upatterned surface. For example, the unpatterned surface can include an adjunct stimulatory molecule such as IL-21 connected to the surface. The primary activating molecular ligands, co-activating molecular ligands, and/or additional ligands may be linked to the surface as described above for the proto-antigen-presenting surfaces. As used herein, a surface having a ligand "distributed evenly" thereon is characterized in that no portion of the surface having a size of 10% the total surface area, or greater, has a statistically significant higher concentration of ligand as compared to the average ligand concentration of the total surface area of the surface.

**Patterned surface.** In various embodiments, the proto-antigen-presenting synthetic surface may be patterned and may have a plurality of regions, each region including a plurality of the primary activating molecular ligands comprising MHC molecules, where the plurality of regions is separated by a region configured to substantially exclude the primary activating molecular ligands. The proto-antigen-presenting synthetic surface may be a planar surface. In some embodiments, each of the plurality of regions including the at least a plurality of the primary activating molecular ligands may further include a plurality of the co-activating molecular ligands, e.g., a TCR co-activating molecule and/or an adjunct TCR activating molecule. The coactivating molecular ligands may be any of the co-activating molecular ligands as described above and in any combination. The primary activating molecular ligands and/or co-activating molecular ligands may be linked to the surface as described above for the proto-antigen-presenting surfaces. The density of the primary activating molecular ligands and/or the co-activating molecular ligands in each of the regions containing the primary activating molecular ligands and/or the co-activating molecular ligands may be in the same range as the densities described above for proto-antigen-presenting surfaces. In some embodiments, each of the plurality of regions comprising at least the plurality of the primary activating molecular ligands has an area of about 0.10 square microns to about 4.0 square microns. In other embodiments, the area of each of the plurality of regions may be about 0.20 square microns to about 0.8 square microns. The plurality of regions may be separated from each other by about 2 microns, about 3 microns, about 4 microns, or about 5 microns. The pitch between each region of the plurality and its neighbor may be about 2 microns, about 3 microns, about 4 microns, about 5 microns, or about 6 microns. SeeFIGS. 7A and 7B showing two embodiments of a patterned surface.

In various embodiments, the region configured to substantially exclude the primary activating molecular ligands comprising MHC molecules may also be configured to substantially exclude TCR coactivating molecules and/or adjunct TCR activating molecules.

In some embodiments, the region configured to substantially exclude the primary activating molecular ligands and optionally the TCR co-activating molecules and/or adjunct TCR activating molecules may also be configured to include one or more of surface-blocking molecular ligands, growth stimulatory molecules, additional stimulatory molecules, and adhesion stimulatory molecular ligands. In some embodiments, the growth stimulatory molecules and/or additional stimulatory molecules include a cytokine or fragment thereof, and may further include IL-21 or fragment thereof. In some embodiments, the region configured to substantially exclude the primary activating molecular ligands and optionally the TCR coactivating molecules and/or adjunct TCR activating molecules may further be configured to include one or more supportive moieties. The supportive moieties may provide adhesive motifs to support T lymphocyte growth or may provide hydrophilic moieties providing a generally supportive environment for cell growth. The moiety providing adhesive support may include a peptide sequence including a RGD motif. In other embodiments, the moiety providing adhesive support may be an ICAM sequence. A moiety providing hydrophilicity may be a moiety such as a PEG moiety or carboxylic acid substituted PEG moiety.

**Microfluidic device.** In some embodiments, a microfluidic device comprises a patterned proto-antigen-presenting synthetic surface having a plurality of regions according to any of the foregoing embodiments. While the proto-antigen-presenting surface of microfluidic device may be any microfluidic (or nanofluidic) device as described herein, the disclosure is not so limited. Other classes of microfluidic devices, including but not limited to microfluidic devices including microwells or microchambers such as described in WO2014/153651, WO2016/115337, or WO2017/124101, may be modified to either incorporate an antigen presenting surface as described in this section, or may be used in combination with the proto-antigen-presenting beads or proto-antigen-presenting wafers as described herein in the methods described in this disclosure.

In some embodiments, the proto-antigen-presenting synthetic surface is an inner surface of a microfluidic device comprising one or more sequestration pens and a channel. At least part of a surface within one or more such sequestration pens may comprise a plurality of primary activating molecular ligands and a plurality of co-activating molecular ligands, e.g., comprising TCR co-activating molecules and/or adjunct TCR activating molecules. The primary activating molecular ligands and the co-activating molecular ligands may be any described above for proto-antigen-presenting surfaces, and may be present in any concentration or combination as described above. The nature of the ligands attachment to the surface of the microfluidic device may be any described above as for proto-antigen-presenting surfaces. In some embodiments, this surface within the one or more such sequestration pens can further comprise one or more of surface-blocking molecular ligands, growth stimulatory molecular ligands, additional stimulatory molecular ligands, and adhesion stimulatory molecular ligands. At least part of a surface of the channel may comprise surface-blocking molecular ligands, e.g., any of the regions configured to substantially exclude the primary activating molecular ligands described herein. In some embodiments, the surface of the channel comprises surface-blocking molecular ligands and optionally other non-stimulatory ligands, but is substantially free of other ligands present on the surface of the sequestration pen, e.g., primary activating molecular ligands and coactivating molecular ligands.

**Modulation of cell-to-surface adhesion.** In some embodiments, it can be useful to modulate the capacity for cells to adhere to surfaces within the microfluidic device. A surface that has substantially hydrophilic character may not provide anchoring points for cells requiring mechanical stress of adherence to grow and expand appropriately. A surface that presents an excess of such anchoring moieties may prevent successfully growing adherent cells from being exported from within a sequestration pen and out of the microfluidic device. In some embodiments, a covalently bound surface modification comprises surface contact moieties to help anchor adherent cells. The structures of the surfaces described herein and the methods of preparing them provide the ability to select the amount of anchoring moieties that may be desirable for a particular use. A very small percentage of adherent type motifs may be needed to provide a sufficiently adhesion enhancing environment. In some embodiments, the adhesion enhancing moieties are prepared before cells are introduced to the microfluidic device. Alternatively, an adhesion enhancing modified surface may be provided before introducing cells, and a further addition of another adhesion enhancing moiety may be made, which is designed to attach to the first modified surface either covalently or non-covalently (e.g., as in the base of biotin/streptavidin binding).

In some embodiments, adhesion enhancing surface modifications may modify the surface in a random pattern of individual molecules of a surface modifying ligand. In some other embodiments, a more concentrated pattern of adhesion enhancing surface modifications may be introduced by using polymers containing multiple adhesion enhancing motifs such as positively charged lysine side chains, which can create small regions of surface modification surrounded by the remainder of the surface, which may have hydrophilic surface modifications to modulate the adhesion enhancement. This may be further elaborated by use of dendritic polymers, having multiple adhesion enhancing ligands. A dendritic polymer type surface modifying compound or reagent may be present in a very small proportion relative to a second surface modification having only hydrophilic surface contact moieties, while still providing adhesion enhancement. Further a dendritic polymer type surface modifying compound or reagent may itself have a mixed set of end functionalities which can additionally modulate the behavior of the overall surface.

In some embodiments, it may be desirable to provide regioselective introduction of surfaces. For example, in the context of a microfluidic device comprising a microfluidic channel and sequestration pens, it may be desirable to provide a first type of surface within the microfluidic channel while providing a surface within the sequestration pens opening off of the channel that provides the ability to both culture adherent-type cells successfully as well as easily export them (e.g., using dielectrophoretic or other forces) when desired. In some embodiments, the adhesion enhancing modifications may include cleavable moieties. The cleavable moieties may be cleavable under conditions compatible with the cells being cultured within, such that at any desired timepoint, the cleavable moiety may be cleaved and the nature of the surface may alter to be less enhancing for adhesion. The underlying cleaved surface may be usefully non-fouling such that export is enhanced at that time. While the examples discussed herein focus on modulating adhesion and motility, the use of these regioselectively modified surfaces are not so limited. Different surface modifications for any kind of benefit for cells being cultured therein may be incorporated into the surface having a first and a second surface modification according to the disclosure.

Exemplary adherent motifs that may be used include poly-L-lysine, amine and the like, and the tripeptide sequence RGD, which is available as a biotinylated reagent and is easily adaptable to the methods described herein. Other larger biomolecules that may be used include fibronectin, laminin or collagen, amongst others. A surface modification having a structure of Formula XXVI as defined in WO2017/205830, including a polyglutamic acid surface contact moiety, can induce adherent cells to attach and grow viably. Another motif that may assist in providing an adherent site is an Elastin Like Peptide (ELP), which includes a repeat sequence of VPGXG, where X is a variable amino acid which can modulate the effects of the motif.

In some embodiments, in the context of a microfluidic device comprising a microfluidic channel and sequestration pens, a surface of the flow region (e.g., microfluidic channel) may be modified with a first covalently bound surface modification and a surface of the at least one sequestration pen may be modified with a second covalently bound surface modification, wherein the first and the second covalently bound surface modification have different surface contact moieties, different reactive moieties, or a combination thereof. The first and the second covalently bound surface modifications may be selected from any of Formula XXX, Formula V, Formula VII, Formula XXXI, Formula VIII, and/or Formula IX, all of which are as defined in WO2017/205830. When the first and the second covalently bound surface modifications both include functionalized surface of Formula XXX, Formula V, or Formula VII as defined in WO2017/205830, then orthogonal reaction chemistries are selected for the choice of the first reactive moiety and the second reactive moiety. In various embodiments, all the surfaces of the flow region may be modified with the first covalent surface modification and all the surfaces of the at least one sequestration pen may be modified with the second covalent modification.

The proto-antigen-presenting surfaces described herein can be used to prepare an antigen-presenting surface that presents a peptide antigen, e.g., by reacting the peptide antigen with the proto-antigen-presenting surface, wherein the exchange factor or initial peptide is substantially displaced and the peptide antigen becomes associated with the MHC molecules.

### Exchange Factors.

Exchange factors are provided in various kits and surfaces described herein and are used in various methods and uses described herein. The following description is provided with respect to all disclosed embodiments herein involving exchange factors.

An exchange factor is a compound of the general formula A-B, wherein A comprises one or more amino acid residues and B comprises a C-terminal amino acid residue, wherein the side chain of the C-terminal amino acid residue comprises at least three non-hydrogen atoms (e.g., carbon, nitrogen, oxygen, and/or sulfur). In some embodiments, A and B are linked by a peptide bond. In some embodiments, A and B are linked through an alternative linkage, such as a lactam or piperazinone. In some embodiments, one or more amino acid residues of the exchange factor are nonstandard amino acid residues (i.e., different from the 20 canonical amino acid residues that are specified by the standard genetic code). Exemplary nonstandard amino acid residues include norleucine, homoleucine, and cyclohexylalanine (in which a proton of the methyl side chain of alanine is substituted with a cyclohexyl). In some embodiments, the penultimate residue from the C-terminus of the exchange factor (e.g., the N-terminal residue of a dipeptide) has a side chain comprising 0, 1, or 2 non-hydrogen atoms (e.g., G, A, S, or C). The penultimate residue from the C-terminus is the residue immediately adjacent to the C-terminal residue. In some embodiments, the N-terminal residue of the exchange factor (e.g., the N-terminal residue of a dipeptide) has a free N-terminal amine. In some embodiments, the C-terminal residue of the exchange factor is Leu, Phe, Ile, Val, Arg, or Met. In some embodiments, the C-terminal residue of the exchange factor is homoleucine, norleucine, or cyclohexylalanine. In some embodiments, the penultimate residue from the C-terminus of the exchange factor is Gly. In some embodiments, the penultimate residue from the C-terminus of the exchange factor is Ala. In some embodiments, the exchange factor is a dipeptide, such as GL, GF, GV, GR, GM, G(homoleucine), G(cyclohexylalanine), G(Norleucine), GK, GI, AL, AF, AV, AR, AM, A(homoleucine), A(cyclohexylalanine), A(Norleucine), AK, or Al. The A or G in any of the foregoing may alternatively be substituted with S or C.

See Saini et al., Proc Nat'l Acad Sci USA (2013) 110, 15383-88, and Saini et al., Proc Nat'l Acad Sci USA (2015) 112, 202-07, for discussion of exemplary exchange factors and their use to displace an initial peptide from an MHC and then undergo displacement by a subsequent peptide.

### Major Histocompatibility Complexes (MHC).

The following description is provided with respect to any embodiment (e.g., surface, kit, use, or method) described herein involving an MHC. In some embodiments, the MHC molecule is an MHC Class I molecule. In some embodiments, the MHC molecule is an MHC Class II molecule.

Many different MHC Class I alleles are known and have been sequenced. MHC Class I sequences for the HLA-A, HLA-B, and HLA-C heavy chains are available, e.g., through the hla.alleles.org website (see hla.alleles.org/data/hla-a.html, hla.alleles.org/data/hla-b.html, and hla.alleles.org/data/hla-c.html for links to HLA nucleotide and amino acid sequences). In some embodiments, the MHC comprises an HLA-A. In some embodiments, the MHC comprises an HLA-B. In some embodiments, the MHC comprises an HLA-C.

In some embodiments, the HLA-A is an HLA-A*01, HLA-A*02, HLA-A*03, HLA-A*11, HLA-A*23, HLA-A*24, HLA-A*25, HLA-A*26, HLA-A*29, HLA-A*30, HLA-A*31, HLA-A*32, HLA-A*33, HLA-A*34, HLA-A*43, HLA-A*66, HLA-A*68, HLA-A*69, HLA-A*74, or HLA-A*80.

In some embodiments, the HLA-B is an HLA-B*07, HLA-B*08, HLA-B*13, HLA-B*14, HLA-B*15, HLA-B*18, HLA-B*27, HLA-B*35, HLA-B*37, HLA-B*38, HLA-B*39, HLA-B*40, HLA-B*41, HLA-B*42, HLA-B*44, HLA-B*45, HLA-B*46, HLA-B*47, HLA-B*48, HLA-B*49, HLA-B*50, HLA-B*51, HLA-B*52, HLA-B*53, HLA-B*54, HLA-B*55, HLA-B*56, HLA-B*57, HLA-B*58, HLA-B*59, HLA-B*67, HLA-B*73, HLA-B*78, HLA-B*81, HLA-B*82, or HLA-B*83.

In some embodiments, the HLA-C is an HLA-C*01, HLA-C*02, HLA-C*03, HLA-C*04, HLA-C*05, HLA-C*06, HLA-C*07, HLA-C*08, HLA-C*12, HLA-C*14, HLA-C*15, HLA-C*16, HLA-C*17, or HLA-C*18.

In some embodiments, an initial peptide is bound to an MHC molecule, e.g., in a kit described herein or during or at the beginning of a method described herein (e.g., before an exchange reaction). The initial peptide may be any of the initial peptides described herein.

### Initial Peptides.

An initial peptide is provided in various kits and surfaces described herein and is used in various methods and uses described herein. The following description is provided with respect to all disclosed embodiments herein involving initial peptides.

In some embodiments, the initial peptide comprises at least 4 or 5 amino acid residues. In some embodiments, the initial peptide has a length of about 4, 5, 6, 7, 8, 9, 10, 11, 12, 13, 14, 15, 16, 17, or 18 amino acid residues. In some embodiments, the initial peptide has a length that ranges from 8 to 10 amino acid residues or 13 to 15 amino acid residues.

In some embodiments, the initial peptide comprises a lysine as the fourth or fifth amino acid residue (counting from the N-terminus, i.e., wherein the N-terminal residue is the first residue). In some embodiments, the initial peptide comprises a label. In some embodiments, the fourth or fifth amino acid residue (e.g., lysine) is labeled. In some embodiments, the label is a fluorescent label. In some embodiments, the label is radioactive. In some embodiments, the label is a chemical moiety (e.g., dinitrophenyl (DNP)) which can be specifically bound by an detection agent (e.g., a labeled antibody) when the initial peptide is bound to MHC. Where an initial peptide is labeled, it can facilitate monitoring of the progress of an exchange reaction to exchange an initial peptide initially bound to an MHC molecule for a peptide antigen in the presence of an exchange factor, wherein the extent of exchange of the initial peptide for the peptide antigen can be determined by detecting the extent to which the label is associated with the MHC molecule.

In some embodiments, the initial peptide comprises a sequence from a naturally occurring (e.g., mammalian or human) polypeptide. In some embodiments, the sequence of the initial peptide consists of sequence from a naturally occurring (e.g., mammalian or human) polypeptide (e.g., a sequence that appears in a wild-type (e.g., mammalian or human) polypeptide). In some embodiments, the initial peptide is non-immunogenic in the organism from which it originated, e.g., in a mammal or in humans. In some embodiments, the sequence of the initial peptide comprises or consists of sequence from a highly conserved protein (e.g., a protein with a below average mutation rate; in some embodiments the mutation rate is at least one or two standard deviations below the average amino acid mutation rate in the organism). In some embodiments, the sequence of the initial peptide comprises or consists of sequence from a cytoskeletal polypeptide, e.g., an actin or tubulin polypeptide. In some embodiments, the sequence of the initial peptide comprises or consists of sequence from a ribosomal polypeptide, e.g., the RPSA, RPS2, RPL3, RPL4, RPL5, RPL6, RPL7A, or RPP0 polypeptides. Ribosomal and cytoskeletal polypeptides are examples of highly conserved polypeptides, which should be non-immunogenic because of tolerization. It can be beneficial to use such polypeptides because, in the event that a residual amount of the initial polypeptide remains bound to the MHC molecule following an exchange reaction, the MHC molecules comprising the initial polypeptide will not result in stimulation of antigen-specific T cells because T cells specific for tolerized polypeptides generally do not exist.

Exemplary initial peptide sequences are shown in Table 1 below.

| **Protein Source** | **UniProt ID** | **Peptide Position** | **Sequence** | **SEQ ID NO** |
|---|---|---|---|---|
| **ACTB** | P60709 | 46-54 | GMGQKDSYV | 1 |
| **ACTB** | P60709 | 312-320 | RMQKEITAL | 2 |
| **MYH9** | P35579 | 653-661 | QLAKLMATL | 3 |
| **TBA1A** | Q71U36 | 397-405 | LMYAKRAFV | 4 |

An initial peptide may be used that binds the MHC molecule with high affinity and/or a low off-rate or long half-life. In some embodiments, the binding of the initial peptide to the MHC molecule has a half-life of at least about 4, 6, 8, 10, 12, 14, 16, 18, 20, 24, 28, 32, 36, or 48 hours. In some embodiments, the binding of the initial peptide to the MHC molecule has a half-life in the range of about 4-12, 8-16, 12-20, 20-28, 24-32, 28-36, 32-40, 36-48, or 48-72 hours. Half-lives may be determined in the absence of an exchange factor and/or using the conditions described for the stability assay of Example 27. It can be beneficial to the stability of the MHC to be bound to an initial peptide with high affinity and/or a low off-rate or long half-life. For example, a MHC molecule including a beta macroglobulin (e.g., a beta-2-microglobulin) may lose the beta microglobulin subunit if the initial peptide dissociates prior to an exchange reaction, which may adversely impact the function of the MHC molecule. The high affinity and/or a low off-rate or long half-life does not pose a substantial obstacle to the exchange reaction because the exchange reaction can be driven by stoichiometry (i.e., an excess of the exchange factor and peptide antigen).

### Peptide Antigens.

Peptide antigens are provided in various kits and surfaces described herein and are used in various methods and uses described herein. The following description is provided with respect to all disclosed embodiments herein involving peptide antigens. As noted herein, peptide antigens include candidate peptide antigens that may or may not be immunogenic when presented by an MHC, in addition to peptide antigens with known or verifiable immunogenicity, where immunogenicity refers to the ability of a peptide antigen to contribute to activation of a T lymphocyte, such as a cytotoxic T lymphocyte, when the peptide antigen is bound in the antigen-binding pocket of a major histocompatibility complex (MHC), e.g., a class I MHC.

In some embodiments, a peptide antigen is 7-11 amino acid residues in length, e.g., 7, 8, 9, 10, or 11 amino acid residues in length. In some embodiments, a peptide antigen is 8, 9, or 10 amino acid residues in length. In some embodiments, a peptide antigen comprises a tumor associated antigen. Some non-limiting examples of tumor associated antigens include MART1 (peptide sequence ELAGIGILTV (SEQ ID NO: 7)), for melanoma, NYESO1 (peptide sequence SLLMWITQV (SEQ ID NO: 8)), involved in melanoma and some carcinomas, SLC45A2, TCL1, and VCX3A, but the disclosure is not so limited. Additional examples of tumor associated antigens include peptides comprising a segment of amino acid sequence from a protein expressed on the surface of a tumor cell such as CD19, CD20, CLL-1, TRP-2, LAGE-1, HER2, EphA2, FOLR1, MAGE-A1, mesothelin, SOX2, PSM, CA125, T antigen, etc. The peptide can be from an extracellular domain of the tumor associated antigen. An antigen is considered tumor associated if it is expressed at a higher level on a tumor cell than on a healthy cell of the type from which the tumor cell was derived. The T cell which recognizes this tumor associated antigen is an antigen specific T cell. Any tumor associated antigen may be utilized in the antigen presenting surface described herein. In some embodiments, the tumor associated antigen is a neoantigenic peptide, e.g., encoded by a mutant gene in a tumor cell. For detailed discussion of neoantigenic peptides, see, e.g., US 2011/0293637.

Upon reaction with a proto-antigen-presenting surface, the peptide antigen (e.g., tumor associated antigen) may become noncovalently associated with the primary activating molecular ligand (e.g., MHC molecule), e.g., through binding in the antigen-binding pocket of the MHC molecule. Such binding may involve displacement of the previous occupant of the pocket (an exchange factor, or an initial peptide whose displacement is catalyzed by an exchange factor). The peptide antigen may be presented by the primary activating molecular ligand (e.g., MHC molecule) in an orientation which can initiate activation of a T lymphocyte.

In some embodiments, a population of peptide antigens is provided, e.g., in one or more pools. For example, such a population can be prepared from material from a tumor sample, and may be enriched for tumor associated antigens and/or neoantigenic peptides. The one or more pools can be used together with one or more proto-antigen-presenting surfaces described herein to generate a population of antigen-presenting surfaces, e.g., for use in screening the members of the population of peptide antigens for immunogenicity.

### Methods of forming a proto-antigen-presenting synthetic surface.

A method of forming a proto-antigen-presenting synthetic surface for activating a T lymphocyte (T cell), is provided, comprising: synthesizing a plurality of major histocompatibility complex (MHC) molecules in the presence of initial peptide, thereby forming a plurality of complexes each comprising an MHC molecule and an initial peptide; or synthesizing a plurality of major histocompatibility complex (MHC) molecules in the presence of exchange factor, thereby forming a plurality of complexes each comprising an MHC molecule and an exchange factor; or reacting a plurality of MHC molecules with exchange factor, thereby forming a plurality of complexes each comprising an MHC molecule and an exchange factor; wherein:
a plurality of primary activating molecules comprise the MHC molecules and first reactive moieties, and the method further comprises reacting the first reactive moieties of the plurality of primary activating molecules with a first plurality of binding moieties disposed on a covalently functionalized synthetic surface, thereby forming the proto-antigen-presenting surface.

In some embodiments, the method further comprises reacting the plurality of MHC molecules synthesized in the presence of the initial peptide with exchange factor, optionally in the presence of a peptide antigen.

In some embodiments, before reacting a plurality of MHC molecules with the exchange factor, an initial peptide is bound to the MHC molecule. The initial peptide may be any of the embodiments of initial peptide described elsewhere herein.

In some embodiments, a plurality of co-activating molecular ligands, each including a TCR co-activating molecule or an adjunct TCR activating molecule, are present on the covalently functionalized synthetic surface or are added to the covalently functionalized synthetic surface by reacting a plurality of co-activating molecules, each including second reactive moiety and a TCR co-activating molecule or an adjunct TCR activating molecule, with a second plurality of binding moieties of the covalently functionalized synthetic surface configured for binding the second reactive moieties.

In some embodiments, the covalently functionalized synthetic surface presents a plurality of azido groups. In such embodiments, the first reactive moieties can be configured to react with the azido groups of the covalently functionalized synthetic surface so as to form covalent bonds. Where present, the second reactive moieties can also be configured to react with the azido groups of the covalently functionalized synthetic surface so as to form covalent bonds.

In some embodiments, the covalently functionalized synthetic surface presents a plurality of biotin-binding agents, and wherein the first reactive moieties are configured to specifically bind to the biotin-binding agent. In some such embodiments, the first reactive moieties comprise or consist essentially of biotin. Where present, the second reactive moieties can also comprise or consist essentially of biotin. The biotin-binding agent may be covalently attached to the covalently functionalized synthetic surface or noncovalently attached to the covalently functionalized synthetic surface, e.g., through biotin functionalities.

The covalently functionalized synthetic surface used to prepare a proto-antigen-presenting surface may be any of the surface types described herein, e.g., a bead, wafer, inner surface of a microfluidic device, or tube (e.g., glass or polymer tube). The surface material may comprise, e.g., metal, glass, ceramic, polymer, or a metal oxide. The microfluidic device may be any microfluidic device as described herein, and may have any combination of features. The bead can be a bead with a surface-area that is within 10% of the surface-area of a sphere of an equal volume or diameter, as discussed herein in the section regarding proto-antigen-presenting synthetic surfaces. In some embodiments, the bead may be a bead having a surface area that exceeds 10% of the surface area of a sphere of an equal volume or diameter, as discussed herein for antigen presenting surfaces. In some embodiments, the bead is not a bead that has a surface area that exceeds 10% of the surface area of a sphere of an equal volume or diameter, as discussed herein for antigen presenting surfaces.

The primary activating molecules and co-activating molecules may each be any such molecule described herein, and any combination thereof may be used. Thus, a primary activating molecule can comprise an MHC molecule and, optionally, an initial peptide or exchange factor; and a co-activating molecule can comprise any of the TCR co-activating molecules described herein or any of the adjunt TCR activating molecules described herein. As noted above, the MHC molecules may be synthesized in the presence of the exchange factor, e.g., so that they bind the exchange factor in the antigen-binding pocket upon folding. Alternatively, the MHC molecules may be reacted with the exchange factor after synthesis. This approach can displace an initially bound peptide from the antigen-binding pocket.

Where the MHC molecules are synthesized in the presence of the exchange factor, they are subsequently incorporated into primary activating molecules through a process comprising adding a first reactive moiety (e.g., biotin or moieties configured to react with azido groups), as discussed in detail elsewhere herein. Such a method further comprises reacting the first reactive moieties of the plurality of primary activating molecules with a first plurality of binding moieties disposed on a covalently functionalized synthetic surface, thereby forming the proto-antigen-presenting surface.

Where the MHC molecules are reacted with the exchange factor after synthesis, such a reaction may occur before or after being incorporated into primary activating molecules through a process comprising adding a first reactive moiety (e.g., biotin), as discussed in detail elsewhere herein. Such a reaction may also occur before or after reacting the first reactive moieties of the plurality of primary activating molecules with a first plurality of binding moieties disposed on a covalently functionalized synthetic surface. That is, the exchange factor can be reacted with the MHC molecules in solution or when they are already associated with a surface.

In some embodiments, reacting a plurality of primary activating molecules with a first plurality of binding moieties of a covalently functionalized synthetic surface comprising binding moieties comprises forming a noncovalent association between the primary activating molecules and the binding moieties. For example, the primary activating molecules can comprise biotin and the binding moieties can comprise a biotin-binding agent such as streptavidin (e.g., which may be covalently bound to the surface or which may be non-covalently bound to a second biotin which itself is covalently bound to the surface). In some embodiments, the biotin-binding agent such as streptavidin is linked to the surface through a series of about 15, 20, 25, 30, 35, 40, 45, 50, 55, 60, 65, 70, 75, 80, 85, 95, 100, 200 bond lengths, or any number of bond lengths therebetwteen. For example, the biotin-binding agent may be linked to the surface through a series of one or more linkers having a selected length as described. In another example, both the binding moieties and the primary activating molecules can comprise biotin and a free, multivalent biotin-binding agent, such as streptavidin, can be used as a noncovalent linking agent. Any other suitable noncovalent binding pair, such as those described elsewhere herein, can also be used.

Alternatively, reacting a plurality of primary activating molecules with a first plurality of binding moieties of a covalently functionalized synthetic surface comprising binding moieties can comprise forming a covalent bond. For example, an azide-alkyne reaction (such as any of those described elsewhere herein) can be used to form the covalent bond, where the primary activating molecules and the binding moieties comprise, respectively, an azide and an alkyne, or an alkyne and an azide. Other reaction pairs may be used, as is known in the art, including but not limited to maleimide and sulfides. More generally, exemplary functionalities useful for forming covalent bonds include azide, carboxylic acid and active esters thereof, succiniimide ester, maleimide, keto, sulfonyl halides, sulfonic acid, dibenzocyclooctyne, alkene, alkyne, and the like. Skilled artisans are familiar with appropriate combinations and reaction conditions for forming covalent bonds using such moieties.

Where the covalently functionalized synthetic surface comprises a covalently associated biotin, the surface can further comprise noncovalently associated biotin-binding agent (e.g., streptavidin), such that the surface can be reacted with primary activating molecules and co-activating molecules that comprise biotin moieties. In some embodiments, the method of preparing a proto-antigen-presenting synthetic surface comprises reacting a covalently functionalized synthetic surface comprising a covalently associated biotin with a biotin-binding agent (e.g., streptavidin), and then with primary activating molecules and co-activating molecules comprising biotin moieties. In some embodiments, the biotin of the covalently functionalized surface is linked to the surface through a series of about 15, 20, 25, 30, 35, 40, 45, 50, 55, 60, 65, 70, 75, 80, 85, 95, 100, 200 bond lengths, or any number of bond lengths therebetween..

In some embodiments, the reaction provides any of the densities described herein of primary activating molecular ligands on the surface, such as about 4X 10² to about 3X 10⁴, 4X 10² to about 2X 10³, about 5X 10³ to about 3X 10⁴, about 5X 10³ to about 2X 10⁴, or about 1X 10⁴ to about 2X 10⁴ molecules per square micron.

In some embodiments, reacting a plurality of co-activating molecules, each comprising: a T cell receptor (TCR) co-activating molecule; or an adjunct TCR activating molecule, with a second plurality of binding moieties of the covalently functionalized synthetic surface comprises forming a noncovalent association between the co-activating molecules and the binding moieties. Any of the embodiments described above or set forth in any embodiments disclosed herein with respect to primary activating molecules involving noncovalent binding pairs such as biotin and a biotin-binding agent such as streptavidin may be used.

Alternatively, reacting a plurality of co-activating molecules with a second plurality of binding moieties of the covalently functionalized synthetic surface can comprise forming a covalent bond. For example, an azide-alkyne reaction (such as any of those described elsewhere herein) can be used to form the covalent bond, where the primary activating molecules and the binding moieties comprise, respectively, an azide and an alkyne, or an alkyne and an azide.

In some embodiments, the reaction provides any of the densities described herein of co-activating molecular ligands on the surface, such as from about 4X 10² to about 3X 10⁴, 4X 10² to about 2X 10³, about 5X 10³ to about 3X 10⁴, about 5X 10³ to about 2X 10⁴, or about 1X 10⁴ to about 2X 10⁴ molecules per square micron.

In some embodiments, the reaction provides TCR co-activating molecules and adjunct TCR activating molecules on the surface in any of the ratios described herein, such as 100:1 to 1:100, 10:1 to 1:20, 5:1 to 1:5, or 3:1 to 1:3, wherein each of the foregoing values can be modified by "about."

In some embodiments, the reactions described above or set forth in any embodiments disclosed herein provide primary activating molecular ligands and co-activating molecular ligands on the surface in any of the ratios described herein, such as about 1:1 to about 2:1; about 1:1; or about 3:1 to about 1:3.

In some embodiments, a method of preparing a proto-antigen-presenting surface further comprises reacting a plurality of surface-blocking molecules with a third plurality of binding moieties of the covalently functionalized surface, wherein each of the binding moieties of the third plurality is configured for binding the surface-blocking molecule. Any surface-blocking molecule described elsewhere herein may be used. Any of the reaction approaches described herein for forming noncovalent associations or a covalent bond may be used.

In some embodiments, a method of preparing a proto-antigen-presenting surface further comprises reacting a plurality of adhesion stimulatory molecular ligands, wherein each adhesion stimulatory molecular ligand includes a ligand for a cell adhesion receptor including an ICAM protein sequence, with a fourth plurality of binding moieties of the covalently functionalized bead, wherein each of the binding moieties of the fourth plurality is configured for binding with the cell adhesion receptor ligand molecule. Any of the reaction approaches described herein for forming noncovalent associations or a covalent bond may be used.

In some embodiments, a method of preparing a proto-antigen-presenting surface further comprises producing the intermediate reactive surface. This can include, e.g., reacting at least a first portion of surface-exposed moieties disposed at a surface of a synthetic reactive surface with a plurality of intermediate preparation molecules including reactive moieties, thereby producing the intermediate reactive surface. Methods of preparing a covalently functionalized surface, which can be used as the intermediate reactive surface, are described in detail elsewhere herein. Producing the intermediate reactive surface can comprise any of the features described herein with respect to methods of preparing a covalently functionalized surface.

In some embodiments, the methods further comprise modulating the capacity for cells to adhere to surfaces within the microfluidic device, e.g., by providing anchoring points for cells requiring mechanical stress of adherence to grow and expand appropriately. This can be accomplished by introducing a covalently bound surface modification comprising surface contact moieties to help anchor adherent cells. Any of the surface contact moieties described elsewhere herein can be used.

The covalently functionalized synthetic surface can comprise moieties suitable for use in any of the reactions described herein.

### Methods of preparing a covalently functionalized surface.

In some embodiments, preparation of a proto-antigen-presenting surface from a covalently functionalized surface further comprises preparing a covalently functionalized surface including a plurality of streptavidin or biotin functionalities and at least a first plurality of surface-blocking molecular ligands. In some embodiments, preparing the covalently functionalized surface comprises reacting at least a first subset of reactive moieties of an intermediate reactive synthetic surface with a plurality of linking reagents, each linking reagent including streptavidin or biotin; and reacting at least a second subset of reactive moieties of the intermediate reactive synthetic surface with a plurality of surface-blocking molecules, thereby providing the covalently functionalized synthetic surface including at the least one plurality of streptavidin or biotin functionalities and at the least first plurality of surface-blocking molecular ligands. Generally only one or the other of a linking reagent including streptavidin or a linking reagent including biotin is used. The intermediate reactive synthetic surface may be any of the surface types described herein, e.g., a bead, wafer, inner surface of a microfluidic device, or tube (e.g., glass or polymer tube). The surface material may comprise, e.g., metal, glass, ceramic, polymer, or a metal oxide. The microfluidic device may be any microfluidic device as described herein, and may have any combination of features. The bead can be a bead with a surface-area is within 10% of the surface-area of a sphere of an equal volume or diameter, as discussed herein in the section regarding proto-antigen-presenting synthetic surfaces.

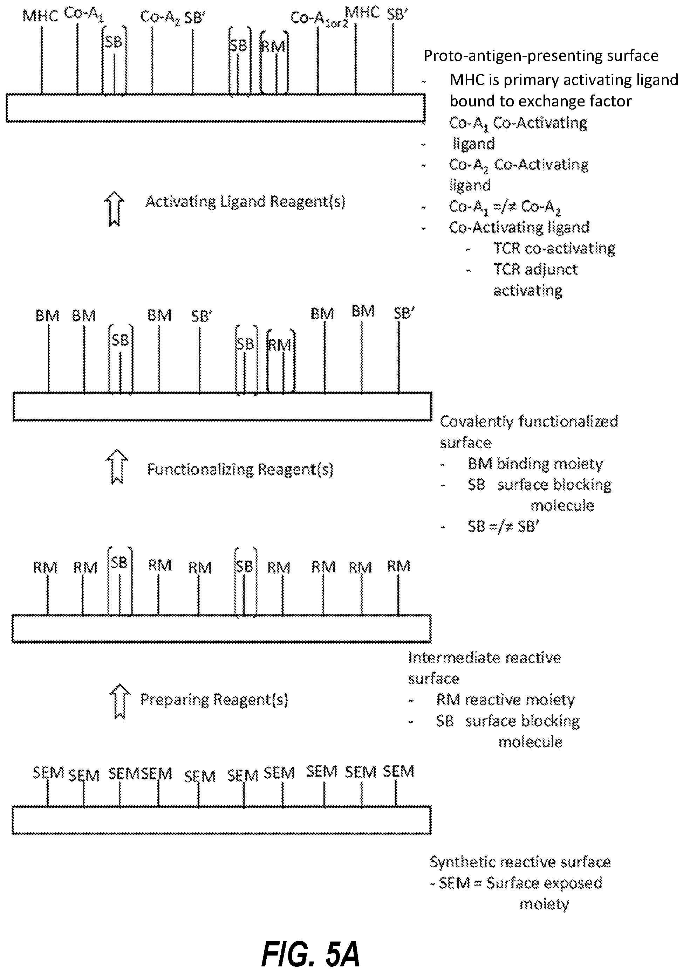

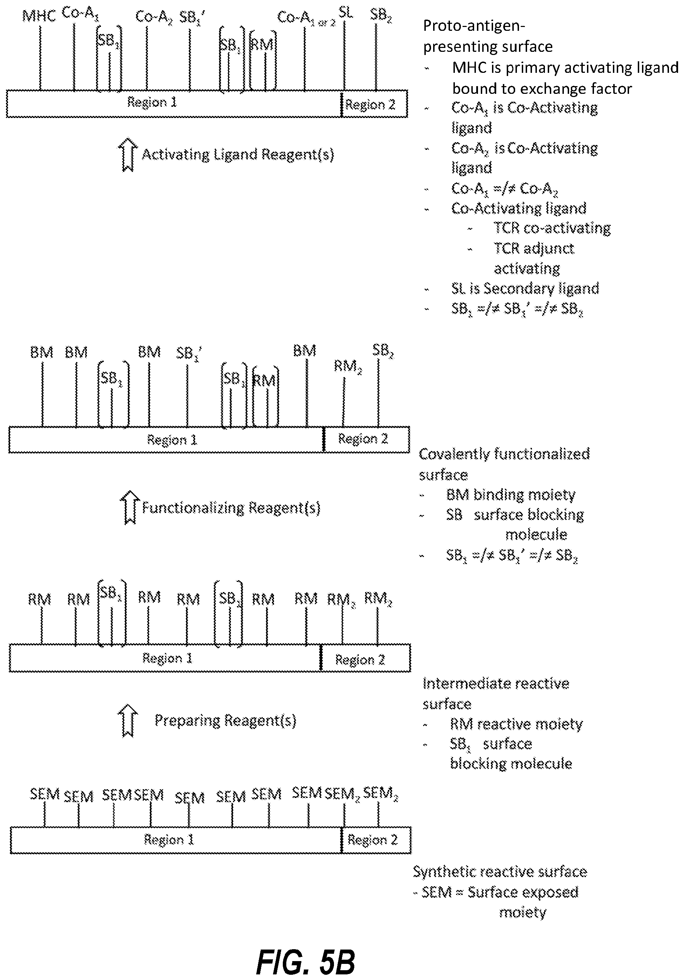

FIGS. 5A and 5B show the structure of a proto-antigen-presenting synthetic surface as it is constructed from an unmodified surface according to certain exemplary methods, comprising adding the activating, co-activating and surface-blocking molecular ligands in one or more steps. FIG. 5A shows the process and structure for a proto-antigen-presenting synthetic surface having a single region, while FIG. 5B shows the process and structure of each intermediate and final product for a proto-antigen-presenting synthetic surface having two regions.

Turning to Fig. 5A, the schematic representation illustrates an exemplary procedure for preparing a proto-antigen-presenting surface starting with a synthetic reactive surface comprising a plurality of surface-exposed moieties (SEM). Reactive moieties RM and surface-blocking molecular ligands SB, if added at this point in the preparation, are introduced by reacting the SEMs with appropriate preparing reagent(s), providing an intermediate reactive surface. The reactive moieties RM introduced to the intermediate reactive surface may be any reactive moiety described herein and may be linked to the intermediate reactive surface by any linker described herein. The intermediate reactive surface includes at least reactive moieties RM, and, in some embodiments, may include surface-blocking molecular ligands SB, which may be any surface-blocking molecular ligand as described herein.

The intermediate reactive surface is then treated with functionalizing reagents including binding moieties BM, where the functionalizing reagents react with the reactive moieties RM to introduce binding moiety BM ligands. The binding moieties so introduced may be any binding moiety BM described herein. The binding moiety BM may be streptaviding or biotin. In some embodiments, the binding moiety BM is streptavidin which is covalently attached via a linker to the covalently functionalized surface, through a reaction with a reactive moiety RM. In some other embodiments, the covalently functionalized surface may introduce a streptavidin binding moiety non-covalently, in a two part structure. This two part structure is introduced by contacting the intermediate reactive surface with a first functionalizing reagent to introduce a biotin moiety covalently attached via a linker through reaction with the reactive moieties RM. Subsequent introduction of streptavidin, as a second functionalizing reagent, provides the covalently functionalized surface wherein the binding moiety BM, streptavidin, is non-covalently attached to a biotin moiety which itself is covalently attached to the surface. Surface-blocking molecular ligands SB' may be introduced at the same time as the introduction of the binding moieties or may be introduced to the covalently functionalized surface subsequent to the introduction of the binding moieties. The surface-blocking molecular ligands SB' may be any surface-blocking molecular ligand as described herein and may be the same as or different from surface-blocking molecular ligands SB, if surface-blocking molecular ligands SB are present. In some embodiments, surface-blocking molecular ligands SB may be present and there may be no surface-blocking molecular ligands SB'. Alternatively, there may be surface-blocking molecular ligands SB' but no surface-blocking molecular ligands SB. In some embodiments, both surface-blocking molecular ligands SB and SB' are present. Without being bound by theory, there may be some reactive moieties RM left unreacted upon the covalently functionalized surface but there are insufficient numbers of reactive moieties RM present to prevent the product antigen presenting synthetic surface from functioning. Primary activating ligands MHC and Co-Activating Ligands Co-A₁ and Co-A₂ are introduced by reacting the binding moieties BM of the covalently functionalized surface with appropriate activating ligand reagents, providing the proto-antigen-presenting synthetic surface in the case where the MHC at the time of reaction with the binding moieties comprises an exchange factor. Alternatively, the MHC at the time of reaction with the binding moieties may comprise an initial peptide and the proto-antigen-presenting surface can be provided by contacting the MHC (already associated with the surface) with an exchange factor, e.g., at molar excess under conditions suitable for displacement of the initial peptide by the exchange factor. Co-A₁ and Co-A₂ may be the same or different co-activating ligands. For example, Co-A₁ and Co-A₂ can comprise one, the other, or collectively both of a TCR co-activating molecule and a TCR adjunct activating molecule. Co-A₁ and/or Co-A₂, may be any combination of TCR co-activating molecule and a TCR adjunct activating molecule as described herein. In some embodiments, the primary activating ligand MHC may be introduced to the covalently functionalized surface, before the covalently functionalized surface is contacted with the co-activating ligands Co-A₁ and/or Co-A₂. In other embodiments, the primary activating ligand MHC may be introduced to the covalently functionalized surface concurrently with or subsequently to the introduction of the Co-Activating ligands Co-A₁ and Co-A₂. In some embodiments, not shown in FIG 5A, after introduction of the primary activating ligand MHC and co-activating ligands Co-A₁ and/or Co-A₂, surface-blocking molecular ligands SB may be introduced to the antigen presenting synthetic surface by reacting surface-blocking molecules with remaining reactive moieties RM still present on the proto-antigen-presenting synthetic surface. Also included but not illustrated in FIG. 5A, is the introduction of Secondary Ligands SL, which may be one or more growth stimulatory molecular ligands and/or adhesion stimulatory molecular ligands. Secondary Ligands SL may be any of these classes of ligands.

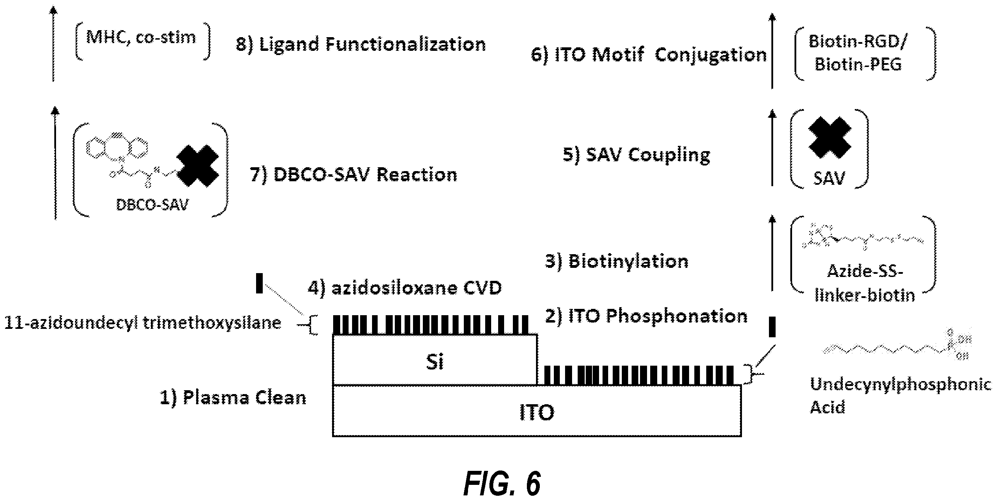

Fig. 5B provides a schematic illustration of an exemplary procedure for preparing a proto-antigen-presenting surface comprising first and second regions starting with a synthetic reactive surface comprising a plurality of surface-exposed moieties (SEM). The surface exposed moieties SEM in Region 1 may be different from the surface exposed moieties SEM₂ in Region 2, as shown in FIG. 6, where different materials may be present at the surface of the synthetic reactive surface. Reactive moieties RM are introduced in region 1 and substantially not in region 2, while reactive moieties RM₂ are introduced in region 2,and substantially not in region 1, due to the use of orthogonal chemistries for each of SEM and SEM₂. For example, as shown in FIG 6, the SEM of region 1 may be reacted with an alkoxysiloxane reagent comprising an azide RM, while the SEM2 of region 2 may be reacted with a phosphonic acid reagent comprising an alkynyl RM. Surface-blocking molecular ligands SB₁ are introduced in region 1, and substantially not in region 2, by reacting the SEMs with appropriate preparing reagent(s (e.g, for a surface like region 1 of FIG. 6, the reagent would be an alkoxysiloxane reagnt including a surface-blocking group SB). An intermediate reactive surface having differentiated reactive moieties result from this process. Based on the differentiated reactive moieties RM and RM₂, further orthogonal chemistries can introduce binding moieties BM and surface-blocking molecular ligands SB₁' in region 1 and not substantially in region 2, and surface-blocking molecular ligands SB₂ are introduced in region 2, and not substantially in region 1. Thus, a covalently functionalized surface having two different regions is provided. The SB₁' may be the same as or different from SB₁; SB₁' may be the same as or different from SB₂; and SB₂ may be the same as or different from SB₁. Primary activating ligands MHC and Co-Activating Ligands Co-A₁ and Co-A₂ are introduced in region 1 by reacting binding moieties BM with appropriate activating ligand reagents, and secondary ligands SL are formed in region 2 by reacting RMs with appropriate reagent(s), providing the proto-antigen-presenting synthetic surface in the case where the MHC at the time of reaction with the binding moieties comprises an exchange factor. Alternatively, the MHC at the time of reaction with the binding moieties may comprise an initial peptide and the proto-antigen-presenting surface can be provided by contacting the MHC (already associated with the surface) with an exchange factor, e.g., at molar excess under conditions suitable for displacement of the initial peptide by the exchange factor. Secondary Ligands SL may be any of the classes of molecular ligands as described for FIG. 5A. The primary activating ligand MHC may be introduced before introducing the Co-Activating Ligands, similarly to the process described for FIG. 5A. Co-A₁ and Co-A₂ may be the same or different co-activating ligands. For example, Co-A₁ and Co-A₂ can comprise one, the other, or collectively both of a TCR co-activating molecule and a TCR adjunct activating molecule. Each of SEM, RM, SB, primary activating ligand MHC, Co-Activating Ligands Co-A₁ and Co-A₂, and secondary ligands SL may be any SEM, RM, SB, primary activating ligand MHC, Co-Activating Ligands Co-A₁ and Co-A₂, and secondary ligands SL described herein.

In embodiments in which the linking reagents include biotin, the method can further comprise noncovalently associating streptavidin with the biotin. In such embodiments, with reference to FIG. 5A, the conversion of a reactive moiety RM to a binding moiety BM can comprise covalently attaching a biotin (corresponding to the additional biotin in the above description) through reaction with the RM and then associating a streptavidin noncovalently with the covalently attached biotin.

In some embodiments, the reactive moieties of an intermediate reactive synthetic surface are linked to the surface through a series of 5, 10, 11, 12, 13, 14, 15, 16, 17, 18, 19, 20, or, in some embodiments, greater numbers of bonds. For example, the reactive moieties can be linked through a series of 15 bonds, e.g., using (11-(X)undecyl)trimethoxy silane, where X is the reactive moiety (e.g., X can be azido). With respect to linking reagents including biotin, biotin can then be covalently associated using a linking reagent such as one having the general structure DBCO-PEG₄-biotin (commercially available from BroadPharm). In some embodiments, the biotin is linked to the surface through a series of about 15, 20, 25, 30, 35, 40, 45, 50, 55, 60, 65, 70, 75, 80, 85, 95, 100, 200 bond lengths, or any number of bond lengths therebetwteen.. With respect to linking reagents including streptavidin, streptavidin can then be covalently associated using a linking reagent such as one having the general structure DBCO-PEG₁₃-succinimide, followed by reaction of streptavidin with the succinimide. In some embodiments, the streptavidin is linked to the surface through a series of about 15, 20, 25, 30, 35, 40, 45, 50, 55, 60, 65, 70, 75, 80, 85, 95, 100, 200 bond lengths, or any number of bond lengths therebetwteen.. The number of bonds through which a moiety is linked to a surface can be varied, e.g., by using reagents similar to those mentioned above but with alkylene and/or PEG chains of different lengths.

In some embodiments, the reactive moieties of at least first region of the intermediate reactive synthetic surface include azide moieties. In some embodiments, covalent bonds are formed through an azide-alkyne reaction, such as any azide-alkyne reaction described elsewhere herein.

In some embodiments, the covalently functionalized synthetic surface includes a second region wherein the plurality of streptavidin functionalities is excluded. In some embodiments, the at least first plurality of surface-blocking molecular ligands are disposed in the second region of the covalently functionalized synthetic surface.

In some embodiments, a method further includes reacting a second plurality of surface-blocking molecules with a second subset of reactive moieties in the at least first region of the intermediate reactive synthetic surface.

In some embodiments, the reacting of the plurality of streptavidin functionalities and the reacting of the at least first plurality of surface-blocking molecules is performed at a plurality of sub-regions of the at least first region of the covalently prepared synthetic surface including reactive moieties.

In some embodiments, the second portion of the reactive synthetic surface includes surface exposed moieties configured to substantially not react with the pluralities of the primary activating and co-activating molecules.

In some embodiments, a method further includes preparing the intermediate reactive synthetic surface, including: reacting at least a first surface preparing reagent including azide reactive moieties with surface-exposed moieties disposed at at least a first region of a reactive synthetic surface.

In some embodiments, the surface-exposed moieties are nucleophilic moieties. In some embodiments, the nucleophilic moiety of the surface is a hydroxide, amino or thiol. In some other embodiments, the nucleophilic moiety of the surface may be a hydroxide.

In some embodiments, the surface-exposed moieties are displaceable moieties.

In some embodiments, where two modifying reagents are used, the reaction of the first modifying reagent and the reaction of the second modifying reagent with the surface may occur at random locations upon the surface. In other embodiments, the reaction of the first modifying reagent may occurs within a first region of the surface and reaction of the second modifying reagent may occur within a second region of the surface abutting the first region. For example, the surfaces within the channel of a microfluidic device may be selectively modified with a first surface modification and the surfaces within the sequestration pen, which abut the surfaces within the channel, may be selectively modified with a second, different surface modification.

In yet other embodiments, the reaction of the first modifying reagent may occurs within a plurality of first regions separated from each other on the at least one surface, and the reaction of the second modifying reaction may occur at a second region surrounding the plurality of first regions separated from each other.

In various embodiments, modification of one or more surfaces of a microfluidic device to introduce a combination of a first surface modification and a second surface modification may be performed after the microfluidic device has been assembled. For one nonlimiting example, the first and second surface modification may be introduced by chemical vapor deposition after assembly of the microfluidic device. In another nonlimiting example, a functionalized surface having a first surface modification having a first reactive moiety and a second surface modification having a second, orthogonal reactive moiety may be introduced. Differential conversion to two different surface modifying ligands having two different surface contact moieties can follow.

In some embodiments, at least one of the combination of first and second surface modification may be performed before assembly of the microfluidic device. In some embodiments, modifying the at least one surface may be performed after assembly of the microfluidic device.

In some embodiments, a covalently functionalized surface is prepared comprising a binding agent. In some embodiments, the distribution of the plurality of binding agent (e.g., plurality of multivalent binding agent, such as a tetravalent binding agent, e.g., streptavidin functionalities, which may be covalently associated or noncovalently associated with a covalently bound biotin) on the covalently functionalized synthetic surface is from about 6X 10² to about 5X 10³ molecules per square micron, in each region where it is attached. In some embodiments, the distribution of the plurality of binding agent (e.g., plurality of multivalent binding agent, such as a trivalent binding agent) is about about 1.5X 10³ to about 1X 10⁴, about 1.5X 10³ to about 7.5X 10³, or about 3X 10³ to about 7.5X 10³ molecules per square micron, in each region where it is attached. In some embodiments, the distribution of the plurality of binding agent (e.g., plurality of multivalent binding agent, such as a divalent binding agent) is about 2.5X 10³ to about 1.5X 10⁴, about 2.5X 10³ to about 1X 10⁴, or about 5X 10³ to about 1X 10⁴ molecules per square micron, in each region where it is attached. In some embodiments, the distribution of the plurality of binding agent (e.g., plurality of monovalent binding agent) is about 5X 10³ to about 3X 10⁴, about 5X 10³ to about 2X 10⁴, or about 1X 10⁴ to about 2X 10⁴ molecules per square micron, in each region where it is attached..

In some embodiments, a covalently functionalized surface is prepared comprising a binding agent, in which the distribution of the plurality of binding agent (e.g., streptavidin functionalities, which may be covalently associated or noncovalently associated with a covalently bound biotin) on the covalently functionalized synthetic surface is from about 1x10⁴ to about 1x 10⁶ molecules per square micron, in each region where it is attached.

In some embodiments, a combined method comprising preparing a covalently functionalized surface and then preparing a proto-antigen-presenting synthetic surface is provided. As such, any suitable combination of steps for preparing the covalently functionalized surface and steps for preparing the proto-antigen-presenting synthetic surface may be used.

### Additional aspects of surface preparation and covalently functionalized surfaces.

Any method of preparing a surface described herein, including methods of preparing an proto-antigen-presenting synthetic surface, may further comprise one or more of the following aspects. A covalently functionalized surface may further comprise one or more of the following aspects applicable to such surfaces, such as reactive groups.

**Azide-alkyne reactions.** In some embodiments, covalent bonds are formed by reacting an alkyne, such as an acyclic alkyne, with an azide. For example, a "Click" cyclization reaction may be performed, which is catalyzed by a copper (I) salt. When a copper (I) salt is used to catalyze the reaction, the reaction mixture may optionally include other reagents which can enhance the rate or extent of reaction. When an alkyne, e.g., of a surface modifying reagent or a functionalized surface is a cyclooctyne, the "Click" cyclization reaction with an azide of the corresponding functionalized surface or the surface modifying reagent may be copper-free. A "Click" cyclization reaction can thereby be used to couple a surface modifying ligand to a functionalized surface to form a covalently modified surface.

**Copper catalysts.** Any suitable copper (I) catalyst may be used. In some embodiments, copper (I) iodide, copper (I) chloride, copper (I) bromide or another copper (I) salt. In other embodiments, a copper (II) salt may be used in combination with a reducing agent such as ascorbate to generate a copper (I) species in situ. Copper sulfate or copper acetate are non-limiting examples of a suitable copper (II) salt. In other embodiments, a reducing agent such as ascorbate may be present in combination with a copper (I) salt to ensure sufficient copper (I) species during the course of the reaction. Copper metal may be used to provide Cu(I) species in a redox reaction also producing Cu(ll) species. Coordination complexes of copper such as [CuBr(PPh₃)₃], silicotungstate complexes of copper, [Cu(CH₃CN)₄]PF₆, or (Eto)₃P Cul may be used. In yet other embodiments, silica supported copper catalyst, copper nanoclusters or copper /cuprous oxide nanoparticles may be employed as the catalyst.

**Other reaction enhancers.** As described above, reducing agents such as sodium ascorbate may be used to permit copper (I) species to be maintained throughout the reaction, even if oxygen is not rigorously excluded from the reaction. Other auxiliary ligands may be included in the reaction mixture, to stabilize the copper (I) species. Triazolyl containing ligands can be used, including but not limited to tris(benzyl-1H-1,2,3-triazol-4-yl) methylamine (TBTA) or 3 [tris(3-hydroxypropyltriazolylmethyl)amine (THPTA). Another class of auxiliary ligand that can be used to facilitate reaction is a sulfonated bathophenanthroline, which is water soluble, as well, and can be used when oxygen can be excluded. Other chemical couplings as are known in the art may be used to couple a surface modifying reagent to a functionalized surface.

**Cleaning the surface.** The surface to be modified may be cleaned before modification to ensure that the nucleophilic moieties on the surface are freely available for reaction, e.g., not covered by oils or adhesives. Cleaning may be accomplished by any suitable method including treatment with solvents including alcohols or acetone, sonication, steam cleaning and the like. Alternatively, or in addition, such pre-cleaning can include cleaning (e.g., of the cover, the microfluidic circuit material, and/or the substrate in the context of components of a microfluidic device) in an oxygen plasma cleaner, which can remove various impurities, while at the same time introducing an oxidized surface (e.g. oxides at the surface, which may be covalently modified as described herein). Alternatively, liquid-phase treatments, such as a mixture of hydrochloric acid and hydrogen peroxide or a mixture of sulfuric acid and hydrogen peroxide (e.g., piranha solution, which may have a ratio of sulfuric acid to hydrogen peroxide from about 3:1 to about 7:1) may be used in place of an oxygen plasma cleaner. This can advantageously provide more sites for modification on the surface, thereby providing a more closely packed modified surface layer.

**Components of microfluidic devices.** A surface of a material that may be used as a component of a microfluidic device may be modified before assembly thereof. Alternatively, a partially or completely constructed microfluidic device may be modified such that all surfaces that will contact biomaterials including biomolecules and/or micro-objects (which may include biological micro-objects) are modified at the same time. In some embodiments, the entire interior of a device and/or apparatus may be modified, even if there are differing materials at different surfaces within the device and/or apparatus. This discussion also applies to the methods of preparing an proto-antigen-presenting synthetic surface described herein.

When an interior surface of a microfluidic device reacted with a surface modifying reagent, the reaction may be performed by flowing a solution of the surface modifying reagent into and through the microfluidic device.

**Surface modifying reagent solutions and reaction conditions.** In various embodiments, the surface modifying reagent may be used in a liquid phase surface modification reaction, e.g., wherein the surface modifying reagent is provided in solution, such as an aqueous solution. Other useful solvents include aqueous dimethyl sulfoxide (DMSO), DMF, acetonitrile, or an alcohol may be used. For example, surfaces activated with tosyl groups or labeled with epoxy groups can be modified in liquid phase reactions. Reactions to couple biotin or proteins such as antibodies, MHCs, or streptavidin to a binding moiety can also be performed as liquid phase reactions.

The reaction may be performed at room temperature or at elevated temperatures. In some embodiments, the reaction is performed at a temperature in a range from about 15°C to about 60°C; about 15°C to about 55°C; about 15°C to about 50°C; about 20°C to about 45°C. In some embodiments, the reaction to convert a functionalized surface of a microfluidic device to a covalently modified surface is performed at a temperature of about 15°C, 20°C, 25°C, 30°C, 35°C, 40°C, 45°C, 50°C, 55°C, or about 60°C.

Alternatively, a surface modifying reagent may be used in a vapor phase surface modification reaction. For example, silica surfaces and other surfaces comprising hydroxyl groups can be modified in a vapor phase reaction. In some embodiments, a surface (e.g., a silicon surface) is treated with plasma (e.g., using an oxygen plasma cleaner; see the Examples for exemplary treatment conditions). In some embodiments, a surface, such as a plasma treated and/or silicon surface, is reacted under vacuum with a preparing reagent, e.g., comprising a methoxysilane and an azide, such as (11-azidoundecyl) trimethoxy silane. The preparing reagent can be provided initially in liquid form in a vessel separate from the surface and can be vaporized to render it available for reaction with the surface. A water source such as a hydrated salt, e.g., magnesium sulfate heptahydrate can also be provided, e.g., in a further separate vessel. For example, foil boat(s) in the bottom of a vacuum reactor can be used as the separate vessel(s). Exemplary reaction conditions and procedures include pumping the chamber to about 750 mTorr using a vacuum pump and then sealing the chamber. The vacuum reactor can then be incubated at a higher-than ambient temperature for an appropriate length of time, e.g., by placing it within an oven heated at 110°C for 24- 48 h. Following the reaction period, the chamber can be allowed to cool and an inert gas such as argon can be introduced to the evacuated chamber. The surface can be rinsed with one or more appropriate liquids such as acetone and/or isopropanol, and then dried under a stream of inert gas such as nitrogen. Confirmation of introduction of the modified surface can be obtained using techniques such as ellipsometry and contact angle goniometry.

Additional modified surfaces, surface-modifying reagents, and related methods that can be employed in accordance with this disclosure are described in WO2017/205830, published November 30, 2017, which is incorporated herein by reference for all purposes.

### Methods of activating a T lymphocyte.

A method of activating T lymphocytes is provided, comprising: preparing an antigen-presenting surface as described herein; contacting a plurality of T lymphocytes with the antigen-presenting synthetic surface; and, culturing the plurality of T lymphocytes in contact with the proto-antigen-presenting synthetic surface, thereby converting at least a portion of the plurality of T Lymphocytes to activated T lymphocytes. Any proto-antigen-presenting surface described herein may be used to generate the antigen-presenting surface. In some embodiments, the MHC molecule is an MHC Class 1 molecule. In various embodiments, the plurality of MHC molecules may each include an amino acid sequence, and further may be connected to the surface via a C-terminal connection of the amino acid sequence. Alternatively, the MHC molecule can be connected to the surface through a noncovalent association. Any noncovalent association can be used, e.g., biotinylation of the MHC and binding thereof to streptavidin on the surface. In various embodiments, the MHC molecule may further include a peptide antigen following displacement of an exchange factor, such as any of the exchange factors described herein. In some embodiments, the peptide antigen is a tumor associated antigen, e.g., any of the tumor associated antigens described herein.

In some embodiments, the co-activating molecules may be connected to the proto-antigen-presenting synthetic surface, as described herein. The T cell receptor (TCR) co-activating molecule or an adjunct TCR activating molecule of the plurality of co-activating molecules may be any TCR co-activating molecule or any adjunct TCR activating molecule as described herein and may be provided in any ratio described herein.

In various embodiments the method may further include contacting the plurality of T lymphocytes with a plurality of growth stimulatory molecular ligands. In some embodiments, each of the growth stimulatory molecular ligands may includs a growth factor receptor ligand. In some embodiments, contacting the plurality of T lymphocytes with the plurality of growth stimulatory molecular ligands may be performed after a first period of culturing of at least one day. In some embodiments, the plurality of growth stimulatory molecular ligands may include IL-21 or a fragment thereof. In various embodiments, the plurality of growth stimulatory molecular ligands may be connected to the antigen-presenting synthetic surface. In some embodiments, the plurality of growth stimulatory molecular ligands may be connected to a surface (e.g., of a bead) that is a different surface than the antigen-presenting synthetic surface including the biomolecules including MHC molecules. In some embodiments, the plurality of growth stimulatory molecular ligands may be connected to the antigen-presenting synthetic surface including MHC molecules.

In various embodiments, the method may include using antigen presenting surfaces on beads. When beads having antigen presenting surfaces are used, the ratio of beads to T lymphocytes may be about 1:1; about 3:1; about 5:1; about 7:1 or about 10:1. The beads may have antigen presenting MHC molecules and anti-CD28 antibodies attached thereto in any method as described herein. In some embodiments, IL-21 may also be attached to the antigen presenting surface of the bead. In other embodiments, IL-21 may be attached to a second bead that has IL-21 as the only biomolecule contributing to activation.

In other embodiments, the method may be performed using a planar surface which may be patterned or unpatterned.

In various embodiments, a first period of culturing may be performed for 4, 5, 6, 7, or 8 days. During the first period of culturing, growth stimulatory molecules such as IL-21, IL-2, and/or IL-7 may be added in solution or may be added on bead to feed the T lymphocytes.

At the end of a first period of culture, the population of cells may include a mixture of unactivated and activated T lymphocytes. Flow cytometry using multiple cell surface markers can be performed to determine the extent of activation and the phenotype of the cells analyzed.

A second period of culture can be performed. If the antigen presenting surfaces are beads, a second aliquot of beads containing the primary activating molecular ligand including the MHC molecule, which includes the tumor associated antigen and co-activating molecules (e.g., TCR co-activating molecules and/or adjunct TCR activating molecules, such as anti-CD28 antibodies and/ot anti-CD2 antibodies, respectively) may be provided to the T lymphocutes, e.g., by addition to the wellplate, chamber of the fluidic circuit containing device, or microfluidic device having sequestration pens as described herein. The antigen presenting beads may further include additional growth stimulatory molecules, e.g., IL-21, connected thereto. The antigen presenting beads may be added to the cells being cultured in about a 1:1; about 3:1, about 5:1; or about 10:1 ratio to the cells. In some embodiments, a second aliquot of IL-21 may be added as a second set of beads having IL-21 connected thereto, or further, may be added as a solution. IL-2 and IL-7 may also be added during the second period of culturing to activate additional numbers of T lymphocytes.

When a patterned or unpatterned wafer, inner surface of a fluidic circuit containing device, inner surface of a tube, or inner surface of a microfluidic device having sequestration pens is used, a second period of culturing may be accomplished by continuing to culture in contact with same antigen presenting surface. Alternatively, a new antigen presenting surface may be brought into contact with the T lymphocytes resultant from the first period of culturing. In other embodiments, antigen presenting beads, like any described above or set forth in any embodiments disclosed herein, may be added to the wells or interior chamber of a fluidic circuit containing device or the sequestration pens of a microfluidic device. Growth stimulatory molecules such as IL-21, IL-2, IL-7, or a combination thereof may be added in solution or on beads. In some embodiments, IL-2 and IL-7 are added.

At the conclusion of the second culturing period, flow cytometry analysis can be performed to determine the extent of activation and to determine the phenotype of the further activated T lymphocytes present at that time.

In some embodiments, a third period of culturing may be included. The third period may have any of the features described herein with respect to the second period. In some embodiments, the third period is performed in the same way as the second period. For example, and all of the actions employed in the second period of culturing may be repeated to further activate T lymphocytes in the wells of the wellplate, in a tube, or in the chamber of a fluidic circuit containing device or a microfluidic device having sequestration pens.

In some embodiments, the T lymphocytes being activated comprise CD8+ T lymphocytes, such as naïve CD8+T lymphocytes. In some embodiments, the T lymphocytes being activated are enriched for CD8+ T lymphocytes, such as naïve CD8+ T lymphocytes. Alternatively, in some embodiments, the T lymphocytes being activated comprise CD4+ T lymphocytes, such as naïve CD4+T lymphocytes. In some embodiments, the T lymphocytes being activated are enriched for CD4+ T lymphocytes, such as naïve CD4+ T lymphocytes. CD4+T lymphocytes can be used, e.g., if T cells specific for a Class II-restricted antigen are desired.

In some embodiments, the method produces activated T lymphocytes that are CD45RO+. In some embodiments, the method produces activated T lymphocytes that are CD28+. In some embodiments, the method produces activated T lymphocytes that are CD28+ CD45RO+. In some embodiments, the method produces activated T lymphocytes that are CD197+. In some embodiments, the method produces activated T lymphocytes that are CD127+. In some embodiments, the method produces activated T lymphocytes that are positive for CD28, CD45RO, CD127 and CD197, or at least any combination of three of the foregoing markers, or at least any combination of two of the foregoing markers. The activated T lymphocytes with any of the foregoing phenotypes can further be CD8+. In some embodiments, any of the foregoing phenotypes that is CD28+ comprises a CD28high phenotype.

In some embodiments, the method produces a population of T cells comprising antigen-specific T cells, wherein at least 65%, 70%, 75%, 80%, 85%, 86%, 87%, 88%, 89%, 90%, 91%, 92%, 93%, 94%, 95%, 96%, 97%, or 98% of the antigen-specific T cells are CD45RO+/CD28High cells, wherein each of the foregoing values can be modified by "about." Alternatively or in addition, in some embodiments, the method produces a population of T cells wherein at least 1%, 1.5%, 2%, 3,%, 4%, 5%, 6%, 7%, 8%, 9%, or 10% of the T cells are antigen-specific T cells; or wherein 1%-2%, 2%-3%, 3%-4%, 4%-5%, 5%-6%, 6%-7%, 7%-8%, 8%-9%, 9%-10%, 10%-11%, or 11%-12% of the T cells are antigen-specific T cells, wherein each of the foregoing values can be modified by "about." The content of the population of T cells can be determined on the "crude" product of the method following contact with the antigen-presenting surface and optionally further expansion steps, i.e., before/without enriching or separating product T cells having a particular phenotype. The determination of antigen-specificity and/or T cell marker phenotype can exclude dead cells.

In some embodiments, the method provides a population of T cells in which the fraction of T cells that are antigen-specific is increased relative to the starting population.

### Cells and Compositions.

An activated T lymphocyte produced by any method described herein is provided.

In some embodiments, the activated T lymphocytes are CD45RO+. In some embodiments, the activated T lymphocytes are CD28+. In some embodiments, the activated T lymphocytes are CD28+ CD45RO+. In some embodiments, the activated T lymphocytes are CD197+. In some embodiments, the activated T lymphocytes are CD127+. In some embodiments, the activated T lymphocytes are positive for CD28, CD45RO, CD127 and CD197, at least any combination of three of the foregoing markers, or at least any combination of two of the foregoing markers. The activated T lymphocytes with any of the foregoing phenotypes can further be CD8+. In some embodiments, any of the foregoing phenotypes that is CD28+ comprises a CD28high phenotype.

In some embodiments, a population of T cells comprising activated T cells produced by any method described herein is provided. The population can have any of the features described above for T cell populations.

In some embodiments, a microfluidic device is provided comprising a population of T cells provided herein. The microfluidic device can be any of the antigen-presenting microfluidic devices or other microfluidic devices described herein.

In some embodiments, a pharmaceutical composition is provided comprising a population of T cells provided herein. The pharmaceutical composition can further comprise, e.g., saline, glucose, and/or Human Serum AlbuminThe composition may be an aqueous composition and can be provided in frozen or liquid form. A pharmaceutical composition can be provided as a single dose, e.g., within a syringe, and can comprise 10 million, 100 million, 1 billion, or 10 billion cells. The number of cells administered is indication specific, patient specific (e.g., size of patient), and will also vary with the purity and phenotype of the administered cells.

### Methods of Treatment.

Provided herein is a method of treating a subject in need of treating a cancer; including: obtaining a sample comprising T lymphocytes from the subject; separating the T lymphocytes from other cells in the sample; contacting the T lymphocytes with a antigen-presenting synthetic surface including MHC molecules, wherein the antigen-presenting synthetic surface is prepared according to any method described herein, where the MHC molecules include an antigen specific for the cancer of the subject; producing a plurality of T lymphocytes activated to be specific against the cancer of the subject; separating the plurality of specific activated T lymphocytes from non-activated T lymphocytes; and, introducing the plurality of specific activated T lymphocytes into the subject. Also provided herein is a plurality of specific activated T lymphocytes for use in treating a cancer, wherein the plurality is prepared by a method including: obtaining a sample comprising T lymphocytes from the subject; separating the T lymphocytes from other cells in the sample; contacting the T lymphocytes with a antigen-presenting synthetic surface including MHC molecules according to any method described herein, where the MHC molecules inlcude an antigen specific for the cancer of the subject; producing a plurality of T lymphocytes activated to be specific against the cancer of the subject; and separating the plurality of specific activated T lymphocytes from non-activated T lymphocytes. Also provided herein is the use of a plurality of specific activated T lymphocytes for the manufacture of a medicament for treating a cancer, wherein the plurality is prepared by a method including: obtaining a sample comprising T lymphocytes from the subject; separating the T lymphocytes from other cells in the sample; contacting the T lymphocytes with a antigen-presenting synthetic surface including MHC molecules according to any method described herein, where the MHC molecules inlcude an antigen specific for the cancer of the subject; producing a plurality of T lymphocytes activated to be specific against the cancer of the subject; and separating the plurality of specific activated T lymphocytes from non-activated T lymphocytes.

Also provided is a method of treating a subject in need of treating a cancer; including introducing a plurality of specific activated T lymphocytes into the subject, wherein the plurality of specific activated T lymphocytes were produced by a method described herein. Also provided is a method of treating a subject in need of treating a cancer, including introducing a population of specific activated T lymphocytes described herein into the subject. Such methods can further comprise separating activated T lymphocytes from non-activated T lymphocytes. Also provided is a plurality of specific activated T lymphocytes for use in treating a subject in need of treating a cancer, wherein the plurality of specific activated T lymphocytes were produced by a method described herein. Also provided is a population of specific activated T lymphocytes described herein for use in treating a subject in need of treating a cancer. Also provided is a use of a plurality of specific activated T lymphocytes for the manufacture of a medicament for treating a subject in need of treating a cancer, wherein the plurality of specific activated T lymphocytes were produced by a method described herein. Also provided is a use of a population of specific activated T lymphocytes described herein for the manufacture of a medicament for treating a subject in need of treating a cancer. Such a plurality or population of specific activated T lymphocytes can be further prepared by separating activated T lymphocytes from non-activated T lymphocytes.

In some embodiments, separating the plurality of specific activated T lymphocytes may further include detecting surface biomarkers of the specific activated T lymphocytes.

In some embodiments, the specific activated T lymphocytes are autologous (i.e., derived from the subject to which they are to be administered).

In various embodiments, the methods or the preparation of the plurality or population of specific activated T lymphocytes may further include rapidly expanding the activated T lymphocytes to provide an expanded population of activated T lymphocytes. In some embodiments, the rapid expansion may be performed after separating the specific activated T lymphocytes from the non-activated T lymphocytes. The generation of sufficient levels of T lymphocytes may be achieved using rapid expansion methods described herein or known in the art. See, e.g., the Examples below; Riddell, US 5,827,642; Riddell et al., US Patent No. 6,040,177, and Yee and Li, PCT Patent App. Pub. No. WO2009/045308 A2.

Uses of T cells in treatment of human subjects (e.g., for adoptive cell therapy) are known in the art. T cells prepared according to the methods described herein can be used in such methods. For example, adoptive cell therapy using tumor-infiltrating lymphocytes including MART-1 antigen specific T cells have been tested in the clinic (Powell et al., Blood 105:241-250, 2005). Also, administration of T cells coactivated with anti-CD3 monoclonal antibody and IL-2 was described in Chang et al., J. Clinical Oncology 21:884-890, 2003. Additional examples and/or discussion of T cell administration for the treatment of cancer are provided in Dudley et al., Science 298:850-854, 2002; Roszkowski et al., Cancer Res 65(4): 1570-76, 2005; Cooper et al., Blood 101: 1637-44, 2003; Yee, US Patent App. Pub. No. 2006/0269973; Yee and Li, PCT Patent App. Pub. No. WO2009/045308 A2; Gruenberg et al., US Patent App. Pub. No. 2003/0170238; Rosenberg, US Patent No. 4,690,915; and Alajez et al., Blood 105:4583-89, 2005.

In some embodiments, the cells are formulated by first harvesting them from their culture medium and then washing and concentrating the cells in a medium and container system suitable for administration (a "pharmaceutically acceptable" carrier) in a treatment-effective amount. Suitable infusion medium can be any isotonic medium formulation, typically normal saline, Normosol R (Abbott) of Plasma-Lyte A (Baxter), but also 5% dextrose in water or Ringer's lactate can be utilized. The infusion medium can be supplemented with human serum albumin.

In some embodiments, the number of cells in the composition is at least 10⁹, or at least 10¹⁰ cells. In some embodiments, a single dose can comprise at least 10 million, 100 million, 1 billion, or 10 billion cells. The number of cells administered is indication specific, patient specific (e.g., size of patient), and will also vary with the purity and phenotype of the administered cells. The number of cells will depend upon the ultimate use for which the composition is intended as will the type of cells included therein. For example, if cells that are specific for a particular antigen are desired, then the population will contain greater than 70%, generally greater than 80%, 85% and 90-95% of such cells. For uses provided herein, the cells are generally in a volume of a liter or less, can be 500 mis or less, even 250 mls or 100 mls or less. Hence the density of the desired cells may be greater than 10⁶ cells/ml, greater than 10⁷ cells/ml, or 10⁶ cells/ml or greater. The clinically relevant number of immune cells can be apportioned into multiple infusions that cumulatively equal or exceed 10⁹, 10¹⁰ or 10¹¹ cells.

In some embodiments, T lymphocytes described herein or prepared according to a method described herein may be used to confer immunity to individuals against a tumor or cancer cells. By "immunity" is meant a lessening of one or more physical symptoms associated with cancer cells or a tumor against an antigen of which the lymphocytes have been activated. The cells may be administered by infusion, with each infusion in a range of at least 10⁶ to 10¹⁰ cells/m², e.g., in the range of at least 10⁷ to 10⁹ cells/m². The clones may be administered by a single infusion, or by multiple infusions over a range of time. However, since different individuals are expected to vary in responsiveness, the type and amount of cells infused, as well as the number of infusions and the time range over which multiple infusions are given are determined by the attending physician, and can be determined by examination.

Following the transfer of cells back into patients, methods may be employed to maintain their viability by treating patients with cytokines that could include IL-21 and IL-2 (Bear et al., Cancer Immunol. Immunother.50:269-74, 2001; and Schultze et al., Br. J. Haematol. 113:455-60, 2001). In another embodiment, cells are cultured in the presence of IL-21 before administration to the patient. See, e.g., Yee, US Patent App. Pub. No. 2006/0269973. IL-21 can increase T cell frequency in a population comprising activated T cells to levels that are high enough for expansion and adoptive transfer without further antigen-specific T cell enrichment. Accordingly, such a step can further decrease the time to therapy and/or obviate a need for further selection and/or cloning.

### Kits for generating an antigen-presenting synthetic surface.

A kit is also provided for generating an antigen-presenting synthetic surface for activating a T lymphocyte (T cell), including: a covalently functionalized surface, such as any covalently functionalized synthetic surface described herein; a primary activating molecule that includes a major histocompatibility complex (MHC) molecule configured to bind to a T cell receptor (TCR), and a first reactive moiety configured to react with or bind to the covalently functionalized surface; and at least one of: an exchange factor (e.g., provided separately from the primary activating molecule); and an exchange factor bound to the MHC molecule or an initial peptide bound to the MHC molecule, optionally wherein the initial peptide is non-immunogenic. The kit may further comprise at least one co-activating molecule that includes a second reactive moiety configured to react with or bind to the covalently functionalized surface, wherein each co-activating molecule is selected from a TCR co-activating molecule and an adjunct TCR activating molecule and/or an exchange factor. In some embodiments, the exchange factor is provided separately from the primary activating molecule. For example, where an initial peptide (e.g., any of the initial peptides described herein) is bound to the MHC molecule, the exchange factor may be provided as a separate reagent. Alternatively, the exchange factor may be bound to the MHC molecule, e.g., bound in the antigen-binding pocket of the MHC molecule. In some embodiments, the covalently functionalized surface comprises a plurality of first coupling agents. The first coupling agent may be a biotin-binding agent. The primary activating molecular ligand may be configured to bind a first subset of the plurality of first coupling agents. The biotin-binding agent may be streptavidin. In some embodiments, each of the plurality of MHC molecules may further include at least one biotin functionality. Other coupling chemistries may be used, as is known in the art, wherein other site specific protein tags may be attached to the MHC protein, which are configured to covalently attach to recognition protein based species attached to the bead. These coupling strategies can provide the equivalent site specific and specifically orienting attachment of the MHC molecule as provided by C-terminal biotinylation of the MHC molecule. The covalently functionalized synthetic surface may be a wafer, a bead, at least one inner surface of a microfluidic device, or a tube.

Such a kit may be intended for use with one or more peptide antigens supplied by the user. In some embodiments, the kit further includes a buffer suitable for performing an exchange reaction wherein a peptide antigen displaces the exchange factor and/or instructions for performing an exchange reaction wherein a peptide antigen displaces the exchange factor. Exemplary conditions for performing an exchange reaction wherein a peptide antigen displaces the exchange factor include those described, e.g., in Saini et al., Proc Nat'l Acad Sci USA (2013) 110, 15383-88, and Saini et al., Proc Nat'l Acad Sci USA (2015) 112, 202-07.

In some embodiments, the kit further comprises a surface-blocking molecule capable of covalently binding to the covalently functionalized synthetic surface. For example, the surface-blocking molecule can be a PEG acid such as (PEG)₄-COOH. Other surface-blocking molecules, such as those described elsewhere herein, may also be provided.

The kit may further include a reagent including a plurality of co-activating molecules, each configured to bind one of a second subset of the plurality of first coupling agents, e.g., noncovalently or covalently associated biotin-binding agents of the covalently functionalized synthetic surface. In some embodiments, each of the plurality of co-activating molecules may include a biotin functionality. Each of the co-activating molecules may include a T cell receptor (TCR) co-activating molecule, an adjunct TCR activating molecule, or any combination thereof. In some embodiments, the reagent is provided in individual containers containing the T cell receptor (TCR) co-activating molecule and/or an adjunct TCR activating molecule. Alternatively, the reagent including the plurality of co-activating molecules may be provided in one container containing the TCR co-activating molecules and/or the adjunct TCR activating molecules of the plurality of co-activating molecular ligands in a ratio from about 100:1 to 1:100. In some embodiments the reagent including the plurality of co-activating molecules includes a mixture of TCR co-activating molecules and adjunct TCR activating molecules wherein the ratio of the TCR co-activating molecules to the adjunct TCR activating molecules of the plurality of co-activating molecular ligands is 100:1 to 90:1, 90:1 to 80:1, 80:1 to 70:1, 70:1 to 60:1, 60:1 to 50:1, 50:1 to 40:1, 40:1 to 30:1, 30:1 to 20:1, 20:1 to 10:1, 10:1 to 1:1, 1:1 to 1:10, 1:10 to 1:20, 1:20 to 1:30, 1:30 to 1:40, 1:40 to 1:50, 1:50 to 1:60, 1:60 to 1:70, 1:70 to 1:80, 1:80 to 1:90, or 1:90 to 1:100, wherein each of the foregoing values is modified by "about". In some embodiments, the reagent including a plurality of co-activating molecules contains the TCR co-activating molecules and the adjunct TCR activating molecules of the plurality of co-activating molecular ligands in a ratio from about 20:1 to about 1:20.

In some embodiments, the kit for preparing an antigen presenting synthetic surface may further include a reagent including adhesion stimulatory molecules, wherein each adhesion stimulatory molecule includes a ligand for a cell adhesion receptor including an ICAM protein sequence configured to react with a third subset of the plurality of noncovalently or covalently associated biotin-binding agent functionalities of the covalently functionalized synthetic surface. In some embodiments, the adhesion stimulatory molecule may include a biotin functionality.

In some embodiments, the kit for preparing an antigen presenting synthetic surface may further include a reagent including growth stimulatory molecules, wherein each growth stimulatory molecule may include a growth factor receptor ligand. In some embodiments, the growth factor receptor ligand may include a cytokine or a fragment thereof. In some embodiments, the cytokine may include IL-21 or a fragment thereof. In some embodiments, the growth stimulatory molecule may be attached to a covalently modified bead.

In some embodiments, the kit for preparing an antigen presenting synthetic surface may further include a reagent including one or more additional growth-stimulatory molecules. In some embodiments, the one or more additional growth-stimulatory molecules include IL2 and/or IL7, or fragments thereof. In some embodiments, the growth stimulatory molecule may be attached to a covalently modified bead.

### Kits for activating T lymphocytes.

Also provided is a kit for activating T lymphocytes, including a proto-antigen-presenting synthetic surface as described herein. The kit can further comprise instructions for performing an exchange reaction wherein a peptide antigen displaces the exchange factor bound to the primary activating ligand of the proto-antigen-presenting synthetic surface and/or a buffer suitable for performing an exchange reaction wherein a peptide antigen displaces the exchange factor. Such a kit may be intended for use with one or more peptide antigens supplied by the user. The kit can further comprise growth stimulatory molecules, wherein each growth stimulatory molecule may include a growth factor receptor ligand. The growth stimulatory molecules can be provided as free molecules, attached to the antigen presenting synthetic surface (in the same or a different region than the primary activating molecular ligand), or attached to a different covalently modified synthetic surface. For example, the kit can further comprise a plurality of covalently modified beads comprising an adjunct stimulatory molecule. In some embodiments, the growth factor receptor ligand molecule may include a cytokine or a fragment thereof. In some embodiments, the growth factor receptor ligandmay include IL-21. In other embodiments, the kit may include one or more additional (e.g., a second or second and third) growth stimulatory molecules ). In some embodiments, the one or more additional growth stimulatory molecules may include IL-2 and/or IL-7, or fragments thereof. Additional growth stimulatory molecules can be provided as a free molecule, attached to the antigen presenting synthetic surface (in the same or a different region than the primary activating molecular ligand), or attached to a different covalently modified synthetic surface, such as a bead.

### Methods of screening a plurality of peptide antigens for T cell activation.

Also provided herein are methods of screening a plurality of peptide antigens for T cell activation. The proto-antigen-presenting surfaces can be used to rapidly generate antigen-presenting surfaces comprising various peptide antigens of interest, e.g., which may be immunogenic in the context of T cell activation. Such methods can comprise reacting a plurality of different peptide antigens with a plurality of proto-antigen-presenting surfaces, such as any proto-antigen-presenting surfaces described herein, thereby substantially displacing the exchange factors or initial peptides and forming a plurality of antigen-presenting surfaces; contacting a plurality of T cells with the antigen-presenting surfaces; and monitoring the T cells for activation, wherein activation of a T cell indicates that a peptide antigen associated with the surface with which the T cell was contacted is able to contribute to T cell activation.

The proto-antigen-presenting surfaces can be any of the surface types described herein, such as beads, surfaces of a microfluidic device, well plate, etc. To be clear, where the surfaces are surfaces of a larger article such as a microfluidic device or well plate, the plurality of surfaces may be surfaces at different locations on a single article (e.g., well plate or microfluidic device) or surfaces of different articles. For example, a plurality of proto-antigen-presenting surfaces of a microfluidic device can be separated by regions of non-antigen-presenting surface. In some embodiments, the proto-antigen-presenting surfaces can be in different sequestration pens of the microfluidic device while the non-antigen-presenting surface can be in a channel or region connecting the openings of the sequestration pens.

In some embodiments, the proto-antigen-presenting surfaces are reacted separately with the plurality of different peptide antigens, thereby generating a plurality of different antigen-presenting surfaces. In this approach, individual surfaces comprise an individual peptide antigen and thus the extent of T cell activation attributable to that surface provides a readout of the immunogenicity of that particular peptide antigen.

In some embodiments, the proto-antigen-presenting surfaces are reacted separately with pools of members of the plurality of different peptide antigens, thereby generating a plurality of different antigen-presenting surfaces. In this approach, the individual antigen-presenting surfaces comprise more than one peptide antigen, and thus the extent of T cell activation attributable to that surface provides a readout of the immunogenicity of one or more of the peptide antigens associated with the surface. The particular peptide antigen or antigens responsible for the T cell activation can be identified by further analysis, e.g., using the approach of preparing individual surfaces comprising individual antigens described above.

The pools can be overlapping or non-overlapping pools. Overlapping pools provide more information about the individual peptide antigens being tested, in that when activation occurs with a subset of tested surfaces, it can be possible to identify a subset of peptide antigens most likely to be responsible based on which antigens were present on the surfaces that exhibited activation. Non-overlapping pools provide more bandwidth, in that a greater total number of peptide antigens can be tested using a given number of pools, pool sizes, and surfaces when the pools are non-overlapping. A possible workflow for identifying immunogenic peptide antigens from an initial candidate set is to first perform screening using non-overlapping pools, then generate overlapping sub-pools from members of the initial pool sets that showed activation, and then screen individual peptide antigens that the overlapping sub-pool results indicate are potentially immunogenic.

Where beads are used as the surface, T cells may be contacted separately with members of the plurality of different antigen-presenting beads. For example, an individual bead can be contacted with one or more T cells, e.g., in a chamber, such as a sequestration pen or well, while other individual beads are contacted with other T cells in other chambers. Alternatively, T cells can be contacted with a pool of the different antigen-presenting beads. In another alternative, T cells can contacted with a plurality of pools of the different antigen-presenting beads. For example, T cells in a first chamber (such as a sequestration pen or well) can be contacted with a first pool and T cells in a second chamber (such as a sequestration pen or well) can be contacted with a second pool. The first and second pools may be overlapping or non-overlapping.

In some embodiments, the plurality of proto-antigen-presenting surfaces is a plurality of proto-antigen-presenting surfaces in wells of one or more well plates. In such embodiments, the wells may also comprise non-antigen-presenting regions. This can be beneficial through reducing the amount of reagents needed to prepare the antigen-presenting surfaces within the wells and/or through avoiding overstimulation of the T cells.

Monitoring the T cells for activation in any screening method described herein may comprise detecting one or more of various markers consistent with activation (e.g., in combination with being antigen-specific). For example, T cells that are CD45RO+, CD28+, CD28^{High}, CD127+, and/or CD197+ may be detected. In some embodiments, the T cells are or include CD8+ T cells.

### Methods of analyzing stability of a complex comprising a major histocompatibility complex (MHC) molecule and a peptide antigen

Also provided herein are methods of analyzing stability of a complex comprising a major histocompatibility complex (MHC) molecule configured to bind to a T cell receptor (TCR) and a peptide antigen. In some embodiments, the method comprises contacting a plurality of the MHC molecules with the peptide antigen and an exchange factor, thereby forming peptide antigen-bound MHC molecules. An initial peptide (e.g., as described elsewhere herein) may be bound to the MHC molecule before contact with the peptide antigen and exchange factor. The contacting step may be performed over a period of time sufficient for the peptide antigen to substantially displace the initial peptide from the MHC molecules and/or become for the MHC molecules to become bound to the peptide antigen, e.g., at room temperature for about 4 hours or more, or under refrigeration (e.g., about 4 °C) overnight or for about 10, 12, or 15 hours or more. In some embodiments, a plurality of primary activating molecular ligands comprise the MHC molecules and the plurality of primary activating molecular ligands are specifically bound to a covalently functionalized synthetic surface. In other embodiments, (1) a plurality of primary activating molecules comprise the MHC molecules and first reactive moieties or (2) a plurality of primary activating molecules is prepared by adding first reactive moieties to the MHC molecules; and the method further comprises reacting the first reactive moieties of the plurality of primary activating molecules with a first plurality of binding moieties disposed on a covalently functionalized synthetic surface. The method further comprises measuring total binding and/or an extent of dissociation of the peptide antigen from the MHC molecule. The covalently functionalized surface may be any such surface described herein. In some embodiments, the covalently functionalized surface is the surface of a bead.

Measuring the total binding and/or extent of dissociation can comprise, e.g., measuring binding of an agent (e.g., antibody, such as that produced by Biolegend Clone W6/32) to the MHC molecule, wherein the agent specifically binds to (i) the initial peptide, and/or (ii) a peptide-bound conformation of the MHC molecule. The peptide-bound conformation is the conformation that exists when a peptide (e.g., an antigenic peptide or an initial peptide) is bound in the peptide binding cleft formed by the alpha chain of the MHC molecule. Typically, for a MHC Class I molecule, the peptide binding cleft binds to peptides having a length of 8-10 amino acid residues, whereas for an MHC Class II molecule, the peptide binding cleft binds to peptides having a length of 13-18 amino acid residues. In some embodiments, a beta microglobulin (e.g., beta-2-microglobulin) is part of the MHC molecule in its peptide-bound conformation. The beta microglobulin may dissociate from the MHC molecule as part of a transition to a peptide-unbound conformation, e.g., simultaneous with or upon dissociation of the peptide antigen from the MHC molecule. Thus, the agent can be used to discriminate between MHC molecules that retain the peptide antigen and those that do not. The agent may be labeled directly (e.g., by conjugation to a label) or indirectly (e.g., by binding of a secondary antibody comprising a label). The label may be a fluorescent label.

Various approaches for measuring label (e.g., fluorescence) levels associated with a surface may be employed. In some embodiments, measuring the total binding and/or extent of dissociation comprises performing flow cytometry. Flow cytometry can rapidly and accurately quantify the amount of a labeled agent as discussed above that is bound to an MHC molecule associated with an appropriate solid support, such as a bead. Observing changes in such binding over time can permit analysis of stability, e.g., in terms of an appropriate kinetic parameter, such as a half-life or off-rate.

Such methods can be useful to evaluate the suitability of a peptide antigen for preparing and using an antigen-presenting surface as described herein. Peptide antigens that form more stable complexes with MHC molecules can provide more effective stimulation of T cells because the complexes are longer lived and therefore have more time to interact with the T cells. For example, in some embodiments, a peptide antigen is identified as being capable of forming a complex with an MHC molecule that has a half-life of at least about 4 hours (e.g., at least about 6, 8, 10, 12, 14, 16, or 18 hours), or a half-life in the range of about 4 to about 40 hours (e.g., about 4 to about 10 hours, about 10 to about 15 hours, about 15 to about 20 hours, about 20 to about 25 hours, about 25 to about 30 hours, about 30 to about 35, or about 35 to about 40 hours).

### Additional aspects of microfluidic device structure, loading, and operation; related systems.

Microfluidic devices and uses thereof described herein may have any of the following features, and can be used in conjunction with systems described below.

**Methods of loading.** Loading of biological micro-objects or micro-objects such as, but not limited to, beads, can involve the use of fluid flow, gravity, a dielectrophoresis (DEP) force, electrowetting, a magnetic force, or any combination thereof as described herein. The DEP force can be generated optically, such as by an optoelectronic tweezers (OET) configuration and/or electrically, such as by activation of electrodes/electrode regions in a temporal/spatial pattern. Similarly, electrowetting force may be provided optically, such as by an opto-electro wetting (OEW) configuration and/or electrically, such as by activation of electrodes/electrode regions in a temporal spatial pattern.

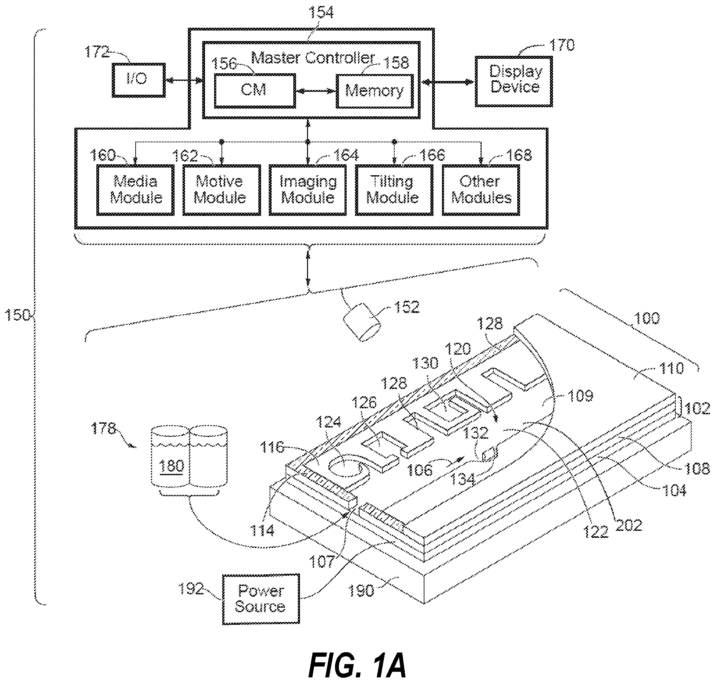

**Microfluidic devices and systems for operating and observing such devices.** Figure 1A illustrates an example of a microfluidic device 100 and a system 150 which can be used for maintaining, isolating, assaying or culturing biological micro-objects. A perspective view of the microfluidic device 100 is shown having a partial cut-away of its cover 110 to provide a partial view into the microfluidic device 100. The microfluidic device 100 generally comprises a microfluidic circuit 120 comprising a flow path 106 through which a fluidic medium 180 can flow, optionally carrying one or more micro-objects (not shown) into and/or through the microfluidic circuit 120. Although a single microfluidic circuit 120 is illustrated in Figure 1A, suitable microfluidic devices can include a plurality (e.g., 2 or 3) of such microfluidic circuits. Regardless, the microfluidic device 100 can be configured to be a nanofluidic device. As illustrated in Figure 1A, the microfluidic circuit 120 may include a plurality of microfluidic sequestration pens 124, 126, 128, and 130, where each sequestration pens may have one or more openings in fluidic communication with flow path 106. In some embodiments of the device of Figure 1A, the sequestration pens may have only a single opening in fluidic communication with the flow path 106. As discussed further below, the microfluidic sequestration pens comprise various features and structures that have been optimized for retaining micro-objects in the microfluidic device, such as microfluidic device 100, even when a medium 180 is flowing through the flow path 106. Before turning to the foregoing, however, a brief description of microfluidic device 100 and system 150 is provided.

As generally illustrated in Figure 1A, the microfluidic circuit 120 is defined by an enclosure 102. Although the enclosure 102 can be physically structured in different configurations, in the example shown in Figure 1A the enclosure 102 is depicted as comprising a support structure 104 (e.g., a base), a microfluidic circuit structure 108, and a cover 110. The support structure 104, microfluidic circuit structure 108, and cover 110 can be attached to each other. For example, the microfluidic circuit structure 108 can be disposed on an inner surface 109 of the support structure 104, and the cover 110 can be disposed over the microfluidic circuit structure 108. Together with the support structure 104 and cover 110, the microfluidic circuit structure 108 can define the elements of the microfluidic circuit 120.

The support structure 104 can be at the bottom and the cover 110 at the top of the microfluidic circuit 120 as illustrated in Figure 1A. Alternatively, the support structure 104 and the cover 110 can be configured in other orientations. For example, the support structure 104 can be at the top and the cover 110 at the bottom of the microfluidic circuit 120. Regardless, there can be one or more ports 107 each comprising a passage into or out of the enclosure 102. Examples of a passage include a valve, a gate, a pass-through hole, or the like. As illustrated, port 107 is a pass-through hole created by a gap in the microfluidic circuit structure 108. However, the port 107 can be situated in other components of the enclosure 102, such as the cover 110. Only one port 107 is illustrated in Figure 1A but the microfluidic circuit 120 can have two or more ports 107. For example, there can be a first port 107 that functions as an inlet for fluid entering the microfluidic circuit 120, and there can be a second port 107 that functions as an outlet for fluid exiting the microfluidic circuit 120. Whether a port 107 function as an inlet or an outlet can depend upon the direction that fluid flows through flow path 106.

The support structure 104 can comprise one or more electrodes (not shown) and a substrate or a plurality of interconnected substrates. For example, the support structure 104 can comprise one or more semiconductor substrates, each of which is electrically connected to an electrode (e.g., all or a subset of the semiconductor substrates can be electrically connected to a single electrode). The support structure 104 can further comprise a printed circuit board assembly ("PCBA"). For example, the semiconductor substrate(s) can be mounted on a PCBA.

The microfluidic circuit structure 108 can define circuit elements of the microfluidic circuit 120. Such circuit elements can comprise spaces or regions that can be fluidly interconnected when microfluidic circuit 120 is filled with fluid, such as flow regions (which may include or be one or more flow channels), chambers, pens, traps, and the like. In the microfluidic circuit 120 illustrated in Figure 1A, the microfluidic circuit structure 108 comprises a frame 114 and a microfluidic circuit material 116. The frame 114 can partially or completely enclose the microfluidic circuit material 116. The frame 114 can be, for example, a relatively rigid structure substantially surrounding the microfluidic circuit material 116. For example, the frame 114 can comprise a metal material.

The microfluidic circuit material 116 can be patterned with cavities or the like to define circuit elements and interconnections of the microfluidic circuit 120. The microfluidic circuit material 116 can comprise a flexible material, such as a flexible polymer (e.g. rubber, plastic, elastomer, silicone, polydimethylsiloxane ("PDMS"), or the like), which can be gas permeable. Other examples of materials that can compose microfluidic circuit material 116 include molded glass, an etchable material such as silicone (e.g. photo-patternable silicone or "PPS"), photo-resist (e.g., SU8), or the like. In some embodiments, such materials-and thus the microfluidic circuit material 116-can be rigid and/or substantially impermeable to gas. Regardless, microfluidic circuit material 116 can be disposed on the support structure 104 and inside the frame 114.

The cover 110 can be an integral part of the frame 114 and/or the microfluidic circuit material 116. Alternatively, the cover 110 can be a structurally distinct element, as illustrated in Figure 1A. The cover 110 can comprise the same or different materials than the frame 114 and/or the microfluidic circuit material 116. Similarly, the support structure 104 can be a separate structure from the frame 114 or microfluidic circuit material 116 as illustrated, or an integral part of the frame 114 or microfluidic circuit material 116. Likewise, the frame 114 and microfluidic circuit material 116 can be separate structures as shown in Figure 1A or integral portions of the same structure.

In some embodiments, the cover 110 can comprise a rigid material. The rigid material may be glass or a material with similar properties. In some embodiments, the cover 110 can comprise a deformable material. The deformable material can be a polymer, such as PDMS. In some embodiments, the cover 110 can comprise both rigid and deformable materials. For example, one or more portions of cover 110 (e.g., one or more portions positioned over sequestration pens 124, 126, 128, 130) can comprise a deformable material that interfaces with rigid materials of the cover 110. In some embodiments, the cover 110 can further include one or more electrodes. The one or more electrodes can comprise a conductive oxide, such as indium-tin-oxide (ITO), which may be coated on glass or a similarly insulating material. Alternatively, the one or more electrodes can be flexible electrodes, such as single-walled nanotubes, multi-walled nanotubes, nanowires, clusters of electrically conductive nanoparticles, or combinations thereof, embedded in a deformable material, such as a polymer (e.g., PDMS). Flexible electrodes that can be used in microfluidic devices have been described, for example, in U.S. 2012/0325665 (Chiou et al.), the contents of which are incorporated herein by reference. In some embodiments, the cover 110 can be modified (e.g., by conditioning all or part of a surface that faces inward toward the microfluidic circuit 120) to support cell adhesion, viability and/or growth. The modification may include a coating of a synthetic or natural polymer. In some embodiments, the cover 110 and/or the support structure 104 can be transparent to light. The cover 110 may also include at least one material that is gas permeable (e.g., PDMS or PPS).

Figure 1A also shows a system 150 for operating and controlling microfluidic devices, such as microfluidic device 100. System 150 includes an electrical power source 192, an imaging device (incorporated within imaging module 164, and not explicitly illustrated in Figure 1A), and a tilting device (part of tilting module 166, and not explicitly illustrated in Figure 1A).

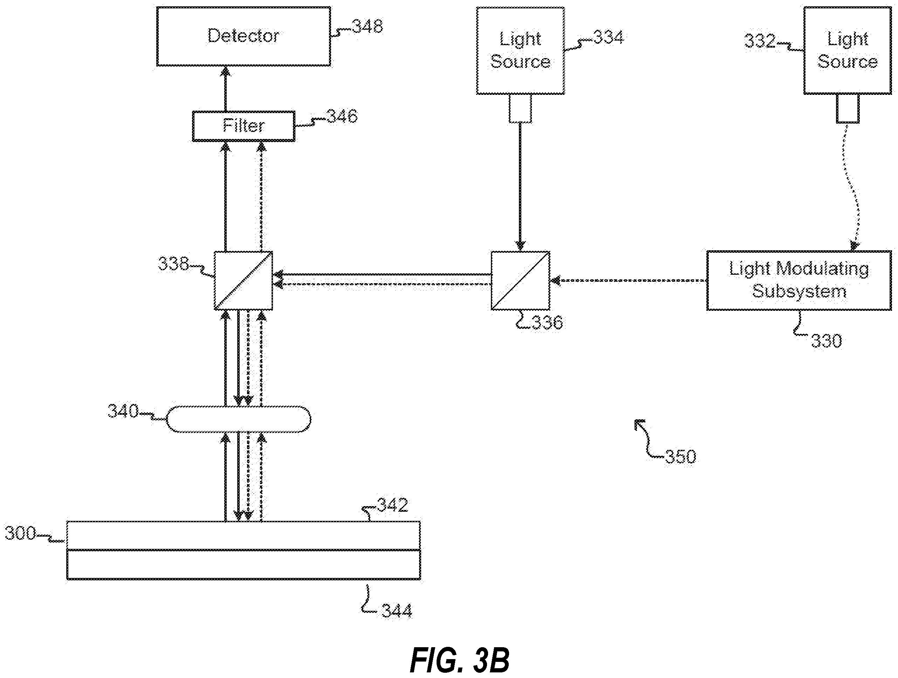

The electrical power source 192 can provide electric power to the microfluidic device 100 and/or tilting device 190, providing biasing voltages or currents as needed. The electrical power source 192 can, for example, comprise one or more alternating current (AC) and/or direct current (DC) voltage or current sources. The imaging device 194 (part of imaging module 164, discussed below) can comprise a device, such as a digital camera, for capturing images inside microfluidic circuit 120. In some instances, the imaging device 194 further comprises a detector having a fast frame rate and/or high sensitivity (e.g. for low light applications). The imaging device 194 can also include a mechanism for directing stimulating radiation and/or light beams into the microfluidic circuit 120 and collecting radiation and/or light beams reflected or emitted from the microfluidic circuit 120 (or micro-objects contained therein). The emitted light beams may be in the visible spectrum and may, e.g., include fluorescent emissions. The reflected light beams may include reflected emissions originating from an LED or a wide spectrum lamp, such as a mercury lamp (e.g. a high pressure mercury lamp) or a Xenon arc lamp. As discussed with respect to Figure 3B, the imaging device 194 may further include a microscope (or an optical train), which may or may not include an eyepiece.

System 150 further comprises a tilting device 190 (part of tilting module 166, discussed below) configured to rotate a microfluidic device 100 about one or more axes of rotation. In some embodiments, the tilting device 190 is configured to support and/or hold the enclosure 102 comprising the microfluidic circuit 120 about at least one axis such that the microfluidic device 100 (and thus the microfluidic circuit 120) can be held in a level orientation (i.e. at 0° relative to x- and y-axes), a vertical orientation (i.e. at 90° relative to the x-axis and/or the y-axis), or any orientation therebetween. The orientation of the microfluidic device 100 (and the microfluidic circuit 120) relative to an axis is referred to herein as the "tilt" of the microfluidic device 100 (and the microfluidic circuit 120). For example, the tilting device 190 can tilt the microfluidic device 100 at 0.1°, 0.2°, 0.3°, 0.4°, 0.5°, 0.6°, 0.7°, 0.8°, 0.9°, 1°, 2°, 3°, 4°, 5°, 10°, 15°, 20°, 25°, 30°, 35°, 40°, 45°, 50°, 55°, 60°, 65°, 70°, 75°, 80°, 90° relative to the x-axis or any degree therebetween. The level orientation (and thus the x-and y-axes) is defined as normal to a vertical axis defined by the force of gravity. The tilting device can also tilt the microfluidic device 100 (and the microfluidic circuit 120) to any degree greater than 90° relative to the x-axis and/or y-axis, or tilt the microfluidic device 100 (and the microfluidic circuit 120) 180° relative to the x-axis or the y-axis in order to fully invert the microfluidic device 100 (and the microfluidic circuit 120). Similarly, in some embodiments, the tilting device 190 tilts the microfluidic device 100 (and the microfluidic circuit 120) about an axis of rotation defined by flow path 106 or some other portion of microfluidic circuit 120.

In some instances, the microfluidic device 100 is tilted into a vertical orientation such that the flow path 106 is positioned above or below one or more sequestration pens. The term "above" as used herein in the context of microfluidic devices denotes that the flow path 106 is positioned higher than the one or more sequestration pens on a vertical axis defined by the force of gravity (i.e. an object in a sequestration pen above a flow path 106 would have a higher gravitational potential energy than an object in the flow path). The term "below" as used herein in the context of microfluidic devices denotes that the flow path 106 is positioned lower than the one or more sequestration pens on a vertical axis defined by the force of gravity (i.e. an object in a sequestration pen below a flow path 106 would have a lower gravitational potential energy than an object in the flow path).

In some instances, the tilting device 190 tilts the microfluidic device 100 about an axis that is parallel to the flow path 106. Moreover, the microfluidic device 100 can be tilted to an angle of less than 90° such that the flow path 106 is located above or below one or more sequestration pens without being located directly above or below the sequestration pens. In other instances, the tilting device 190 tilts the microfluidic device 100 about an axis perpendicular to the flow path 106. In still other instances, the tilting device 190 tilts the microfluidic device 100 about an axis that is neither parallel nor perpendicular to the flow path 106.

System 150 can further include a media source 178. The media source 178 (e.g., a container, reservoir, or the like) can comprise multiple sections or containers, each for holding a different fluidic medium 180. Thus, the media source 178 can be a device that is outside of and separate from the microfluidic device 100, as illustrated in Figure 1A. Alternatively, the media source 178 can be located in whole or in part inside the enclosure 102 of the microfluidic device 100. For example, the media source 178 can comprise reservoirs that are part of the microfluidic device 100.

Figure 1A also illustrates simplified block diagram depictions of examples of control and monitoring equipment 152 that constitute part of system 150 and can be utilized in conjunction with a microfluidic device 100. As shown, examples of such control and monitoring equipment 152 include a master controller 154 comprising a media module 160 for controlling the media source 178, a motive module 162 for controlling movement and/or selection of micro-objects (not shown) and/or medium (e.g., droplets of medium) in the microfluidic circuit 120, an imaging module 164 for controlling an imaging device 194 (e.g., a camera, microscope, light source or any combination thereof) for capturing images (e.g., digital images), and a tilting module 166 for controlling a tilting device 190. The control equipment 152 can also include other modules 168 for controlling, monitoring, or performing other functions with respect to the microfluidic device 100. As shown, the equipment 152 can further include a display device 170 and an input/output device 172.

The master controller 154 can comprise a control module 156 and a digital memory 158. The control module 156 can comprise, for example, a digital processor configured to operate in accordance with machine executable instructions (e.g., software, firmware, source code, or the like) stored as non-transitory data or signals in the memory 158. Alternatively, or in addition, the control module 156 can comprise hardwired digital circuitry and/or analog circuitry. The media module 160, motive module 162, imaging module 164, tilting module 166, and/or other modules 168 can be similarly configured. Thus, functions, processes acts, actions, or steps of a process discussed herein as being performed with respect to the microfluidic device 100 or any other microfluidic apparatus can be performed by any one or more of the master controller 154, media module 160, motive module 162, imaging module 164, tilting module 166, and/or other modules 168 configured as discussed above. Similarly, the master controller 154, media module 160, motive module 162, imaging module 164, tilting module 166, and/or other modules 168 may be communicatively coupled to transmit and receive data used in any function, process, act, action or step discussed herein.

The media module 160 controls the media source 178. For example, the media module 160 can control the media source 178 to input a selected fluidic medium 180 into the enclosure 102 (e.g., through an inlet port 107). The media module 160 can also control removal of media from the enclosure 102 (e.g., through an outlet port (not shown)). One or more media can thus be selectively input into and removed from the microfluidic circuit 120. The media module 160 can also control the flow of fluidic medium 180 in the flow path 106 inside the microfluidic circuit 120. For example, in some embodiments media module 160 stops the flow of media 180 in the flow path 106 and through the enclosure 102 prior to the tilting module 166 causing the tilting device 190 to tilt the microfluidic device 100 to a desired angle of incline.

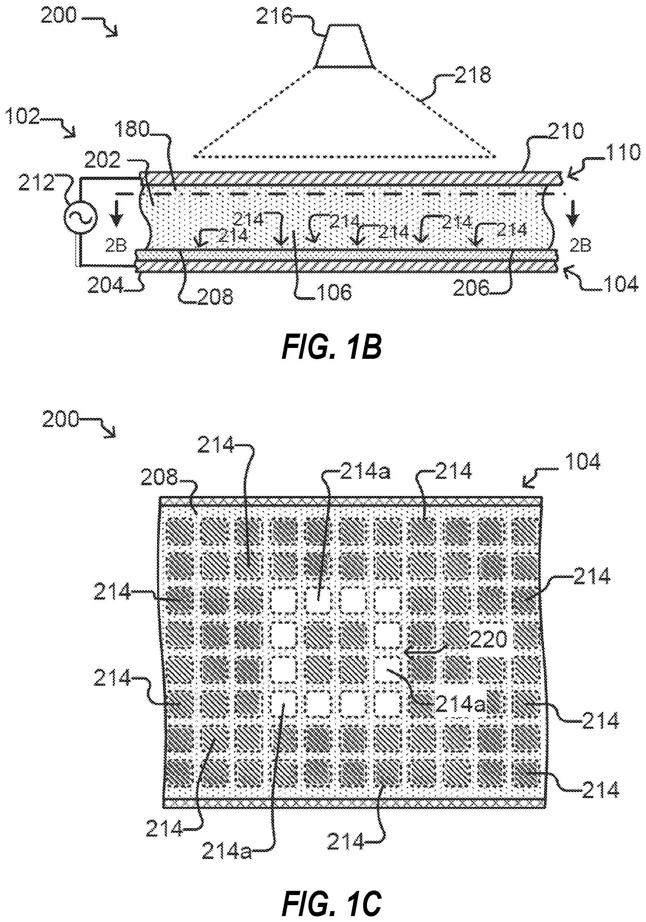

The motive module 162 can be configured to control selection, trapping, and movement of micro-objects (not shown) in the microfluidic circuit 120. As discussed below with respect to Figures 1B and 1C, the enclosure 102 can comprise a dielectrophoresis (DEP), optoelectronic tweezers (OET) and/or optoelectrowetting (OEW) configuration (not shown in Figure 1A), and the motive module 162 can control the activation of electrodes and/or transistors (e.g., phototransistors) to select and move micro-objects (not shown) and/or droplets of medium (not shown) in the flow path 106 and/or sequestration pens 124, 126, 128, 130.

The imaging module 164 can control the imaging device 194. For example, the imaging module 164 can receive and process image data from the imaging device 194. Image data from the imaging device 194 can comprise any type of information captured by the imaging device 194 (e.g., the presence or absence of micro-objects, droplets of medium, accumulation of label, such as fluorescent label, etc.). Using the information captured by the imaging device 194, the imaging module 164 can further calculate the position of objects (e.g., micro-objects, droplets of medium) and/or the rate of motion of such objects within the microfluidic device 100.

The tilting module 166 can control the tilting motions of tilting device 190. Alternatively, or in addition, the tilting module 166 can control the tilting rate and timing to optimize transfer of micro-objects to the one or more sequestration pens via gravitational forces. The tilting module 166 is communicatively coupled with the imaging module 164 to receive data describing the motion of micro-objects and/or droplets of medium in the microfluidic circuit 120. Using this data, the tilting module 166 may adjust the tilt of the microfluidic circuit 120 in order to adjust the rate at which micro-objects and/or droplets of medium move in the microfluidic circuit 120. The tilting module 166 may also use this data to iteratively adjust the position of a micro-object and/or droplet of medium in the microfluidic circuit 120.

In the example shown in Figure 1A, the microfluidic circuit 120 is illustrated as comprising a microfluidic channel 122 and sequestration pens 124, 126, 128, 130. Each pen comprises an opening to channel 122, but otherwise is enclosed such that the pens can substantially isolate micro-objects inside the pen from fluidic medium 180 and/or micro-objects in the flow path 106 of channel 122 or in other pens. The walls of the sequestration pen extend from the inner surface 109 of the base to the inside surface of the cover 110 to provide enclosure. The opening of the pen to the microfluidic channel 122 is oriented at an angle to the flow 106 of fluidic medium 180 such that flow 106 is not directed into the pens. The flow may be tangential or orthogonal to the plane of the opening of the pen. In some instances, pens 124, 126, 128, 130 are configured to physically corral one or more micro-objects within the microfluidic circuit 120. Sequestration pens in accordance with the present disclosure can comprise various shapes, surfaces and features that are optimized for use with DEP, OET, OEW, fluid flow, and/or gravitational forces, as will be discussed and shown in detail below.

The microfluidic circuit 120 may comprise any number of microfluidic sequestration pens. Although five sequestration pens are shown, microfluidic circuit 120 may have fewer or more sequestration pens. As shown, microfluidic sequestration pens 124, 126, 128, and 130 of microfluidic circuit 120 each comprise differing features and shapes which may provide one or more benefits useful for maintaining, isolating, assaying or culturing biological micro-objects. In some embodiments, the microfluidic circuit 120 comprises a plurality of identical microfluidic sequestration pens.

In the embodiment illustrated in Figure 1A, a single channel 122 and flow path 106 is shown. However, other embodiments may contain multiple channels 122, each configured to comprise a flow path 106. The microfluidic circuit 120 further comprises an inlet valve or port 107 in fluid communication with the flow path 106 and fluidic medium 180, whereby fluidic medium 180 can access channel 122 via the inlet port 107. In some instances, the flow path 106 comprises a single path. In some instances, the single path is arranged in a zigzag pattern whereby the flow path 106 travels across the microfluidic device 100 two or more times in alternating directions.

In some instances, microfluidic circuit 120 comprises a plurality of parallel channels 122 and flow paths 106, wherein the fluidic medium 180 within each flow path 106 flows in the same direction. In some instances, the fluidic medium within each flow path 106 flows in at least one of a forward or reverse direction. In some instances, a plurality of sequestration pens is configured (e.g., relative to a channel 122) such that the sequestration pens can be loaded with target micro-objects in parallel.

In some embodiments, microfluidic circuit 120 further comprises one or more micro-object traps 132. The traps 132 are generally formed in a wall forming the boundary of a channel 122, and may be positioned opposite an opening of one or more of the microfluidic sequestration pens 124, 126, 128, 130. In some embodiments, the traps 132 are configured to receive or capture a single micro-object from the flow path 106. In some embodiments, the traps 132 are configured to receive or capture a plurality of micro-objects from the flow path 106. In some instances, the traps 132 comprise a volume approximately equal to the volume of a single target micro-object.

The traps 132 may further comprise an opening which is configured to assist the flow of targeted micro-objects into the traps 132. In some instances, the traps 132 comprise an opening having a height and width that is approximately equal to the dimensions of a single target micro-object, whereby larger micro-objects are prevented from entering into the micro-object trap. The traps 132 may further comprise other features configured to assist in retention of targeted micro-objects within the trap 132. In some instances, the trap 132 is aligned with and situated on the opposite side of a channel 122 relative to the opening of a microfluidic sequestration pen, such that upon tilting the microfluidic device 100 about an axis parallel to the microfluidic channel 122, the trapped micro-object exits the trap 132 at a trajectory that causes the micro-object to fall into the opening of the sequestration pen. In some instances, the trap 132 comprises a side passage 134 that is smaller than the target micro-object in order to facilitate flow through the trap 132 and thereby increase the likelihood of capturing a micro-object in the trap 132.

In some embodiments, dielectrophoretic (DEP) forces are applied across the fluidic medium 180 (e.g., in the flow path and/or in the sequestration pens) via one or more electrodes (not shown) to manipulate, transport, separate and sort micro-objects located therein. For example, in some embodiments, DEP forces are applied to one or more portions of microfluidic circuit 120 in order to transfer a single micro-object from the flow path 106 into a desired microfluidic sequestration pen. In some embodiments, DEP forces are used to prevent a micro-object within a sequestration pen (e.g., sequestration pen 124, 126, 128, or 130) from being displaced therefrom. Further, in some embodiments, DEP forces are used to selectively remove a micro-object from a sequestration pen that was previously collected in accordance with the embodiments of the current disclosure. In some embodiments, the DEP forces comprise optoelectronic tweezer (OET) forces.

In other embodiments, optoelectrowetting (OEW) forces are applied to one or more positions in the support structure 104 (and/or the cover 110) of the microfluidic device 100 (e.g., positions helping to define the flow path and/or the sequestration pens) via one or more electrodes (not shown) to manipulate, transport, separate and sort droplets located in the microfluidic circuit 120. For example, in some embodiments, OEW forces are applied to one or more positions in the support structure 104 (and/or the cover 110) in order to transfer a single droplet from the flow path 106 into a desired microfluidic sequestration pen. In some embodiments, OEW forces are used to prevent a droplet within a sequestration pen (e.g., sequestration pen 124, 126, 128, or 130) from being displaced therefrom. Further, in some embodiments, OEW forces are used to selectively remove a droplet from a sequestration pen that was previously collected in accordance with the embodiments of the current disclosure.

In some embodiments, DEP and/or OEW forces are combined with other forces, such as flow and/or gravitational force, so as to manipulate, transport, separate and sort micro-objects and/or droplets within the microfluidic circuit 120. For example, the enclosure 102 can be tilted (e.g., by tilting device 190) to position the flow path 106 and micro-objects located therein above the microfluidic sequestration pens, and the force of gravity can transport the micro-objects and/or droplets into the pens. In some embodiments, the DEP and/or OEW forces can be applied prior to the other forces. In other embodiments, the DEP and/or OEW forces can be applied after the other forces. In still other instances, the DEP and/or OEW forces can be applied at the same time as the other forces or in an alternating manner with the other forces.

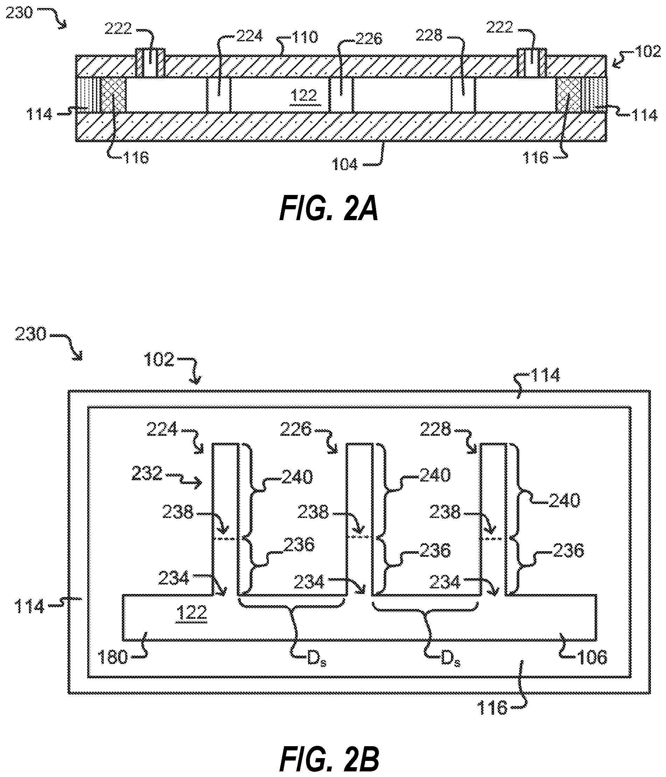

Figures 1B, 1C, and 2A-2H illustrates various embodiments of microfluidic devices that can be used in the practice of the embodiments of the present disclosure. Figure 1B depicts an embodiment in which the microfluidic device 200 is configured as an optically-actuated electrokinetic device. A variety of optically-actuated electrokinetic devices are known in the art, including devices having an optoelectronic tweezer (OET) configuration and devices having an opto-electrowetting (OEW) configuration. Examples of suitable OET configurations are illustrated in the following U.S. patent documents, each of which is incorporated herein by reference in its entirety: U.S. Patent No. RE 44,711 (Wu et al.) (originally issued as U.S. Patent No. 7,612,355); and U.S. Patent No. 7,956,339 (Ohta et al.). Examples of OEW configurations are illustrated in U.S. Patent No. 6,958,132 (Chiou et al.) and U.S. Patent Application Publication No. 2012/0024708 (Chiou et al.), both of which are incorporated by reference herein in their entirety. Yet another example of an optically-actuated electrokinetic device includes a combined OET/OEW configuration, examples of which are shown in U.S. Patent Publication Nos. 20150306598 (Khandros et al.) and 20150306599 (Khandros et al.) and their corresponding PCT Publications WO2015/164846 and WO2015/164847, all of which are incorporated herein by reference in their entirety.

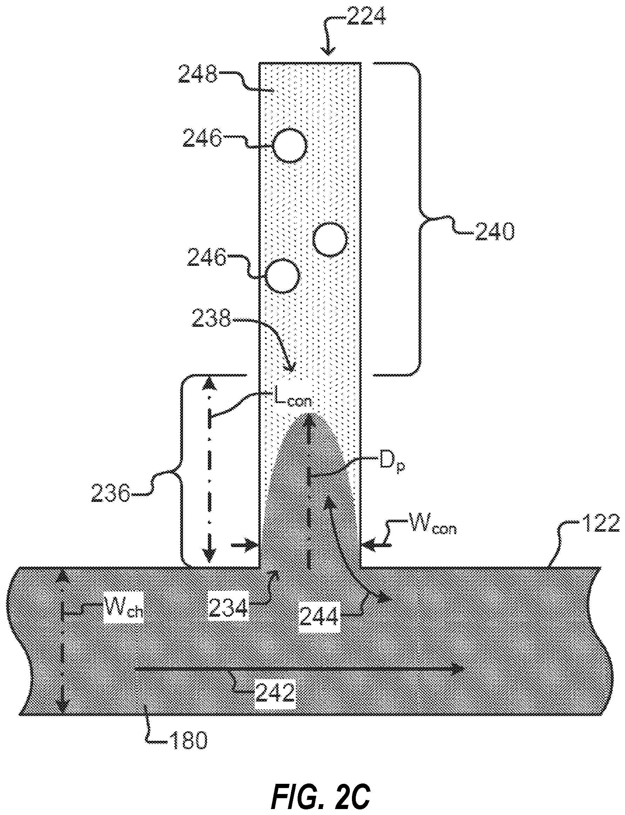

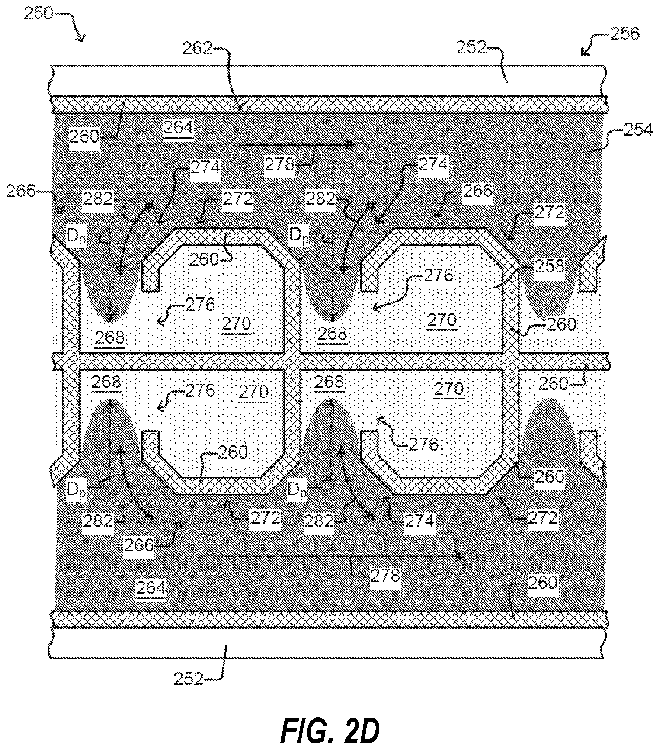

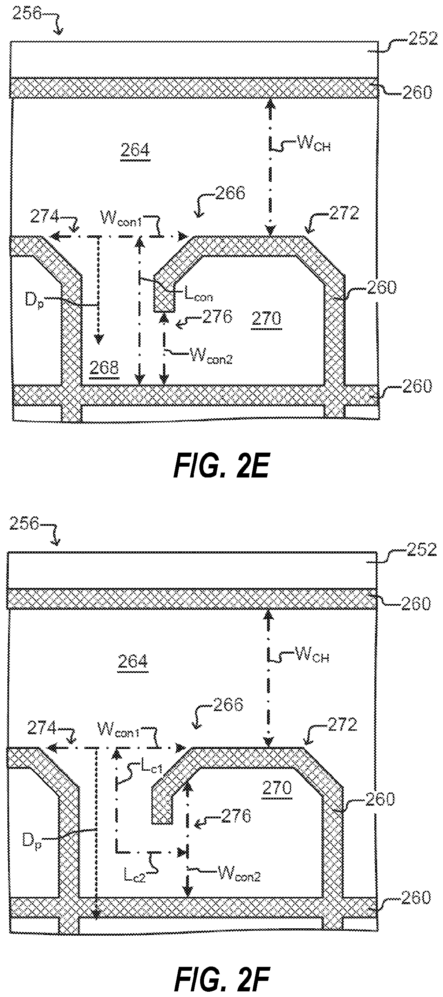

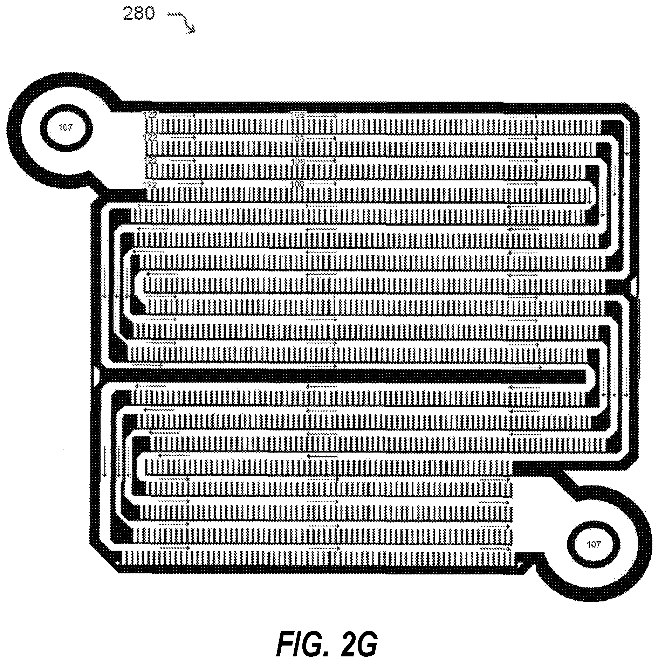

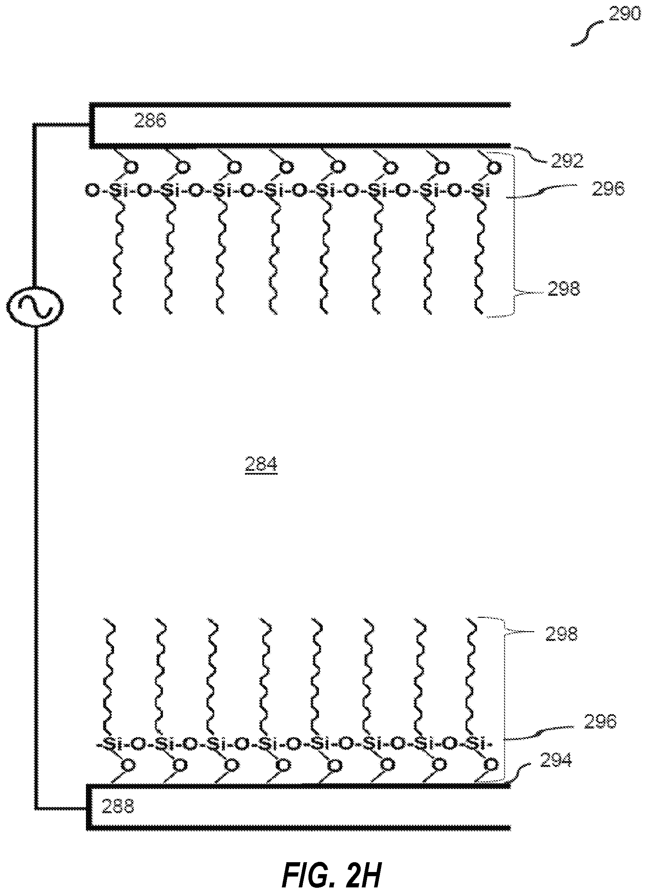

Examples of microfluidic devices having pens in which biological micro-objects can be placed, cultured, and/or monitored have been described, for example, in US 2014/0116881 (application no. 14/060,117, filed October 22, 2013), US 2015/0151298 (application no. 14/520,568, filed October 22, 2014), and US 2015/0165436 (application no. 14/521,447, filed October 22, 2014), each of which is incorporated herein by reference in its entirety. US application nos. 14/520,568 and 14/521,447 also describe exemplary methods of analyzing secretions of cells cultured in a microfluidic device. Each of the foregoing applications further describes microfluidic devices configured to produce dielectrophoretic (DEP) forces, such as optoelectronic tweezers (OET) or configured to provide opto-electro wetting (OEW). For example, the optoelectronic tweezers device illustrated in Figure 2 of US 2014/0116881 is an example of a device that can be utilized in embodiments of the present disclosure to select and move an individual biological micro-object or a group of biological micro-objects.

**Microfluidic device motive configurations.** As described above, the control and monitoring equipment of the system can comprise a motive module for selecting and moving objects, such as micro-objects or droplets, in the microfluidic circuit of a microfluidic device. The microfluidic device can have a variety of motive configurations, depending upon the type of object being moved and other considerations. For example, a dielectrophoresis (DEP) configuration can be utilized to select and move micro-objects in the microfluidic circuit. Thus, the support structure 104 and/or cover 110 of the microfluidic device 100 can comprise a DEP configuration for selectively inducing DEP forces on micro-objects in a fluidic medium 180 in the microfluidic circuit 120 and thereby select, capture, and/or move individual micro-objects or groups of micro-objects. Alternatively, the support structure 104 and/or cover 110 of the microfluidic device 100 can comprise an electrowetting (EW) configuration for selectively inducing EW forces on droplets in a fluidic medium 180 in the microfluidic circuit 120 and thereby select, capture, and/or move individual droplets or groups of droplets.

One example of a microfluidic device 200 comprising a DEP configuration is illustrated in Figures 1B and 1C. While for purposes of simplicity Figures 1B and 1C show a side cross-sectional view and a top cross-sectional view, respectively, of a portion of an enclosure 102 of the microfluidic device 200 having a region/chamber 202, it should be understood that the region/chamber 202 may be part of a fluidic circuit element having a more detailed structure, such as a growth chamber, a sequestration pen, a flow region, or a flow channel. Furthermore, the microfluidic device 200 may include other fluidic circuit elements. For example, the microfluidic device 200 can include a plurality of growth chambers or sequestration pens and/or one or more flow regions or flow channels, such as those described herein with respect to microfluidic device 100. A DEP configuration may be incorporated into any such fluidic circuit elements of the microfluidic device 200, or select portions thereof. It should be further appreciated that any of the above or below described microfluidic device components and system components may be incorporated in and/or used in combination with the microfluidic device 200. For example, system 150 including control and monitoring equipment 152, described above, may be used with microfluidic device 200, including one or more of the media module 160, motive module 162, imaging module 164, tilting module 166, and other modules 168.

As seen in Figure 1B, the microfluidic device 200 includes a support structure 104 having a bottom electrode 204 and an electrode activation substrate 206 overlying the bottom electrode 204, and a cover 110 having a top electrode 210, with the top electrode 210 spaced apart from the bottom electrode 204. The top electrode 210 and the electrode activation substrate 206 define opposing surfaces of the region/chamber 202. A medium 180 contained in the region/chamber 202 thus provides a resistive connection between the top electrode 210 and the electrode activation substrate 206. A power source 212 configured to be connected to the bottom electrode 204 and the top electrode 210 and create a biasing voltage between the electrodes, as required for the generation of DEP forces in the region/chamber 202, is also shown. The power source 212 can be, for example, an alternating current (AC) power source.

In certain embodiments, the microfluidic device 200 illustrated in Figures 1B and 1C can have an optically-actuated DEP configuration. Accordingly, changing patterns of light 218 from the light source 216, which may be controlled by the motive module 162, can selectively activate and deactivate changing patterns of DEP electrodes at regions 214 of the inner surface 208 of the electrode activation substrate 206. (Hereinafter the regions 214 of a microfluidic device having a DEP configuration are referred to as "DEP electrode regions.") As illustrated in Figure 1C, a light pattern 218 directed onto the inner surface 208 of the electrode activation substrate 206 can illuminate select DEP electrode regions 214a (shown in white) in a pattern, such as a square. The non-illuminated DEP electrode regions 214 (cross-hatched) are hereinafter referred to as "dark" DEP electrode regions 214. The relative electrical impedance through the DEP electrode activation substrate 206 (i.e., from the bottom electrode 204 up to the inner surface 208 of the electrode activation substrate 206 which interfaces with the medium 180 in the flow region 106) is greater than the relative electrical impedance through the medium 180 in the region/chamber 202 (i.e., from the inner surface 208 of the electrode activation substrate 206 to the top electrode 210 of the cover 110) at each dark DEP electrode region 214. An illuminated DEP electrode region 214a, however, exhibits a reduced relative impedance through the electrode activation substrate 206 that is less than the relative impedance through the medium 180 in the region/chamber 202 at each illuminated DEP electrode region 214a.

With the power source 212 activated, the foregoing DEP configuration creates an electric field gradient in the fluidic medium 180 between illuminated DEP electrode regions 214a and adjacent dark DEP electrode regions 214, which in turn creates local DEP forces that attract or repel nearby micro-objects (not shown) in the fluidic medium 180. DEP electrodes that attract or repel micro-objects in the fluidic medium 180 can thus be selectively activated and deactivated at many different such DEP electrode regions 214 at the inner surface 208 of the region/chamber 202 by changing light patterns 218 projected from a light source 216 into the microfluidic device 200. Whether the DEP forces attract or repel nearby micro-objects can depend on such parameters as the frequency of the power source 212 and the dielectric properties of the medium 180 and/or micro-objects (not shown).

The square pattern 220 of illuminated DEP electrode regions 214a illustrated in Figure 1C is an example only. Any pattern of the DEP electrode regions 214 can be illuminated (and thereby activated) by the pattern of light 218 projected into the microfluidic device 200, and the pattern of illuminated/activated DEP electrode regions 214 can be repeatedly changed by changing or moving the light pattern 218.

In some embodiments, the electrode activation substrate 206 can comprise or consist of a photoconductive material. In such embodiments, the inner surface 208 of the electrode activation substrate 206 can be featureless. For example, the electrode activation substrate 206 can comprise or consist of a layer of hydrogenated amorphous silicon (a-Si:H). The a-Si:H can comprise, for example, about 8% to 40% hydrogen (calculated as 100 * the number of hydrogen atoms / the total number of hydrogen and silicon atoms). The layer of a-Si:H can have a thickness of about 500 nm to about 2.0 µm. In such embodiments, the DEP electrode regions 214 can be created anywhere and in any pattern on the inner surface 208 of the electrode activation substrate 206, in accordance with the light pattern 218. The number and pattern of the DEP electrode regions 214 thus need not be fixed, but can correspond to the light pattern 218. Examples of microfluidic devices having a DEP configuration comprising a photoconductive layer such as discussed above have been described, for example, in U.S. Patent No. RE 44,711 (Wu et al.) (originally issued as U.S. Patent No. 7,612,355), the entire contents of which are incorporated herein by reference.

In other embodiments, the electrode activation substrate 206 can comprise a substrate comprising a plurality of doped layers, electrically insulating layers (or regions), and electrically conductive layers that form semiconductor integrated circuits, such as is known in semiconductor fields. For example, the electrode activation substrate 206 can comprise a plurality of phototransistors, including, for example, lateral bipolar phototransistors, each phototransistor corresponding to a DEP electrode region 214. Alternatively, the electrode activation substrate 206 can comprise electrodes (e.g., conductive metal electrodes) controlled by phototransistor switches, with each such electrode corresponding to a DEP electrode region 214. The electrode activation substrate 206 can include a pattern of such phototransistors or phototransistor-controlled electrodes. The pattern, for example, can be an array of substantially square phototransistors or phototransistor-controlled electrodes arranged in rows and columns, such as shown in Fig. 2B. Alternatively, the pattern can be an array of substantially hexagonal phototransistors or phototransistor-controlled electrodes that form a hexagonal lattice. Regardless of the pattern, electric circuit elements can form electrical connections between the DEP electrode regions 214 at the inner surface 208 of the electrode activation substrate 206 and the bottom electrode 210, and those electrical connections (i.e., phototransistors or electrodes) can be selectively activated and deactivated by the light pattern 218. When not activated, each electrical connection can have high impedance such that the relative impedance through the electrode activation substrate 206 (i.e., from the bottom electrode 204 to the inner surface 208 of the electrode activation substrate 206 which interfaces with the medium 180 in the region/chamber 202) is greater than the relative impedance through the medium 180 (i.e., from the inner surface 208 of the electrode activation substrate 206 to the top electrode 210 of the cover 110) at the corresponding DEP electrode region 214. When activated by light in the light pattern 218, however, the relative impedance through the electrode activation substrate 206 is less than the relative impedance through the medium 180 at each illuminated DEP electrode region 214, thereby activating the DEP electrode at the corresponding DEP electrode region 214 as discussed above. DEP electrodes that attract or repel micro-objects (not shown) in the medium 180 can thus be selectively activated and deactivated at many different DEP electrode regions 214 at the inner surface 208 of the electrode activation substrate 206 in the region/chamber 202 in a manner determined by the light pattern 218.

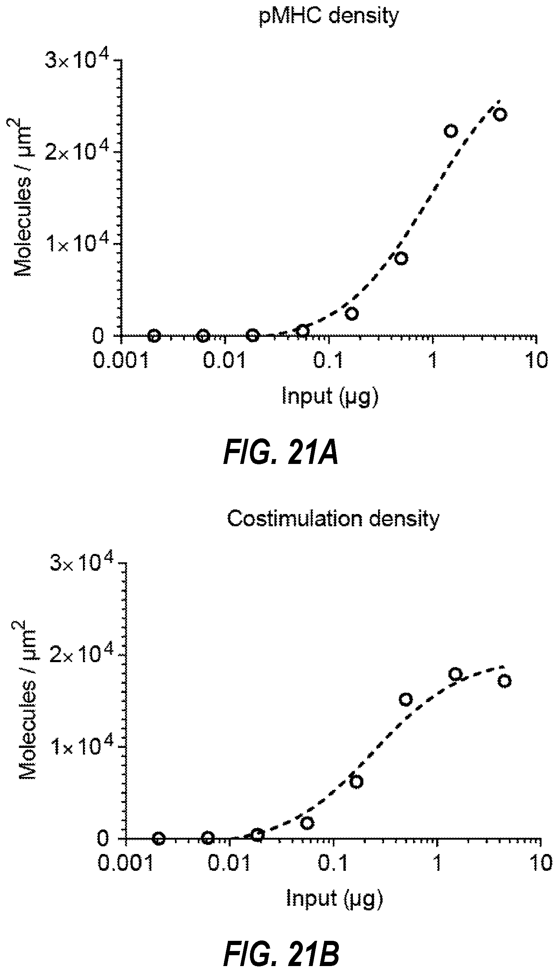

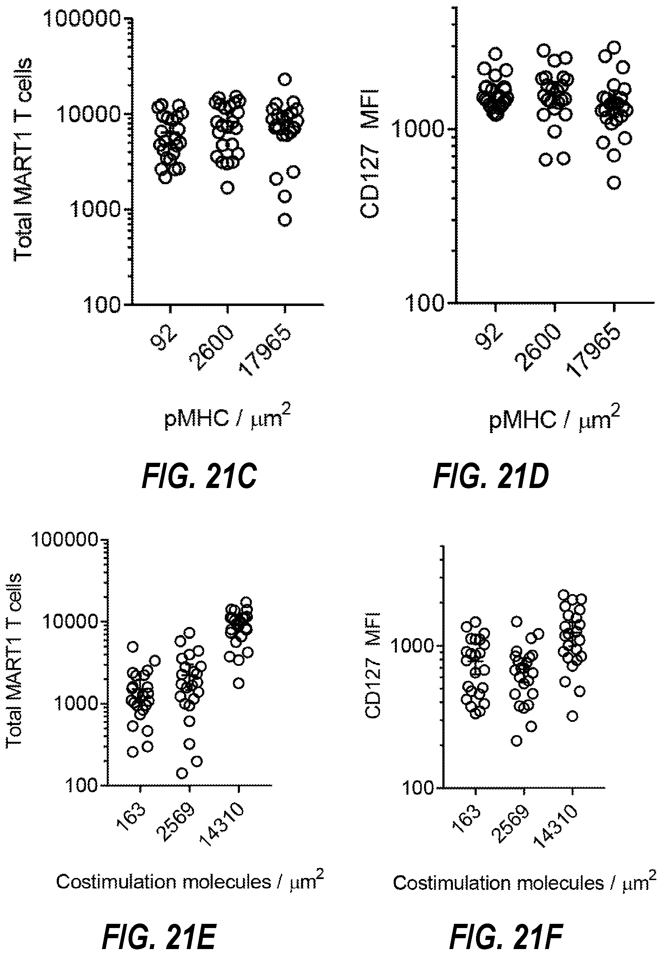

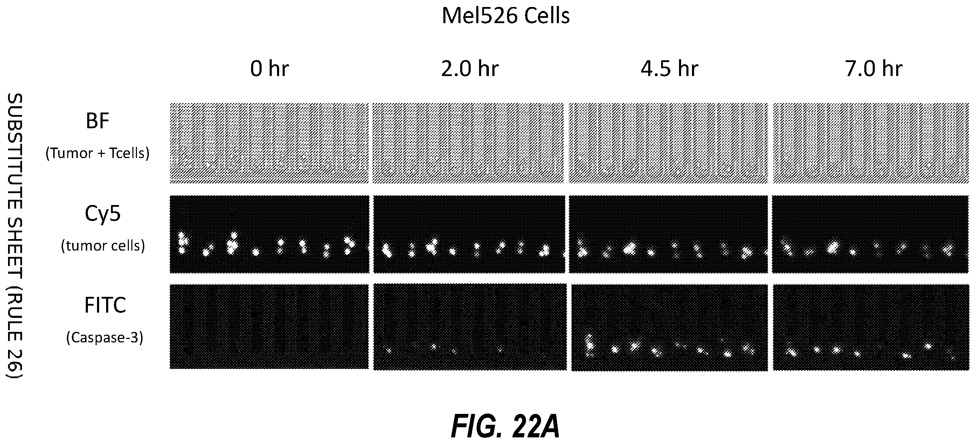

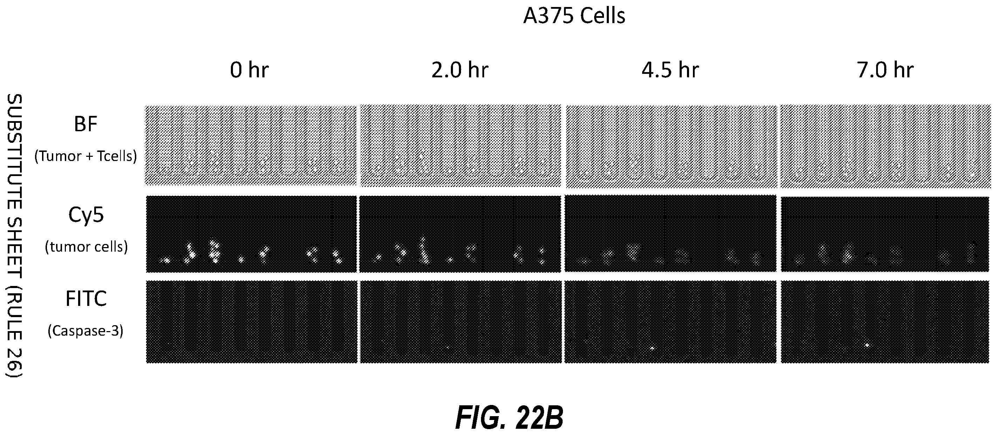

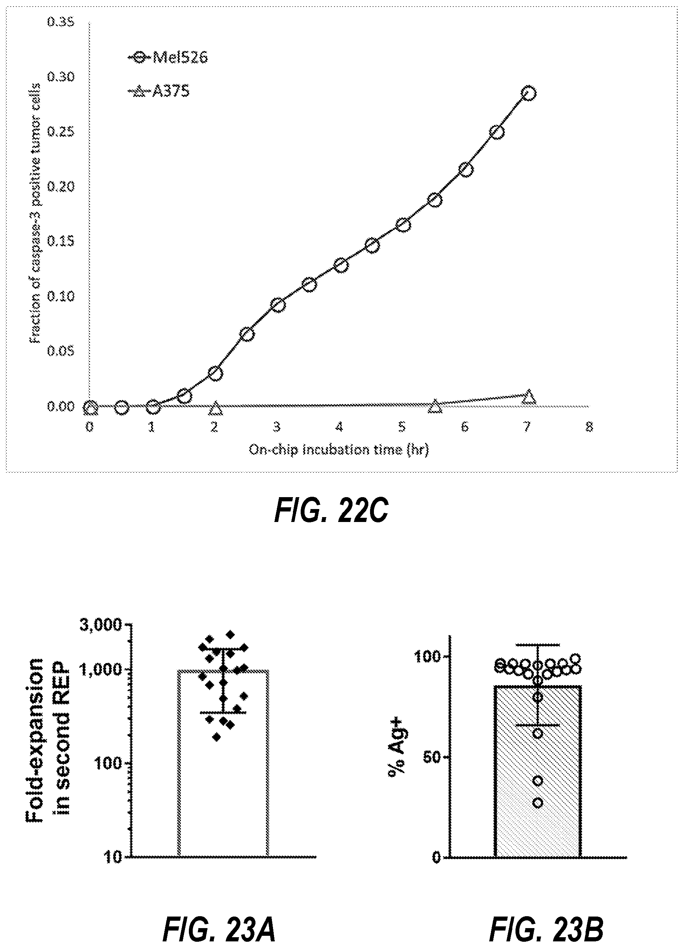

Examples of microfluidic devices having electrode activation substrates that comprise phototransistors have been described, for example, in U.S. Patent No. 7,956,339 (Ohta et al.) (see, e.g., device 300 illustrated in Figures 21 and 22, and descriptions thereof), the entire contents of which are incorporated herein by reference. Examples of microfluidic devices having electrode activation substrates that comprise electrodes controlled by phototransistor switches have been described, for example, in U.S. Patent Publication No. 2014/0124370 (Short et al.) (see, e.g., devices 200, 400, 500, 600, and 900 illustrated throughout the drawings, and descriptions thereof), the entire contents of which are incorporated herein by reference.

In some embodiments of a DEP configured microfluidic device, the top electrode 210 is part of a first wall (or cover 110) of the enclosure 102, and the electrode activation substrate 206 and bottom electrode 204 are part of a second wall (or support structure 104) of the enclosure 102. The region/chamber 202 can be between the first wall and the second wall. In other embodiments, the electrode 210 is part of the second wall (or support structure 104) and one or both of the electrode activation substrate 206 and/or the electrode 210 are part of the first wall (or cover 110). Moreover, the light source 216 can alternatively be used to illuminate the enclosure 102 from below.

With the microfluidic device 200 of Figures 1B-1C having a DEP configuration, the motive module 162 can select a micro-object (not shown) in the medium 180 in the region/chamber 202 by projecting a light pattern 218 into the microfluidic device 200 to activate a first set of one or more DEP electrodes at DEP electrode regions 214a of the inner surface 208 of the electrode activation substrate 206 in a pattern (e.g., square pattern 220) that surrounds and captures the micro-object. The motive module 162 can then move the in situ-generated captured micro-object by moving the light pattern 218 relative to the microfluidic device 200 to activate a second set of one or more DEP electrodes at DEP electrode regions 214. Alternatively, the microfluidic device 200 can be moved relative to the light pattern 218.

In other embodiments, the microfluidic device 200 can have a DEP configuration that does not rely upon light activation of DEP electrodes at the inner surface 208 of the electrode activation substrate 206. For example, the electrode activation substrate 206 can comprise selectively addressable and energizable electrodes positioned opposite to a surface including at least one electrode (e.g., cover 110). Switches (e.g., transistor switches in a semiconductor substrate) may be selectively opened and closed to activate or inactivate DEP electrodes at DEP electrode regions 214, thereby creating a net DEP force on a micro-object (not shown) in region/chamber 202 in the vicinity of the activated DEP electrodes. Depending on such characteristics as the frequency of the power source 212 and the dielectric properties of the medium (not shown) and/or micro-objects in the region/chamber 202, the DEP force can attract or repel a nearby micro-object. By selectively activating and deactivating a set of DEP electrodes (e.g., at a set of DEP electrodes regions 214 that forms a square pattern 220), one or more micro-objects in region/chamber 202 can be trapped and moved within the region/chamber 202. The motive module 162 in Figure 1A can control such switches and thus activate and deactivate individual ones of the DEP electrodes to select, trap, and move particular micro-objects (not shown) around the region/chamber 202. Microfluidic devices having a DEP configuration that includes selectively addressable and energizable electrodes are known in the art and have been described, for example, in U.S. Patent Nos. 6,294,063 (Becker et al.) and 6,942,776 (Medoro), the entire contents of which are incorporated herein by reference.

As yet another example, the microfluidic device 200 can have an electrowetting (EW) configuration, which can be in place of the DEP configuration or can be located in a portion of the microfluidic device 200 that is separate from the portion which has the DEP configuration. The EW configuration can be an opto-electrowetting configuration or an electrowetting on dielectric (EWOD) configuration, both of which are known in the art. In some EW configurations, the support structure 104 has an electrode activation substrate 206 sandwiched between a dielectric layer (not shown) and the bottom electrode 204. The dielectric layer can comprise a hydrophobic material and/or can be coated with a hydrophobic material, as described below. For microfluidic devices 200 that have an EW configuration, the inner surface 208 of the support structure 104 is the inner surface of the dielectric layer or its hydrophobic coating.

The dielectric layer (not shown) can comprise one or more oxide layers, and can have a thickness of about 50 nm to about 250 nm (e.g., about 125 nm to about 175 nm). In certain embodiments, the dielectric layer may comprise a layer of oxide, such as a metal oxide (e.g., aluminum oxide or hafnium oxide). In certain embodiments, the dielectric layer can comprise a dielectric material other than a metal oxide, such as silicon oxide or a nitride. Regardless of the exact composition and thickness, the dielectric layer can have an impedance of about 10 kOhms to about 50 kOhms.

In some embodiments, the surface of the dielectric layer that faces inward toward region/chamber 202 is coated with a hydrophobic material. The hydrophobic material can comprise, for example, fluorinated carbon molecules. Examples of fluorinated carbon molecules include perfluoro-polymers such as polytetrafluoroethylene (e.g., TEFLON^{®}) or poly(2,3-difluoromethylenyl-perfluorotetrahydrofuran) (e.g., CYTOP^{™}). Molecules that make up the hydrophobic material can be covalently bonded to the surface of the dielectric layer. For example, molecules of the hydrophobic material can be covalently bound to the surface of the dielectric layer by means of a linker such as a siloxane group, a phosphonic acid group, or a thiol group. Thus, in some embodiments, the hydrophobic material can comprise alkyl-terminated siloxane, alkyl-termination phosphonic acid, or alkyl-terminated thiol. The alkyl group can be long-chain hydrocarbons (e.g., having a chain of at least 10 carbons, or at least 16, 18, 20, 22, or more carbons). Alternatively, fluorinated (or perfluorinated) carbon chains can be used in place of the alkyl groups. Thus, for example, the hydrophobic material can comprise fluoroalkyl-terminated siloxane, fluoroalkyl-terminated phosphonic acid, or fluoroalkyl-terminated thiol. In some embodiments, the hydrophobic coating has a thickness of about 10 nm to about 50 nm. In other embodiments, the hydrophobic coating has a thickness of less than 10 nm (e.g., less than 5 nm, or about 1.5 to 3.0 nm).

In some embodiments, the cover 110 of a microfluidic device 200 having an electrowetting configuration is coated with a hydrophobic material (not shown) as well. The hydrophobic material can be the same hydrophobic material used to coat the dielectric layer of the support structure 104, and the hydrophobic coating can have a thickness that is substantially the same as the thickness of the hydrophobic coating on the dielectric layer of the support structure 104. Moreover, the cover 110 can comprise an electrode activation substrate 206 sandwiched between a dielectric layer and the top electrode 210, in the manner of the support structure 104. The electrode activation substrate 206 and the dielectric layer of the cover 110 can have the same composition and/or dimensions as the electrode activation substrate 206 and the dielectric layer of the support structure 104. Thus, the microfluidic device 200 can have two electrowetting surfaces.

In some embodiments, the electrode activation substrate 206 can comprise a photoconductive material, such as described above. Accordingly, in certain embodiments, the electrode activation substrate 206 can comprise or consist of a layer of hydrogenated amorphous silicon (a-Si:H). The a-Si:H can comprise, for example, about 8% to 40% hydrogen (calculated as 100 * the number of hydrogen atoms / the total number of hydrogen and silicon atoms). The layer of a-Si:H can have a thickness of about 500 nm to about 2.0 µm. Alternatively, the electrode activation substrate 206 can comprise electrodes (e.g., conductive metal electrodes) controlled by phototransistor switches, as described above. Microfluidic devices having an opto-electrowetting configuration are known in the art and/or can be constructed with electrode activation substrates known in the art. For example, U.S. Patent No. 6,958,132 (Chiou et al.), the entire contents of which are incorporated herein by reference, discloses opto-electrowetting configurations having a photoconductive material such as a-Si:H, while U.S. Patent Publication No. 2014/0124370 (Short et al.), referenced above, discloses electrode activation substrates having electrodes controlled by phototransistor switches.

The microfluidic device 200 thus can have an opto-electrowetting configuration, and light patterns 218 can be used to activate photoconductive EW regions or photoresponsive EW electrodes in the electrode activation substrate 206. Such activated EW regions or EW electrodes of the electrode activation substrate 206 can generate an electrowetting force at the inner surface 208 of the support structure 104 (i.e., the inner surface of the overlaying dielectric layer or its hydrophobic coating). By changing the light patterns 218 (or moving microfluidic device 200 relative to the light source 216) incident on the electrode activation substrate 206, droplets (e.g., containing an aqueous medium, solution, or solvent) contacting the inner surface 208 of the support structure 104 can be moved through an immiscible fluid (e.g., an oil medium) present in the region/chamber 202.

In other embodiments, microfluidic devices 200 can have an EWOD configuration, and the electrode activation substrate 206 can comprise selectively addressable and energizable electrodes that do not rely upon light for activation. The electrode activation substrate 206 thus can include a pattern of such electrowetting (EW) electrodes. The pattern, for example, can be an array of substantially square EW electrodes arranged in rows and columns, such as shown in Fig. 2B. Alternatively, the pattern can be an array of substantially hexagonal EW electrodes that form a hexagonal lattice. Regardless of the pattern, the EW electrodes can be selectively activated (or deactivated) by electrical switches (e.g., transistor switches in a semiconductor substrate). By selectively activating and deactivating EW electrodes in the electrode activation substrate 206, droplets (not shown) contacting the inner surface 208 of the overlaying dielectric layer or its hydrophobic coating can be moved within the region/chamber 202. The motive module 162 in Figure 1A can control such switches and thus activate and deactivate individual EW electrodes to select and move particular droplets around region/chamber 202. Microfluidic devices having a EWOD configuration with selectively addressable and energizable electrodes are known in the art and have been described, for example, in U.S. Patent No. 8,685,344 (Sundarsan et al.), the entire contents of which are incorporated herein by reference.

Regardless of the configuration of the microfluidic device 200, a power source 212 can be used to provide a potential (e.g., an AC voltage potential) that powers the electrical circuits of the microfluidic device 200. The power source 212 can be the same as, or a component of, the power source 192 referenced in Fig. 1. Power source 212 can be configured to provide an AC voltage and/or current to the top electrode 210 and the bottom electrode 204. For an AC voltage, the power source 212 can provide a frequency range and an average or peak power (e.g., voltage or current) range sufficient to generate net DEP forces (or electrowetting forces) strong enough to trap and move individual micro-objects (not shown) in the region/chamber 202, as discussed above, and/or to change the wetting properties of the inner surface 208 of the support structure 104 (i.e., the dielectric layer and/or the hydrophobic coating on the dielectric layer) in the region/chamber 202, as also discussed above. Such frequency ranges and average or peak power ranges are known in the art. See, e.g., US Patent No. 6,958,132 (Chiou et al.), US Patent No. RE44,711 (Wu et al.) (originally issued as US Patent No. 7,612,355), and US Patent Application Publication Nos. US2014/0124370 (Short et al.), US2015/0306598 (Khandros et al.), and US2015/0306599 (Khandros et al.).

**Sequestration pens.** Non-limiting examples of generic sequestration pens 224, 226, and 228 are shown within the microfluidic device 230 depicted in Figures 2A-2C. Each sequestration pen 224, 226, and 228 can comprise an isolation structure 232 defining an isolation region 240 and a connection region 236 fluidically connecting the isolation region 240 to a channel 122. The connection region 236 can comprise a proximal opening 234 to the microfluidic channel 122 and a distal opening 238 to the isolation region 240. The connection region 236 can be configured so that the maximum penetration depth of a flow of a fluidic medium (not shown) flowing from the microfluidic channel 122 into the sequestration pen 224, 226, 228 does not extend into the isolation region 240. Thus, due to the connection region 236, a micro-object (not shown) or other material (not shown) disposed in an isolation region 240 of a sequestration pen 224, 226, 228 can thus be isolated from, and not substantially affected by, a flow of medium 180 in the microfluidic channel 122.

The sequestration pens 224, 226, and 228 of Figures 2A-2C each have a single opening which opens directly to the microfluidic channel 122. The opening of the sequestration pen opens laterally from the microfluidic channel 122. The electrode activation substrate 206 underlays both the microfluidic channel 122 and the sequestration pens 224, 226, and 228. The upper surface of the electrode activation substrate 206 within the enclosure of a sequestration pen, forming the floor of the sequestration pen, is disposed at the same level or substantially the same level of the upper surface the of electrode activation substrate 206 within the microfluidic channel 122 (or flow region if a channel is not present), forming the floor of the flow channel (or flow region, respectively) of the microfluidic device. The electrode activation substrate 206 may be featureless or may have an irregular or patterned surface that varies from its highest elevation to its lowest depression by less than about 3 microns, 2.5 microns, 2 microns, 1.5 microns, 1 micron, 0.9 microns, 0.5 microns, 0.4 microns, 0.2 microns, 0.1 microns or less. The variation of elevation in the upper surface of the substrate across both the microfluidic channel 122 (or flow region) and sequestration pens may be less than about 3%, 2%, 1%. 0.9%, 0.8%, 0.5%, 0.3% or 0.1% of the height of the walls of the sequestration pen or walls of the microfluidic device. While described in detail for the microfluidic device 200, this also applies to any of the microfluidic devices 100, 230, 250, 280, 290 described herein.

The microfluidic channel 122 can thus be an example of a swept region, and the isolation regions 240 of the sequestration pens 224, 226, 228 can be examples of unswept regions. As noted, the microfluidic channel 122 and sequestration pens 224, 226, 228 can be configured to contain one or more fluidic media 180. In the example shown in Figures 2A-2B, the ports 222 are connected to the microfluidic channel 122 and allow a fluidic medium 180 to be introduced into or removed from the microfluidic device 230. Prior to introduction of the fluidic medium 180, the microfluidic device may be primed with a gas such as carbon dioxide gas. Once the microfluidic device 230 contains the fluidic medium 180, the flow 242 of fluidic medium 180 in the microfluidic channel 122 can be selectively generated and stopped. For example, as shown, the ports 222 can be disposed at different locations (e.g., opposite ends) of the microfluidic channel 122, and a flow 242 of medium can be created from one port 222 functioning as an inlet to another port 222 functioning as an outlet.

Figure 2C illustrates a detailed view of an example of a sequestration pen 224 according to the present disclosure. Examples of micro-objects 246 are also shown.

As is known, a flow 242 of fluidic medium 180 in a microfluidic channel 122 past a proximal opening 234 of sequestration pen 224 can cause a secondary flow 244 of the medium 180 into and/or out of the sequestration pen 224. To isolate micro-objects 246 in the isolation region 240 of a sequestration pen 224 from the secondary flow 244, the length L_{con} of the connection region 236 of the sequestration pen 224 (i.e., from the proximal opening 234 to the distal opening 238) should be greater than the penetration depth Dₚ of the secondary flow 244 into the connection region 236. The penetration depth Dₚ of the secondary flow 244 depends upon the velocity of the fluidic medium 180 flowing in the microfluidic channel 122 and various parameters relating to the configuration of the microfluidic channel 122 and the proximal opening 234 of the connection region 236 to the microfluidic channel 122. For a given microfluidic device, the configurations of the microfluidic channel 122 and the opening 234 will be fixed, whereas the rate of flow 242 of fluidic medium 180 in the microfluidic channel 122 will be variable. Accordingly, for each sequestration pen 224, a maximal velocity Vₘₐₓ for the flow 242 of fluidic medium 180 in channel 122 can be identified that ensures that the penetration depth Dₚ of the secondary flow 244 does not exceed the length L_{con} of the connection region 236. As long as the rate of the flow 242 of fluidic medium 180 in the microfluidic channel 122 does not exceed the maximum velocity Vₘₐₓ, the resulting secondary flow 244 can be limited to the microfluidic channel 122 and the connection region 236 and kept out of the isolation region 240. The flow 242 of medium 180 in the microfluidic channel 122 will thus not draw micro-objects 246 out of the isolation region 240. Rather, micro-objects 246 located in the isolation region 240 will stay in the isolation region 240 regardless of the flow 242 of fluidic medium 180 in the microfluidic channel 122.

Moreover, as long as the rate of flow 242 of medium 180 in the microfluidic channel 122 does not exceed Vₘₐₓ, the flow 242 of fluidic medium 180 in the microfluidic channel 122 will not move miscellaneous particles (e.g., microparticles and/or nanoparticles) from the microfluidic channel 122 into the isolation region 240 of a sequestration pen 224. Having the length L_{con} of the connection region 236 be greater than the maximum penetration depth Dₚ of the secondary flow 244 can thus prevent contamination of one sequestration pen 224 with miscellaneous particles from the microfluidic channel 122 or another sequestration pen (e.g., sequestration pens 226, 228 in Fig. 2D).

Because the microfluidic channel 122 and the connection regions 236 of the sequestration pens 224, 226, 228 can be affected by the flow 242 of medium 180 in the microfluidic channel 122, the microfluidic channel 122 and connection regions 236 can be deemed swept (or flow) regions of the microfluidic device 230. The isolation regions 240 of the sequestration pens 224, 226, 228, on the other hand, can be deemed unswept (or non-flow) regions. For example, components (not shown) in a first fluidic medium 180 in the microfluidic channel 122 can mix with a second fluidic medium 248 in the isolation region 240 substantially only by diffusion of components of the first medium 180 from the microfluidic channel 122 through the connection region 236 and into the second fluidic medium 248 in the isolation region 240. Similarly, components (not shown) of the second medium 248 in the isolation region 240 can mix with the first medium 180 in the microfluidic channel 122 substantially only by diffusion of components of the second medium 248 from the isolation region 240 through the connection region 236 and into the first medium 180 in the microfluidic channel 122. In some embodiments, the extent of fluidic medium exchange between the isolation region of a sequestration pen and the flow region by diffusion is greater than about 90%, 91%, 92%, 93%, 94% 95%, 96%, 97%, 98%, or greater than about 99% of fluidic exchange. The first medium 180 can be the same medium or a different medium than the second medium 248. Moreover, the first medium 180 and the second medium 248 can start out being the same, then become different (e.g., through conditioning of the second medium 248 by one or more cells in the isolation region 240, or by changing the medium 180 flowing through the microfluidic channel 122).

The maximum penetration depth Dₚ of the secondary flow 244 caused by the flow 242 of fluidic medium 180 in the microfluidic channel 122 can depend on a number of parameters, as mentioned above. Examples of such parameters include: the shape of the microfluidic channel 122 (e.g., the microfluidic channel can direct medium into the connection region 236, divert medium away from the connection region 236, or direct medium in a direction substantially perpendicular to the proximal opening 234 of the connection region 236 to the microfluidic channel 122); a width W_{ch} (or cross-sectional area) of the microfluidic channel 122 at the proximal opening 234; and a width W_{con} (or cross-sectional area) of the connection region 236 at the proximal opening 234; the velocity V of the flow 242 of fluidic medium 180 in the microfluidic channel 122; the viscosity of the first medium 180 and/or the second medium 248, or the like.

In some embodiments, the dimensions of the microfluidic channel 122 and sequestration pens 224, 226, 228 can be oriented as follows with respect to the vector of the flow 242 of fluidic medium 180 in the microfluidic channel 122: the microfluidic channel width W_{ch} (or cross-sectional area of the microfluidic channel 122) can be substantially perpendicular to the flow 242 of medium 180; the width W_{con} (or cross-sectional area) of the connection region 236 at opening 234 can be substantially parallel to the flow 242 of medium 180 in the microfluidic channel 122; and/or the length L_{con} of the connection region can be substantially perpendicular to the flow 242 of medium 180 in the microfluidic channel 122. The foregoing are examples only, and the relative position of the microfluidic channel 122 and sequestration pens 224, 226, 228 can be in other orientations with respect to each other.

As illustrated in Figure 2C, the width W_{con} of the connection region 236 can be uniform from the proximal opening 234 to the distal opening 238. The width W_{con} of the connection region 236 at the distal opening 238 can thus be any of the values identified herein for the width W_{con} of the connection region 236 at the proximal opening 234. Alternatively, the width W_{con} of the connection region 236 at the distal opening 238 can be larger than the width W_{con} of the connection region 236 at the proximal opening 234.

As illustrated in Figure 2C, the width of the isolation region 240 at the distal opening 238 can be substantially the same as the width W_{con} of the connection region 236 at the proximal opening 234. The width of the isolation region 240 at the distal opening 238 can thus be any of the values identified herein for the width W_{con} of the connection region 236 at the proximal opening 234. Alternatively, the width of the isolation region 240 at the distal opening 238 can be larger or smaller than the width W_{con} of the connection region 236 at the proximal opening 234. Moreover, the distal opening 238 may be smaller than the proximal opening 234 and the width W_{con} of the connection region 236 may be narrowed between the proximal opening 234 and distal opening 238. For example, the connection region 236 may be narrowed between the proximal opening and the distal opening, using a variety of different geometries (e.g. chamfering the connection region, beveling the connection region). Further, any part or subpart of the connection region 236 may be narrowed (e.g. a portion of the connection region adjacent to the proximal opening 234).

Figures 2D-2F depict another exemplary embodiment of a microfluidic device 250 containing a microfluidic circuit 262 and flow channels 264, which are variations of the respective microfluidic device 100, circuit 132 and channel 134 of Figure 1A. The microfluidic device 250 also has a plurality of sequestration pens 266 that are additional variations of the above-described sequestration pens 124, 126, 128, 130, 224, 226 or 228. In particular, it should be appreciated that the sequestration pens 266 of device 250 shown in Figures 2D-2F can replace any of the above-described sequestration pens 124, 126, 128, 130, 224, 226 or 228 in devices 100, 200, 230, 280, 290. Likewise, the microfluidic device 250 is another variant of the microfluidic device 100, and may also have the same or a different DEP configuration as the above-described microfluidic device 100, 200, 230, 280, 290, as well as any of the other microfluidic system components described herein.

The microfluidic device 250 of Figures 2D-2F comprises a support structure (not visible in Figures 2D-2F, but can be the same or generally similar to the support structure 104 of device 100 depicted in Figure 1A), a microfluidic circuit structure 256, and a cover (not visible in Figures 2D-2F, but can be the same or generally similar to the cover 122 of device 100 depicted in Figure 1A). The microfluidic circuit structure 256 includes a frame 252 and microfluidic circuit material 260, which can be the same as or generally similar to the frame 114 and microfluidic circuit material 116 of device 100 shown in Figure 1A. As shown in Figure 2D, the microfluidic circuit 262 defined by the microfluidic circuit material 260 can comprise multiple channels 264 (two are shown but there can be more) to which multiple sequestration pens 266 are fluidically connected.

Each sequestration pen 266 can comprise an isolation structure 272, an isolation region 270 within the isolation structure 272, and a connection region 268. From a proximal opening 274 at the microfluidic channel 264 to a distal opening 276 at the isolation structure 272, the connection region 268 fluidically connects the microfluidic channel 264 to the isolation region 270. Generally, in accordance with the above discussion of Figures 2B and 2C, a flow 278 of a first fluidic medium 254 in a channel 264 can create secondary flows 282 of the first medium 254 from the microfluidic channel 264 into and/or out of the respective connection regions 268 of the sequestration pens 266.

As illustrated in Figure 2E, the connection region 268 of each sequestration pen 266 generally includes the area extending between the proximal opening 274 to a channel 264 and the distal opening 276 to an isolation structure 272. The length L_{con} of the connection region 268 can be greater than the maximum penetration depth Dₚ of secondary flow 282, in which case the secondary flow 282 will extend into the connection region 268 without being redirected toward the isolation region 270 (as shown in Figure 2D). Alternatively, at illustrated in Figure 2F, the connection region 268 can have a length L_{con} that is less than the maximum penetration depth Dₚ, in which case the secondary flow 282 will extend through the connection region 268 and be redirected toward the isolation region 270. In this latter situation, the sum of lengths L_{c1} and L_{c2} of connection region 268 is greater than the maximum penetration depth Dₚ, so that secondary flow 282 will not extend into isolation region 270. Whether length L_{con} of connection region 268 is greater than the penetration depth Dₚ, or the sum of lengths L_{c1} and L_{c2} of connection region 268 is greater than the penetration depth Dₚ, a flow 278 of a first medium 254 in channel 264 that does not exceed a maximum velocity Vₘₐₓ will produce a secondary flow having a penetration depth Dₚ, and micro-objects (not shown but can be the same or generally similar to the micro-objects 246 shown in Figure 2C) in the isolation region 270 of a sequestration pen 266 will not be drawn out of the isolation region 270 by a flow 278 of first medium 254 in channel 264. Nor will the flow 278 in channel 264 draw miscellaneous materials (not shown) from channel 264 into the isolation region 270 of a sequestration pen 266. As such, diffusion is the only mechanism by which components in a first medium 254 in the microfluidic channel 264 can move from the microfluidic channel 264 into a second medium 258 in an isolation region 270 of a sequestration pen 266. Likewise, diffusion is the only mechanism by which components in a second medium 258 in an isolation region 270 of a sequestration pen 266 can move from the isolation region 270 to a first medium 254 in the microfluidic channel 264. The first medium 254 can be the same medium as the second medium 258, or the first medium 254 can be a different medium than the second medium 258. Alternatively, the first medium 254 and the second medium 258 can start out being the same, then become different, e.g., through conditioning of the second medium by one or more cells in the isolation region 270, or by changing the medium flowing through the microfluidic channel 264.

As illustrated in Figure 2E, the width W_{ch} of the microfluidic channels 264 (i.e., taken transverse to the direction of a fluid medium flow through the microfluidic channel indicated by arrows 278 in Figure 2D) in the microfluidic channel 264 can be substantially perpendicular to a width W_{con1} of the proximal opening 274 and thus substantially parallel to a width W_{con2} of the distal opening 276. The width W_{con1} of the proximal opening 274 and the width W_{con2} of the distal opening 276, however, need not be substantially perpendicular to each other. For example, an angle between an axis (not shown) on which the width W_{con1} of the proximal opening 274 is oriented and another axis on which the width W_{con2} of the distal opening 276 is oriented can be other than perpendicular and thus other than 90°. Examples of alternatively oriented angles include angles of: about 30° to about 90°, about 45° to about 90°, about 60° to about 90°, or the like.

In various embodiments of sequestration pens (e.g. 124, 126, 128, 130, 224, 226, 228, or 266), the isolation region (e.g. 240 or 270) is configured to contain a plurality of micro-objects. In other embodiments, the isolation region can be configured to contain only one, two, three, four, five, or a similar relatively small number of micro-objects. Accordingly, the volume of an isolation region can be, for example, at least 1x10⁶, 2x10⁶, 4x10⁶, 6x10⁶ cubic microns, or more.

In various embodiments of sequestration pens, the width W_{ch} of the microfluidic channel (e.g., 122) at a proximal opening (e.g. 234) can be about 50-1000 microns, 50-500 microns, 50-400 microns, 50-300 microns, 50-250 microns, 50-200 microns, 50-150 microns, 50-100 microns, 70-500 microns, 70-400 microns, 70-300 microns, 70-250 microns, 70-200 microns, 70-150 microns, 90-400 microns, 90-300 microns, 90-250 microns, 90-200 microns, 90-150 microns, 100-300 microns, 100-250 microns, 100-200 microns, 100-150 microns, or 100-120 microns. In some other embodiments, the width W_{ch} of the microfluidic channel (e.g., 122) at a proximal opening (e.g. 234) can be about 200-800 microns, 200-700 microns, or 200-600 microns. The foregoing are examples only, and the width W_{ch} of the microfluidic channel 122 can be any width within any of the endpoints listed above. Moreover, the W_{ch} of the microfluidic channel 122 can be selected to be in any of these widths in regions of the microfluidic channel other than at a proximal opening of a sequestration pen.

In some embodiments, a sequestration pen has a height of about 30 to about 200 microns, or about 50 to about 150 microns. In some embodiments, the sequestration pen has a cross-sectional area of about 1 x10⁴ - 3 x10⁸ square microns, 2 x10⁴ - 2 x10⁶ square microns, 4 x10⁴ - 1 x10⁶ square microns, 2 x10⁴- 5 x10⁵ square microns, 2 x10⁴- 1 x10⁵ square microns or about 2 x10⁵ - 2x10⁶ square microns.

In various embodiments of sequestration pens, the height H_{ch} of the microfluidic channel (e.g., 122) at a proximal opening (e.g., 234) can be a height within any of the following heights: 20-100 microns, 20-90 microns, 20-80 microns, 20-70 microns, 20-60 microns, 20-50 microns, 30-100 microns, 30-90 microns, 30-80 microns, 30-70 microns, 30-60 microns, 30-50 microns, 40-100 microns, 40-90 microns, 40-80 microns, 40-70 microns, 40-60 microns, or 40-50 microns. The foregoing are examples only, and the height H_{ch} of the microfluidic channel (e.g.,122) can be a height within any of the endpoints listed above. The height H_{ch} of the microfluidic channel 122 can be selected to be in any of these heights in regions of the microfluidic channel other than at a proximal opening of a sequestration pen.

In various embodiments of sequestration pens a cross-sectional area of the microfluidic channel ( e.g., 122) at a proximal opening (e.g., 234) can be about 500-50,000 square microns, 500-40,000 square microns, 500-30,000 square microns, 500-25,000 square microns, 500-20,000 square microns, 500-15,000 square microns, 500-10,000 square microns, 500-7,500 square microns, 500-5,000 square microns, 1,000-25,000 square microns, 1,000-20,000 square microns, 1,000-15,000 square microns, 1,000-10,000 square microns, 1,000-7,500 square microns, 1,000-5,000 square microns, 2,000-20,000 square microns, 2,000-15,000 square microns, 2,000-10,000 square microns, 2,000-7,500 square microns, 2,000-6,000 square microns, 3,000-20,000 square microns, 3,000-15,000 square microns, 3,000-10,000 square microns, 3,000-7,500 square microns, or 3,000 to 6,000 square microns. The foregoing are examples only, and the cross-sectional area of the microfluidic channel (e.g., 122) at a proximal opening (e.g., 234) can be any area within any of the endpoints listed above.

In various embodiments of sequestration pens, the length L_{con} of the connection region (e.g., 236) can be about 1-600 microns, 5-550 microns, 10-500 microns, 15-400 microns, 20-300 microns, 20-500 microns, 40-400 microns, 60-300 microns, 80-200 microns, or about 100-150 microns. The foregoing are examples only, and length L_{con} of a connection region (e.g., 236) can be in any length within any of the endpoints listed above.

In various embodiments of sequestration pens the width W_{can} of a connection region (e.g., 236) at a proximal opening (e.g., 234) can be about 20-500 microns, 20-400 microns, 20-300 microns, 20-200 microns, 20-150 microns, 20-100 microns, 20-80 microns, 20-60 microns, 30-400 microns, 30-300 microns, 30-200 microns, 30-150 microns, 30-100 microns, 30-80 microns, 30-60 microns, 40-300 microns, 40-200 microns, 40-150 microns, 40-100 microns, 40-80 microns, 40-60 microns, 50-250 microns, 50-200 microns, 50-150 microns, 50-100 microns, 50-80 microns, 60-200 microns, 60-150 microns, 60-100 microns, 60-80 microns, 70-150 microns, 70-100 microns, or 80-100 microns. The foregoing are examples only, and the width W_{con} of a connection region (e.g., 236) at a proximal opening (e.g., 234) can be different than the foregoing examples (e.g., any value within any of the endpoints listed above).

In various embodiments of sequestration pens, the width W_{con} of a connection region (e.g., 236) at a proximal opening (e.g., 234) can be at least as large as the largest dimension of a micro-object (e.g., biological cell which may be a T cell, or B cell) that the sequestration pen is intended for. The foregoing are examples only, and the width W_{con} of a connection region (e.g., 236) at a proximal opening (e.g., 234) can be different than the foregoing examples (e.g., a width within any of the endpoints listed above).

In various embodiments of sequestration pens, the width Wₚᵣ of a proximal opening of a connection region may be at least as large as the largest dimension of a micro-object (e.g., a biological micro-object such as a cell) that the sequestration pen is intended for. For example, the width Wₚᵣ may be about 50 microns, about 60 microns, about 100 microns, about 200 microns, about 300 microns or may be about 50-300 microns, about 50-200 microns, about 50 -100 microns, about 75- 150 microns, about 75-100 microns, or about 200- 300 microns.

In various embodiments of sequestration pens, a ratio of the length L_{con} of a connection region (e.g., 236) to a width W_{can} of the connection region (e.g., 236) at the proximal opening 234 can be greater than or equal to any of the following ratios: 0.5, 1.0, 1.5, 2.0, 2.5, 3.0, 3.5, 4.0, 4.5, 5.0, 6.0, 7.0, 8.0, 9.0, 10.0, or more. The foregoing are examples only, and the ratio of the length L_{con} of a connection region 236 to a width W_{con} of the connection region 236 at the proximal opening 234 can be different than the foregoing examples.

In various embodiments of microfluidic devices 100, 200, 23, 250, 280, 290, Vₘₐₓ can be set around 0.2, 0.5, 0.7, 1.0, 1.3, 1.5, 2.0, 2.5, 3.0, 3.5, 4.0, 4.5, 5.0, 5.5, 6.0, 6.7, 7.0, 7.5, 8.0, 8.5, 9.0, 10, 11, 12, 13, 14, or 15 microliters/sec.

In various embodiments of microfluidic devices having sequestration pens, the volume of an isolation region (e.g., 240) of a sequestration pen can be, for example, at least 5x10⁵, 8x10⁵, 1x10⁶, 2x10⁶, 4x10⁶, 6x10⁶, 8x10⁶, 1x10⁷, 5x10⁷, 1x10⁸, 5x10⁸, or 8x10⁸ cubic microns, or more. In various embodiments of microfluidic devices having sequestration pens, the volume of a sequestration pen may be about 5x10⁵, 6x10⁵, 8x10⁵, 1x10⁶, 2x10⁶, 4x10⁶, 8x10⁶, 1x10⁷, 3x10⁷, 5x10⁷, or about 8x10⁷ cubic microns, or more. In some other embodiments, the volume of a sequestration pen may be about 1 nanoliter to about 50 nanoliters, 2 nanoliters to about 25 nanoliters, 2 nanoliters to about 20 nanoliters, about 2 nanoliters to about 15 nanoliters, or about 2 nanoliters to about 10 nanoliters.

In various embodiment, the microfluidic device has sequestration pens configured as in any of the embodiments discussed herein where the microfluidic device has about 5 to about 10 sequestration pens, about 10 to about 50 sequestration pens, about 100 to about 500 sequestration pens; about 200 to about 1000 sequestration pens, about 500 to about 1500 sequestration pens, about 1000 to about 2000 sequestration pens, about 1000 to about 3500 sequestration pens, about 3000 to about 7000 sequestration pens, about 5000 to about 10,000 sequestration pens, about 9,000 to about 15,000 sequestration pens, or about 12, 000 to about 20,000 sequestration pens. The sequestration pens need not all be the same size and may include a variety of configurations (e.g., different widths, different features within the sequestration pen).

Figure 2G illustrates a microfluidic device 280 according to one embodiment. The microfluidic device 280 illustrated in Figure 2G is a stylized diagram of a microfluidic device 100. In practice the microfluidic device 280 and its constituent circuit elements (e.g. channels 122 and sequestration pens 128) would have the dimensions discussed herein. The microfluidic circuit 120 illustrated in Figure 2G has two ports 107, four distinct channels 122 and four distinct flow paths 106. The microfluidic device 280 further comprises a plurality of sequestration pens opening off of each channel 122. In the microfluidic device illustrated in Figure 2G, the sequestration pens have a geometry similar to the pens illustrated in Figure 2C and thus, have both connection regions and isolation regions. Accordingly, the microfluidic circuit 120 includes both swept regions (e.g. channels 122 and portions of the connection regions 236 within the maximum penetration depth Dₚ of the secondary flow 244) and non-swept regions (e.g. isolation regions 240 and portions of the connection regions 236 not within the maximum penetration depth Dₚ of the secondary flow 244).

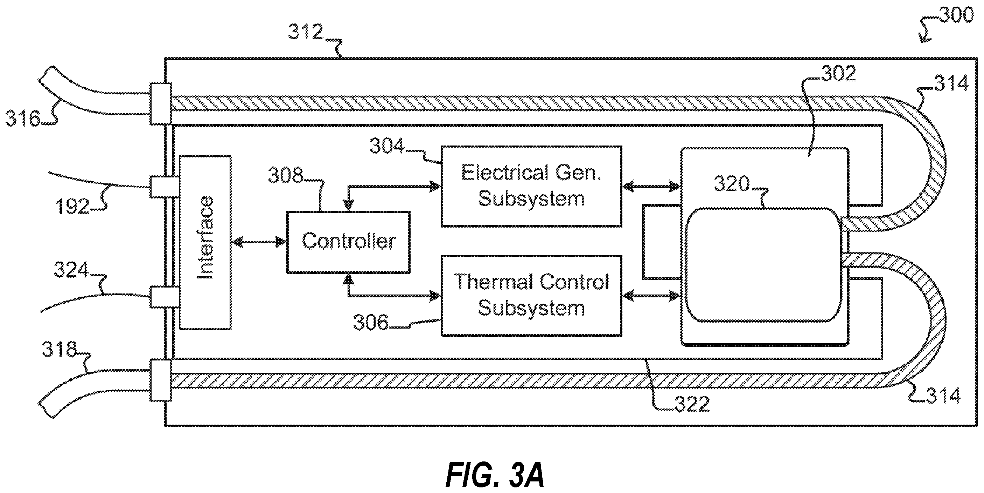

Figures 3A through 3B shows various embodiments of system 150 which can be used to operate and observe microfluidic devices (e.g. 100, 200, 230, 250, 280, 290) according to the present disclosure. As illustrated in Figure 3A, the system 150 can include a structure ("nest") 300 configured to hold a microfluidic device 100 (not shown), or any other microfluidic device described herein. The nest 300 can include a socket 302 capable of interfacing with the microfluidic device 320 (e.g., an optically-actuated electrokinetic device 100) and providing electrical connections from power source 192 to microfluidic device 320. The nest 300 can further include an integrated electrical signal generation subsystem 304. The electrical signal generation subsystem 304 can be configured to supply a biasing voltage to socket 302 such that the biasing voltage is applied across a pair of electrodes in the microfluidic device 320 when it is being held by socket 302. Thus, the electrical signal generation subsystem 304 can be part of power source 192. The ability to apply a biasing voltage to microfluidic device 320 does not mean that a biasing voltage will be applied at all times when the microfluidic device 320 is held by the socket 302. Rather, in most cases, the biasing voltage will be applied intermittently, e.g., only as needed to facilitate the generation of electrokinetic forces, such as dielectrophoresis or electro-wetting, in the microfluidic device 320.

As illustrated in Figure 3A, the nest 300 can include a printed circuit board assembly (PCBA) 322. The electrical signal generation subsystem 304 can be mounted on and electrically integrated into the PCBA 322. The exemplary support includes socket 302 mounted on PCBA 322, as well.

Typically, the electrical signal generation subsystem 304 will include a waveform generator (not shown). The electrical signal generation subsystem 304 can further include an oscilloscope (not shown) and/or a waveform amplification circuit (not shown) configured to amplify a waveform received from the waveform generator. The oscilloscope, if present, can be configured to measure the waveform supplied to the microfluidic device 320 held by the socket 302. In certain embodiments, the oscilloscope measures the waveform at a location proximal to the microfluidic device 320 (and distal to the waveform generator), thus ensuring greater accuracy in measuring the waveform actually applied to the device. Data obtained from the oscilloscope measurement can be, for example, provided as feedback to the waveform generator, and the waveform generator can be configured to adjust its output based on such feedback. An example of a suitable combined waveform generator and oscilloscope is the Red Pitaya^{™}.

In certain embodiments, the nest 300 further comprises a controller 308, such as a microprocessor used to sense and/or control the electrical signal generation subsystem 304. Examples of suitable microprocessors include the Arduino^{™} microprocessors, such as the Arduino Nano^{™}. The controller 308 may be used to perform functions and analysis or may communicate with an external master controller 154 (shown in Figure 1A) to perform functions and analysis. In the embodiment illustrated in Figure 3A the controller 308 communicates with a master controller 154 through an interface 310 (e.g., a plug or connector).

In some embodiments, the nest 300 can comprise an electrical signal generation subsystem 304 comprising a Red Pitaya^{™} waveform generator/oscilloscope unit ("Red Pitaya unit") and a waveform amplification circuit that amplifies the waveform generated by the Red Pitaya unit and passes the amplified voltage to the microfluidic device 100. In some embodiments, the Red Pitaya unit is configured to measure the amplified voltage at the microfluidic device 320 and then adjust its own output voltage as needed such that the measured voltage at the microfluidic device 320 is the desired value. In some embodiments, the waveform amplification circuit can have a +6.5V to -6.5V power supply generated by a pair of DC-DC converters mounted on the PCBA 322, resulting in a signal of up to 13 Vpp at the microfluidic device 100.

As illustrated in Figure 3A, the support structure 300 (e.g., nest) can further include a thermal control subsystem 306. The thermal control subsystem 306 can be configured to regulate the temperature of microfluidic device 320 held by the support structure 300. For example, the thermal control subsystem 306 can include a Peltier thermoelectric device (not shown) and a cooling unit (not shown). The Peltier thermoelectric device can have a first surface configured to interface with at least one surface of the microfluidic device 320. The cooling unit can be, for example, a cooling block (not shown), such as a liquid-cooled aluminum block. A second surface of the Peltier thermoelectric device (e.g., a surface opposite the first surface) can be configured to interface with a surface of such a cooling block. The cooling block can be connected to a fluidic path 314 configured to circulate cooled fluid through the cooling block. In the embodiment illustrated in Figure 3A, the support structure 300 comprises an inlet 316 and an outlet 318 to receive cooled fluid from an external reservoir (not shown), introduce the cooled fluid into the fluidic path 314 and through the cooling block, and then return the cooled fluid to the external reservoir. In some embodiments, the Peltier thermoelectric device, the cooling unit, and/or the fluidic path 314 can be mounted on a casing 312of the support structure 300. In some embodiments, the thermal control subsystem 306 is configured to regulate the temperature of the Peltier thermoelectric device so as to achieve a target temperature for the microfluidic device 320. Temperature regulation of the Peltier thermoelectric device can be achieved, for example, by a thermoelectric power supply, such as a Pololu^{™} thermoelectric power supply (Pololu Robotics and Electronics Corp.). The thermal control subsystem 306 can include a feedback circuit, such as a temperature value provided by an analog circuit. Alternatively, the feedback circuit can be provided by a digital circuit.

In some embodiments, the nest 300 can include a thermal control subsystem 306 with a feedback circuit that is an analog voltage divider circuit (not shown) which includes a resistor (e.g., with resistance 1 kOhm+/-0.1 %, temperature coefficient +/-0.02 ppm/C0) and a NTC thermistor (e.g., with nominal resistance 1 kOhm+/-0.01 %). In some instances, the thermal control subsystem 306 measures the voltage from the feedback circuit and then uses the calculated temperature value as input to an on-board PID control loop algorithm. Output from the PID control loop algorithm can drive, for example, both a directional and a pulse-width-modulated signal pin on a Pololu^{™} motor drive (not shown) to actuate the thermoelectric power supply, thereby controlling the Peltier thermoelectric device.

The nest 300 can include a serial port 324 which allows the microprocessor of the controller 308 to communicate with an external master controller 154 via the interface 310 (not shown). In addition, the microprocessor of the controller 308 can communicate (e.g., via a Plink tool (not shown)) with the electrical signal generation subsystem 304 and thermal control subsystem 306. Thus, via the combination of the controller 308, the interface 310, and the serial port 324, the electrical signal generation subsystem 304 and the thermal control subsystem 306 can communicate with the external master controller 154. In this manner, the master controller 154 can, among other things, assist the electrical signal generation subsystem 304 by performing scaling calculations for output voltage adjustments. A Graphical User Interface (GUI) (not shown) provided via a display device 170 coupled to the external master controller 154, can be configured to plot temperature and waveform data obtained from the thermal control subsystem 306 and the electrical signal generation subsystem 304, respectively. Alternatively, or in addition, the GUI can allow for updates to the controller 308, the thermal control subsystem 306, and the electrical signal generation subsystem 304.

As discussed above, system 150 can include an imaging device 194. In some embodiments, the imaging device 194 comprises a light modulating subsystem 330 (See Figure 3B). The light modulating subsystem 330 can include a digital mirror device (DMD) or a microshutter array system (MSA), either of which can be configured to receive light from a light source 332 and transmits a subset of the received light into an optical train of microscope 350. Alternatively, the light modulating subsystem 330 can include a device that produces its own light (and thus dispenses with the need for a light source 332), such as an organic light emitting diode display (OLED), a liquid crystal on silicon (LCOS) device, a ferroelectric liquid crystal on silicon device (FLCOS), or a transmissive liquid crystal display (LCD). The light modulating subsystem 330 can be, for example, a projector. Thus, the light modulating subsystem 330 can be capable of emitting both structured and unstructured light. In certain embodiments, imaging module 164 and/or motive module 162 of system 150 can control the light modulating subsystem 330.

In certain embodiments, the imaging device 194 further comprises a microscope 350. In such embodiments, the nest 300 and light modulating subsystem 330 can be individually configured to be mounted on the microscope 350. The microscope 350 can be, for example, a standard research-grade light microscope or fluorescence microscope. Thus, the nest 300 can be configured to be mounted on the stage 344 of the microscope 350 and/or the light modulating subsystem 330 can be configured to mount on a port of microscope 350. In other embodiments, the nest 300 and the light modulating subsystem 330 described herein can be integral components of microscope 350.

In certain embodiments, the microscope 350 can further include one or more detectors 348. In some embodiments, the detector 348 is controlled by the imaging module 164. The detector 348 can include an eye piece, a charge-coupled device (CCD), a camera (e.g., a digital camera), or any combination thereof. If at least two detectors 348 are present, one detector can be, for example, a fast-frame-rate camera while the other detector can be a high sensitivity camera. Furthermore, the microscope 350 can include an optical train configured to receive reflected and/or emitted light from the microfluidic device 320 and focus at least a portion of the reflected and/or emitted light on the one or more detectors 348. The optical train of the microscope can also include different tube lenses (not shown) for the different detectors, such that the final magnification on each detector can be different.

In certain embodiments, imaging device 194 is configured to use at least two light sources. For example, a first light source 332 can be used to produce structured light (e.g., via the light modulating subsystem 330) and a second light source 334 can be used to provide unstructured light. The first light source 332 can produce structured light for optically-actuated electrokinesis and/or fluorescent excitation, and the second light source 334 can be used to provide bright field illumination. In these embodiments, the motive module 164 can be used to control the first light source 332 and the imaging module 164 can be used to control the second light source 334. The optical train of the microscope 350 can be configured to (1) receive structured light from the light modulating subsystem 330 and focus the structured light on at least a first region in a microfluidic device, such as an optically-actuated electrokinetic device, when the device is being held by the nest 300, and (2) receive reflected and/or emitted light from the microfluidic device and focus at least a portion of such reflected and/or emitted light onto detector 348. The optical train can be further configured to receive unstructured light from a second light source and focus the unstructured light on at least a second region of the microfluidic device, when the device is held by the nest 300. In certain embodiments, the first and second regions of the microfluidic device can be overlapping regions. For example, the first region can be a subset of the second region. In other embodiments, the second light source 334 may additionally or alternatively include a laser, which may have any suitable wavelength of light. The representation of the optical system shown in Figure 3B is a schematic representation only, and the optical system may include additional filters, notch filters, lenses and the like. When the second light source 334 includes one or more light source(s) for brightfield and/or fluorescent excitation, as well as laser illumination the physical arrangement of the light source(s) may vary from that shown in Figure 3B, and the laser illumination may be introduced at any suitable physical location within the optical system. The schematic locations of light source 334 and light source 332/light modulating subsystem 330 may be interchanged as well.

In Figure 3B, the first light source 332 is shown supplying light to a light modulating subsystem 330, which provides structured light to the optical train of the microscope 350 of system 355 (not shown). The second light source 334 is shown providing unstructured light to the optical train via a beam splitter 336. Structured light from the light modulating subsystem 330 and unstructured light from the second light source 334 travel from the beam splitter 336 through the optical train together to reach a second beam splitter (or dichroic filter 338, depending on the light provided by the light modulating subsystem 330), where the light gets reflected down through the objective 336 to the sample plane 342. Reflected and/or emitted light from the sample plane 342 then travels back up through the objective 340, through the beam splitter and/or dichroic filter 338, and to a dichroic filter 346. Only a fraction of the light reaching dichroic filter 346 passes through and reaches the detector 348.

In some embodiments, the second light source 334 emits blue light. With an appropriate dichroic filter 346, blue light reflected from the sample plane 342 is able to pass through dichroic filter 346 and reach the detector 348. In contrast, structured light coming from the light modulating subsystem 330 gets reflected from the sample plane 342, but does not pass through the dichroic filter 346. In this example, the dichroic filter 346 is filtering out visible light having a wavelength longer than 495 nm. Such filtering out of the light from the light modulating subsystem 330 would only be complete (as shown) if the light emitted from the light modulating subsystem did not include any wavelengths shorter than 495 nm. In practice, if the light coming from the light modulating subsystem 330 includes wavelengths shorter than 495 nm (e.g., blue wavelengths), then some of the light from the light modulating subsystem would pass through filter 346 to reach the detector 348. In such an embodiment, the filter 346 acts to change the balance between the amount of light that reaches the detector 348 from the first light source 332 and the second light source 334. This can be beneficial if the first light source 332 is significantly stronger than the second light source 334. In other embodiments, the second light source 334 can emit red light, and the dichroic filter 346 can filter out visible light other than red light (e.g., visible light having a wavelength shorter than 650 nm).

**Coating solutions and coating agents.** Without intending to be limited by theory, maintenance of a biological micro-object (e.g., a biological cell) within a microfluidic device (e.g., a DEP-configured and/or EW-configured microfluidic device) may be facilitated (i.e., the biological micro-object exhibits increased viability, greater expansion and/or greater portability within the microfluidic device) when at least one or more inner surfaces of the microfluidic device have been conditioned or coated so as to present a layer of organic and/or hydrophilic molecules that provides the primary interface between the microfluidic device and biological micro-object(s) maintained therein. In some embodiments, one or more of the inner surfaces of the microfluidic device (e.g. the inner surface of the electrode activation substrate of a DEP-configured microfluidic device, the cover of the microfluidic device, and/or the surfaces of the circuit material) may be treated with or modified by a coating solution and/or coating agent to generate the desired layer of organic and/or hydrophilic molecules.

The coating may be applied before or after introduction of biological micro-object(s), or may be introduced concurrently with the biological micro-object(s). In some embodiments, the biological micro-object(s) may be imported into the microfluidic device in a fluidic medium that includes one or more coating agents. In other embodiments, the inner surface(s) of the microfluidic device (e.g., a DEP-configured microfluidic device) are treated or "primed" with a coating solution comprising a coating agent prior to introduction of the biological micro-object(s) into the microfluidic device.

In some embodiments, at least one surface of the microfluidic device includes a coating material that provides a layer of organic and/or hydrophilic molecules suitable for maintenance and/or expansion of biological micro-object(s) (e.g. provides a conditioned surface as described below). In some embodiments, substantially all the inner surfaces of the microfluidic device include the coating material. The coated inner surface(s) may include the surface of a flow region (e.g., channel), chamber, or sequestration pen, or a combination thereof. In some embodiments, each of a plurality of sequestration pens has at least one inner surface coated with coating materials. In other embodiments, each of a plurality of flow regions or channels has at least one inner surface coated with coating materials. In some embodiments, at least one inner surface of each of a plurality of sequestration pens and each of a plurality of channels is coated with coating materials.

**Coating agent/Solution.** Any convenient coating agent/coating solution can be used, including but not limited to: serum or serum factors, bovine serum albumin (BSA), polymers, detergents, enzymes, and any combination thereof.

**Polymer-based coating materials.** The at least one inner surface may include a coating material that comprises a polymer. The polymer may be covalently or non-covalently bound (or may be non-specifically adhered) to the at least one surface. The polymer may have a variety of structural motifs, such as found in block polymers (and copolymers), star polymers (star copolymers), and graft or comb polymers (graft copolymers), all of which may be suitable for the methods disclosed herein.

The polymer may include a polymer including alkylene ether moieties. A wide variety of alkylene ether containing polymers may be suitable for use in the microfluidic devices described herein. One non-limiting exemplary class of alkylene ether containing polymers are amphiphilic nonionic block copolymers which include blocks of polyethylene oxide (PEO) and polypropylene oxide (PPO) subunits in differing ratios and locations within the polymer chain. Pluronic^{®} polymers (BASF) are block copolymers of this type and are known in the art to be suitable for use when in contact with living cells. The polymers may range in average molecular mass M_{w} from about 2000Da to about 20KDa. In some embodiments, the PEO-PPO block copolymer can have a hydrophilic-lipophilic balance (HLB) greater than about 10 (e.g. 12-18). Specific Pluronic^{®} polymers useful for yielding a coated surface include Pluronic^{®} L44, L64, P85, and F127 (including F127NF). Another class of alkylene ether containing polymers is polyethylene glycol (PEG M_{w} <100,000Da) or alternatively polyethylene oxide (PEO, M_{w}>100,000). In some embodiments, a PEG may have an M_{w} of about 88Da, 100Da, 132Da, 176Da, 200Da, 220Da, 264Da, 308Da, 352Da, 396Da, 440Da, 500Da, 600Da, 700Da, 800Da, 900Da, 1000Da, 1500Da, 2000Da, 5000Da, 10,000Da or 20,000Da, or may have a Mw that falls within a range defined by any two of the foregoing values.

In other embodiments, the coating material may include a polymer containing carboxylic acid moieties. The carboxylic acid subunit may be an alkyl, alkenyl or aromatic moiety containing subunit. One non-limiting example is polylactic acid (PLA). In other embodiments, the coating material may include a polymer containing phosphate moieties, either at a terminus of the polymer backbone or pendant from the backbone of the polymer. In yet other embodiments, the coating material may include a polymer containing sulfonic acid moieties. The sulfonic acid subunit may be an alkyl, alkenyl or aromatic moiety containing subunit. One non-limiting example is polystyrene sulfonic acid (PSSA) or polyanethole sulfonic acid. In further embodiments, the coating material may include a polymer including amine moieties. The polyamino polymer may include a natural polyamine polymer or a synthetic polyamine polymer. Examples of natural polyamines include spermine, spermidine, and putrescine.

In other embodiments, the coating material may include a polymer containing saccharide moieties. In a non-limiting example, polysaccharides such as xanthan gum or dextran may be suitable to form a material which may reduce or prevent cell sticking in the microfluidic device. For example, a dextran polymer having a size about 3kDa may be used to provide a coating material for a surface within a microfluidic device.

In other embodiments, the coating material may include a polymer containing nucleotide moieties, i.e. a nucleic acid, which may have ribonucleotide moieties or deoxyribonucleotide moieties, providing a polyelectrolyte surface. The nucleic acid may contain only natural nucleotide moieties or may contain unnatural nucleotide moieties which comprise nucleobase, ribose or phosphate moiety analogs such as 7-deazaadenine, pentose, methyl phosphonate or phosphorothioate moieties without limitation.

In yet other embodiments, the coating material may include a polymer containing amino acid moieties. The polymer containing amino acid moieties may include a natural amino acid containing polymer or an unnatural amino acid containing polymer, either of which may include a peptide, a polypeptide or a protein. In one non-limiting example, the protein may be bovine serum albumin (BSA) and/or serum (or a combination of multiple different sera) comprising albumin and/or one or more other similar proteins as coating agents. The serum can be from any convenient source, including but not limited to fetal calf serum, sheep serum, goat serum, horse serum, and the like. In certain embodiments, BSA in a coating solution is present in a concentration from about 1 mg/mL to about 100 mg/mL, including 5 mg/mL, 10 mg/mL, 20 mg/mL, 30 mg/mL, 40 mg/mL, 50 mg/mL, 60 mg/mL, 70 mg/mL, 80 mg/mL, 90 mg/mL, or more or anywhere in between. In certain embodiments, serum in a coating solution may be present in a concentration of about 20% (v/v) to about 50% v/v, including 25%, 30%, 35%, 40%, 45%, or more or anywhere in between. In some embodiments, BSA may be present as a coating agent in a coating solution at 5 mg/mL, whereas in other embodiments, BSA may be present as a coating agent in a coating solution at 70 mg/mL. In certain embodiments, serum is present as a coating agent in a coating solution at 30%. In some embodiments, an extracellular matrix (ECM) protein may be provided within the coating material for optimized cell adhesion to foster cell growth. A cell matrix protein, which may be included in a coating material, can include, but is not limited to, a collagen, an elastin, an RGD-containing peptide (e.g. a fibronectin), or a laminin. In yet other embodiments, growth factors, cytokines, hormones or other cell signaling species may be provided within the coating material of the microfluidic device.

In some embodiments, the coating material may include a polymer containing more than one of alkylene oxide moieties, carboxylic acid moieties, sulfonic acid moieties, phosphate moieties, saccharide moieties, nucleotide moieties, or amino acid moieties. In other embodiments, the polymer conditioned surface may include a mixture of more than one polymer each having alkylene oxide moieties, carboxylic acid moieties, sulfonic acid moieties, phosphate moieties, saccharide moieties, nucleotide moieties, and/or amino acid moieties, which may be independently or simultaneously incorporated into the coating material.

In addition, in embodiments in which a covalently modified surface is used in conjunction with coating agents, the anions, cations, and/or zwitterions of the covalently modified surface can form ionic bonds with the charged portions of non-covalent coating agents (e.g. proteins in solution) that are present in a fluidic medium (e.g. a coating solution) in the enclosure.

Further details of appropriate coating treatments and modifications may be found at U.S. Application Serial No. 15/135,707, filed on April 22, 2016, and is incorporated by reference in its entirety.

**Additional system components for maintenance of viability of cells within the sequestration pens of the microfluidic device.** In order to promote growth and/or expansion of cell populations, environmental conditions conducive to maintaining functional cells may be provided by additional components of the system. For example, such additional components can provide nutrients, cell growth signaling species, pH modulation, gas exchange, temperature control, and removal of waste products from cells.

### Additional disclosed items

Item 1 is a kit for generating an antigen-presenting surface, the kit comprising:
(a) a covalently functionalized synthetic surface; (b) a primary activating molecule that includes a major histocompatibility complex (MHC) Class I molecule configured to bind to a T cell receptor (TCR), and a first reactive moiety configured to react with or bind to the covalently functionalized surface; (c) at least one co-activating molecule that includes a second reactive moiety configured to react with or bind to the covalently functionalized surface, wherein each co-activating molecular ligand is selected from a TCR co-activating molecule and an adjunct TCR activating molecule; and
(d) an exchange factor, optionally wherein the exchange factor is bound to the MHC Class I molecule.

Item 2 is the kit of item 2 further comprising one or more of:
a surface-blocking molecule capable of covalently binding to the covalently functionalized synthetic surface;
a buffer suitable for performing an exchange reaction wherein a peptide antigen displaces the exchange factor;
or instructions for performing an exchange reaction wherein a peptide antigen displaces the exchange factor.

Item 3 is a method of forming a proto-antigen-presenting surface, the method comprising:
synthesizing a plurality of major histocompatibility complex (MHC) Class I molecules in the presence of exchange factor, thereby forming a plurality of complexes each comprising an MHC Class I molecule and an exchange factor; or
reacting a plurality of MHC Class I molecules with exchange factor, thereby forming a plurality of complexes each comprising an MHC Class I molecule and an exchange factor; wherein:
   (a) a plurality of primary activating molecular ligands comprise the MHC Class I molecules and the plurality of primary activating molecular ligands are specifically bound to a covalently functionalized synthetic surface; or
   (b)(i)(A) a plurality of primary activating molecules comprise the MHC Class I molecules and first reactive moieties or (B) a plurality of primary activating molecules is prepared by adding first reactive moieties to the MHC Class I molecules, and (ii) the method further comprises reacting the first reactive moieties of the plurality of primary activating molecules with a first plurality of binding moieties disposed on a covalently functionalized synthetic surface, thereby forming the proto-antigen-presenting surface.

Item 4 is the method or kit of any one of the preceding items, wherein the covalently functionalized synthetic surface presents a plurality of azido groups.

Item 5 is the method or kit of item 4, wherein the first reactive moieties are configured to react with the azido groups of the covalently functionalized synthetic surface so as to form covalent bonds.

Item 6 is the method or kit of any one of items 1-3, wherein the covalently functionalized synthetic surface presents a plurality of biotin-binding agents, and wherein the first reactive moieties are configured to specifically bind to the biotin-binding agent.

Item 7 is the method or kit of item 6, wherein the first reactive moieties comprise or consist essentially of biotin.

Item 8 is the method or kit of item 6 or 7, wherein the biotin-binding agent is covalently attached to the covalently functionalized synthetic surface.

Item 9 is the method or kit of item 6 or 7, wherein the biotin-binding agent is noncovalently attached to the covalently functionalized synthetic surface through biotin functionalities.

Item 10 is the method or kit of any one of items 6 to 9, wherein the biotin-binding agent is streptavidin.

Item 11 is the method of any one of items 3-10, wherein a plurality of co-activating molecular ligands, each including a TCR co-activating molecule or an adjunct TCR activating molecule, are present on the covalently functionalized synthetic surface or are added to the covalently functionalized synthetic surface by reacting a plurality of co-activating molecules, each including second reactive moiety and a TCR co-activating molecule or an adjunct TCR activating molecule, with a second plurality of binding moieties of the covalently functionalized synthetic surface configured for binding the second reactive moieties.

Item 12 is the kit of any one of items 4-5, or the method of item 11, wherein the covalently functionalized synthetic surface presents a plurality of azido groups, and wherein the second reactive moieties are configured to react with the azido groups of the covalently functionalized synthetic surface so as to form covalent bonds.

Item 13 is the kit of any one of items 6-10, or the method of item 11, wherein the covalently functionalized synthetic surface presents a plurality of biotin-binding agents, and wherein the second reactive moieties are configured to specifically bind to the biotin-binding agent.

Item 14 is the method or kit of item 6, wherein the first reactive moieties comprise or consist essentially of biotin.

Item 15 is a proto-antigen-presenting surface, the surface comprising:
a plurality of primary activating molecular ligands, wherein each primary activating molecular ligand includes a major histocompatibility complex (MHC) Class I molecule configured to bind to a T cell receptor (TCR) of a T cell, and wherein an exchange factor is bound to the MHC Class I molecules; and
a plurality of co-activating molecular ligands each including a TCR co-activating molecule or an adjunct TCR activating molecule.

Item 16 is the proto-antigen-presenting surface of item 15, wherein each of the plurality of primary activating molecular ligands and the plurality of co-activating molecular ligands is specifically bound to the antigen presenting surface.

Item 17 is the surface, kit, or method of any one of the preceding items, wherein the exchange factor comprises Leu, Phe, Val, Arg, Met, Lys, Ile, homoleucine, cyclohexylalanine, or Norleucine as its C-terminal amino acid residue.

Item 18 is the surface, kit, or method of any one of the preceding items, wherein the exchange factor comprises a free N-terminal amine.

Item 19 is the surface, kit, or method of any one of the preceding items, wherein the exchange factor comprises Gly, Ala, Ser, or Cys as its penultimate C-terminal residue.

Item 20 is the surface, kit, or method of item 19, wherein the exchange factor comprises Gly as its penultimate C-terminal residue.

Item 21 is the surface, kit, or method of any one of the preceding items, wherein the exchange factor is 2, 3, 4, or 5 amino acid residues in length.

Item 22 is the surface, kit, or method of item 21, wherein the exchange factor is 2 amino acid residues in length.

Item 23 is the surface, kit, or method of any one of the preceding items, wherein the exchange factor comprises a linkage between its C-terminal and penultimate C-terminal residues which is a peptide bond, lactam, or piperazinone.

Item 24 is the surface, kit, or method of item 23, wherein the exchange factor comprises a peptide bond between its C-terminal and penultimate C-terminal residues.

Item 25 is the surface, kit, or method of any one of the preceding items, wherein the covalently functionalized synthetic surface or the proto-antigen-presenting surface further comprises at least one plurality of surface-blocking molecular ligands covalently attached to the surface.

Item 26 is the surface, kit, or method of item 25, wherein:
(i) each of the plurality of surface-blocking molecular ligands includes a hydrophilic moiety, an amphiphilic moiety, a zwitterionic moiety, and/or a negatively charged moiety;
(ii) each of the plurality of surface-blocking molecular ligands includes a linker and a terminal surface-blocking group, optionally wherein the linkers of the plurality of surface-blocking molecular ligands are of the same length or are of different lengths; or
(iii) each of the plurality of surface-blocking molecular ligands includes a linker and a terminal surface-blocking group, wherein the terminal surface-blocking group comprises a hydrophilic moiety, amphiphilic moiety, zwitterionic moiety, and/or negatively charged moiety, optionally wherein the linkers of the plurality of surface-blocking molecular ligands are of the same length or are of different lengths;
(iv) each of the plurality of surface-blocking molecular ligands is covalently bound to the covalently functionalized synthetic surface or the proto-antigen-presenting surface and/or
(v) the plurality of the surface-blocking molecular ligands and may include 2, 3, or 4 different surface-blocking groups and/or 2, 3, 4, or more different lengths of linkers, chosen in any combination.

Item 27 is the surface, kit, or method of item 25 or 26, wherein:
(i) the plurality of surface-blocking molecular ligands all have the same terminal surface-blocking group; or
(ii) the plurality of surface-blocking molecular ligands have a mixture of terminal surface-blocking groups; optionally wherein each of the plurality of surface-blocking molecular ligands includes a polyethylene glycol (PEG) moiety, a carboxylic acid moiety, or a combination thereof.

Item 28 is the surface, kit, or method of item 27, wherein the PEG moiety of each of the surface-blocking molecular ligands has a backbone linear chain length of about 10 atoms to about 100 atoms.

Item 29 is the surface, kit, or method of item 27 or 28, wherein the PEG moiety comprises a carboxylic acid moiety.

Item 30 is the surface, kit, or method of item 29, wherein the PEG moiety comprises (PEG)₄-COOH.

Item 31 is the surface, kit, or method of any one of the preceding items, wherein a plurality of biotin or biotin-binding agent functionalities is attached to the covalently functionalized synthetic surface or the proto-antigen-presenting surface via a linker.

Item 32 is the surface, kit, or method of item 31, wherein the linker linking the biotin or biotin-binding agent functionality has a length of about 20 Angstroms to about 100 Angstroms.

Item 33 is the surface, kit, or method of item 31 or 32, wherein the linker links the biotin or biotin-binding agent functionality to the covalently functionalized synthetic surface or the proto-antigen-presenting surface through a series of about 15, 20, 25, 30, 35, 40, 45, 50, 55, 60, 65, 70, 75, 80, 85, 95, 100, 200 bond lengths, or any number of bond lengths therebetween.

Item 34 is the surface, kit, or method of any one of items 31-33, wherein the linker of each biotin or biotin-binding agent functionality includes a polyethylene glycol (PEG) moiety.

Item 35 is the surface, kit, or method of item 34, wherein the PEG linker includes a (PEG)₁₃ repeating sequence, optionally wherein the covalently functionalized synthetic surface or the proto-antigen-presenting surface includes the plurality of biotin-binding agent functionalities.

Item 36 is the surface, kit, or method of item 34, wherein the PEG linker includes a (PEG)₄ repeating sequence, optionally wherein the covalently functionalized synthetic surface or the proto-antigen-presenting surface includes the plurality of biotin functionalities.

Item 37 is the surface, kit, or method of any one of items 31-36, wherein the biotin-binding agent functionalities are streptavidin moieties.

Item 38 is the surface, kit, or method of item 37, wherein the at least one plurality of streptavidin moieties is disposed upon the covalently functionalized synthetic surface or the proto-antigen-presenting surface in a density from about 4X 10² to about 3X 10⁴ molecules per square micron, in each portion or sub-region where it is attached.

Item 39 is the surface, kit, or method of item 37, wherein the at least one plurality of streptavidin moieties is disposed upon the covalently functionalized synthetic surface or the proto-antigen-presenting surface in a density from about 5X 10³ to about 3X 10⁴ molecules per square micron, in each portion or sub-region where it is attached.

Item 40 is the surface, kit, or method of item 37, wherein the at least one plurality of streptavidin moieties is disposed upon the covalently functionalized synthetic surface or the proto-antigen-presenting surface from about 6X 10² to about 5X 10³ molecules per square micron, about 5X 10³ to about 2X 10⁴ molecules per square micron, about 1X 10⁴ to about 2X 10⁴ molecules per square micron, or about 1.25X 10⁴ to about 1.75X 10⁴ molecules per square micron , in each portion or sub-region where it is attached.

Item 41 is the surface, kit, or method of any one of items 31-40, wherein the at least one plurality of biotin-binding agent or biotin moieties is disposed upon substantially all of the covalently functionalized synthetic surface or the proto-antigen-presenting surface.

Item 42 is the surface, kit, or method of any one of items 31-40, wherein the covalently functionalized synthetic surface or the proto-antigen-presenting surface further includes a first portion and a second portion, wherein the distribution of the at least one plurality of biotin-binding agent or biotin functionalities is located in the first portion of the covalently modified synthetic surface, and the distribution of the at least one plurality of the surface-blocking molecular ligands is located in the second portion.

Item 43 is the surface, kit, or method of item 42, wherein a second plurality of surface-blocking molecular ligands is disposed in the first portion of the covalently functionalized synthetic surface or the proto-antigen-presenting surface.

Item 44 is the surface, kit, or method of item 42 or 43, wherein the first portion of the covalently functionalized synthetic surface or the proto-antigen-presenting surface further includes a plurality of first regions, each first region including at least a subset of the plurality of the biotin-binding agent or biotin functionalities, wherein each of the plurality of first regions is separated from another of the plurality of first regions by the second region configured to substantially exclude the streptavidin or biotin functionalities.

Item 45 is the surface, kit, or method of item 44, wherein each of the plurality of first regions including at least the subset of the plurality of the streptavidin or biotin functionalities has an area of about 0.10 square microns to about 4.0 square microns.

Item 46 is the surface, kit, or method of item 44, wherein the area of each of the plurality of first regions including at least the subset of the plurality of the primary activating molecular ligands is about 4.0 square microns to about 0.8 square microns.

Item 47 is the surface, kit, or method of any one of the preceding items, wherein the covalently functionalized synthetic surface or the proto-antigen-presenting surface includes glass, polymer, metal, ceramic, and/or a metal oxide.

Item 48 is the surface, kit, or method of any one of the preceding items, wherein the covalently functionalized synthetic surface or the proto-antigen-presenting surface is a wafer, an inner surface of a tube, or an inner surface of a microfluidic device.

Item 49 is the surface, kit, or method of item 42, wherein the tube is a glass or polymer tube.

Item 50 is the surface, kit, or method of any one of items 1-41, wherein the covalently functionalized synthetic surface or the proto-antigen-presenting surface is a bead.

Item 51 is the surface, kit, or method of item 44, wherein the bead includes a magnetic material.

Item 52 is the surface, kit, or method of item 44 or 45, wherein the bead has a surface area within 10% of the surface area of a sphere of an equal volume or diameter.

Item 53 is the surface, kit, or method of item 48, wherein the covalently functionalized synthetic surface or the proto-antigen-presenting surface is at least one inner surface of a microfluidic device.

Item 54 is the surface, kit, or method of item 53, wherein the inner surface of the microfluidic device is within a chamber of the microfluidic device.

Item 55 is the surface, kit, or method of any one of items 44-46, wherein each of the plurality of first regions including at least a subset of the plurality of biotin-binding agent or biotin functionalities includes at least one surface within a chamber of the microfluidic device.

Item 56 is the surface, kit, or method of item 54 or 55, wherein the chamber is a sequestration pen.

Item 57 is the surface, kit, or method of item 56, wherein the microfluidic device further comprises a flow region for containing a flow of a first fluidic medium; and the sequestration pen comprises an isolation region for containing a second fluidic medium, the isolation region having a single opening, wherein the isolation region of the sequestration pen is an unswept region of the microfluidic device; and a connection region fluidically connecting the isolation region to the flow region; optionally wherein the microfluidic device comprises a microfluidic channel comprising at least a portion of the flow region.

Item 58 is the surface, kit, or method of item 57, wherein the microfluidic device comprises a microfluidic channel comprising at least a portion of the flow region, and the connection region comprises a proximal opening into the microfluidic channel having a width W_{con} ranging from about 20 microns to about 100 microns and a distal opening into the isolation region, and wherein a length L_{con} of the connection region from the proximal opening to the distal opening is at least 1.0 times a width W_{con} of the proximal opening of the connection region.

Item 59 is the surface, kit, or method of item 58, wherein the length L_{con} of the connection region from the proximal opening to the distal opening is at least 1.5 times the width W_{con} of the proximal opening of the connection region.

Item 60 is the surface, kit, or method of item 58, wherein the length L_{con} of the connection region from the proximal opening to the distal opening is at least 2.0 times the width W_{con} of the proximal opening of the connection region.

Item 61 is the surface, kit, or method of any one of items 58-60, wherein the width W_{con} of the proximal opening of the connection region ranges from about 20 microns to about 60 microns.

Item 62 is the surface, kit, or method of any one of items 58-61, wherein the length L_{con} of the connection region from the proximal opening to the distal opening is between about 20 microns and about 500 microns.

Item 63 is the surface, kit, or method of any one of items 58-62, wherein a width of the microfluidic channel at the proximal opening of the connection region is between about 50 microns and about 500 microns.

Item 64 is the surface, kit, or method of any one of items 58-63, wherein a height of the microfluidic channel at the proximal opening of the connection region is between 20 microns and 100 microns.

Item 65 is the surface, kit, or method of any one of items 57-64, wherein the volume of the isolation region ranges from about 2 x 10⁴ to about 2 x 10⁶ cubic microns.

Item 66 is the surface, kit, or method of any one of items 57-65, wherein the proximal opening of the connection region is parallel to a direction of the flow of the first medium in the flow region.

Item 67 is the surface, kit, or method of any one of items 57-66, wherein the microfluidic device comprises an enclosure comprising a base, a microfluidic circuit structure disposed on the base, and a cover which collectively define a microfluidic circuit, and the microfluidic circuit comprises the flow region, the microfluidic channel, and the sequestration pen.

Item 68 is the surface, kit, or method of any one of items 57-67, wherein the microfluidic circuit further comprises one or more inlets through which the first medium can be input into the flow region and one or more outlets through which the first medium can be removed from the flow region.

Item 69 is the surface, kit, or method of item 67, wherein the cover is an integral part of the microfluidic circuit structure.

Item 70 is the surface, kit, or method of any one of items 67 or 68, wherein barriers defining the microfluidic sequestration pen extend from a surface of the base of the microfluidic device to a surface of the cover of the microfluidic device.

Item 71 is the surface, kit, or method of any one of items 67-70, wherein the cover and the base are part of a dielectrophoresis (DEP) mechanism for selectively inducing DEP forces on a micro-object.

Item 72 is the surface, kit, or method of any one of items 67-71, wherein the microfluidic device further comprises a first electrode, an electrode activation substrate, and a second electrode, wherein the first electrode is part of a first wall of the enclosure and the electrode activation substrate and the second electrode is part of a second wall of the enclosure, wherein the electrode activation substrate comprises a photoconductive material, semiconductor integrated circuits, or phototransistors.

Item 73 is the surface, kit, or method of item 72, wherein the first wall of the microfluidic device is the cover, and wherein the second wall of the microfluidic device is the base.

Item 74 is the surface, kit, or method of item 72 or 73, wherein the electrode activation substrate comprises phototransistors.

Item 75 is the surface, kit, or method of any one of items 67-74, wherein the cover and/or the base is transparent to light.

Item 76 is the surface, kit, or method of any one of items 53-75, wherein the covalently functionalized surface or the proto-antigen-presenting surface includes a portion configured to exclude biotin-binding agent or biotin functionalities which is disposed at at least one surface of a microfluidic channel of the microfluidic device.

Item 77 is the surface, kit, or method of any one of items 1-2 or 4-76, wherein the plurality of co-activating molecular ligands comprises TCR co-activating molecules and adjunct TCR activating molecules.

Item 78 is the surface, kit, or method of item 77, wherein a ratio of the TCR co-activating molecules to the adjunct TCR activating molecules of the plurality of co-activating molecular ligands is about 100:1 to about 1:100.

Item 79 is the surface, kit, or method of item 77, wherein a ratio of the TCR co-activating molecules to the adjunct TCR activating molecules of the plurality of co-activating molecular ligands is 100:1 to 90:1, 90:1 to 80:1, 80:1 to 70:1, 70:1 to 60:1, 60:1 to 50:1, 50:1 to 40:1, 40:1 to 30:1, 30:1 to 20:1, 20:1 to 10:1, 10:1 to 1:1, 1:1 to 1:10, 1:10 to 1:20, 1:20 to 1:30, 1:30 to 1:40, 1:40 to 1:50, 1:50 to 1:60, 1:60 to 1:70, 1:70 to 1:80, 1:80 to 1:90, or 1:90 to 1:100, wherein each of the foregoing values is modified by "about."

Item 80 is the surface, kit, or method of item 77, wherein a ratio of the TCR co-activating molecules to the adjunct TCR activating molecules of the plurality of co-activating molecular ligands is about 10:1 to about 1:20.

Item 81 is the surface, kit, or method of item 77, wherein a ratio of the TCR co-activating molecules to the adjunct TCR activating molecules of the plurality of co-activating molecular ligands is about 10:1 to about 1:10.

Item 82 is the surface, kit, or method of any one of the preceding items, wherein the MHC molecule includes an MHC protein sequence and a beta microglobulin.

Item 83 is the surface, kit, or method of item 82, wherein the MHC Class I molecule comprises a human leukocyte antigen A (HLA-A) heavy chain.

Item 84 is the surface, kit, or method of item 83, wherein the HLA-A heavy chain is an HLA-A*01, HLA-A*02, HLA-A*03, HLA-A*11, HLA-A*23, HLA-A*24, HLA-A*25, HLA-A*26, HLA-A*29, HLA-A*30, HLA-A*31, HLA-A*32, HLA-A*33, HLA-A*34, HLA-A*43, HLA-A*66, HLA-A*68, HLA-A*69, HLA-A*74, or HLA-A*80 heavy chain.

Item 85 is the surface, kit, or method of item 82, wherein the MHC Class I molecule comprises a human leukocyte antigen B (HLA-B) heavy chain.

Item 86 is the surface, kit, or method of item 85, wherein the HLA-B heavy chain is an HLA-B*07, HLA-B*08, HLA-B*13, HLA-B*14, HLA-B*15, HLA-B*18, HLA-B*27, HLA-B*35, HLA-B*37, HLA-B*38, HLA-B*39, HLA-B*40, HLA-B*41, HLA-B*42, HLA-B*44, HLA-B*45, HLA-B*46, HLA-B*47, HLA-B*48, HLA-B*49, HLA-B*50, HLA-B*51, HLA-B*52, HLA-B*53, HLA-B*54, HLA-B*55, HLA-B*56, HLA-B*57, HLA-B*58, HLA-B*59, HLA-B*67, HLA-B*73, HLA-B*78, HLA-B*81, HLA-B*82, or HLA-B*83 heavy chain.

Item 87 is the surface, kit, or method of item 82, wherein the MHC Class I molecule comprises a human leukocyte antigen C (HLA-C) heavy chain.

Item 88 is the surface, kit, or method of item 87, wherein the HLA-C heavy chain is an HLA-C*01, HLA-C*02, HLA-C*03, HLA-C*04, HLA-C*05, HLA-C*06, HLA-C*07, HLA-C*08, HLA-C*12, HLA-C*14, HLA-C*15, HLA-C*16, HLA-C*17, or HLA-C*18 heavy chain.

Item 89 is the surface, kit, or method of any one of items 1-2 or 4-88, wherein the TCR co-activating molecule includes a protein.

Item 90 is the surface, kit, or method of item 89, wherein the TCR co-activating molecule further comprises a site-specific C-terminal biotin moiety.

Item 91 is the surface, kit, or method of item 88 or 89, wherein the TCR co-activating protein molecule includes a CD28 binding protein or a fragment thereof which retains binding ability with CD28.

Item 92 is the surface, kit, or method of item 91, wherein the CD28 binding protein includes a CD80 molecule or a fragment thereof, wherein the fragment retains binding ability to CD28.

Item 93 is the surface, kit, or method of item 88 or 89, wherein the TCR co-activating molecule includes an anti-CD28 antibody or fragment thereof, wherein the fragment retains binding activity with CD28.

Item 94 is the surface, kit, or method of any one of items 1-2 or 4-93, wherein the adjunct TCR activating molecule is configured to provide adhesion stimulation.

Item 95 is the surface, kit, or method of any one of items 1-2 or 4-94, wherein the adjunct TCR activating molecular ligand includes a CD2 binding protein or a fragment thereof, wherein the fragment retains binding ability with CD2.

Item 96 is the surface, kit, or method of item 95, wherein the CD2 binding protein further comprises a site-specific C-terminal biotin moiety.

Item 97 is the surface, kit, or method of any one of items 95 or 96, wherein the adjunct TCR activating molecular ligand includes a CD58 molecule or fragment thereof, wherein the fragment retains binding activity with CD2.

Item 98 is the surface, kit, or method of any one of items 95 or 96, wherein the adjunct TCR activating molecule includes an anti-CD2 antibody or a fragment thereof, wherein the fragment retains binding activity with CD2.

Item 99 is the proto-antigen-presenting surface of any one of items 15-98, wherein the plurality of primary activating molecular ligands is disposed upon at least a portion of the antigen-presenting surface at a density from about 4X 10² to about 3X 10⁴ molecules per square micron, in each portion or sub-region where it is attached.

Item 100 is the proto-antigen-presenting surface of item 99, wherein the plurality of primary activating molecular ligands is disposed upon at least a portion of the antigen-presenting surface at a density from about 4X 10² to about 2X 10³ molecules per square micron.

Item 101 is the proto-antigen-presenting surface of item 99, wherein the plurality of primary activating molecular ligands is disposed upon at least a portion of the antigen-presenting surface at a density from about 2X 10³ to about 5X 10³ molecules per square micron.

Item 102 is the proto-antigen-presenting surface of item 99, wherein the plurality of primary activating molecular ligands is disposed upon at least a portion of a surface of the antigen-presenting surface at a density from about 5X 10³ to about 2X 10⁴ molecules per square micron, about 1X 10⁴ to about 2X 10⁴ molecules per square micron, or about 1.25X 10⁴ to about 1.75X 10⁴ molecules per square micron.

Item 103 is the proto-antigen-presenting surface of any one of items 99-102, wherein the plurality of primary activating molecular ligands is disposed upon substantially all of the antigen-presenting surface at the stated density.

Item 104 is the proto-antigen-presenting surface of any one of items 15-103, wherein the plurality of co-activating molecular ligands is disposed upon at least a portion the antigen-presenting surface at a density from about 5X 10² to about 2X 10⁴ molecules per square micron or about 5X 10² to about 1.5 X 10⁴ molecules per square micron.

Item 105 is the proto-antigen-presenting surface of item 104, wherein the plurality of co-activating molecular ligands is disposed upon at least a portion of the antigen-presenting surface at a density from about 5X 10³ to about 2X 10⁴ molecules per square micron, about 5X 10³ to about 1.5X 10⁴ molecules per square micron, about 1X 10⁴ to about 2X 10⁴ molecules per square micron, about 1X 10⁴ to about 1.5X 10⁴ molecules per square micron, about 1.25X 10⁴ to about 1.75X 10⁴ molecules per square micron, or about 1.25X 10⁴ to about 1.5X 10⁴ molecules per square micron.

Item 106 is the proto-antigen-presenting surface of any one of items 15-103, wherein the plurality of co-activating molecular ligands is disposed upon at least a portion of the antigen-presenting surface at a density from about 2X 10³ to about 5X 10³ molecules per square micron.

Item 107 is the proto-antigen-presenting surface of any one of items 15-103, wherein the plurality of co-activating molecular ligands is disposed upon at least a portion of a surface of the antigen-presenting surface at a density from about 5X 10² to about 2X 10³ molecules per square micron.

Item 108 is the proto-antigen-presenting surface of any one of items 104-107, wherein the plurality of co-activating molecular ligands is disposed upon substantially all of the antigen-presenting surface at the stated density.

Item 109 is the proto-antigen-presenting surface of any one of items 15-108, wherein a ratio of the primary activating molecular ligands to the co-activating molecular ligands present on the antigen-presenting surface is about 1:10 to about 2:1, about 1:5 to about 2:1, about 1:2 to about 2:1, about 1:10 to about 1:1, about 1:5 to about 1:1, about 1:1 to about 2:1, or about 1:2 to about 1:1.

Item 110 is the proto-antigen-presenting surface of any one of items 15-109, wherein each of the plurality of primary activating molecular ligands is noncovalently bound to a binding moiety, and further wherein the binding moiety is covalently bound to the antigen-presenting surface.

Item 111 is the proto-antigen-presenting surface of item 110, wherein each of the plurality of primary activating molecular ligands comprises a biotin and is noncovalently bound to a biotin-binding agent, and further wherein the biotin-binding agent is covalently bound to the antigen-presenting surface.

Item 112 is the proto-antigen-presenting surface of any one of items 15-109, wherein each of the plurality of primary activating molecular ligands is noncovalently bound to a binding moiety, and further wherein the binding moiety is noncovalently bound to the antigen-presenting surface.

Item 113 is the proto-antigen-presenting surface of item 112, wherein each of the plurality of primary activating molecular ligands comprises a biotin moiety, the binding moiety comprises a biotin-binding agent, and the biotin-binding agent is noncovalently bound to a second biotin moiety covalently attached to the antigen-presenting surface.

Item 114 is the proto-antigen-presenting surface of 111 or 113, wherein the biotin-binding agent is streptavidin.

Item 115 is the proto-antigen-presenting surface of any one of items 15-114, wherein each of the plurality of co-activating molecular ligands is non-covalently attached to a streptavidin and the streptavidin is non-covalently attached to a streptavidin binding molecule, further wherein the streptavidin binding molecule is covalently attached via a linker to the proto-antigen-presenting surface, optionally wherein the streptavidin binding molecule comprises biotin.

Item 116 is the proto-antigen-presenting surface of any one of items 15-114, wherein each of the plurality of co-activating molecular ligands is covalently connected to the surface via a linker.

Item 117 is the proto-antigen-presenting surface of item 115 or 116, wherein the linker links the streptavidin binding molecule and/or co-activating molecular ligands to the surface through a series of about 15, 20, 25, 30, 35, 40, 45, 50, 55, 60, 65, 70 , 75, 80, 85, 95, 100, 200 bond lengths, or any number of bond lengths therebetween bonds.

Item 118 is the proto-antigen-presenting surface of any one of items 15-114, wherein each of the plurality of co-activating molecular ligands is non-covalently attached to a streptavidin moiety; and the streptavidin moiety is covalently attached to the antigen-presenting surface.

Item 119 is the proto-antigen-presenting surface of item 118, wherein the streptavidin moiety is linked by a linker to the surface through a series of about 15, 20, 25, 30, 35, 40, 45, 50, 55, 60, 65, 70, 75, 80, 85, 95, 100, 200 bond lengths, or any number of bond lengths therebetween.

Item 120 is the proto-antigen-presenting surface of any one of items 15-119, wherein the proto-antigen-presenting surface further comprises a plurality of surface-blocking molecular ligands.

Item 121 is the proto-antigen-presenting surface of item 120, wherein:
(i) each of the plurality of surface-blocking molecular ligands includes a hydrophilic moiety, an amphiphilic moiety, a zwitterionic moiety, and/or a negatively charged moiety;
(ii) each of the plurality of surface-blocking molecular ligands includes a linker and a terminal surface-blocking group, optionally wherein the linkers of the plurality of surface-blocking molecular ligands are of the same length or are of different lengths; or
(iii) each of the plurality of surface-blocking molecular ligands includes a linker and a terminal surface-blocking group, wherein the terminal surface-blocking group comprises a hydrophilic moiety, amphiphilic moiety, zwitterionic moiety, and/or negatively charged moiety, optionally wherein the linkers of the plurality of surface-blocking molecular ligands are of the same length or are of different lengths.

Item 122 is the proto-antigen-presenting surface of item 120 or 121, wherein:
(i) the plurality of surface-blocking molecular ligands all have the same terminal surface-blocking group; or
(ii) the plurality of surface-blocking molecular ligands have a mixture of terminal surface-blocking groups; optionally wherein each of the plurality of surface-blocking molecular ligands includes a polyethylene glycol (PEG) moiety, a carboxylic acid moiety, or a combination thereof, further optionally wherein the PEG moiety of each of the surface-blocking molecular ligands has a backbone linear chain length of about 10 atoms to about 100 atoms.

Item 123 is the proto-antigen-presenting surface of any one of items 120-122, wherein:
(i) each of the plurality of surface-blocking molecular ligands is covalently connected to the antigen-presenting surface; and/or
(ii) the plurality of the surface-blocking molecular ligands and may include 2, 3, or 4 different surface-blocking groups and/or 2, 3, 4, or more different lengths of linkers, chosen in any combination.

Item 124 is the proto-antigen-presenting surface of any one of items 15-123, further including a plurality of adhesion stimulatory molecular ligands, optionally wherein each adhesive molecular ligand includes a ligand for a cell adhesion receptor comprising an ICAM protein sequence.

Item 125 is the proto-antigen-presenting surface of item 124, wherein the adhesion stimulatory molecular ligand is covalently connected to the antigen-presenting surface via a linker.

Item 126 is the proto-antigen-presenting surface of item 125, wherein the adhesion stimulatory molecular ligand is non-covalently attached to a streptavidin moiety, wherein the streptavidin moiety is covalently attached via a linker to the antigen-presenting surface.

Item 127 is the proto-antigen-presenting surface of item 125, wherein the adhesion stimulatory molecular ligand is non-covalently attached to a streptavidin, wherein the streptavidin is noncovalently attached to a biotin and the biotin is covalently attached via a linker to the antigen-presenting surface.

Item 128 is the proto-antigen-presenting surface of any one of items 15-127, wherein the ratio of the TCR co-activating molecules to the adjunct TCR activating molecules of the plurality of co-activating molecular ligands is from about 3:1 to about 1:3.

Item 129 is the proto-antigen-presenting surface of any one of items 15-128, wherein the ratio of the TCR co-activating molecules to the adjunct TCR activating molecules of the plurality of co-activating molecular ligands is about 1:2 to about 2:1.

Item 130 is the proto-antigen-presenting surface of any one of items 15-129, wherein the ratio of the TCR co-activating molecules to the adjunct TCR activating molecules of the plurality of co-activating molecular ligands is about 1:1.

Item 131 is the proto-antigen-presenting surface of any one of items 15-130, further including a plurality of growth-stimulatory molecular ligands, wherein each of the growth-stimulatory molecular ligands includes a growth factor receptor ligand.

Item 132 is the proto-antigen-presenting surface of item 131, wherein the growth factor receptor ligand includes a cytokine or fragment thereof, wherein the fragment retains receptor binding ability, optionally wherein the cytokine comprises IL-21.

Item 133 is the proto-antigen-presenting surface of any one of items 15-102, 104-107, or 109-132, further including a first portion and a second portion, wherein the distribution of the plurality of primary activating molecular ligands and the distribution of the plurality of co-activating molecular ligands are located in the first portion of the antigen-presenting surface, and the second portion is configured to substantially exclude the primary activating molecular ligands.

Item 134 is the proto-antigen-presenting surface of item 133, wherein at least one plurality of surface-blocking molecular ligands is located in the second portion of the at least one inner surface of the antigen-presenting surface.

Item 135 is the proto-antigen-presenting surface of item 132 or 133, wherein the first portion of the antigen-presenting surface further includes a plurality of first regions, each first region including at least a subset of the plurality of the primary activating molecular ligands, wherein each of the plurality of first regions is separated from another of the plurality of first region by the second portion configured to substantially exclude primary activating molecular ligands.

Item 136 is the proto-antigen-presenting surface of item 135, wherein each of the plurality of first regions including the at least a subset of the plurality of the primary activating molecular ligands further includes a subset of the plurality of the co-activating molecular ligands.

Item 137 is the proto-antigen-presenting surface of item 135 or 136, wherein each of the plurality of first regions including at least the subset of the plurality of the primary activating molecular ligands has an area of about 0.10 square microns to about 4.0 square microns.

Item 138 is the proto-antigen-presenting surface of any one of items 135-137, wherein the area of each of the plurality of first regions including at least the subset of the plurality of the primary activating molecular ligands is about 4.0 square microns to about 0.8 square microns.

Item 139 is the proto-antigen-presenting surface of any one of items 135-138, wherein each of the plurality of first regions further includes at least a subset of a plurality of adhesion stimulatory molecular ligands, and optionally wherein each of the adhesion stimulatory molecular ligands includes a ligand for a cell adhesion receptor comprising an ICAM protein sequence.

Item 140 is the proto-antigen-presenting surface of any one of items 133-139, wherein the second portion configured to substantially exclude the primary activating molecular ligands is also configured to substantially exclude co-activating molecular ligands.

Item 141 is the proto-antigen-presenting surface of any one of items 133-140, wherein the second portion configured to substantially exclude the primary activating molecular ligands is further configured to include a plurality of growth stimulatory molecular ligands, wherein each of the growth stimulatory molecular ligands includes a growth factor receptor ligand.

Item 142 is the proto-antigen-presenting surface of any one of items 133-141, wherein the second portion configured to substantially exclude the primary activating molecular ligands includes a plurality of adhesion stimulatory molecular ligands, wherein each of the adhesion stimulatory molecular ligands includes a ligand for a cell adhesion receptor including an ICAM protein sequence.

Item 143 is the proto-antigen-presenting surface of any one of items 133-142, which is an antigen-presenting surface of a microfluidic device and each of the plurality of first regions including at least a subset of the plurality of primary activating molecular ligands is disposed at least one surface within a chamber of the antigen-presenting microfluidic device.

Item 144 is the kit or method of item 43, wherein the second plurality of surface-blocking molecular ligands limits the density of functionalizing moieties of an antigen-presenting synthetic surface formed from the covalently functionalized synthetic surface.

Item 145 is the method of any one of items 3-98 or 144, further comprising reacting a plurality of surface-blocking molecules with a first additional plurality of binding moieties of the covalently functionalized surface, wherein each of the binding moieties of the first additional plurality is configured for binding the surface-blocking molecule.

Item 146 is the method of any one of items 3-98 or 144-145, further comprising reacting a plurality of adhesion stimulatory molecular ligands, wherein each adhesion stimulatory molecular ligand includes a ligand for a cell adhesion receptor including an ICAM protein sequence, with a second additional plurality of binding moieties of the covalently functionalized surface, wherein each of the binding moieties of the second additional plurality is configured for binding with the cell adhesion receptor ligand molecule.

Item 147 is the kit of any one of items 1-2, 4-98, or 144, further comprising a plurality of surface-blocking molecules, wherein the covalently functionalized surface further comprises a first additional plurality of binding moieties configured for binding the surface-blocking molecule.

Item 148 is the kit of any one of items 1-2, 4-98, 144, or 148, further comprising a plurality of adhesion stimulatory molecular ligands, wherein each adhesion stimulatory molecular ligand includes a ligand for a cell adhesion receptor including an ICAM protein sequence, and the covalently functionalized surface further comprises a second additional plurality of binding moieties configured for binding the cell adhesion receptor ligand molecule.

Item 149 is the kit of any one of items 1-2, 4-98, 144, or 148-149, further comprising a peptide antigen.

Item 150 is a method of preparing an antigen-presenting surface comprising a peptide antigen, the method comprising reacting the peptide antigen with a proto-antigen-presenting surface according to any one of items 15-143, wherein the exchange factor is substantially displaced and the peptide antigen becomes associated with the MHC Class I molecules.

Item 151 is the kit or method of item 149 or 150, wherein the peptide antigen comprises a tumor-associated antigen.

Item 152 is the kit or method of any one of items 149-151, wherein the peptide antigen comprises a segment of amino acid sequence from a protein expressed on the surface of a tumor cell.

Item 153 is the kit or method of item 152, wherein the segment comprises 5, 6, 7, 8, 9, or 10 amino acid residues or is 5, 6, 7, 8, 9, or 10 amino acid residues in length.

Item 154 is the kit or method of item 152 or 153, wherein the protein expressed on the surface of a tumor cell is CD19, CD20, CLL-1, TRP-2, LAGE-1, HER2, EphA2, FOLR1, MAGE-A1, mesothelin, SOX2, PSM, CA125, or T antigen.

Item 155 is the kit or method of any one of items 149-154, wherein the peptide antigen is a neoantigenic peptide.

Item 156 is the kit or method of any one of items 149-155, wherein the peptide antigen is 7, 8, 9, 10, or 11 amino acids in length.

Item 157 is the kit or method of item 156, wherein the peptide antigen is 8, 9, or 10 amino acids in length.

Item 158 is the method of any one of items 150-157, further comprising contacting a T lymphocyte with the antigen-presenting surface comprising the peptide antigen.

Item 159 is the method of item 158, wherein a plurality of T lymphocytes are contacted with the antigen-presenting surface.

Item 160 is the method of item 158 or 159, wherein a sample comprising unactivated T cells is enriched for T cells prior to activation.

Item 161 is the method of any one of items 158-160, wherein a sample comprising unactivated T cells is enriched for CD8⁺ T cells prior to activation.

Item 162 is the method of item 160 or 161, wherein the sample comprising unactivated T cells is a peripheral blood sample.

Item 163 is the method of any one of items 160-162, wherein the sample is from a subject in need of treatment for cancer.

Item 164 is the method of any one of items 158-163, wherein the T lymphocyte or the plurality of T lymphocytes is CD8⁺.

Item 165 is the method of any one of items 158-164, wherein the T lymphocyte or the plurality of T lymphocytes are obtained from a subject in need of treating a cancer.

Item 166 is the method of any one of items 158-165, wherein the T lymphocyte becomes an activated T lymphocyte following contact with the antigen-presenting surface.

Item 167 is the method of any one of items 159-165, wherein a plurality of the T lymphocytes become activated T lymphocytes following contact with the antigen-presenting surface.

Item 168 is the method of any one of items 166-167, wherein the activated T lymphocyte(s) is CD28+.

Item 169 is the method of any one of items 166-168, wherein the activated T lymphocyte(s) is CD45RO+.

Item 170 is the method of any one of items 166-169, wherein the activated T lymphocyte(s) is CD127+.

Item 171 is the method of any one of items 166-170, wherein the activated T lymphocyte(s) is CD197+.

Item 172 is the method of any one of items 167-171, wherein the method produces a population of T cells, wherein at least about 1%, about 1.5%, about 2%, about 3%, about 4%, about 5%, about 6%, about 7%, about 8%, about 9%, or about 10% of the population of T cells are antigen-specific T cells.

Item 173 is the method of item 172, wherein 1%-2%, 2%-3%, 3%-4%, 4%-5%, 5%-6%, 6%-7%, 7%-8%, 8%-9%, 9%-10%, 10%-11%, or 11%-12% of the T cells are antigen-specific T cells wherein each of the foregoing values are modified by "about."

Item 174 is the method of item 172 or 173, wherein at least about 65%, about 70%, about 75%, about 80%, about 85%, about 86%, about 87%, about 88%, about 89%, about 90%, about 91%, about 92%, about 93%, about 94%, about 95%, about 96%, about 97%, or about 98% of the antigen-specific T cells are CD45RO+/CD28^{High} cells.

Item 175 is the method of any one of items 172-174, further comprising rapidly expanding the antigen-specific T cells to provide an expanded population of antigen-specific T cells.

Item 176 is the method of any one of items 167-175, further comprising separating activated T lymphocytes from unactivated T lymphocytes.

Item 177 is the method of item 176, wherein separating activated T cells includes detecting a plurality of surface biomarkers of the activated T cells.

Item 178 is One or more activated T lymphocytes produced by the method of any one of items 158-177.

Item 179 is a population of T cells comprising activated T cells produced by the method of any one of items 158-177.

Item 180 is the cell or population of item 178 or 179, wherein the activated T cells are CD45RO+.

Item 181 is the cell or population of any one of items 178-180, wherein the activated T cells are CD28+.

Item 182 is the cell or population of any one of items 178-181, wherein the activated T cells are CD28^{high}.

Item 183 is the cell or population of any one of items 178-182, wherein the activated T cells are CD127+.

Item 184 is the cell or population of any one of items 178-183, wherein the activated T cells are CD197+.

Item 185 is the cell or population of any one of items 178-184, wherein the activated T cells are CD8+.

Item 186 is a microfluidic device comprising the cell or population of any one of items 178-185.

Item 187 is a pharmaceutical composition comprising the cell or population of any one of items 178-185.

Item 188 is a method of screening a plurality of peptide antigens for T-cell activation, the method comprising:
reacting a plurality of different peptide antigens with a plurality of proto-antigen-presenting surfaces according to any one of items 15-143, thereby substantially displacing the exchange factors and forming a plurality of antigen-presenting surfaces;
contacting a plurality of T cells with the antigen-presenting surfaces; and
monitoring the T cells for activation, wherein activation of a T cell indicates that a peptide antigen associated with the surface with which the T cell was contacted is able to contribute to T cell activation.

Item 189 is the method of item 188, wherein the proto-antigen-presenting surfaces are reacted separately with the plurality of different peptide antigens, thereby generating a plurality of different antigen-presenting surfaces.

Item 190 is the method of item 188, wherein the proto-antigen-presenting surfaces are reacted separately with pools of members of the plurality of different peptide antigens, thereby generating a plurality of different antigen-presenting surfaces.

Item 191 is the method of item 190, wherein the pools of members of the plurality of different peptide antigens comprise overlapping pools.

Item 192 is the method of item 190, wherein the pools of members of the plurality of different peptide antigens comprise non-overlapping pools.

Item 193 is the method of any one of items 188-192, wherein the plurality of proto-antigen-presenting surfaces is a plurality of proto-antigen-presenting beads.

Item 194 is the method of item 193, wherein T cells are contacted separately with members of the plurality of different antigen-presenting beads.

Item 195 is the method of item 193, wherein T cells are contacted with a pool of the different antigen-presenting beads.

Item 196 is the method of item 193, wherein T cells are contacted with a plurality of pools of the different antigen-presenting beads.

Item 197 is the method of item 196, wherein the plurality of pools of the different antigen-presenting beads comprises overlapping pools.

Item 198 is the method of item 196, wherein the plurality of pools of the different antigen-presenting beads comprises non-overlapping pools.

Item 199 is the method of any one of items 193-197, wherein the T cells are in wells of a well plate when contacted with the antigen-presenting beads.

Item 200 is the method of any one of items 193-197, wherein the T cells are in a microfluidic device when contacted with the antigen-presenting beads.

Item 201 is the method of any one of items 193-197, wherein the T cells are in sequestration pens of a microfluidic device when contacted with the antigen-presenting beads.

Item 202 is the method of any one of items 193-201, further comprising (i) determining that T cells contacted with a pool of antigen-presenting beads underwent activation and (ii) contacting additional T cells with a member or subset of members of the pool, or with one or more additional antigen-presenting surfaces comprising the same peptide antigen or peptide antigens as a member or subset of members of the pool.

Item 203 is the method of any one of items 188-192, wherein the plurality of proto-antigen-presenting surfaces is a plurality of proto-antigen-presenting surfaces of a microfluidic device.

Item 204 is the method of item 203, wherein the plurality of proto-antigen-presenting surfaces of the microfluidic device are separated by regions of non-antigen-presenting surface.

Item 205 is the method of item 203 or 204, wherein the plurality of proto-antigen-presenting surfaces of a microfluidic device are in sequestration pens of the microfluidic device.

Item 206 is the method of any one of items 203-205, wherein individual antigen-presenting surfaces of the microfluidic device comprise pools of peptide antigens and the method further comprises (i) determining that T cells contacted with one or more of the antigen-presenting surfaces of the microfluidic device underwent activation and (ii) contacting additional T cells with one or more additional antigen-presenting surfaces comprising a member or subset of members of the peptide antigens associated with the one or more antigen-presenting surfaces of the microfluidic device.

Item 207 is the method of any one of items 188-192, wherein the plurality of proto-antigen-presenting surfaces is a plurality of proto-antigen-presenting surfaces in wells of one or more well plates.

Item 208 is the method of item 207, wherein the wells comprise non-antigen-presenting regions.

Item 209 is the method of item 207 or 208, wherein individual antigen-presenting surfaces of the one or more well plates comprise pools of peptide antigens and the method further comprises (i) determining that T cells contacted with one or more of the antigen-presenting surfaces one or more well plates underwent activation and (ii) contacting additional T cells with one or more additional antigen-presenting surfaces comprising a member or subset of members of the peptide antigens associated with the one or more antigen-presenting surfaces of the one or more well plates.

Item 210 is the method of any one of items 188-209, wherein the T cells include CD8+ T cells.

Item 211 is the method of any one of items 188-210, wherein monitoring the T cells for activation comprises detecting a CD45RO+ activated T cell.

Item 212 is the method of any one of items 188-211, wherein monitoring the T cells for activation comprises detecting a CD28+ activated T cell.

Item 213 is the method of any one of items 188-212, wherein monitoring the T cells for activation comprises detecting a CD28^{high} activated T cell.

Item 214 is the method of any one of items 188-213, wherein monitoring the T cells for activation comprises detecting a CD127+ activated T cell.

Item 215 is the method of any one of items 188-214, wherein monitoring the T cells for activation comprises detecting a CD197+ activated T cell.

Item 216 is a method of treating a subject in need of treating a cancer, comprising introducing a plurality of activated T cells according to any one of items 178-185 into the subject, wherein the activated T cells are antigen-specific against the cancer of the subject.

Item 217 is a method of treating a subject in need of treating a cancer, comprising preparing a plurality of activated T cells according to the method of any one of items 158-177, wherein the activation is configured to be specific against the cancer of the subject, and introducing the activated T cells into the subject.

Item 218 is the method of item 216 or 217, wherein the activation is configured to be specific against one or more cancer-specific antigens from the subject.

Item 219 is the method of item 218, wherein the one or more cancer-specific antigens from the subject are obtained from a sample of cancer cells from the subject.

Item 220 is the method of item 218 or 219, wherein one or more cancer-specific antigens from the subject are screened according to the method of any one of items 188-215 prior to preparing the plurality of activated T cells.

Item 221 is the method of any one of items 216-220, wherein the method comprises reacting the one or more cancer-specific antigens from the subject with one or more proto-antigen-presenting surfaces according to any one of items 15-143 prior to preparing the plurality of activated T cells.

Item 222 is the method of any one of items 216-221, wherein the T cells are autologous T cells.

Item 223 is the method of any one of items 216-222, wherein the cancer is melanoma, breast cancer, or lung cancer.

### EXAMPLES

### General Materials and Methods.

**System and Microfluidic device:** An OptoSelect chip, a microfluidic (or nanofluidic) device manufactured by Berkeley Lights, Inc. and controlled by an optical instrument which was also manufactured by Berkeley Lights, Inc. The instrument included: a mounting stage for the chip coupled to a temperature controller; a pump and fluid medium conditioning component; and an optical train including a camera and a structured light source suitable for activating phototransistors within the chip. The OptoSelect^{™} chip included a substrate configured with OptoElectroPositioning (OEP^{™}) technology, which provides a phototransistor-activated OET force. The chip also included a plurality of microfluidic channels, each having a plurality of NanoPen^{™} chambers (or sequestration pens) fluidically connected thereto. The volume of each sequestration pen was around 1x10⁶ cubic microns.

**Priming solution:** Complete growth medium containing 0.1% Pluronic^{®} F127 ((Life Technologies^{®} Cat# P6866).

Preparation for culturing: The microfluidic device having a modified surface was loaded onto the system and purged with 100% carbon dioxide at 15 psi for 5 min. Immediately following the carbon dioxide purge, the priming solution was perfused through the microfluidic device at 5 microliters/sec for 8 min. Culture medium was then flowed through the microfluidic device at 5 microliters/sec for 5 min.

**Priming regime.** 250 microliters of 100% carbon dioxide was flowed in at a rate of 12 microliters/sec. This was followed by 250 microliters of PBS containing 0.1% Pluronic^{®} F27 (Life Technologies^{®} Cat# P6866), flowed in at 12 microliters/sec. The final step of priming included 250 microliters of PBS, flowed in at 12 microliters/sec. Introduction of the culture medium follows.

**Perfusion regime.** The perfusion method was either of the following two methods:
1. Perfuse at 0.01 microliters/sec for 2h; perfuse at 2 microliters/sec for 64 sec; and repeat.
2. Perfuse at 0.02 microliters/sec for 100 sec; stop flow 500 sec; perfuse at 2 microliters/sec for 64 sec; and repeat.

### Example 1. Preparation of a functionalized surface of an unpatterned silicon wafer.

A silicon wafer (780 microns thick, 1cm by 1cm) was treated in an oxygen plasma cleaner (Nordson Asymtek) for 5 min, using 100W power, 240 mTorr pressure and 440 sccm oxygen flow rate. The plasma treated silicon wafer was treated in a vacuum reactor with (11-azidoundecyl) trimethoxy silane (300 microliters) in a foil boat in the bottom of the vacuum reactor in the presence of magnesium sulfate heptahydrate (0.5g, Acros Cat. # 10034-99-8), as a water reactant source in a separate foil boat in the bottom of the vacuum reactor. The chamber was then pumped to 750 mTorr using a vacuum pump and then sealed. The vacuum reactor was placed within an oven heated at 110°C for 24-48 h. This introduced a modified surface to the wafer, where the modified surface had a structure of Formula I:

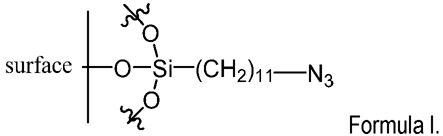

After cooling to room temperature and introducing argon to the evacuated chamber, the wafer was removed from the reactor. The wafer was rinsed with acetone, isopropanol, and dried under a stream of nitrogen. Confirmation of introduction of the modified surface was made by ellipsometry and contact angle goniometry.

Alternatively, the silicon wafer was cut to size to fit within the bottom of a flat bottomed wellplate before introducing the functionalized surface of Formula I upon it, and a plurality of the formatted silicon wafers were functionalized at the same time.

### Example 2. Preparation of a planar unpatterned silicon wafer having a streptavidin functionalized surface.

The product silicon wafer from Example 1, having a surface of Formula I as described above, was treated with dibenzylcyclooctynyl (DBCO) Streptavidin (SAV), Nanocs, Cat. # SV1-DB-1, where there are 2-7 DBCO for each molecule of SAV) by contacting the silicon wafer with an aqueous solution containing a 2 micromolar solution of the commercially available DBCO-SAV. The reaction was allowed to proceed at room temperature for at least 1 h. The unpatterned silicon wafer having a modified surface of Formula II was then rinsed with 1xPBS.

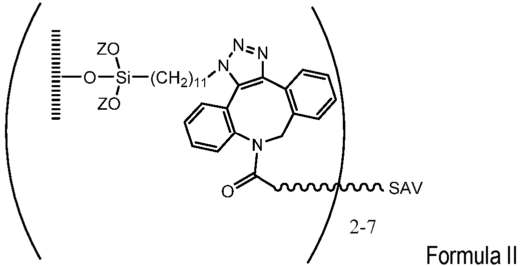

### Example 3. Synthesis of DBCO-Labeled Streptavidin (SAV) Compound 1

5 mg of lyophilized SAV (ThermoFisher PN#S888) was dissolved into 1 mL of 1X PBS (Gibco) and 1 mL of 2 mM Na₂CO₃ (Acros) in 1X PBS. 10 mg of neat DBCO-PEG13-NHS (Compound 2, Click Chemistry Tools PN# 1015-10) was dissolved into 0.4 mL of dry DMSO. 16 uL of the DBCO-PEG13-NHS solution was added to the SAV solution and mixed at 400 RPM at 25°C for 4 h on an Eppendorf ThermoMixer. The labeled SAV (Compound 1) was purified from the DBCO-PEG13-NHS by passing the reaction mixture through Zeba size exclusion chromatography spin columns (ThermoFisher PN# 89882), and used without further purification.

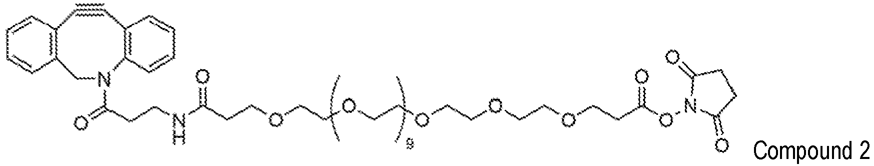

### Example 4. Preparation of a planar unpatterned silicon wafer having a streptavidin functionalized surface.

The product silicon wafer from Example 1, having a surface of Formula I as described above, was treated with Compound 1 (DBCO linked Streptavidin (SAV), product of Example 3, having a PEG 13 linker, where there are 2-7 DBCO for each molecule of SAV) by contacting the silicon wafer with an aqueous solution containing 2 micromolar Compound 1. The reaction was allowed to proceed at room temperature for at least 1 h. The unpatterned silicon wafer having a streptavidin covalently functionalized surface including a PEG 13 linker was then rinsed with 1xPBS.

### Example 5. Comparison of functionalization of silicon wafers with commercially available DBCO linked SAV compared with functionalization using Compound 1.

Comparison of the SAV modified surfaces was made by ellipsometry and contact angle goniometry after each step of introduction of reactive azide moieties (Example 1); introduction of respective SAV layers; followed by introduction of biotinylated anti-CD28, where the concentrations of reagents and reaction conditions were the same. Sample 2, using the DBCO SAV reagent having a linker including a PEG13 moiety, clearly provided a more robust functionalization of SAV than that of Sample 1, and subsequently, more robust functionalization by biotinylated anti-CD2 binding to the SAV binding sites.

| Sample | Thickness of azide layer (Angstroms) | Overall Thickness after DBCO SAV coupling (Angstroms) | Overall Thickness after anti-CD 28 binding (Angstroms) | Thickness of SAV layer introduced (Angstroms) | Thickness of anti-CD28 layer introduced (Angstroms) |
|---|---|---|---|---|---|
| Wafer 1, from Ex. 2 | 13.31 | 25.22 | 28.54 | 11.91 | 3.32 |
| Wafer 2, from Ex.4 | 13.43 | 45.08 | 62.37 | 31.65 | 17.29 |

Without being bound by theory, it was shown that a linker having a length of at least 5 PEG repeat units up to about 20 PEG repeat units (alternatively, a linker having a length from about 20 Angstroms to about 100 Angstroms) may provide superior levels of coupling to the reactive moieties on this reactive surface of the silicon wafer. This is further demonstrated by the additional thickness of the layer of anti-CD28 introduced in Sample Wafer 2, as more SAV binding sites were available.

### Example 6. Preparation of planar patterned surfaces, and further elaboration to provide antigen presenting surfaces within a plurality of regions separated by a differing region having no activation functionalization.

Indium tin oxide (ITO) wafers were fabricated to have a patterned plurality of regions of amorphous silicon upon the ITO. The regions were a.) 1 micron diameter round amorphous silicon regions separated by three microns from each other or b.) 2 micron square amorphous silicon regions separated by 2 microns from each other. FIGS. 6A and 6B show SEM images of portions of each type of patterned surface.

The patterned wafers were cleaned prior to functionalization by sonication for 10 minutes in acetone, rinsed with deionized water, and dried (Step 1 of FIG. 6). The patterned wafers were then treated in an oxygen plasma cleaner (Nordson Asymtek) for 5 min, using 100W power, 240 mTorr pressure and 440 sccm oxygen flow rate.

A **schematic representation of the functionalization process is shown in** **FIG. 6****. Initial covalent modification of the ITO surface.** The indium tin oxide base layer of the wafer was functionalized by reaction with 40 mM undecynyl phosphonic acid (Sikemia Catalog #SIK7110-10) in 50% N-methylpyrrolidine (NMP)/water solution (Step 2 of FIG. 6). The cleaned surface of the wafers was submerged in the solution within a vial and sealed. The vial was maintained in a 50°C water bath overnight. The next day, the wafers were removed and washed with 50% isopropyl alcohol/water, followed by isopropyl alcohol.
**A. Biotinylation of the ITO region of the patterned surface.** The alkyne functionalized ITO region was further covalently modified by reaction with 1.5 mM biotin linked to an azido reactive moiety (azide-S-S-biotin, Broadpharm Catalog # BP-2877), 0.5 mM sodium ascorbate; and 1mM Cu(II)SO₄/THPTA in water (Step 3 of FIG. 6). Care was taken to premix the copper ligand and sodium ascorbate prior to contact with the disulfide containing biotin reagent. The surfaces were allowed to remain in contact with the biotin reagent solution for one hour. The surfaces of the wafers were then washed with water, and dried, in preparation for the next step.

**B. Covalent modification of the surfaces of the plurality of amorphous silicon regions of the patterned wafer.** The patterned wafers having biotinylated surface within the ITO region of the wafer was treated in a vacuum reactor with (11-azidoundecyl) trimethoxy silane (Compound 3, 300 microliters) in a foil boat in the bottom of the vacuum reactor in the presence of magnesium sulfate heptahydrate (0.5g, Acros Cat. # 10034-99-8), as a water reactant source in a separate foil boat in the bottom of the vacuum reactor (Step 4 of FIG. 6). The chamber was then pumped to 750 mTorr using a vacuum pump and then sealed. The vacuum reactor was placed within an oven heated at 110°C overnight. This introduced a modified surface to the plurality of amorphous silicon regions on the wafer, where the modified surface had a structure of Formula I:

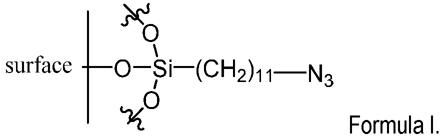

After cooling to room temperature and introducing argon to the evacuated chamber, the wafer was removed from the reactor. The wafer was rinsed with acetone, isopropanol, and dried under a stream of nitrogen. Metrology showed that the biotinylated ITO region of the patterned wafer did not have substantial amounts of contamination of the functionalizing ligands of Formula I; 10% or less contaminant was found.

**C. Covalent modification of the biotin-modified ITO region of the patterned wafer to provide supportive moieties.** The patterned wafers having a plurality of undecyl azido modified amorphous silicon regions separated by a biotin modified ITO region was reacted with a solution of streptavidin (SAV, 3.84 micromolar) in PBS containing 0.02% sodium azide and allowed to incubate for 30 min (Step 5 of FIG. 6). The wafer was then washed with PBS and dried.

The streptavidin modified surface of the ITO region of the patterned wafers is then modified by reaction with a 200 micromolar solution of biotin-RGD (Anaspec Catalog # AS-62347) in PBS containing 0.02% sodium azide, thereby providing adhesive moieties for general improvement in viability of the T lymphocytes when cultured upon these surfaces (Step 6 of FIG. 6). After incubating for 45 min, the wafers are rinsed with PBS and then dried.

**Further generalization.** The streptavidin modified surface of the ITO region of the patterned wafers may alternatively be modified by reaction with a 200 micromolar solution of biotin-PEG-5K (Jenkem Catalog # M-BIOTIN-5000) to provide hydrophilic moieties within this non- activating region of the patterned surface. Further the streptavidin surface may be modified by a mixture of the adhesive and hydrophilic moieties by reacting the streptavidin surface with a mixture of 200 micromolar stock solutions of the biotinylated moieties, in any ratio, e.g., 1:1: 1:10; 10:1 or any ration therebetween.

**D.** Providing a secondary functionalized surface to the plurality of azido functionalized amorphous silicon regions of the patterned wafers. A solution of DBCO-SAV (Nanocs Catalog # SV1-DB-1, 2 micromolar) in PBS containing 0.02% sodium azide was contacted with the patterned wafer having a plurality of azido-functionalized amorphous silicon regions separated by a region of ITO having supportive moieties (e.g., adhesive motifs such as RGD, or hydrophilic moieties such as PEG-5K) covalently attached thereupon, for an incubation period of 30 min, providing a plurality of amorphous regions having a streptavidin functionalized surface separated by the region of supportively modified ITO surface (Step 7 of FIG. 6). The patterned wafers were maintained in PBS/0.02% sodium azide until final introduction of the antigen activating ligands.

**E.** Functionalization of streptavidin modified surfaces of the amorphous silicon regions of the patterned wafer (Step 8 of FIG. 6). Stepwise functionalization is performed similarly as in Example 12, first exposing the patterned surfaces to biotinylated Monomer MHC (HLA-A* 02:01 MART-1 (MBL International Corp., Catalog No. MR01008, ELAGIGILTV) in solution, and incubation for 45 minutes. After rinsing the patterned wafer having a plurality of MHC modified amorphous silicon regions with Wash Buffer, the patterned wafer is then contacted with a solution of biotinylated anti-CD28 (Miltenyi Biotec, Catalog # 130-100-144) and incubated for 30- 45 minutes to provide a plurality of pMHC/anti-CD28 regions separated by a supportively modified ITO region of the patterned wafer.

### Example 7. Activation of CD8+ T lymphocytes using a patterned surface containing a plurality of regions having pMHC and anti-CD28 connected thereto, separated by a region having supportive moieties connected thereto compared to an unpatterned surface.

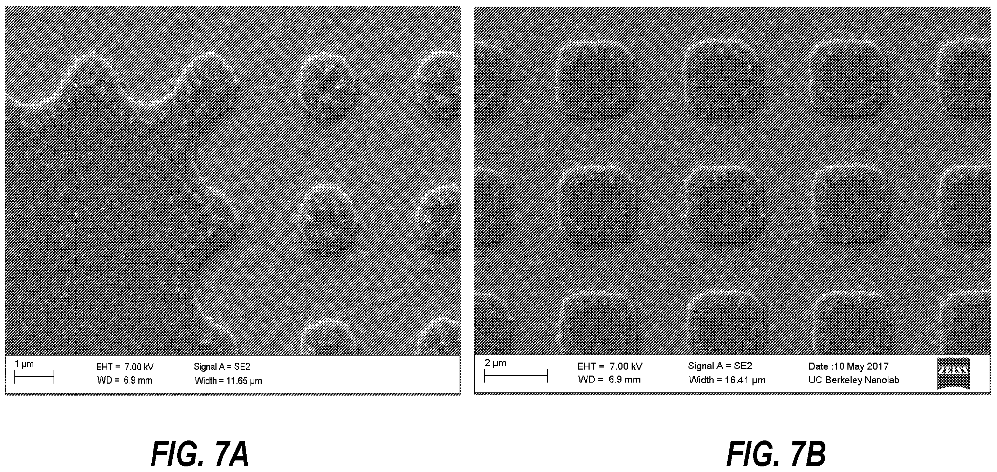

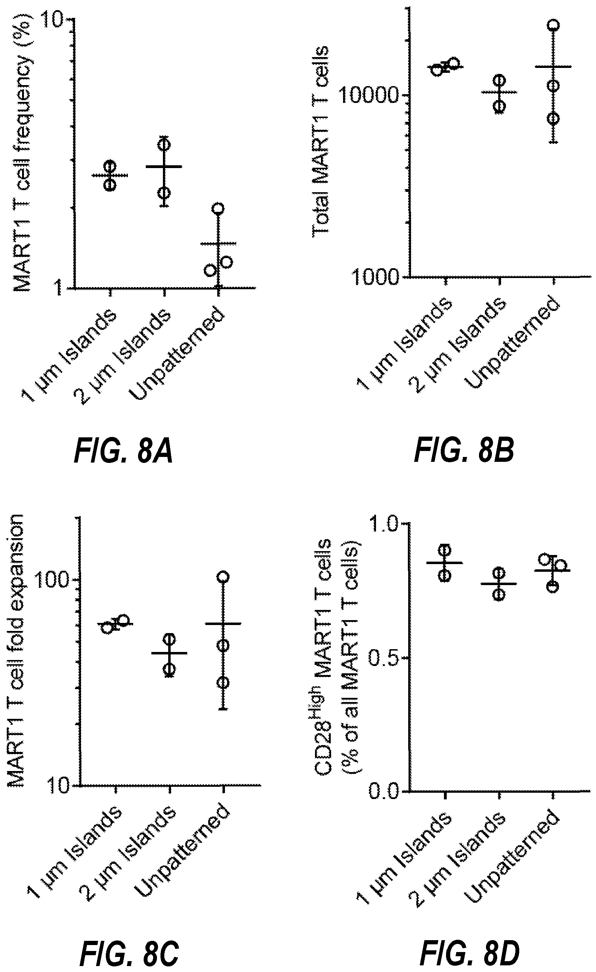

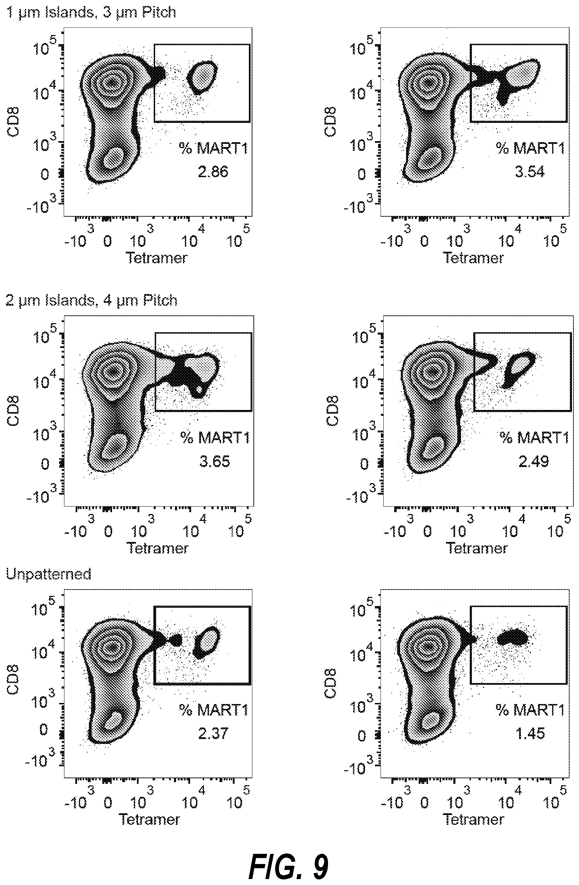

The product patterned wafer of Example 6 was sized for placement within the bottom of a well of a 48-well plate. Two different patterned surfaces were used, the first having circular regions having a diameter of 1 micron, such as shown in FIG. 7A and the second having square regions of 2 microns each side as shown in FIG. 7B. A planar third surface, unpatterned, having a random distribution of the same level of MHC peptide modification and anti-CD28 was used in a third well of a third 48 well plate. Only one level of anti-CD28 antibody loading to the planar surfaces was used. Naïve T-lymphocytes, obtained as described in Example 12 (below) were disposed within the wells of the well plates and in contact with the patterned or unpatterned wafer. The stimulation protocol and culturing protocol were each performed as in Example 12 for days 1-7. Flow cytometry analysis showed activation of the naïve T lymphocytes. The flow cytometry results as shown in FIGS. 8A-8D show that each of the three kinds of surfaces can activate T lymphocytes. Graphical characterization shown in FIGS. 8A-D for these three conditions of activation show that phenotypic specificity is obtained. FIG. 8A shows the percentage of antigen specific (MART1) T cells found in the product cell populations for each of the 1 micron activating island patterns, 2 micron activating island patterns, and unpatterned wafer surface. FIG. 8B shows the total number of MART 1 antigen specific T cells found in each of the resultant cell populations for each surface type. FIG. 8C shows the fold expansion of MART 1 antigen specific T cells for each of the surface type. FIG. 8D shows the percentage of CD28^{high} expressing antigen specific T cells within the antigen specific T cell population for each surface type. The patterned surfaces demonstrate more reproducible and controllable amounts of expansion, phenotype and actual numbers of cells. The smaller 1 micron regions may more effectively mimic the natural preferred arrangement of presented antigen, MHC molecule and anti-CD 28. See FIG. 9.

After the first period of stimulation is complete, the resultant T cells are restimulated for Days 7-14, and optionally for Days 14-21 within the same well of the well plate, adding the cytokine additions as above. Alternatively, on Day 7 and/or Day 14, the resultant T lymphocytes may be moved to a new well having a fresh patterned wafer, and the protocol of Example 12 continued. At the end of the desired repetitions of stimulation, a portion of the cells may be stained and examined by flow cytometry to determine the extent of activation. It is expected that the patterned surface wafers stimulate T cell activation as readily as bead-based activation or activation by antigen presenting Dendritic cells.

### Example 8. Preparation of a microfluidic device having modified interior surfaces of Formula I.

A microfluidic device (Berkeley Lights, Inc.) as described in the general experimental section above, having a first silicon electrode activation substrate and a second ITO substrate on the opposite wall, and photopatterned silicone microfluidic circuit material separating the two substrates, was treated in an oxygen plasma cleaner (Nordson Asymtek) for 1 min, using 100W power, 240 mTorr pressure and 440 sccm oxygen flow rate. The plasma treated microfluidic device was treated in a vacuum reactor with 3-azidoundecyl) trimethoxy silane (300 microliters) in a foil boat in the bottom of the vacuum reactor in the presence of magnesium sulfate heptahydrate (0.5g, Acros), as a water reactant source in a separate foil boat in the bottom of the vacuum reactor. The chamber was then pumped to 750 mTorr using a vacuum pump and then sealed. The vacuum reactor was placed within an oven heated at 110°C for 24- 48 h. This introduced a modified surface to the microfluidic device, where the modified surface had a structure of Formula I:

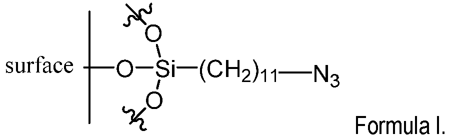

After cooling to room temperature and introducing argon to the evacuated chamber, the microfluidic device was removed from the reactor. The microfluidic device having the functionalized surface was rinsed with at least 250 microliters of deionized water, and was ready for further use.

### Example 9. Introduction of a T-cell activating surface within a microfluidic device.

A. The internal surfaces of an OptoSelect microfluidic device were covalently modified to include azido moieties as in Example 8 (Formula I). To functionalize the surface with streptavidin, the OptoSelect microfluidic device is first flushed repeatedly with 100% carbon dioxide, and then loaded with DBCO-streptavidin solution having a concentration from about 0.5 to about 2 micromolar, as produced in Example 4. After incubation for 15-30 minutes, during which the DBCO and azide groups coupled, the OptoSelect microfluidic device is washed repeatedly with 1X PBS to flush unbound DBCO-modified streptavidin.

This streptavidin surface is then further modified with biotinylated pMHC, and a selection of biotinylated anti CD28, biotinylated anti CD2 or any combination thereof. These molecules are suspended in PBS with 2% Bovine Serum Albumin at concentrations of about 1 - 10 micrograms/mL, in a ratio of pMHC molecules to antiCD28/antiCD2 from about 2:1 to about 1:2. This solution is perfused through the OptoSelect microfluidic device having streptavidin functionalized surfaces, facilitating conjugation to the surface. After one hour of incubation, the OptoSelect microfluidic device is flushed with PBS or media prior to loading cells.

**B.** Alternatively, biomolecules of interest are conjugated via biotin modification of the biomolecules to streptavidin prior to reaction with the azido-modified surfaces of the OptoSelect microfluidic device. DBCO-streptavidin and biotinylated biomolecule are prepared separately in PBS solution at concentrations in the range of 0.5 - 2 micromolar, then mixed at any desired ratio, as described below. After allowing the biotinylated biomolecules to conjugate to the streptavidin for at least 15 minutes, this complex is used to modify the surface of an azido-modified OptoSelect microfluidic device as described above.

Cells may be imported into the microfluidic device having at least one antigen-presenting inner surface and activated during periods of culturing similarly as described for activation of T cells with antigen-presenting beads of Example 19.

### Example 10. Covalent modification and functionalization of silica beads.

**10A. Silica beads having covalent PEG 3 disulfide biotin linked to streptavidin.** Spherical silica beads (2.5 micron, G biosciences Catalog # 786-915, having a substantially simple spherical volume, e.g. the surface area of the bead is within the range predicted by the relationship 4πr² +/- no more than 10%) were dispersed in isopropanol, and then dried. The dried beads were treated in an oxygen plasma cleaner (Nordson Asymtek) for 5 min, using 100W power, 240 mTorr pressure and 440 sccm oxygen flow rate. The cleaned beads were treated in a vacuum reactor with (11-azidoundecyl) trimethoxy silane ( 300 microliters) in a foil boat in the bottom of the vacuum reactor in the presence of magnesium sulfate heptahydrate (0.5g, Acros Cat. # 10034-99-8), as a water reactant source in a separate foil boat in the bottom of the vacuum reactor. The chamber was then pumped to 750 mTorr using a vacuum pump and then sealed. The vacuum reactor was placed within an oven heated at 110°C for 24-48 h. This introduced a covalently modified surface to the beads, where the modified surface had an azide functionalized structure of Formula I:

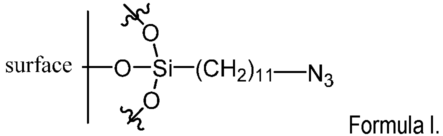

After cooling to room temperature and introducing argon to the evacuated chamber, the covalently modified beads were removed from the reactor. The beads having a covalently modified surface functionalized with azide reactive moieties were rinsed with acetone, isopropanol, and dried under a stream of nitrogen. The covalently modified azide functionalized beads were dispersed at a concentration of 1 mg/20 microliters in a 5.7 mM DMSO solution of dibenzylcyclooctynyl (DBCO) S-S biotin modified-PEG3 (Broadpharm, Cat. # BP-22453) then incubated at 90°C/2000 RPM in a thermomixer for 18 hours. The biotin modified beads were washed three times each in excess DMSO, then rinsed with PBS. The biotin modified beads in PBS were dispersed in PBS solution containing approximately 30 micromoles/700 microliter concentration streptavidin. The reaction mixture was shaken at 30°C/2000 RPM in a thermomixer for 30 minutes. At the completion of the reaction period, the covalently modified beads presenting streptavidin were washed three times in excess PBS. FTIR analysis determined that SAV was added to the surface (Data not shown). The disulfide containing linker may be particularly useful if cleavage from the surface may be desirable. The disulfide linker is susceptible to cleavage with dithiothreitol at concentrations that were found to be compatible with T lymphocyte viability (Data not shown).

10B. Silica beads having covalent **PEG4 biotin linked to streptavidin diluted with PEG5-carboxylic acid surface-blocking molecular ligands.** Beads having a covalently modified surface functionalized with azide reactive moieties of Formula 1, prepared as above in Example 10A, were rinsed with acetone, isopropanol, and dried under a stream of nitrogen. The covalently modified azide functionalized beads were dispersed at a concentration of 1 mg/10 microliters in a DMSO solution of 0.6 mM dibenzylcyclooctynyl (DBCO)-modified-PEG4-biotin (Broadpharm, Cat. # BP-22295), 5.4 mM dibenzylcyclooctynyl (DBCO)-modified-PEG5-carboxylic acid (Broadpharm, Cat. # BP-22449), and 100 mM sodium iodide then incubated at 30°C/1.000 RPM in a thermomixer for 18 hours. The biotin modified beads were washed three times each in excess DMSO, then rinsed with PBS. The biotin modified beads in PBS were dispersed in PBS solution containing approximately 10 nanomoles/1 milliliter concentration streptavidin. The reaction mixture was shaken at 30°C/1000 RPM in a thermomixer for 30 minutes. At the completion of the reaction period, the covalently modified beads presenting streptavidin were washed three times in excess PBS. FTIR analysis determined that SAV was added to the surface (Data not shown).

### Example 11. Preparation of an antigen presenting surface of a polymeric bead.

Streptavidin functionalized (covalently coupled) convoluted spherical polymeric beads (e.g., the actual surface area of the bead is greater than the relationship surface area = 4πr² +/- no more than 10%, DynaBeads^{™} (ThermoFisher Catalog # 11205D, bead stock at 6.67e8/mL)) were delivered (15 microliters; 1e7 beads) to a 1.5 mL microcentrifuge tube with 1mL of Wash Buffer (DPBS (No Magnesium ⁺², No Calcium ⁺², 244 mL); EDTA (1ml, final concentration 2mM); and BSA (5ml of 5%, final concentration 0.1%), and separated using a magnetic DynaBead rack. The wash/separation with 1 mL of the Wash Buffer was repeated, and a further 200 microliters of Wash Buffer was added with subsequent pulse centrifugation. Supernatant Wash Buffer was removed.

Wash Buffer (600 microliters) containing 1.5 micrograms biotinylated Monomer MHC (HLA-A* 02:01 MART-1 (MBL International Corp., Catalog No. MR01008, ELAGIGILTV) was dispensed into the microcentrifuge tube, and the beads were resuspended by pipetting up and down. The monomer was allowed to bind for 30 min at 4°C. After 15 minutes, the mixture was pipetted up and down again. The tube was pulse centrifuged and the supernatant liquid removed, and the tube was placed within the magnetic rack to remove more supernatant without removing beads.

A solution of biotinylated anti-CD28 (Miltenyi Biotec, Catalog # 130-100-144, 22.5 microliters) in 600 microliters Wash Buffer was added to the microcentrifuge tube. The beads were resuspended by pipetting up and down. The beads were incubated at 4°C for 30 min, resuspending after 15 min with another up and down pipetting. At the end of the incubation period, the tube was briefly pulse centrifuged. After placing back into the magnetic rack, and allowing separation for 1 min, the Buffer solution was aspirated away from the functionalized beads. The MHC monomer/anti-CD28 antigen presenting beads were resuspended in 100 microliters Buffer Wash, stored at 4°C, and used without further manipulation. The 1e7 2.80 micron diameter functionalized DynaBeads have a nominal (ideal predicted surface area of a sphere) surface area of about 24e6 square microns available for contact with T lymphocyte cells. However, the convolutions of this class of polymeric bead which are not necessarily accessible by T lymphocyte cells, are also functionalized in this method. Total ligand count may not reflect what is available to contact and activate T lymphocyte cells.

The MHC monomer/anti-CD28 antigen presenting beads were characterized by staining with Alexa Fluor 488- conjugated Rabbit anti Mouse IgG (H+L) Cross- adsorbed secondary antibody (Invitrogen Catalog # A-11059) and APC- conjugated anti-HLA-A2 antibody (Biolegend Catalog # 343307), and characterized by flow cytometry.

### Example 12. Activation of CD8+ T lymphocytes by antigen presenting beads compared to activation CD8+ T lymphocytes by dendritic cells.

Cells: CD8+T lymphocytes were enriched in a medium including RPMI plus 10% fetal bovine serum (FBS) from commercially available PBMCs following manufacturer's directions for EasySep ^{™} Human CD8 Positive Selection Kit II, commercially available kit from StemCell Technologies Canada Inc. (Catalog # 17953), by negative selection.

Dendritic cells: were generated from autologous PBMCs. Autologous PBMCs (10-50 e6) were thawed into 10 mL of pre-warmed RPMI media including 10% FBS. Cells were pelleted by centrifuging for 5 mins at 400xg. Cells were resuspended in RPMI and counted.

The cells were enriched for monocytes using negative bead isolation (EasySep^{™} Human Monocyte Isolation Kit, StemCell Technologies, Catalog # 19359), according to manufacturer's instructions. The resulting monocytes were counted, providing about a 5% yield, and then plated at 1.5- 3 e6 cells per 3mL per well in AIM-V^{®} Medium (ThermoFisher Catalog # 12055091) containing 17ng/mL IL-4 and 53 ng/mL Human Granulocyte Macrophage Colony-Stimulating Fact (GM-CSF, ThermoFisher Catalog # PHC2013)). The cells were incubated for a total of 6 days at 37°C. At Day 2 and Day 4, 100 microliters of feeding media (AIM-V^{®} Medium plus IL-4 (167 U/mL) and GM-CSF (540ng/mL)) was added to each well, and incubation was continued.

On Day 6, 0.5 mL of a maturation cocktail was added to each well. The maturation cocktail included 10ng/mL TNF-alpha; 2ng/mL IL-1B; 1000 U/mL IL-6, 1000 ng/mL PGE2; 167 U/mL IL-4 and 267 U/mL GM-CSF in AIM-V^{®} Medium. The cells were incubated for a further 24h at 37°C. Mature DCs were then collected from the maturation medium, counted, and prepared for further use. The DCs were characterized by staining for CD3 (BD Catalog # 344828), DC-SIGN (CD209, Biolegend Catalog # 330104), CD14 (Biolegend Catalog # 325608), CD86 (BD Catalog # 560359), Fc Block (BioLegend Catalog # 422302), and viability (BD Catalog # 565388); suspended in FACS buffer; and examined by FACS flow cytometry.

Dendritic cells presenting antigen were prepared by plating at a concentration of 2e6/mL in 1% HSA, and pulsing with antigen (MART1 peptide, Anaspec, custom synthesis, 40 micrograms/mL) and beta2-microglobulin (Sigma Aldrich Catalog # M4890, 3 micrograms/mL), and then culturing with agitation for 4h. The pulsed DCs were irradiated in a Faxitron CellRad^{®} x-ray cell irradiator for 30 min before use, with a target dose of 50 greys.

Culture medium and diluent for reagent additions: Advanced RPMI (ThermoFisher Catalog #12633020, 500mL); 1x GlutaMAX (ThermoFisher Catalog # 35050079, 5mL); 10% Human AB serum (zen-bio, Catalog # HSER-ABP 100mL, 50mL); and 50nM beta-mercaptoethanol (ThermoFisher Catalog # 31350010, 50nm stock, 0.5 mL, final conc 50 micromolar).

Experimental Setup: For each activating species, a single 96-well tissue-culture treated plate (VWR Catalog # 10062-902) was used (wellplate 1 (DCs); wellplate 2 (Antigen presenting beads)). CD8+ T lymphocytes (2e5) (80-90% pure) were added to each individual well.

Pulsed DCs were added at 5e3 for each well in wellplate 1, yield a 1:40 ratio of DCs: CD8+ T lymphocytes.

Antigen presenting surface polymeric beads (2e5), prepared as in Example 11, presenting pMHC including MART1 and anti-CD28 antibody, were added to each of the wells in wellplate 2. pMHC was loaded at 1.5 micrograms/1e7 beads. Anti-CD 28 antibody was loaded on the beads at three different levels: 0.25 micrograms/1e7 beads; 0.75 micrograms/1e7 beads; and 2.25 micrograms/1e7 beads.

Each wellplate was cultured at 37°C. On day 0, IL-21 (150ng/milliliter) in CTL media, was added to each well of wellplates 1 and 2, providing a final concentration in each well of 30 ng/mL. On day 2, IL21 was added to each well of the wellplates, to a final concentration of 30ng/mL. Culturing was continued to day 7.

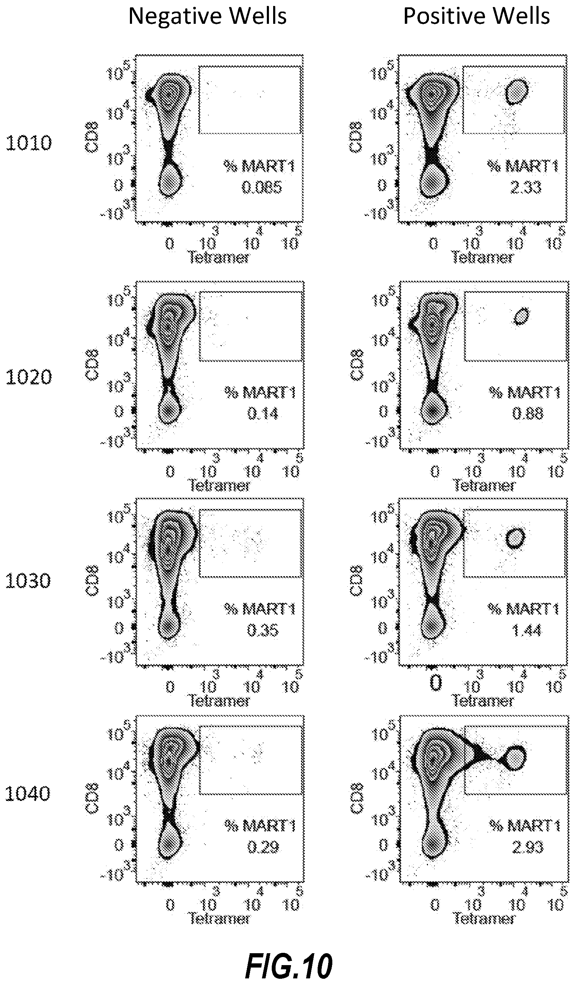

Day 7. A subset of wells from each wellplate was individually stained for MHC tetramer (Tetramer PE, MBL Catalog # T02000, 1microliter/well), CD4 (Biolegend Catalog # 300530, 0.5 microliters/well); CD8 (Biolegend Catalog # 301048, 0.5 microliters/well); CD28 (Biolegend Catalog # 302906, 0.31 microliters/well); CD45RO (Biolegend Catalog # 304210, 0.63 microliters/well); CCR7 (CD197, Biolegend Catalog # 353208, 0.5 microliters/well); and viability (BD Catalog # 565388, 0.125 microliters/well). Each well was resuspended with 150 microliters FACS buffer and 10 microliters of Countbright^{™} beads (ThermoFisher Catalog # C36950). FACS analysis was performed on a FACSCelesta^{™} flow cytometer (BD Biosciences). FIG. 10 shows the zebra plots for the flow cytometry analyses for CD8/MART1 phenotypes. For each row of zebra plots, 1010, 1020, 1030, and 1040, the left hand plot is a representative negative well, and the right hand plot is a representative positive well. Row 1010 are wells from the DC stimulated well plate. Rows 1020, 1030, and 1040 show results from the antigen-presenting bead stimulated well plate. Row 1020 shows the results for 0.25 micrograms/1e7 beads of anti-CD28 antibody loading and 1.5 micrograms/ /1e7 beads of pMHC. Row 1030 shows the results for 0.75 micrograms/1e7 beads anti CD28 antibody loading and 1.5 micrograms/1e7 beads of pMHC. Row 1040 shows the results for 2.25 micrograms/1e7 beads anti CD28 antibody loading and 1.5 micrograms/1e7 beads of pMHC. It can be seen that the antigen presenting beads initiate activation in a dose/responsive manner when varying the levels of anti-CD28 antibody, and that the MHC peptides loaded with MART1 are sufficient in combination with the anti-CD28 loading to activate T lymphocytes similarly to that of DCs.

Day 7. Restimulation. A second aliquot of pulsed DCs or antigen presenting beads, respectively, was delivered to each occupied well in wellplate 1 and wellplate 2. IL21 was added to each well of the wellplate to a final concentration of 30ng/mL. Culturing was continued.

Day 8. Addition of 50 microliters of IL-2 (50 IU/mL) and IL-7 (25ng/mL) was made to each well in wellplate 1 and wellplate 2 to provide a final concentration of 10 IU/mL and 5 ng/mL respectively. Culturing was continued.

Day 9. Addition of 50 microliters of IL-21(150ng/mL) was made to each occupied well of wellplate 1 and wellplate 2 to a final concentration of 30ng/mL. Culturing was continued.

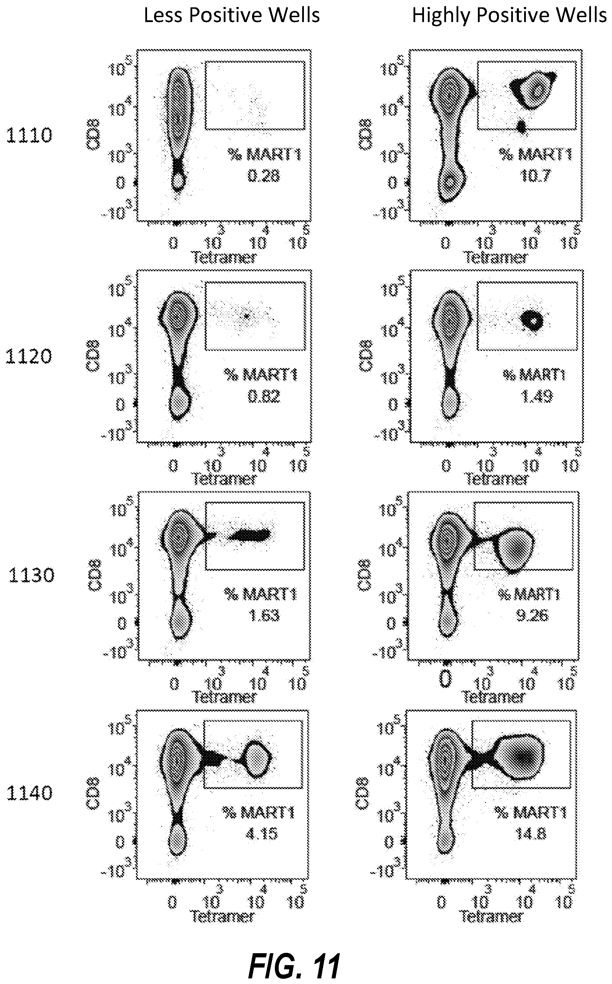

Day 14. A second subset of wells from each wellplate was individually stained and FACS sorted as described for the analysis on Day 7. The flow cytometry results are shown in FIG. 11. For each row 1110, 1120, 1130, 1140 has a representative Less Positive Well (left hand graph of each row) and a Highly Positive Well (right hand graph of each row). Row 1110 shows the amount of activation resulting from DC activation. Rows 1120, 1130, and 1140 represent results for the increasing amounts of anti-CD 28 as discussed for the 7 day results. Row 1120 shows the results for 0.25 micrograms/1e7 beads of anti-CD28 antibody loading and 1.5 micrograms/1e7 beads of pMHC. Row 1130 shows the results for 0.75 micrograms/1e7 beads anti CD28 antibody loading and 1.5 micrograms/1e7 beads of pMHC. Row 1140 shows the results for 2.25 micrograms/1e7 beads anti CD28 antibody loading and 1.5 micrograms/1e7 beads of pMHC. It is notable that for antigen-presenting beads having increasing amounts of costimulatory ligands, there are no wells having no antigen specific T cells. Particularly at the 0.75 microgram and 2.25 microgram CD28 antibody loading levels (Rows 1130 and 1140), there are more significant numbers of antigen specific T cells than for the DC pulsed wells (Row 1110).

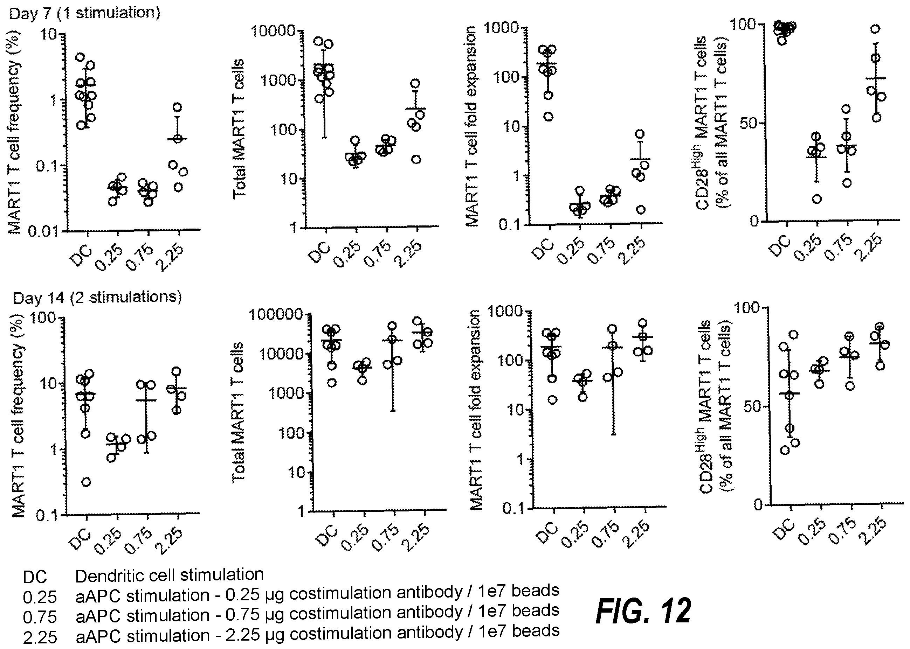

FIG. 12 shows tabularized results from these experiments. Row 1210 shows graphical representations of T cell activation characterization at Day 7. Row 1220 shows graphical representation of T cell activation characterization at Day 14. From left to right in each row, the y axis represents percentage of antigen specific T cells; total number of antigen specific T cells; antigen specific T cell fold expansion; and % of CD28 highly expressing cells within the antigen-specific T cell population. The x-axis for each graph shows the data set of each of DC, 0.25 microgram CD28 loaded beads, 0.75 microgram CD28 beads, and 2.25 microgram CD28 loaded beads. The antigen presenting bead stimulated activation appears to be initially slower than DC stimulation but production reached the same level by the end of the second culturing period. The phenotypic results show a good specificity of activation using the antigen presenting beads. FIG. 12 shows equivalent levels of MART 1 activated T lymphocytes in the antigen presenting bead initiated examples compared to the DC stimulated examples. However, using dendritic cells as activating species, there are wells that have no activated T lymphocytes after 14 days. Therefore, antigen-presenting bead activation provides more controllable and reproducible activation than dendritic cells.

**Third period of culturing.** In another experiment, where antigen presenting surfaces on beads were used as described in the immediately preceding paragraphs, but where comparison was not made with DCs, a third period of stimulation and culturing was performed. Day 14 to Day 21. Restimulation and feeding was performed as above for Day 7-Day 14, during continuation of culture conditions until Day 21. On Day 21, a last subset of wells from each wellplate was individually stained and FACS sorted as described for the analysis on Day 7. Additional activation was observed for the extended third stimulation sequence (Data not shown).

### Example 13. Preparation of covalently functionalized glass beads.

Silica beads (2.5 micron, G biosciences Catalog # 786-915, having a substantially simple spherical surface, e.g. the surface area of the bead is within the range predicted by the relationship 4πr² +/- no more than 10%) were dispersed in isopropanol, and then dried. The dried beads were treated in an oxygen plasma cleaner (Nordson Asymtek) for 5 min, using 100W power, 240 mTorr pressure and 440 sccm oxygen flow rate. The cleaned beads were treated in a vacuum reactor with (11-azidoundecyl) trimethoxy silane (300 microliters) in a foil boat in the bottom of the vacuum reactor in the presence of magnesium sulfate heptahydrate (0.5g, Acros Cat. # 10034-99-8), as a water reactant source in a separate foil boat in the bottom of the vacuum reactor. The chamber was then pumped to 750 mTorr using a vacuum pump and then sealed. The vacuum reactor was placed within an oven heated at 110°C for 24-48 h. This introduced a covalently linked surface presenting reactive azide moieties to the beads, where the modified surface has a structure of Formula I.

After cooling to room temperature and introducing argon to the evacuated chamber, the intermediate reactive azide presenting beads were removed from the reactor and were rinsed with acetone, isopropanol, and dried under a stream of nitrogen. The azide presenting reactive beads (50 mg) were dispersed in 500 microliters DMSO with vigorous vortexing/brief sonication. The beads were pelleted, and 450 microliters of the DMSO were aspirated away from the beads. The pellet, in the remaining 50 microliters DMSO was vortexed vigorously to disperse. DBCO-SAV (52 microliters of 10 micromolar concentration, Compound 1) as synthesized in Example 3, having a PEG13 linker, was added. The beads were dispersed by tip mixing, followed by vortexing. 398 microliters of PBS with 0.02% sodium azide solution was added, followed by additional vortexing. The reaction mixture was incubated overnight on a thermomixer at 30°C, 1000 RPM.

After 16 hrs, 10 microliters of 83.7 mM DBCO-PEG₅-acid were added to each sample and they were incubated an additional 30 minutes at 30°C/1,000 RPM. The beads were washed 3X in PBS/azide, then suspended in 500 microliters of the same.

These covalently functionalized beads are modified to introduce primary activating molecules and co-activating molecules as described below in Example 18.

### Example 14. Preparation of covalently functionalized polystyrene bead.

Divinylbenzene-crosslinked polystyrene beads (14-20 micron, Cospheric Catalog # 786-915) were dispersed in isopropanol, and then dried in a glass petri dish. The dried beads were treated in an oxygen plasma cleaner (Nordson Asymtek) for 40 seconds, using 100W power, 240 mTorr pressure and 440 sccm oxygen flow rate. The cleaned beads were treated in a vacuum oven with (11-azidoundecyl) trimethoxy silane (Compound 5, 900 microliters) in a foil boat on the shelf of the oven in the presence of magnesium sulfate heptahydrate (1 g, Acros Cat. # 10034-99-8), as a water reactant source in a separate foil boat on the same shelf of the oven. The oven was then pumped to 250 mTorr using a vacuum pump and then sealed. The oven was heated at 110°C for 18-24 h. This introduced a covalently modified surface to the beads, where the modified surface had a structure of Formula I:

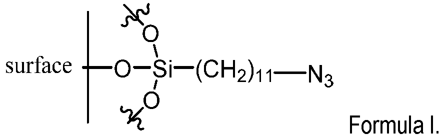

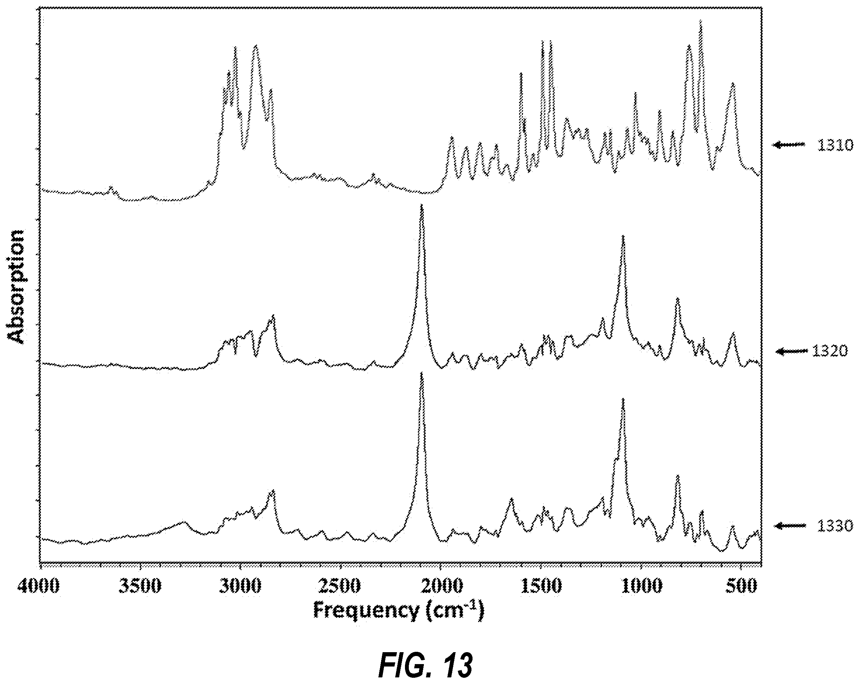

After pump-purging the oven three times, the covalently modified beads were removed from the oven and cooled. The covalently modified azide functionalized beads were dispersed at a concentration 15 mg/50 microliters in DMSO. To this, a 450 microliter solution of DECO-labeled streptavidin (SAV) (Compound 1) at a concentration of 9.9 micromolar were added. The solution was then incubated at 30°C/1000 RPM in a thermomixer for 18 hours. The SAV modified beads were washed three times in PBS, FTIR analysis determined that SAV was added to the surface as shown in FIG. 13.

FIG. 13 shows superimposed FTIR traces of the functionalized bead as the covalently functionalized surface is built up. Trace 1310 showed the original unfunctionalized surface of the polystyrene bead. Trace 1320 showed the FTIR of the surface after introduction of the azide functionalized surface (having a structure of Formula I). Trace 1330 showed the FTIR of the surface after introduction of covalently linked PEG13-streptavidin surface to the polystyrene bead. Traces 1320 and 1330 showed introduction of FTIR absorption bands consistent with the introduction of each set of chemical species in the stepwise synthesis.

### Example 15. Preparation of an antigen presenting surface of a bead with anti-CD28 and anti-CD2.

Streptavidin functionalized (covalently coupled) DynaBeads^{™} (ThermoFisher Catalog # 11205D), bead stock at 6.67e8/mL, convoluted (as described above) polymeric beads) were delivered (15 microliters; 1e7 beads) to 1.5 mL microcentrifuges tube with 1mL of Wash Buffer (DPBS (No Magnesium ⁺², No Calcium ⁺², 244 mL); EDTA (1ml, final concentration 2mM); and BSA (5ml of 5%, final concentration 0.1%), and separated using a magnetic DynaBead rack. The wash/separation with 1 mL of the Wash Buffer was repeated, and a further 200 microliters of Wash Buffer was added with subsequent pulse centrifugation. Supernatant Wash Buffer was removed.

Wash Buffer (600 microliters) containing 0.5 micrograms biotinylated Monomer MHC (HLA-A* 02:01 MART-1 (MBL International Corp., Catalog No. MR01008, ELAGIGILTV) was dispensed into the microcentrifuge tubes, and the beads were resuspended by pipetting up and down. The monomer was allowed to bind for 30 min at 4°C. After 15 minutes, the mixtures were pipetted up and down again. The tubes were pulse-centrifuged and the supernatant liquid removed, and the tubes were placed within the magnetic rack to remove more supernatant without removing beads.

Solutions of biotinylated anti-CD28 (Biolegend, Catalog # 302904) and biotinylated anti-CD2 (Biolegend, Catalog # 300204) in 600 microliters Wash Buffer were added to the microcentrifuge tubes. Solutions contained a total of 3 micrograms of antibody. The solutions contained: 3 micrograms of anti-CD28 and 0 micrograms of anti-CD2, 2.25 micrograms of anti-CD28 and 0.75 micrograms of anti-CD2, 1.5 micrograms of anti-CD28 and 1.5 micrograms of anti-CD2, 0.75 micrograms of anti-CD28 and 2.25 micrograms of anti-CD2, or 0 micrograms of anti-CD28 and 3 micrograms of anti-CD2. The beads were resuspended by pipetting up and down. The beads were incubated at 4°C for 30 min, then resuspended after 15 min with another up and down pipetting. At the end of the incubation period, the tube was briefly pulse centrifuged. After placing back into the magnetic rack, and allowing separation for 1 min, the Buffer solution was aspirated away from the functionalized beads. The MHC monomer/anti CD28 antigen presenting beads were resuspended in 100 microliters Buffer Wash, stored at 4°C, and used without further manipulation. The 1e7 2.80 micron diameter functionalized DynaBeads have a nominal surface area of about 24e6 square microns available for contact with T lymphocyte cells, but as described above, these convoluted spherical beads have a practical surface area of more than 10% above that of the nominal surface area.

### Example 16. Preparation of covalently functionalized polymeric beads. Preparation of an intermediate reactive synthetic surface.

In the first step of the manufacturing process, M-450 Epoxy-functionalized paramagnetic convoluted polymeric beads (DynaBeads^{™} , ThermoFisher Cat. # 14011 (convoluted having the same meaning as above)) were reacted with Tetrabutylammonium Azide to prepare polymeric beads presenting azide reactive moieties capable of reacting with functionalizing reagents having Click chemistry compatible reactive groups.

### Example 17. Preparation of a covalently functionalized synthetic surface of a bead.

The azide-prepared convoluted beads from Example 16 were then reacted with dibenzocyclooctynyl (DBCO)-coupled Streptavidin to attach Streptavidin covalently to the polymeric beads. The DBCO-Streptavidin reagent was generated by reacting Streptavidin with amine-reactive DBCO-polyethylene glycol (PEG)13-NHS Ester, providing more than one attachment site per Streptavidin unit.

**Further reaction with surface-blocking molecules.** The resulting covalently functionalized polymeric beads presenting streptavidin functionalities from Example 17 may subsequently be treated with DBCO functionalized surface-blocking molecules to react with any remaining azide reactive moieties on the polymeric bead. In some instances, the DBCO functionalized surface-blocking molecule may include a PEG molecule. In some instances, the DBCO PEG molecule may be a DBCO PEG5-carboxylic acid. Streptavidin functionalized polymeric beads including additional PEG or PEG-carboxylic acid surface-blocking molecules provide superior physical behavior, demonstrating improved dispersal in aqueous environment. Additionally, the surface-blocking of remaining azide moieties prevents other unrelated/undesired components present in this or following preparation steps or activation steps from also covalently binding to the polymeric bead. Finally, introduction of the surface-blocking molecular ligands can prevent surface molecules present on the T lymphocytes from contacting reactive azide functionalities.

**Further generalization.** It may be desirable to modify the azide functionalized surface of Example 16 with a mixture of DBCO containing ligand molecules. For example, DBCO-polyethylene glycol (PEG)13-streptavidin (Compound 1, prepared as in Example 3) may be mixed with DBCO-PEG5-COOH (surface-blocking molecules) in various ratios, and then placed in contact with the azide functionalized beads. In some instances, the ratio of DBCO-streptavidin molecules to DBCO - PEG5-COOH may be about 1:9; about 1:6, about 1:4 or about 1:3. Without wishing to be bound by theory, surface-blocking molecular ligands prevent excessive loading of streptavidin molecules to the surface of the bead, and further provide enhanced physicochemical behavior by providing additional hydrophilicity. The surface-blocking molecules are not limited to PEG5-COOH but may be any suitable surface-blocking molecule described herein.

### Example 18. Preparation of covalently modified antigen presenting bead. Conjugation of peptide-HLAs and monoclonal antibody co-activating molecules.

**Materials: A. Antigen bearing major histocompatibility complex (MHC) I molecule.** Biotinylated peptide-Human Leukocyte Antigen complexes (pMHC), were commercially available from MBL, Immunitrack or Biolegend. The biotinylated peptide-HLA complex included an antigenic peptide non-covalently bound to the peptide-binding groove of a Class I HLA molecule, which was produced and folded into the HLA complex at the manufacturer. The biotinylated peptide-HLA complex was also non-covalently bound to Beta2-Microglobulin. This complex was covalently biotinylated at the side chain amine of a lysine residue introduced by the BirA enzyme at a recognized location on the C-terminal peptide sequence of the HLA, also performed by the manufacturer.

**B. Co-activating molecules.** Biotinylated antibodies were used for costimulation and were produced from the supernatants of murine hybridoma cultures. The antibodies were conjugated to biotin through multiple amine functionalities of the side chains of lysines, randomly available at the surfaces of the antibodies. The biotinylated antibodies were commercially available (Biolegend, Miltenyi, or Thermo Fisher).

Biotinylated anti-CD28 useful in these experiments were produced from clone CD28.2, 15e8, or 9.3.

Biotinylated anti-CD2 useful in these experiments were produced from clone LT2 or RPA-2.10. Other clones may also be used in construction of covalently modified antigen presenting synthetic surfaces such as these polymeric beads.

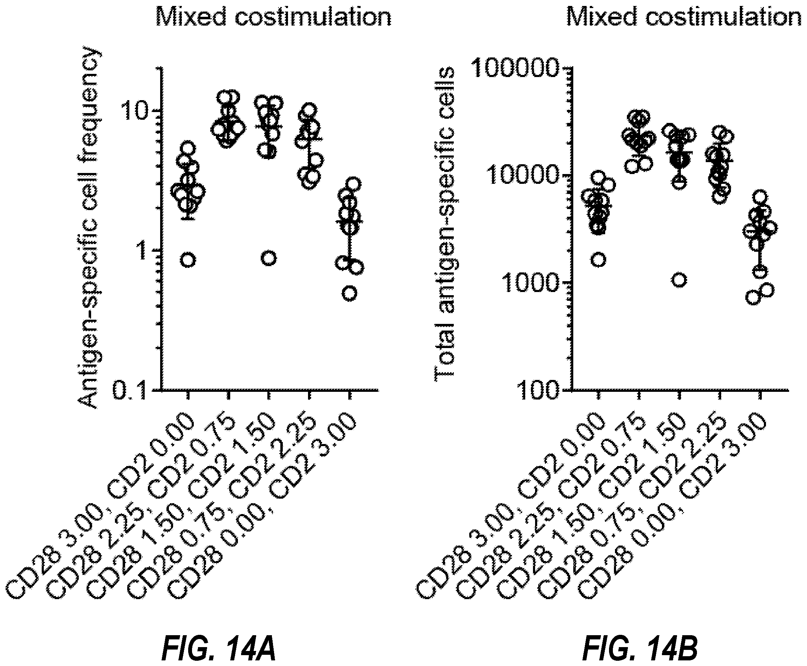

**Conjugation of the primary activating and co-activating molecules to a covalently functionalized surface of a bead.** The MHC molecule (containing the antigenic molecule) and the co-activating molecules were conjugated to beads produced in a two-step process. In various experiments, the ratio of the co-activating molecules-in this case, biotinylated anti-CD28 and biotinylated anti-CD2-may be varied in a range from about 100:1 to about 1:100; or from about 20:1 to about 1:20. In other experiments, the ratio of the co-activating molecules was from about 3:1 to about 1:3 or about 1:1. See FIGS. 14A-D.

**pMHC loading.** Streptavidin functionalized (covalently coupled) DynaBeads^{™} (ThermoFisher Catalog # 11205D), bead stock at 6.67e8/mL, convoluted (as described above) polymeric beads) were washed with Wash Buffer (Dulbecco's Phosphate-Buffered Saline without Calcium or Magnesium; 0.1% Bovine Serum Albumin; 2 mM Ethylenediaminetetraacetic Acid). Wash buffer was pipetted into a tube, to which the Streptavidin beads were added. Typically, ~1e7 beads were pipetted into 1 mL of Wash Buffer. The beads were collected against the wall of the tube using a magnet (e.g., DYNAL DynaMag-2, ThermoFisher Cat. # 12-321-D). After the beads migrated to the wall of the tube, the Wash Buffer was removed via aspiration, avoiding the wall to which the beads were held. This wash process was repeated twice more. After the third wash, the beads were resuspended at 1.67e7 beads/mL in Wash Buffer.

The beads were then mixed with pMHC. The pMHC was added to the beads in Wash Buffer at a final concentration of 0.83 micrograms of pMHC/mL. The beads and pMHC were thoroughly mixed by vortexing, then incubated at 4°C for 15 minutes. The beads were again vortexed, then incubated at 4°C for an additional 15 minutes.

**Co-activating molecule loading.** The pMHC-functionalized beads were again captured via magnet, and the pMHC reagent mixture removed by aspiration. The beads were then brought to 1.67e7/mL in Wash Buffer. Biotinylated Anti-CD28 and biotinylated anti-CD2 (if used) were then added to the beads at a final concentration of 5 micrograms/mL of total antibody. If both anti-CD28 and anti-CD2 were used, then each antibody was added at 2.5 micrograms / mL.

The beads and pMHC were thoroughly mixed by vortexing, then incubated at 4°C for 15 minutes. The beads are again vortexed, then incubated at 4°C for an additional 15 minutes.

After modification by the biotinylated antibodies, the beads were captured via magnet, and the antibody mixture was removed by aspiration. The beads were resuspended in Wash Buffer at a final density of 1e8 beads/mL. The beads were used directly, without further washing.

**Characterization.** To assess the degree of loading and homogeneity of the resulting antigen-presenting beads, the beads were stained with antibodies that bind the pMHC and co-activating CD28/CD2 (if present) antibodies on the beads. The resulting amount of staining antibody was then quantified by flow cytometry. The number of pMHC and costimulatory antibodies on the beads was then determined using a molecular quantification kit (Quantum Simply Cellular, Bangs Labs) according to the manufacturer's instructions.

To analyze the beads, 2e5 beads were added to each of two microcentrifuge tubes with 1 mL of Wash Buffer. The pMHC quantification, and costimulation antibody quantification were performed in separate tubes. In each separate experiment, the beads were collected against the wall of the tube using a magnet, and the Wash Buffer removed. The beads were resuspended in the respective tubes in 0.1 mL of Wash Buffer, and each tube was briefly vortexed to separate the beads from the wall of the tube. To detect pMHC, 0.5 microliters of anti-HLA-A conjugated to APC (Clone BB7.2, Biolegend) was added to the first tube. The first tube was again vortexed briefly to mix the beads and detection antibody. To detect the costimulation antibodies, 0.5 microliters of anti-mouse IgG conjugated to APC was added to the second tube. Depending on the costimulation antibody clones used, different anti-mouse antibodies were used, e.g., RMG1-1 (Biolegend) is used to detect CD28.2 (anti-CD28) and RPA-2.10 (anti-CD2). The detection antibodies were incubated with the beads for 30 minutes in the dark at room temperature for each tube.

For each tube, the beads were then captured against the wall of the tube via magnet, and the staining solution was removed by aspiration. 1 mL of Wash Buffer was added to each tube, then aspirated to remove any residual staining antibody. The beads in each tube were resuspended in 0.2 mL of Wash Buffer and then the beads from each tube was transferred to a 5 mL Polystyrene tube, keeping the two sets of beads separate.

To quantify the loading of the different species, the beads were analyzed on a flow cytometer (FACS Aria or FACS Celesta, BD Biosciences). First, a sample of unstained product antigen-bearing beads is collected. A gate is drawn around the singlet and doublet beads. Doublet beads are discriminated from singlet beads based on their higher forward and side scatter amplitudes. Typically, approximately 10,000 bead events were recorded. The beads stained for pMHC and costimulation antibodies are then analyzed in separate experiments. Again, approximately 10,000 bead events were collected for each sample, and the APC median fluorescence intensity (MFI) and coefficient of variation of the APC MFI (100*[Standard Deviation of the MFI]/[MFI]) of the singlet bead events was recorded.

To determine the number of pMHC and costimulation antibodies per bead, a molecular quantification kit is used. The kit (Quantum Simply Cellular (Bangs Laboratories) included a set of beads with specified antibody binding capacities (determined by the manufacturer). These beads are used to capture the detection antibody. Briefly, the quantification beads are incubated with saturating amounts of the detection binding, then washed thoroughly to remove excess antibody. The beads with different binding capacities are mixed, along with negative control beads and resuspended in Wash Buffer. The mixed beads are then analyzed by Flow Cytometry. The APC MFI of each bead with specified binding capacity is recorded, and a linear fit of the MFI vs binding capacity is generated. The MFI of the aAPCs is then used to determine the number of detection antibodies bound per aAPC. This number is equal to the number of (pMHC or costimulation) antibodies on the bead. The following table shows results for:
A. Antigen-presenting convoluted polymeric beads produced in Examples 16-17 and functionalized above in this experiment.
B. Antigen-presenting substantially spherical silica beads as produced in Example 10B

| **Condition** | **Labeling** | **Ligands / Bead** | **Ligands / sq um** | **Target antibodies (ug) / mg beads** |
|---|---|---|---|---|
| **A. Polymeric bead** | HLA | 487209 | 19781 | 8.1 |
| **A. Polymeric bead** | Costim | 426992 | 17336 | 7.1 |
| **B. 4 micron monodisperse silica (10B)** | HLA | 604471 | 12026 | 2.35 |
| **B. 4 micron monodisperse silica (10B)** | Costim | 760489 | 15129 | 2.96 |

### Example 19. Stimulation by antigen-presenting beads.

**Input cell populations.** To increase the number of antigen-specific, CD8⁺ T cells plated per well, CD8+T cells were first isolated from Peripheral Blood Mononuclear Cells using commercially available reagents. The cells can be isolated using negative selection, e.g., EasySep^{™} Human CD8+T Cell Isolation Kit (StemCell Technologies) or by positive selection, e.g., CliniMACS CD8 Reagent (Miltenyi Biotec). The CD8⁺ T cells were isolated according to the manufacturers recommended protocol. Alternatively, different subsets of T cells can be isolated, e.g., Naïve CD8⁺ T cells only, or a less-stringent purification can be performed, e.g., depletion of Monocytes by CliniMACS CD14 Reagent (Miltenyi Biotec). Alternatively, if T cells specific for a Class II-restricted antigen are desired, CD4⁺ T cells can be isolated by corresponding methods.

**First T cell stimulation period.** The enriched CD8+T cells were resuspended at 1e6/mL in media with IL-21 at 30 nanograms/mL (R&D Systems). The media used for T cells was Advanced RPMI 1640 Medium (Thermo Fisher) supplemented with 10 % Human AB Serum (Corning CellGro) plus GlutaMax (Thermo Fisher) and 50 micromolar Beta-MercaptoEthanol (Thermo Fisher) or ImmunoCult^{™}-XF T Cell Expansion Medium (StemCell Technologies).

Antigen-presenting beads were prepared as described in Example 18, where the convoluted polymeric beads were loaded at a final concentration of 0.83 micrograms of pMHC/mL. The resulting lot of beads was split into five portions, loading the costimulating ligands in the following proportions:
Set 1: CD28 at 3.00 micrograms/mL and CD2 at zero concentration.
Set 2: CD28 at 2.25 micrograms/mL and CD2 at 0.75 micrograms/mL.
Set 3: CD28 at 1.50 micrograms/mL and CD2 at 2.25 micrograms/mL.
Set 4: CD28 at 0.75 micrograms/mL and CD2 at 2.25 micrograms/mL.
Set 5: CD28 at 0.00 micrograms/mL and CD2 at 3.00 micrograms/mL.

To the T cells in media, an aliquot of each set of antigen-presenting beads were added to separate wells to a final concentration of 1 antigen-presenting bead per cell. The cells and antigen-presenting beads were mixed and seeded into tissue culture-treated, round-bottom, 96-well microplates. 0.2 mL (2e5 cells) was added to each well of the plate, which was then placed in a standard 5% CO₂, 37°C incubator. Typically, 48-96 wells were used per plate. Two days later, IL-21 was diluted to 150 nanograms/mL in media. 50 microliters of IL-21 diluted in media was added to each well, and the plate was returned to the incubator.

After culturing the cells for an additional 5 days (seven days total), the cells were analyzed for antigen-specific T cell expansion. Alternatively, the cells were-stimulated in a second stimulation period as described in the following paragraphs to continue expanding antigen specific T cells.

**Second T cell stimulation period.** From each well of the above well plate at the conclusion of the first stimulation period, 50 microliters of media were removed, being careful not to disturb the cell pellet at the bottom of the well. IL-21 was diluted to 150 ng/mL in fresh media, and the antigen-presenting beads as produced above were added to the IL-21/media mixture at a final density of 4e6 antigen-presenting beads/mL. 50 microliters of this IL-21/antigen-presenting bead/media mixture was added to each well, resulting in an additional 2e5 antigen-presenting beads being added to each well. Optionally, the wellplate can be centrifuged for 5 minutes at 400xg to pellet the antigen-presenting beads onto the cells. The wellplate was returned to the incubator.

The next day (8 days from start of stimulation experiments), the wellplate was removed from the incubator, and 50 microliters of media was removed from each well. IL-2 (R&D Systems) was diluted into fresh media to 50 Units/mL. To this, media containing IL-2, IL-7 (R&D Systems) was added to a final concentration of 25 ng/mL. 50 microliters of this IL-2/IL-7/media mixture was added to each well, and the wellplate was returned to the incubator.

The following day (9 days from start of stimulation experiments), the wellplate was removed from the incubator, and 50 microliters of media was again removed from each well. IL-21 was diluted into fresh media to 150 nanograms/mL. 50 microliters of this IL-21/media mixture was added to each well, and the wellplate was returned to the incubator.

After culturing the cells for an additional 5 days (14 days from the start of stimulation experiments), the cells were typically analyzed for antigen-specific T cell expansion. However, the cells can be re-stimulated with more antigen-presenting beads for another period of culturing as above to continue expanding antigen specific T cells.

**Analysis of antigen-specific T cell stimulation and expansion.** Once the desired number of T cell stimulations were performed, the cells were analyzed for expansion of T cells specific for the pMHC complex used to prepare the antigen-presenting beads. Antigen-specific T cells are detected using Phycoerythrin (PE) conjugated Streptavidin, which is bound to 4 pMHC complexes. These complexes are referred to as tetramers. Typically, a tetramer manufactured with the same peptide used in the pMHC of the antigen-presenting beads was used to detect the antigen-specific T cells.

To detect and characterize the antigen-specific T cells, a mixture of PE-tetramer (MBL, Intl) and antibodies specific for various cell surface markers with various fluorophores, e.g., FITC-conjugated anti-CD28, PerCP-Cy5.5-conjugated anti-CD8, was prepared in FACS Buffer (Dulbecco's Phosphate-Buffered Saline without Calcium or Magnesium; 2% Fetal Bovine Serum; 5 mM Ethylenediaminetetraacetic Acid, 10 mM HEPES). The amount of antibody used was determined by titration against standard cell samples. The surface markers typically used for characterization used are: CD4, CD8, CD28, CD45RO, CD127 and CD197. Additionally, a Live/Dead cell discrimination dye, e.g., Zombie Near-IR (Biolegend) and Fc Receptor blocking reagent, e.g., Human TruStain FcX^{™} (Biolegend) were added to distinguish live cells and prevent non-specific antibody staining of any Fc-Receptor expressing cells in the culture, respectively.

Typically, the wells were mixed using a multi-channel micro-pipettor, and 50 microliters of cells from each well were transferred to a fresh, non-treated, round-bottom, 96-well microplate. The cells were washed by addition of 0.2 mL of FACS buffer to each well. Cells were centrifuged at 400xg for 5 minutes at room temperature, and the wash removed. To each well, 25 microliters of the Tetramer, Antibody, Live/Dead, Fc blocking reagent mixture was added. The cells were stained for 30 minutes under foil at room temperature. The cells were then washed again, and finally resuspended in FACS Buffer with CountBright Absolute Counting Beads (Thermo Fisher). The cells were then analyzed by Flow Cytometry (FACS Aria or FACS Celesta, BD Biosciences). The frequency of antigen-specific T cells was determined by gating first on Single/Live cells, then gating on CD8⁺/Tetramer⁺ cells. Appropriate gating conditions were determined from control stains, such as a negative control Tetramer with no known specificity (MBL, Intl) or antibody isotype controls. Within the antigen-specific T cell population, the frequency of CD45RO+/CD28^{High} cells was determined, as well as the number of cells expressing CD127. Activated T cells, which express CD45RO, that continue to express high levels of CD28 and CD127 have been shown to include memory precursor effector cells. Memory precursor cells have been shown to be less differentiated and have higher replicative potential than activated T cells that do not express these markers.

FIG. 14A: The frequency of MART1-specific T cells (percent of live cells) 7 days after stimulation with antigen-presenting beads prepared with the indicated amount (in micrograms) of anti-CD28 and/or anti-CD2. Each point represents a well of a 96-well microplate. Data is pooled from two independent experiments.

FIG. 14B: The total number of MART1-specific T cells 7 days after stimulation with antigen-presenting beads prepared with the indicated amount (in micrograms) of anti-CD28 and/or anti-CD2. Each point represents a well of a 96-well microplate. Data is pooled from two independent experiments.

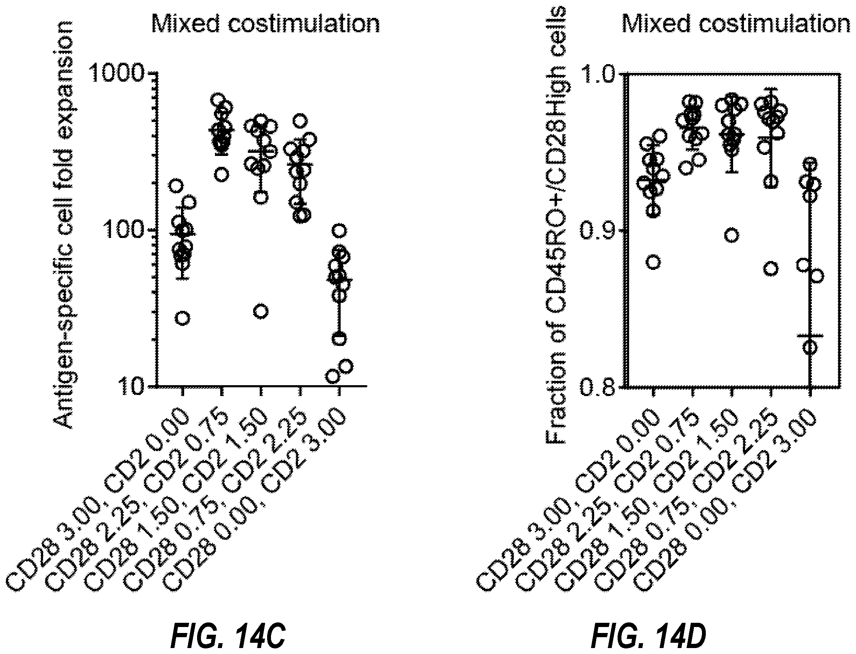

FIG. 14C: The fold expansion of MART1-specific T cells 7 days after stimulation with aAPCs prepared with the indicated amount (in micrograms) of anti-CD28 and/or anti-CD2. Each dot represents a well of a 96-well microplate. Data was pooled from two independent experiments. Fold expansion is calculated by dividing the frequency of MART1 T cells in each well at day 7 by the frequency of MART1 T cells in the sample at day 0.

FIG. 14D: The fraction of MART1-specific T cells that were positive for CD45RO and expressing high levels of CD28 7 days after stimulation with aAPCs prepared with the indicated amount (in micrograms) of anti-CD28 and/or anti-CD2. Each dot represents a well of a 96-well microplate. Data was pooled from two independent experiments. Fold expansion is calculated by dividing the frequency of MART1 T cells in each well at day 7 by the frequency of MART1 T cells in the sample at day 0.

It was observed that production of antigen specific T cells was possible with a wide range of proportions of the costimulatory ligands anti-CD28 and anti-CD2. Production was possible using only one of the two costimulatory ligands. However, a combination of anti-CD28 and anti-CD2, including at ratios of anti-CD28:anti-CD2 from about 3:1 to about 1:3, provided increased measurements of each of the above characteristics.

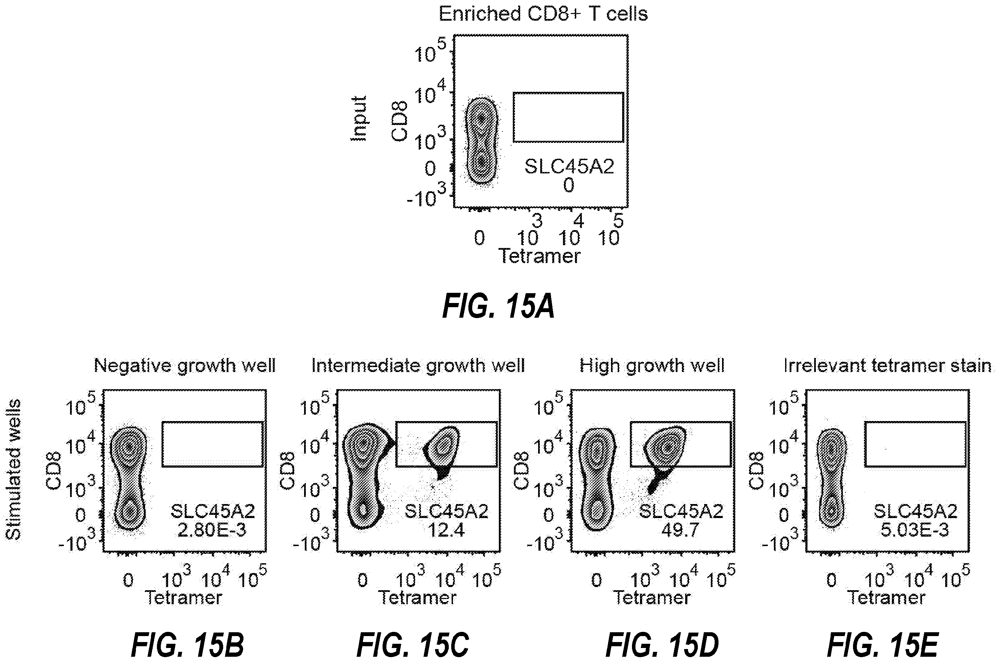

FIGS. 15A-15E: For T cells stimulated as described above, using the SLC45A2 antigen in the antigen-presenting beads produced as described above, exemplary Flow Cytometry graphs are shown. FIG. 15A showed the results of T cells, prior to stimulation ("Input"). Representative stimulated wells are shown in the lower panels: Negative growth well (FIG. 15B); intermediate growth well (FIG. 15C); High growth well (FIG. 15D); and Irrelevant Tetramer staining (FIG. 15E).

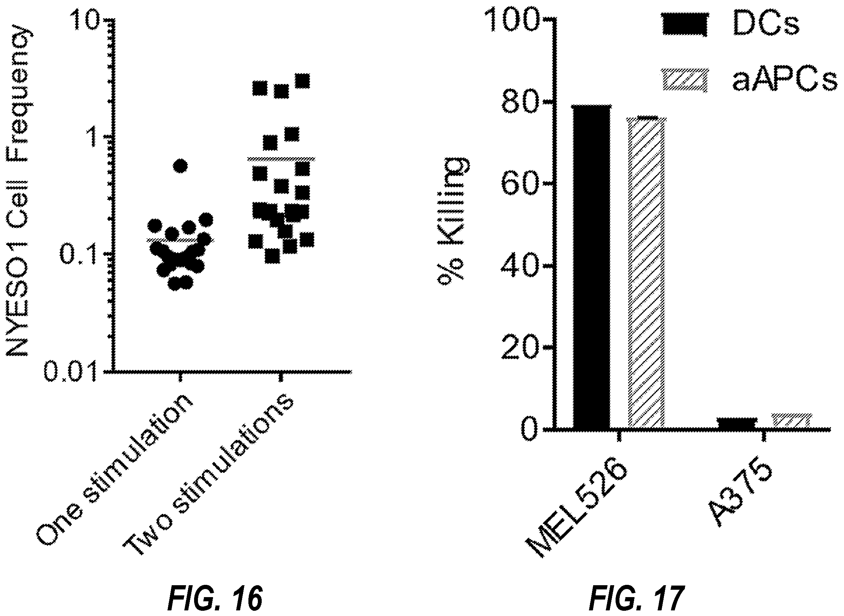

FIG. 16: For T cells stimulated as described above, using the NYESO1 antigen, the frequency of T cells positive for CD45RO and expressing high levels of CD28 are shown respectively after a single period of stimulation (7 days, left column) and after two periods of stimulation as described above (14 days, right column). Increased frequency of antigen specific activated T cells were observed.

**Cytotoxicity:** Killing of target tumor cells and non-target tumor cells by SLC45A2-specific T cells expanded using Dendritic cells pulsed with SLC45A2 antigen (DCs, Black bars) or antigen-presenting beads (presenting SLC45A2 antigen) produced as described above (gray hatched bars). See FIG. 17. Killing was measured by activation of Caspase-3 in target cells. MEL526 tumor cells express SLC45A2 and were killed by T cells expanded using both DCs and the antigen-presenting beads. A375 cells do not express SLC45A2 and were not killed by T cells expanded using DCs or the antigen-presenting beads. The antigen-presenting beads performed as well as the Dendritic cells.

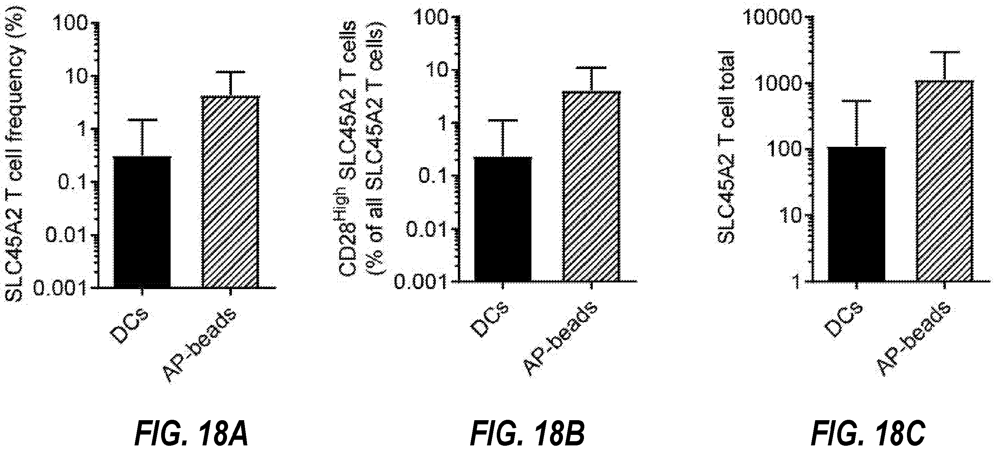

FIGS. 18A-18C show the comparison between the cell product of the dendritic cell stimulation and the antigen-presenting bead stimulated cell product. FIG. 18A showed that the percentage of Antigen Specific (AS) activated T cells is higher in the antigen-presenting bead stimulation experiment. FIG. 18B showed that the cell product of the antigen-presenting bead stimulation experiment has higher percentages of the desired CD45RO positive/highly CD28 positive phenotype, compared to that of the dendritic cell stimulated cell product. FIG. 18C showed that the actual numbers of antigen-specific T cells is higher in the cell product produced by the antigen-presenting bead stimulation experiment. Overall, antigen-presenting bead stimulation provides a more desirable cell product, and is a more controllable and cost effective method of activating T cells than the use of dendritic cell activation.

### Example 20. Preparation of an antigen-presenting bead having protein fragment co-activating ligands.

**20A. Preparation of an antigen presenting surface of a bead with lysine-biotinylated CD80 and CD58.** Streptavidin functionalized (covalently coupled, convoluted ( as described above) polymeric) DynaBeads^{™} (ThermoFisher Catalog # 11205D, bead stock at 6.67e8/mL) were delivered (15 microliters; 1e7 beads) to 1.5 mL microcentrifuges tube with 1mL of Wash Buffer (DPBS (No Magnesium ⁺², No Calcium ⁺², 244 mL); EDTA (1ml, final concentration 2mM); and BSA (5ml of 5%, final concentration 0.1%), and separated using a magnetic DynaBead rack. The wash/separation with 1 mL of the Wash Buffer was repeated, and a further 200 microliters of Wash Buffer was added with subsequent pulse centrifugation. Supernatant Wash Buffer was removed.

Wash Buffer (600 microliters) containing 0.5 micrograms biotinylated Monomer MHC (HLA-A* 02:01 SLC45A2 (Biolegend, Custom Product, SLYSYFQKV) was dispensed into the microcentrifuge tubes, and the beads were resuspended by pipetting up and down. The monomer was allowed to bind for 30 min at 4°C. After 15 minutes, the mixtures were pipetted up and down again. The tubes were pulse centrifuged and the supernatant liquid removed, and the tubes were placed within the magnetic rack to remove more supernatant without removing beads.

A solution of biotinylated recombinant CD80 protein (R&D Systems, Custom Product) and biotinylated recombinant CD58 (R&D Systems, Custom Product) in 600 microliters Wash Buffer was added to the microcentrifuge tubes. The CD80 was prepared as an N-terminal fusion to a human IgG1 Fc domain and biotinylated on random Lysine residues by the manufacturer. The CD58 was biotinylated in the same manner. The solution contained a total of 4.5 micrograms of CD80 and 1.5 micrograms of CD58. The beads were resuspended by pipetting up and down. The beads were incubated at 4C for 30 min, resuspending after 15 min with another up and down pipetting. At the end of the incubation period, the tube was briefly pulse centrifuged. After placing back into the magnetic rack, and allowing separation for 1 min, the Buffer solution was aspirated away from the functionalized beads. The MHC monomer/CD80/CD58 antigen presenting beads were resuspended in 100 microliters Wash Buffer, stored at 4°C, and used without further manipulation. The 1e7 2.80 micron diameter functionalized DynaBeads have a nominal surface area of about 24e6 square microns available for contact with T lymphocyte cells, which, as described herein, does not reflect the total surface occupied by pMHC and costimulatory molecular ligands.

**20B. Preparation of an antigen presenting surface of a bead with BirA biotinylated CD80 and CD58.** Streptavidin functionalized (covalently coupled, convoluted (as described above) polymeric) DynaBeads^{™} (ThermoFisher Catalog # 11205D, bead stock at 6.67e8/mL) were delivered (15 microliters; 1e7 beads) to 1.5 mL microcentrifuges tube with 1mL of Wash Buffer (DPBS (No Magnesium ⁺², No Calcium ⁺², 244 mL); EDTA (1ml, final concentration 2mM); and BSA (5ml of 5%, final concentration 0.1%), and separated using a magnetic DynaBead rack. The wash/separation with 1 mL of the Wash Buffer was repeated, and a further 200 microliters of Wash Buffer was added with subsequent pulse centrifugation. Supernatant Wash Buffer was removed.

Wash Buffer (600 microliters) containing 0.5 micrograms biotinylated Monomer MHC (HLA-A* 02:01 MART1 (Biolegend, Custom Product, ELAGIGILTV) was dispensed into the microcentrifuge tubes, and the beads were resuspended by pipetting up and down. The monomer was allowed to bind for 30 min at 4°C. After 15 minutes, the mixtures were pipetted up and down again. The tubes were pulse centrifuged and the supernatant liquid removed, and the tubes were placed within the magnetic rack to remove more supernatant without removing beads.

A solution of biotinylated recombinant CD80 protein (BPS Biosciences, Catalog Number 71114) and biotinylated recombinant CD58 (BPS Biosciences, Catalog Number 71269) in 600 microliters Wash Buffer was added to the microcentrifuge tubes. The recombinant proteins were prepared as N-terminal fusions to a human IgG1 Fc domain, with a final C-terminal BirA biotinylation site, and biotinylated by the manufacturer. The solution contained a total of 1.5 micrograms of recombinant CD80 and 1.5 micrograms of recombinant CD58 proteins. The beads were resuspended by pipetting up and down. The beads were incubated at 4°C for 30 min, then resuspended after 15 min with another up and down pipetting. At the end of the incubation period, the tube was briefly pulse centrifuged. After placing back into the magnetic rack, and allowing separation for 1 min, the Buffer solution was aspirated away from the functionalized beads. The MHC monomer/CD80/CD58 antigen presenting beads were resuspended in 100 microliters Wash Buffer, stored at 4°C, and used without further manipulation. The 1e7 2.80 micron diameter functionalized DynaBeads have a nominal surface area of about 24e6 square microns available for contact with T lymphocyte cells, which, as described herein, does not reflect the total surface occupied by pMHC and costimulatory molecular ligands.

### Example 21. Activation of CD8+ T lymphocytes by antigen presenting beads with antibody costimulation compared to recombinant protein costimulation.

Cells: CD8+T lymphocytes were enriched in a medium including RPMI plus 10% fetal bovine serum (FBS) from commercially available PBMCs following manufacturer's directions for EasySep ^{™} Human CD8+ T Cell Isolation Kit, commercially available kit from StemCell Technologies Canada Inc. (Catalog # 17953), by negative selection.

Culture medium and diluent for reagent additions: Advanced RPMI (ThermoFisher Catalog #12633020, 500mL); 1x GlutaMAX (ThermoFisher Catalog # 35050079, 5mL); 10% Human AB serum (zen-bio, Catalog # HSER-ABP 100mL, 50mL); and 50nM beta-mercaptoethanol (ThermoFisher Catalog # 31350010, 50nm stock, 0.5 mL, final conc 50 micromolar).

Experimental Setup: For each activating species, a single 96-well tissue-culture treated plate (VWR Catalog # 10062-902) was used:
Wellplate 1. Antigen presenting beads with antibody costimulation).
Wellplate 2. Antigen presenting beads with random biotinylated recombinant protein co-activation.
Wellplate 3. Antigen presenting beads with BirA biotinylated recombinant protein co-activation.
CD8+T lymphocytes (2e5) (80-90% pure) were added to each individual well.

Antigen presenting surface beads (2e5), prepared by a similar preparation as described in Example 15, presenting pMHC including MART1, anti-CD28 antibody and anti-CD2 antibody, were added to each of the wells in wellplate 1. pMHC was loaded at 0.5 micrograms/1e7 beads. Anti-CD28 antibody was loaded at 1.5 micrograms/1e7 beads. Anti-CD2 antibody was loaded at 1.5 micrograms/1e7 beads.

Antigen presenting surface beads (2e5), prepared as in Example 20A, presenting pMHC including MART1, recombinant CD80 and recombinant CD58, were added to each of the wells in wellplate 2. pMHC was loaded at 0.5 micrograms/1e7 beads. Recombinant CD80 was loaded at 4.5 micrograms/1e7 beads. Recombinant CD58 was loaded at 1.5 micrograms/1e7 beads.

Antigen presenting surface beads (2e5), prepared as in Example 20B, presenting pMHC including MART1, BirA biotinylated recombinant CD80 and recombinant CD58, were added to each of the wells in wellplate 3. pMHC was loaded at 0.5 micrograms/1e7 beads. Recombinant CD80 was loaded at 1.5 micrograms/1e7 beads. Recombinant CD58 was loaded at 1.5 micrograms/1e7 beads.

Each wellplate was cultured at 37°C. On day 0, IL-21 (150ng/milliliter) in CTL media, was added to each well of wellplates 1 and 2, providing a final concentration in each well of 30 ng/mL. On day 2, IL21 was added to each well of the wellplates, to a final concentration of 30ng/mL. Culturing was continued to day 7.

Day 7. Restimulation. A second aliquot of antigen presenting beads with antibody costimulation or recombinant protein costimulation was delivered to each occupied well in wellplate 1, wellplate 2 and wellplate 3, respectively. IL21 was added to each well of the wellplate to a final concentration of 30ng/mL. Culturing was continued.

Day 8. Addition of 50 microliters of IL-2 (50 IU/mL) and IL-7 (25ng/mL) was made to each well in each wellplate to provide a final concentration of 10 IU/mL and 5 ng/mL respectively. Culturing was continued.

Day 9. Addition of 50 microliters of IL-21(150ng/mL) was made to each occupied well of each well plate to a final concentration of 30ng/mL. Culturing was continued.

Day 14. The wells from each wellplate were individually stained for MHC tetramer (Tetramer PE, MBL Catalog # T02000, 1 microliter/well), CD4 (Biolegend Catalog # 300530, 0.5 microliters/well); CD8 (Biolegend Catalog # 301048, 0.5 microliters/well); CD28(Biolegend Catalog # 302906, 0.31 microliters/well); CD45RO (Biolegend Catalog # 304210, 0.63 microliters/well); CCR7 (CD197, Biolegend Catalog # 353208, 0.5 microliters/well); and viability (BD Catalog # 565388, 0.125 microliters/well). Resuspend each well with 150 microliters FACS buffer and 10 microliters of Countbright^{™} beads (ThermoFisher Catalog # C36950). FACS analysis was performed on a FACSCelesta^{™} flow cytometer (BD Biosciences).

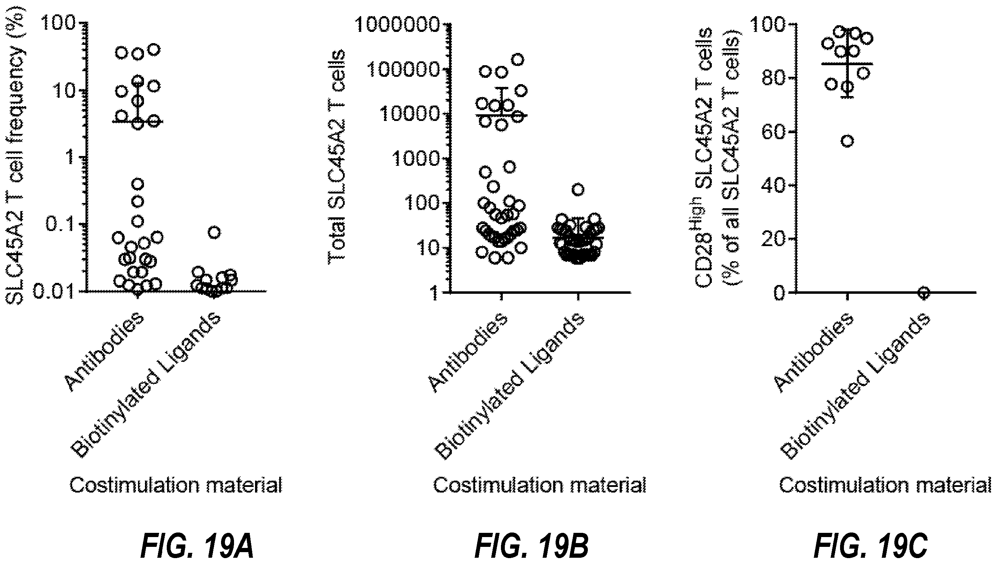

FIG. 19A shows the frequency of MART1-specific T cells (% of all live cells) in each well expanded using Antigen presenting beads with Antibodies or randomly biotinylated recombinant protein ligands. FIG. 19B shows the number of MART1-specific T cells in each well expanded using Antigen presenting beads with Antibodies or randomly biotinylated recombinant protein ligands. FIG. 19C shows the frequency of MART1 T cells that express high levels of CD28, an indicator of a memory precursor phenotype comparing antibody stimulation or randomly biotinylated recombinant protein ligands.

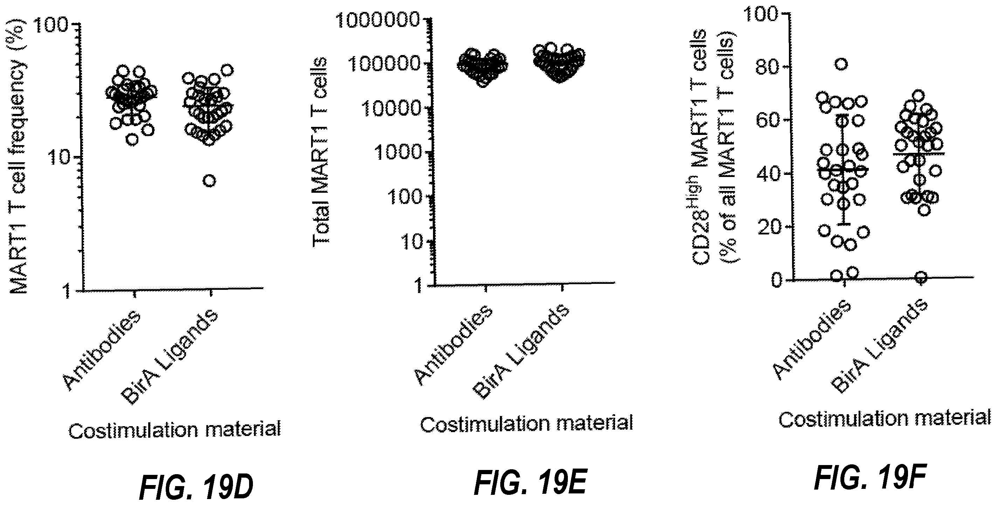

FIG. 19D shows the frequency of MART1-specific T cells (% of all live cells) in each well expanded using Antigen presenting beads with Antibodies or recombinant protein BirA ligands. FIG. 19E shows the number of MART1-specific T cells in each well expanded using Antigen presenting beads with Antibodies or recombinant protein BirA ligands. FIG. 19F shows the frequency of MART1 T cells that express high levels of CD28, an indicator of a memory precursor phenotype. It can be seen that the antigen presenting beads with recombinant protein ligands biotinylated by BirA effectively expand antigen-specific CD8+ T cells and that many of the expanded cells take on a memory precursor phenotype. In contrast, use of randomly biotinylated protein ligands did not lead to significant populations of antigen specific T cells and also did not provide cells with a memory precursor phenotype.

### Example 22. Comparison between loading of and activation with convoluted polymeric beads vs. substantially spherical silica beads.

**Example 22A. Comparison of activating species loading onto Polymer and Silica antigen presenting beads.** Amounts of pMHC and costimulation antibodies that could be deposited onto Polymer and Silica beads was measured.

Streptavidin functionalized (covalently coupled) DynaBeads^{™} (ThermoFisher Catalog # 11205D, bead stock at 6.67e8/mL) were delivered (15 microliters; 1e7 beads) to 1.5 mL microcentrifuges tube with 1mL of Wash Buffer (DPBS (No Magnesium ⁺², No Calcium ⁺², 244 mL); EDTA (1ml, final concentration 2mM); and BSA (5ml of 5%, final concentration 0.1%), and separated using a magnetic DynaBead rack. The wash/separation with 1 mL of the Wash Buffer was repeated, and a further 200 microliters of Wash Buffer was added with subsequent pulse centrifugation. Supernatant Wash Buffer was removed.

Biotin functionalized (covalently coupled) smooth silica beads were first coated with Streptavidin by storage in 100 micromolar Streptavidin. Approximately 5e6 beads were washed by dilution into 1 milliliter of Wash Buffer in a microcentrifuge tube, followed by centrifugation at 1,000xg for 1 minute. The supernatant was carefully removed by aspiration, and the wash process repeated twice more. Supernatant Wash Buffer was removed.

To prepare antigen presenting beads, Wash Buffer (600 microliters) containing 0.5 micrograms biotinylated Monomer MHC (HLA-A* 02:01 SLC45A2 (Biolegend, Custom Product, SLYSYFQKV) was dispensed into the tubes with the DynaBeads and Silica beads, and the beads were resuspended by pipetting up and down. The monomer was allowed to bind for 30 min at 4°C. After 15 minutes, the mixtures were pipetted up and down again. The tubes were centrifuged at 1,000xg for one minute, and the supernatant liquid removed.

Wash Buffer (600 microliters) with 1.5 micrograms of biotinylated anti-CD28 and 1.5 micrograms of biotinylated anti-CD2 was used to resuspend each bead sample, and the beads were resuspended by pipetting up and down. The antibodies were allowed to bind for 30 min at 4C. After 15 minutes, the mixtures were pipetted up and down again. The tubes were centrifuged at 1,000xg for one minute, and the supernatant liquid removed. Finally, the beads were resuspended in 100 microliters of Wash Buffer.

Two samples of approximately 2e5 Polymer antigen presenting beads or 1e5 Silica antigen presenting beads were washed with Wash Buffer (1 milliliter). The bead samples were resuspended in 100 microliters of Wash Buffer and stained by addition of 1 microliter of APC-conjugated anti-HLA-A (Biolegend, Catalog Number 343308) or 1 microliter of APC-conjugated monoclonal anti-Mouse-lgG1 (Biolegend, Catalog Number 406610). The beads were mixed with the antibody and allowed to stain for 30 minutes in the dark. After staining, beads were washed, resuspended in Wash Buffer (200 microliters) and transferred to tubes for analysis by Flow Cytometry.

A set of Quantum Simply Cellular fluorescence quantitation beads (Bangs Labs, Catalog Number 815) was then prepared to determine the number of anti-HLA-A antibodies and anti-Mouse IgG1 antibodies bound to each antigen presenting bead sample. The quantitation beads have antibody binding capacities determined by the manufacturer. A drop of each bead with pre-determined binding capacity was placed in microcentrifuge tubes with 50 microliters of Wash Buffer. To the tubes, 5 microliters of APC-conjugated anti-HLA-A or APC-conjugated anti-Mouse IgG1 was added and mixed by vortexing. The beads were stained for 30 minutes in the dark, washed using the same method as above. The beads with different binding capacities were then pooled into one sample and transferred to a single tube. A drop of blank beads (no antibody binding capacity) was added and the beads were analyzed by Flow Cytometry.

The quantitation beads were analyzed by Flow Cytometry (BD FACSCelesta, Becton Dickinson and Company) by recording 5,000 events. The quantitation beads were identified by Forward Scatter and Side Scatter, and the median intensity in the APC channel of each bead recorded. This data was recorded in a proprietary Excel spreadsheet provided by the manufacturer (Bangs) that calculates a standard curve of APC intensity versus antibody binding capacity. After verifying that the calibration was linear, the antigen presenting bead samples were analyzed. The beads were identified by Forward and Side Scatter, and the median intensity in the APC channel recorded on the spreadsheet. The spreadsheet calculates the number of APC anti-HLA-A antibodies or APC anti-Mouse IgG1 on each antigen presenting bead. Assuming that 1 anti-HLA-A antibody binds to one pMHC on the antigen presenting bead, this value represents the number of pMHC molecules on each bead. Similarly, the number of costimulation antibodies can be determined.

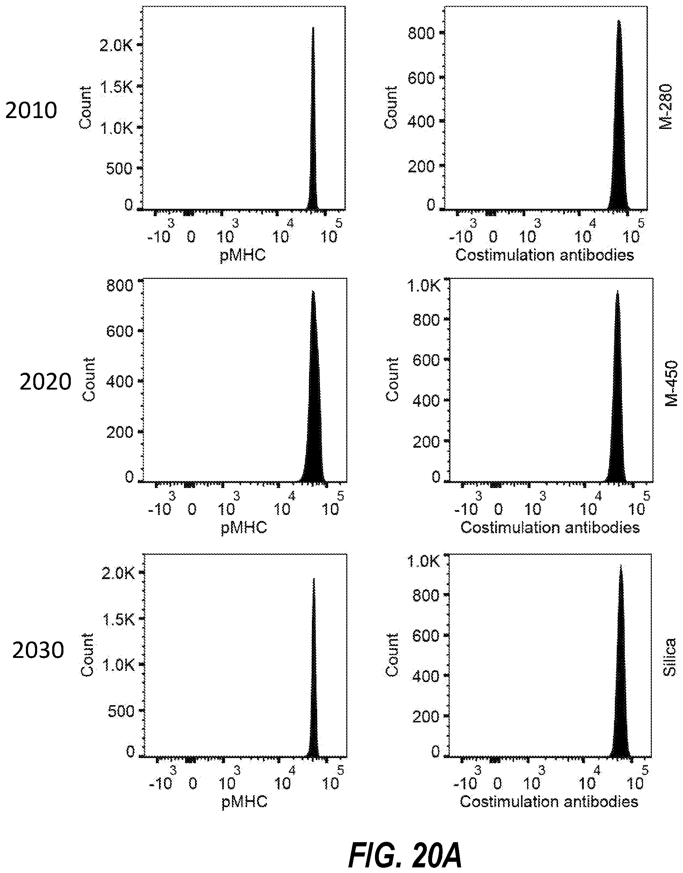

From the nominal surface area of each antigen presenting bead, the density (number of molecules / square micron of bead surface) of each species can be determined. The total number of pMHC on the Silica microspheres was determined to be approximately 800,000 pMHC / antigen presenting bead. The total number of costimulation antibodies was determined to be about 850,000 antibodies / bead. As there is no way to distinguish the anti-CD28 and anti-CD2 clones used to prepare the antigen presenting beads (they are the same isotype), it is assumed that the ratio of the two antibodies is 1:1. Due to the regularity of the Silica bead surfaces, the surface area can be reasonably modeled from a sphere. For a 4.08 micron diameter microsphere, this corresponds to a surface area of about 52.3 square microns. From this, it can be estimated that about 15,000 pMHC and 15,000 costimulation antibodies per square micron of bead surface are presented by the Silica antigen presenting beads as shown in Table 1. The distribution across each bead population for each ligand class is shown in FIG. 20A, where each row 2010, 2020, and 2030 shows the distribution of pMHC in the left hand graph, and the distribution of costimulation antibodies in the right hand graph for each type of bead. Row 2010 shows distribution of the ligands for 2.8 micron diameter convoluted polymer beads (Dynal). Row 2020 shows distribution of ligands for 4.5 micron diameter convoluted polymer beads (Dynal). Row 2030 shows distribution of ligands for a 2.5 micron diameter substantially spherical silica bead as produced in Example 10B. Tightly controlled populations of beads were produced, with the substantially spherical silica beads having even more tightly controlled distribution of ligands over the entire population, and slightly higher median distribution. Thus, the use of substantially spherical silica beads can lead to more reproducible and controllable production of these activating species. Additionally, since all of the ligands are accessible to T lymphocytes, unlike the convoluted polymer bead ligand distribution, more efficient use is made of precious biological ligands such as antibodies.

**Table 1. Ligand quantification and density for convoluted polymer beads and substantially spherical silica beads.**

| **Bead** | **pMHC / bead** | **pMHC Density (molecules / sq micron)** | **Costimulation antibodies / bead** | **Costimulation Antibody Density (molecules / sq micron)** |
|---|---|---|---|---|
| **M-280 Polymer** | 487,209 | 19,781 | 426,992 | 17,336 |
| **Silica** | 807,180 | 14,847 | 845,388 | 15,550 |

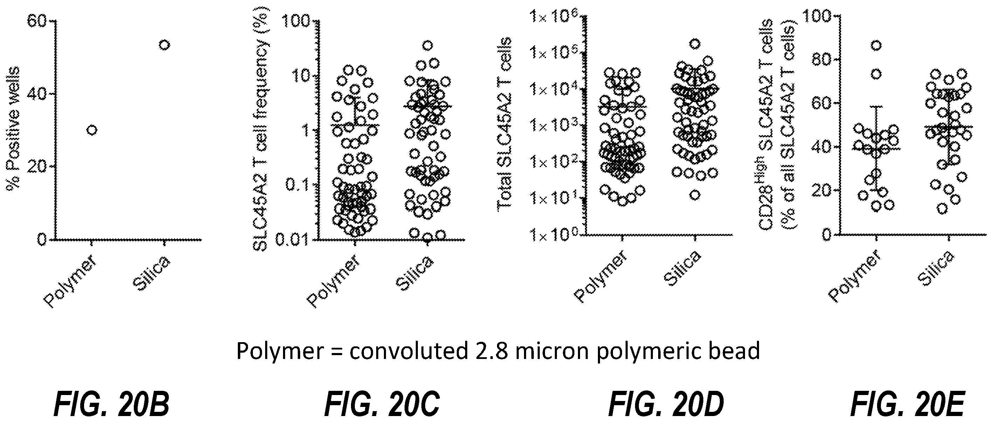

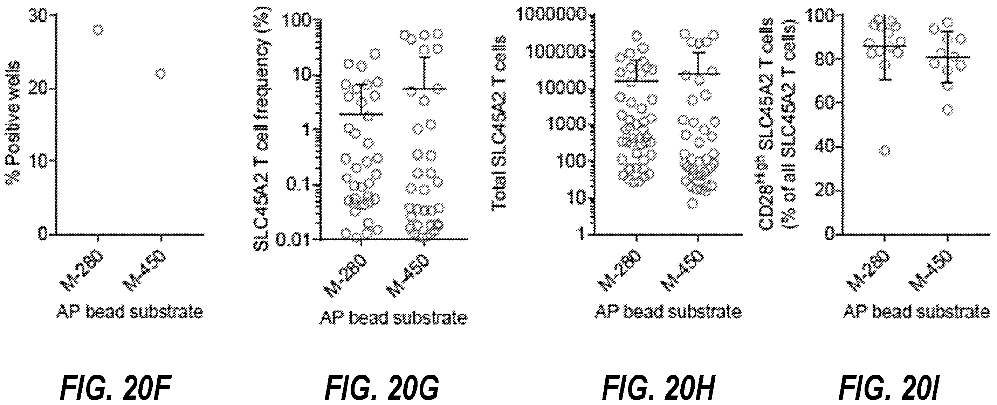

For Polymer beads, the convoluted surface makes the relationship between bead diameter and surface area less straightforward. From the quantitation, it was determined that Polymer antigen presenting beads based on M-280 DynaBeads had about 480,000 pMHC molecules and 425,000 costimulation antibodies on their surface. For a sphere of radius 1.4 microns (equal to the nominal radius of M-280 DynaBeads, this corresponds to about 20,000 pMHC and 17,000 costimulation antibodies per square micron, as shown in Table 1. However, due to the convoluted surface of the Polymer beads, the actual surface area is likely larger, and thus the actual density lower. From Fig. 20E, 20F and 20G, though, it can be seen that these beads can be used as antigen presenting bead substrates to expand large numbers of antigen-specific T cells, where the expansion was performed in a similar manner as in Example 22B. In addition, from Fig. 20H, these antigen presenting beads generated large numbers of antigen-specific T cells with high expression of CD28, indicative of a memory precursor phenotype.

Antigen presenting beads were prepared in the same manner using M-450 Epoxy DynaBeads modified with Streptavidin. From flow cytometry, antigen presenting beads prepared from M-450 beads had approximately the same number of pMHC and costimulation antibody molecules as antigen presenting beads prepared with M-280 DynaBeads. As the M-450 DynaBeads are larger than the M-280 beads, this implies that the density of the activating species on the M-450 antigen presenting beads was about 2-3 times lower than on M-280 antigen presenting beads. However, as can be seen from FIG. 20F, M-450 antigen presenting beads generated positive wells (in which SLC45A2-specific T cells expanded to represent 0.5% or more of the live cells in the well) when used to expand SLC45A2 T cells. From FIGS. 20G and 20H, it can be seen that these wells generated SLC45A2 T cells at high frequencies, and the number of SLC45A2 T cells was comparable to the number obtained from M-280 antigen presenting beads. In addition, from FIG. 20I, the fraction of SLC45A2 T cells expressing high levels of CD28 was comparable when using M-280 or M-450 antigen presenting beads to expand SLC45A2 T cells.

**Example 22B. Expansion of antigen-specific T cells with polymer vs Silica beads.** Expansion of antigen-specific T cells using Silica antigen presenting beads was tested and compared to convoluted polymeric beads (polystyrene).

Streptavidin functionalized (covalently coupled, convoluted) DynaBeads^{™} (ThermoFisher Catalog # 11205D, bead stock at 6.67e8/mL) were delivered (15 microliters; 1e7 beads) to 1.5 mL microcentrifuges tube with 1mL of Wash Buffer (DPBS (No Magnesium ⁺², No Calcium ⁺², 244 mL); EDTA (1ml, final concentration 2mM); and BSA (5ml of 5%, final concentration 0.1%), and separated using a magnetic DynaBead rack. The wash/separation with 1 mL of the Wash Buffer was repeated, and a further 200 microliters of Wash Buffer was added with subsequent pulse centrifugation. Supernatant Wash Buffer was removed.

Biotin functionalized (covalently coupled) smooth silica beads, prepared as in Example 10B, were first coated with Streptavidin by storage in 100 micromolar Streptavidin. Approximately 5e6 beads were washed by dilution into 1 milliliter of Wash Buffer in a microcentrifuge tube, followed by centrifugation at 1,000xg for 1 minute. The supernatant was carefully removed by aspiration, and the wash process repeated twice more. Supernatant Wash Buffer was removed.

To prepare antigen presenting beads, Wash Buffer (600 microliters) containing 0.5 micrograms biotinylated Monomer MHC (HLA-A* 02:01 SLC45A2 (Biolegend, Custom Product, SLYSYFQKV) was dispensed into the tubes with the DynaBeads and Silica beads, and the beads were resuspended by pipetting up and down. The monomer was allowed to bind for 30 min at 4°C. After 15 minutes, the mixtures were pipetted up and down again. The tubes were centrifuged at 1,000xg for one minute, and the supernatant liquid removed.

Wash Buffer (600 microliters) with 1.5 micrograms of biotinylated anti-CD28 and 1.5 micrograms of biotinylated anti-CD2 was used to resuspend each bead sample, and the beads were resuspended by pipetting up and down. The antibodies were allowed to bind for 30 min at 4°C. After 15 minutes, the mixtures were pipetted up and down again. The tubes were centrifuged at 1,000xg for one minute, and the supernatant liquid removed. Finally, the beads were resuspended in 100 microliters of Wash Buffer.

Cells: CD8+T lymphocytes were enriched in a medium including RPMI plus 10% fetal bovine serum (FBS) from commercially available PBMCs following manufacturer's directions for EasySep ^{™} Human CD8+ T Cell Isolation Kit, commercially available kit from StemCell Technologies Canada Inc. (Catalog # 17953), by negative selection.

Culture medium and diluent for reagent additions: Advanced RPMI (ThermoFisher Catalog #12633020, 500mL); 1x GlutaMAX (ThermoFisher Catalog # 35050079, 5mL); 10% Human AB serum (zen-bio, Catalog # HSER-ABP 100mL, 50mL); and 50nM beta-mercaptoethanol (ThermoFisher Catalog # 31350010, 50nm stock, 0.5 mL, final conc 50 micromolar).

Experimental Setup: For each type of antigen presenting bead (Silica or Polymer, as prepared above in this example), a single 96 tissue-culture treated wellplate (VWR Catalog # 10062-902) was used. Silica antigen presenting beads were mixed with CD8+T lymphocytes at ~1:2 beads:cell. CD8+T lymphocytes (2e5) (80-90% pure) were added to each well with approximately 1e5 antigen presenting beads (wellplate 1). Polymer antigen presenting beads were mixed with CD8+T lymphocytes at ~1:1 beads:cell. CD8+T lymphocytes (2e5) (80-90% pure) were added to each well with approximately 2e5 antigen presenting beads (wellplate 2).

Each wellplate was cultured at 37°C. On day 0, IL-21 (150ng/milliliter) in CTL media, was added to each well of wellplates 1 and 2, providing a final concentration in each well of 30 ng/mL. On day 2, IL21 was added to each well of the wellplates, to a final concentration of 30ng/mL. Culturing was continued to day 7.

Day 7. Restimulation. A second aliquot of antigen presenting beads was added to the corresponding wells in wellplate 1 and wellplate 2. For the Silica beads, approximately 1e5 beads (Silica beads as prepared above in this example) were added. For the Polymer beads, approximately 2e5 beads (convoluted polymer beads as prepared above in this example) were added. IL21 was added to each well of the wellplate to a final concentration of 30ng/mL. Culturing was continued.

Day 8. Addition of 50 microliters of IL-2 (50 IU/mL) and IL-7 (25ng/mL) was made to each well in wellplate 1 and wellplate 2 to provide a final concentration of 10 IU/mL and 5 ng/mL respectively. Culturing was continued.

Day 9. Addition of 50 microliters of IL-21(150ng/mL) was made to each occupied well of wellplate 1 and wellplate 2 to a final concentration of 30ng/mL. Culturing was continued.

Day 14. The wells from each wellplate were individually stained for MHC tetramer (Tetramer PE, MBL Catalog # T02000, 1 microliter/well), CD4 (Biolegend Catalog # 300530, 0.5 microliters/well); CD8 (Biolegend Catalog # 301048, 0.5 microliters/well); CD28(Biolegend Catalog # 302906, 0.31 microliters/well); CD45RO (Biolegend Catalog # 304210, 0.63 microliters/well); CCR7 (CD197, Biolegend Catalog # 353208, 0.5 microliters/well); and viability (BD Catalog # 565388, 0.125 microliters/well). Each well was resuspended with 150 microliters FACS buffer and 10 microliters of Countbright^{™} beads (ThermoFisher Catalog # C36950). FACS analysis was performed on a FACSCelesta^{™} flow cytometer (BD Biosciences).

FIG. 20B shows the percentage of positive wells (in which SLC45A2-specific T cells expanded to represent 0.5% or more of the live cells in the well) after expansion using the Polymer or Silica antigen presenting beads. FIG. 20C shows SLC45A2 T cell frequency (% of live cells in each well) after expansion with the Polymer or Silica antigen presenting beads. FIG. 20D shows the total number of SLC454A2 T cells in each of the wells. FIG. 20E shows the percentage of SLC45A2 T cells in the wells that expressed high levels of CD28, indicating the potential for differentiation into a memory T cell. From these plots, it can be seen that the Silica antigen presenting beads generate positive wells, and that the Silica antigen presenting beads expand SLC45A2 T cells as well or better than Polymer antigen presenting beads. In addition, the Silica antigen presenting beads produce cells with high expression of CD28, indicating that they support formation of memory precursor T cells, a desired phenotype for the cellular product.

For Polymer beads, the convoluted surface makes the relationship between bead diameter and surface area less straightforward. From the quantitation, it was determined that Polymer antigen presenting beads based on M-280 DynaBeads had about 480,000 pMHC molecules and 425,000 costimulation antibodies on their surface. For a sphere of radius 1.4 microns (equal to the nominal radius of M-280 DynaBeads, this corresponds to about 20,000 pMHC and 17,000 costimulation antibodies per square micron. However, due to the convoluted surface of the Polymer beads, the actual surface area is likely larger, and thus the actual density lower. However, from FIGS. 20F, 20G and 20H, it can be seen that these beads can be used as antigen presenting bead substrates to expand large numbers of antigen-specific T cells. In addition, from FIG. 20I, these antigen presenting beads generate large numbers of antigen-specific T cells with high expression of CD28, indicative of a memory precursor phenotype.

### Example 23A. Preparation of antigen presenting beads with defined ligand densities.

**Example 23A.1. Preparation of streptavidin presenting beads.** Three-fold serial dilutions of pMHC (HLA-A* 02:01 SLC45A2 (Biolegend, Custom Product, SLYSYFQKV) in Wash Buffer were prepared. 20 microliters of Wash Buffer was added to a microcentrifuge tube for each serial dilution to be performed. Into the first serial dilution tube, 10 microliters of the pMHC were added. The diluted pMHC was mixed by vortexing. 10 uL of the diluted pMHC mixture was then used to prepare the subsequent serial dilution for a total of seven dilutions.
To determine the relationship between concentration of pMHC in solution and the density (molecules / unit area) deposited on the beads, Biotin functionalized (covalently coupled) smooth (e.g., substantially spherical as described above) 4 micron monodisperse silica beads (Cat. # SiO2MS-1.8 4.08um - 1g, Cospheric), prepared as in Example 10B, were first coated with Streptavidin by storage in 100 micromolar Streptavidin. Approximately 1e7 beads were washed by dilution into 1 milliliter of Wash Buffer in a microcentrifuge tube, followed by centrifugation at 1,000xg for 1 minute. The supernatant was carefully removed by aspiration, and the wash process repeated twice more. After washing, approximately 1e6 beads were delivered into eight microcentrifuge tubes, centrifuged again, and the supernatant carefully removed.

**Example 23A.2. Preparation of beads having a range of MHC concentration.** Wash Buffer (120 microliters) containing 4.5 micrograms biotinylated Monomer MHC (HLA-A* 02:01 SLC45A2 (Biolegend, Custom Product, SLYSYFQKV) was dispensed into one of the microcentrifuge tubes, and the beads were resuspended by pipetting up and down. The undiluted pMHC and serial dilutions of pMHC were further diluted into Wash Buffer (120 microliters) and used to resuspend beads, resulting in beads suspended in solutions with 4.5, 1.5, 0.5, 0.167, 0.056, 0.019, 0.006, or 0.002 micrograms of pMHC monomer per 5e6 beads. The monomer was allowed to bind for 30 min at 4°C. After 15 minutes, the mixtures were pipetted up and down again. The tubes were centrifuged, the supernatant liquid removed, and the beads resuspended at approximately 5e7/milliliter.

Approximately 1e5 beads prepared with each concentration of pMHC were washed with Wash Buffer (1 milliliter). The bead samples were resuspended in 100 microliters of Wash Buffer and stained by addition of 1 microliter of APC-conjugated anti-HLA-A (Biolegend, Catalog Number 343308). The beads were mixed with the antibody and allowed to stain for 30 minutes in the dark. After staining, beads were washed, resuspended in Wash Buffer (200 microliters) and transferred to tubes for analysis by Flow Cytometry.

A set of Quantum Simply Cellular fluorescence quantitation beads (Bangs Labs, Catalog Number 815) was then prepared to determine the number of anti-HLA-A antibodies bound to each antigen presenting bead sample. The quantitation beads have antibody binding capacities determined by the manufacturer. A drop of each bead with pre-determined binding capacity was placed in a microcentrifuge tube with 50 microliters of Wash Buffer. To the tube, 5 microliters of APC-conjugated anti-HLA-A was added and mixed by vortexing. The beads were stained for 30 minutes in the dark, washed using the same method as above. The beads with different binding capacities were then pooled into one sample and transferred to a single tube. A drop of blank beads (no antibody binding capacity) was added and the beads were analyzed by Flow Cytometry.

The quantitation beads were analyzed by Flow Cytometry (BD FACSCelesta, Becton Dickinson and Company) by recording 5,000 events. The quantitation beads were identified by Forward Scatter and Side Scatter, and the median intensity in the APC channel of each bead recorded. Quantitation was calculated as described in Example 22A, using the proprietary methodology provided by the quantitation bead manufacturer. From the antigen presenting bead standard, it is then possible to determine the concentration of pMHC in solution with beads that generates antigen presenting beads with a targeted density of pMHC, e.g., beads with approximately 10,000, 1,000, or 100 pMHC molecules per square micron, as seen in FIG. 21A.

**Example 23A.3. Costimulation molecule concentration variation.** Three-fold serial dilutions of biotinylated anti-CD28 and anti-CD2 in Wash Buffer were prepared. 20 microliters of anti-CD28 was mixed with 20 microliters of anti-CD2 in a microcentrifuge tube. Wash Buffer (20 microliters) was then added to a microcentrifuge tube for each serial dilution. The anti-CD28/anti-CD2 mixture (10 microliters) was then added to the first serial dilution tube. The solution was mixed using a vortexer, and 10 uL of the diluted anti-CD28/anti-CD2 mixture was then used to prepare the subsequent serial dilution for a total of seven dilutions.

To quantify the relationship between costimulation antibody in solution and the density (molecules / unit area) deposited on the beads, approximately 1e7 substantially spherical 4 micron silica beads, prepared as in Example 23A.1, having streptavidin binding moieties, were first washed by dilution into 1 milliliter of Wash Buffer in a microcentrifuge tube, followed by centrifugation at 1,000xg for 1 minute. The supernatant was carefully removed by aspiration, and the wash process repeated twice more. After washing, approximately 1e6 beads were delivered into eight microcentrifuge tubes, centrifuged again, and the supernatant carefully removed.

The beads were first functionalized with 1.0 micrograms of pMHC in Wash Buffer (1,200 microliters). After washing, the beads were resuspended in Wash Buffer (1,000 microliters). Into eight microcentrifuge tubes, 100 microliters of pMHC functionalized beads was dispended. The beads were centrifuged, and the supernatant carefully removed.

The undiluted mixed anti-CD28 and anti-CD2 and serial dilutions of anti-CD28/anti-CD2 were further diluted into Wash Buffer (120 microliters) and used to resuspend the beads, resulting in beads suspended in solutions with 4.5, 1.5, 0.5, 0.167, 0.056, 0.019, 0.006, or 0.002 micrograms of mixed costimulation antibodies monomer per 5e6 beads. The monomer was allowed to bind for 30 min at 4°C. After 15 minutes, the mixtures were pipetted up and down again. The tubes were centrifuged, the supernatant liquid removed, and the beads resuspended at approximately 5e7/milliliter.

Approximately 1e5 beads prepared with each concentration of costimulation antibodies was washed with Wash Buffer (1 milliliter). The bead samples were resuspended in 100 microliters of Wash Buffer and stained by addition of 1 microliter of APC-conjugated monoclonal anti-Mouse-IgG1 (Biolegend, Catalog Number 406610). The beads were mixed with the antibody and allowed to stain for 30 minutes in the dark. After staining, beads were washed, resuspended in Wash Buffer (200 microliters) and transferred to tubes for analysis by Flow Cytometry.

A set of Quantum Simply Cellular fluorescence quantitation beads (Bangs Labs, Catalog Number 815) was then prepared to determine the number of APC anti-Mouse IgG1 antibodies bound to each antigen presenting bead sample. A drop of each bead with pre-determined binding capacity was placed in a microcentrifuge tube with 50 microliters of Wash Buffer. To the tube, 5 microliters of APC-conjugated anti-Mouse IgG1 was added and mixed by vortexing. The beads were stained for 30 minutes in the dark, washed using the same method as above. The beads with different binding capacities were then pooled into one sample and transferred to a single tube. A drop of blank beads (no antibody binding capacity) was added and the beads were analyzed by Flow Cytometry.

The quantitation beads were analyzed by Flow Cytometry (BD FACSCelesta, Becton Dickinson and Company) by recording 5,000 events. The quantitation beads were identified by Forward Scatter and Side Scatter, and the median intensity in the APC channel of each bead recorded. Quantitation was performed as described in Example 22A, using proprietary methods provided by the quantitation bead manufacturer. This quantitation method calculates the number of APC anti-Mouse IgG1 antibodies on each antigen presenting bead. Assuming that 1 anti-Mouse IgG1 antibody binds to one costimulation antibody on the antigen presenting bead, this value represents the number of costimulation antibodies on each bead. From the antigen presenting bead standards, it is then possible to determine the concentration of costimulation antibodies in solution with beads that generates antigen presenting beads with a targeted density of costimulation antibodies, e.g., beads with approximately 10,000, 1,000, or 100 costimulation molecules per square micron, as seen in FIG. 21B.

### Example 23B. Expansion of antigen-specific T cells with antigen presenting beads with different ligand densities.

Using the plots of pMHC and costimulation antibody concentration versus density on the resulting antigen presenting beads (FIG. 21A), it was determined what concentration of each pMHC should be used to prepare antigen presenting beads with ~10,000, ~1,000 or ~100 pMHC per square micron of bead surface. This process was repeated to determine the concentration of anti-CD28 and anti-CD2 to be used to prepare antigen presenting beads with ~10,000, ~1,000 or ~100 costimulation antibodies per square micron of bead surface.

**Example 23B.1.** Biotin functionalized (covalently coupled) smooth silica beads, prepared as in Example 23.A.1 were first coated with Streptavidin by storage in 100 micromolar Streptavidin. Approximately 5e7 beads were washed by dilution into 1 milliliter of Wash Buffer in a microcentrifuge tube, followed by centrifugation at 1,000xg for 1 minute. The supernatant was carefully removed by aspiration, and the wash process repeated twice more. After washing, approximately 5e6 beads were delivered into three microcentrifuge tubes, centrifuged again, and the supernatant carefully removed.

**Example 23B.2.** To prepare antigen presenting beads with titrated pMHC, Wash Buffer (600 microliters) containing 0.5, 0.056, or 0.006 micrograms biotinylated Monomer MHC (HLA-A* 02:01 MART-1 (MBL International Corp., Catalog No. MR01008, ELAGIGILTV) was dispensed into three microcentrifuge tubes, and the beads were resuspended by pipetting up and down. The monomer was allowed to bind for 30 min at 4°C. After 15 minutes, the mixtures were pipetted up and down again. The tubes were centrifuged at 1,000xg for one minute, and the supernatant liquid removed.

Wash Buffer (600 microliters) with 1.0 microgram of mixed biotinylated anti-CD28 and biotinylated anti-CD2 was used to resuspend each bead sample, and the beads were resuspended by pipetting up and down. The antibodies were allowed to bind for 30 min at 4°C. After 15 minutes, the mixtures were pipetted up and down again. The tubes were centrifuged at 1,000xg for one minute, and the supernatant liquid removed. Finally, the beads were resuspended in 100 microliters of Wash Buffer. The loading of the beads with the desired order of magnitude of pMHC and antibodies was verified by Flow Cytometry analysis and comparison to quantitation beads.

Wash Buffer (600 microliters) with 1.0 micrograms of anti-CD28 and anti-CD2 was used to resuspend each bead sample, and the beads were resuspended by pipetting up and down. The monomer was allowed to bind for 30 min at 4°C. After 15 minutes, the mixtures were pipetted up and down again. The tubes were centrifuged at 1,000xg for one minute, and the supernatant liquid removed. Finally, the beads were resuspended in 100 microliters of Wash Buffer. The loading of the beads with the desired order of magnitude of pMHC was verified by Flow Cytometry analysis and comparison to quantitation beads.

**Example 23B.3.** To prepare antigen presenting beads with titrated costimulation antibodies, Wash Buffer (1,200 microliters) containing 1.5 micrograms biotinylated Monomer MHC (HLA-A* 02:01 MART-1 (MBL International Corp., Catalog No. MR01008, ELAGIGILTV) was dispensed into a microcentrifuge tube containing 1.5e7 washed beads from Example 23.B.1, and the beads were resuspended by pipetting up and down. The monomer was allowed to bind for 30 min at 4°C. After 15 minutes, the mixtures were pipetted up and down again. The tubes were centrifuged at 1,000xg for one minute, and the supernatant liquid removed.

The beads were then resuspended in Wash Buffer (900 microliters), and 300 microliters of the beads transferred to 3 microcentrifuge tubes.

Wash Buffer (300 microliters) with 1.0 microgram of mixed anti-CD28 and anti-CD2, 0.111 micrograms of mixed antiCD28 and anti-CD2, or 0.012 micrograms of mixed anti-CD28 and anti-CD2 was mixed into the three bead samples, and the beads were thoroughly mixed by pipetting up and down. The antibodies were allowed to bind for 30 min at 4°C. After 15 minutes, the mixtures were pipetted up and down again. The tubes were centrifuged at 1,000xg for one minute, and the supernatant liquid removed. Finally, the beads were resuspended in 100 microliters of Wash Buffer. The loading of the beads with the desired order of magnitude of costimulation antibodies was verified by Flow Cytometry analysis and comparison to quantitation beads.

**Example 23B.4. Stimulation. Cells:** CD8+ T lymphocytes were enriched in a medium including RPMI plus 10% fetal bovine serum (FBS) from commercially available PBMCs following manufacturer's directions for EasySep ^{™} Human CD8+ T Cell Isolation Kit, commercially available kit from StemCell Technologies Canada Inc. (Catalog # 17953), by negative selection.

Culture medium and diluent for reagent additions: Advanced RPMI (ThermoFisher Catalog #12633020, 500mL); 1x GlutaMAX (ThermoFisher Catalog # 35050079, 5mL); 10% Human AB serum (zen-bio, Catalog # HSER-ABP 100mL, 50mL); and 50nM beta-mercaptoethanol (ThermoFisher Catalog # 31350010, 50nm stock, 0.5 mL, final conc 50 micromolar).

Experimental Setup: For each activation species titration (pMHC or costimulation antibodies), a single 96 tissue-culture treated wellplate (VWR Catalog # 10062-902) was used. Antigen presenting beads with ~10,000, ~1,000 or ~100 pMHC per square micron of bead surface and with ~10,000 costimulation antibodies per square micron of bead surface (from Example 23.B.2) were mixed with CD8+ T lymphocytes at ~1:2 beads:cell. CD8+T lymphocytes (2e5) (80-90% pure) were added to each well with approximately 1e5 antigen presenting beads (wellplate 1). Antigen presenting beads with ~10,000 pMHC per square micron of bead surface and with ~10,000, ~1,000 or ~100 costimulation antibodies per square micron of bead surface (from Example 23.B.3) were mixed with CD8+ T lymphocytes at ~1:2 beads:cell. CD8+ T lymphocytes (2e5) (80-90% pure) were added to each well with approximately 1e5 antigen presenting beads (wellplate 2).

Each wellplate was cultured at 37°C. On day 0, IL-21 (150ng/milliliter) in CTL media, was added to each well of wellplates 1 and 2, providing a final concentration in each well of 30 ng/mL. On day 2, IL21 was added to each well of the wellplates, to a final concentration of 30ng/mL. Culturing was continued to day 7.

Day 7. Restimulation. A second aliquot of antigen presenting beads with the targets density of pMHC or costimulation antibody was added to the corresponding wells in wellplate 1 and wellplate 2. IL21 was added to each well of the wellplate to a final concentration of 30ng/mL. Culturing was continued.

Day 8. Addition of 50 microliters of IL-2 (50 IU/mL) and IL-7 (25ng/mL) was made to each well in wellplate 1 and wellplate 2 to provide a final concentration of 10 IU/mL and 5 ng/mL respectively. Culturing was continued.

Day 9. Addition of 50 microliters of IL-21(150ng/mL) was made to each occupied well of wellplate 1 and wellplate 2 to a final concentration of 30ng/mL. Culturing was continued.

Day 14. The wells from each wellplate were individually stained for MHC tetramer (Tetramer PE, MBL Catalog # T02000, 1 microliter/well), CD4 (Biolegend Catalog # 300530, 0.5 microliters/well); CD8 (Biolegend Catalog # 301048, 0.5 microliters/well); CD28 (Biolegend Catalog # 302906, 0.31 microliters/well); CD45RO (Biolegend Catalog # 304210, 0.63 microliters/well); CCR7 (CD197, Biolegend Catalog # 353208, 0.5 microliters/well); and viability (BD Catalog # 565388, 0.125 microliters/well). Each well was resuspended with 150 microliters FACS buffer and 10 microliters of Countbright^{™} beads (ThermoFisher Catalog # C36950). FACS analysis was performed on a FACSCelesta^{™} flow cytometer (BD Biosciences). FIG. 21C shows the number of MART1-specific T cells in each well expanded using antigen presenting beads with various densities of pMHC / square micron. FIG. 21D shows the expression level of CD127, a marker of memory precursor T cells, on the MART1-specific T cells from FIG. 21C. From these plots, it can be seen that the number of MART1-specific T cells and the expression of CD127 on these cells is insensitive to pMHC density when the density is ~100 pMHC / square micron or higher.

FIG. 21E shows the number of MART1-specific T cells in each well expanded using antigen presenting beads with various densities of costimulation antibodies / square micron. FIG. 21F shows the expression level of CD127, a marker of memory precursor T cells, on the MART1-specific T cells from FIG. 21E. From these plots, it can be seen that the number of MART1-specific T cells and the expression of CD127 on these cells is sensitive to costimulation antibody density. Beads prepared with ~10,000 costimulation antibodies per square micron, which nearly saturated the biotin binding sites of the bead (see FIG. 21B), generated the highest number of antigen-specific T cells, and those cells expressed the highest levels of CD127. As the number of costimulatory ligands was decreased to the lower end of the loading regime, primary stimulation by the pMHC was not as effectively co-stimulated, and the phenotype of the cell product is affected.

### Example 24. Performance of an antigen-specific cytotoxicity assay within a microfluidic device

**Experimental Design:** Tumor cell lines obtained from melanoma cells, including Mel 526 cells and A375 cells, were tested in an on-chip T cell killing assay. Each cell line was grown up in vitro according to standard procedures, then labeled with CellTrace^{™} Far Red dye (Cat. #C34572, ThermoFisher Scientific), which provides stable intracellular labelling. Each population of labeled tumor cells were flowed into an individual microfluidic chip (Berkeley Lights, Inc.) in T cell media (Adv. RPMI + 10% Human AB serum (Cat. # 35-060-Cl, Corning) + Gln + 50uM 2-mercaptoethanol (BME, Cat. #31350-010, Gibco, ThermoFisher Scientific) supplemented with 10uM fluorogenic Caspase-3 substrate (DEVD, Green) (Nucview^{®} 488, Cat. #10403, Biotium). Groups of labeled tumor cells (~2-10) were loaded into each of a plurality of sequestration pens on each of the two microfluidic chips (one for Mel 526 cells, and one for A375 cells) by tilting the microfluidic chip and allowing gravity to pull the tumor cells into the sequestration pens, providing a final concentration of the Caspase-3 substrate at 5uM at Time=0 for the assay for each microfluidic chip. The Caspase-3 substrate provides no fluorescent signal until cleaved, so at Time = 0, there was no fluorescent signal due to this reagent. T cells expanded against the SLC45A2 antigen, according to an endogenous T cell (ETC) protocol as described above, were flowed into each of the two microfluidic chips and gravity loaded on top of the tumor cells of each respective chip. Typically, after loading the tumor cells and T cells, each sequestration pen contained 0-5 tumor cells per T cell. As shown in the brightfield image (BF) for each time point and for each microfluidic chip containing SLC45A2-specific T cells and Mel526 tumor cells (FIG. 22A) and SLC45A2-specific T cells and A375 cells (FIG. 22B), respectively, populations of the cells are present. T cell media (Adv. RPMI + 10% Human AB serum + Gln + 50uM BME) supplemented with 5uM Caspase-3 substrate (Green) (Nucview 488 from Biotium) was perfused through the microfluidic channels on each microfluidic chip and images of the sequestration pens were taken every 30 minutes (starting from the end of the T cell loading) for a period of 7 hours. The CellTrace Far Red label and cleaved, now fluorescent Caspase-3 label were visualized using different fluorescent cubes (Cy5, FITC respectively).

The Mel526 melanoma cell line expresses the SLC45A2 tumor-associated antigen and was expected to be targeted and killed by the SLC45A2-specific T cells. The A375 melanoma cell line does not express the SLC45A2 tumor-associated antigen and was not expected to be targeted or killed by the SLC45A2-specific T cells, and thus was used as a negative control for T cell cytotoxicity.

**Results:** Mel526 tumor cells (FIG. 22A) and A375 tumor cells (FIG. 22B) exhibited the CellTrace Far Red signal (Cy5 fluorescent cube) but no signal associated with cleavage of the Caspase-3 substrate (Green fluorescent signal, FITC fluorescent cube) at the 1 hour time point. As time progresses, the green fluorescent signal associated with cleavage of the Caspase-3 substrate increased in the Mel526 tumor cells (FIG. 22A, up to 7 hr. time points shown) but not in the A375 tumor cells (FIG. 22B, up to 7 hrs. time point shown). The results indicate that the Mel526 tumor cells were efficiently killed by the SLC45A2-specific T cells, with 6 of 8 sequestration pens that contain Mel526 tumor cells in Fig. 22A showing high levels of Caspase-3 substrate cleavage. FIG. 22C showed quantification of the extent of antigen-specific Mel526 tumor cell killing vs the extent of cell killing of A375 non-targeted cells over the course of the experiment. A very low amount of SLC45A2-specific T cell (fractional) killing was observed for A375 non-targeted cells, whereas the targeted antigen-specific cell killing of the Mel 526 tumor cells by the SLC45A2-specific T cells approached a 0.25 fractional killing over the same 7 hr. time period. The exposure times for each of the fluorescent images was the same for each microfluidic chip and for each time point. The decrease seen over time of the Cy5 signal from the Cell Trace Far Red stain is often observed for any set of cells; observation of such decrease for each cell type was not unexpected.

### Example 25. Rapid expansion of antigen-specific T lymphocytes after bead stimulation and characterization of cellular product.

Typically after completion of antigen specific T lymphocyte activation as described in the preceding experiments, the antigen specific enriched T cells were sorted by FACS on an FACSAria Fusion System (Becton Dickinson, San Jose, CA) after staining 30min RT in FACS buffer (1XDPBS w/o Ca²⁺Mg²⁺ (Cat. # 4190250, ThermoFisher), 5mM EDTA (Cat. # AM9260G, ThermoFisher), 10mM HEPES (Cat. # 15630080, ThermoFisher), 2% FBS) with anti-CD8-PerCPCy5.5 (Clone RPA-T8, 301032,Biolegend, San Diego, CA), Tetramer-PE (MBL International, Woburn, MA) specific to the antigen, and Zombie NIR (Cat. # 423106, Biolegend, San Diego, CA) to exclude dead cells. Desired cells were purity sorted by gating: size, singles, live, CD8 positive, and Tetramer positive into CTL media (Advanced RPMI (Cat. # 12633020, ThermoFisher), 1x Glutamax (Cat. # 35050079, ThermoFisher), 10% Human Serum (Cat. # MT35060Cl, ThermoFisher), 50uM b-Mercaptoethanol (Cat. # 31350010, ThermoFisher) with 2mM HEPES.

The sorted antigen-specific T cells were then expanded in at least one round of Rapid Expansion Protocol (REP), as described in Riddell, US 5,827,642. Lymphoblastoid Cell Line cells (LCL, the LCL cell line was a gift from Cassian Yee, M.D. Anderson Cancer Center) were irradiated with 100 Gy and PBMC from 3 donors were irradiated with 50 Gy using an X-ray irradiator. Irradiated cells were washed in RPMI containing 10% FBS and mixed in a ratio of 1:5 (LCL:PBMC). These irradiated cells were added to either FACS-sorted T cells (for a first cycle of REP), or to the product of a first cycle of REP in 200 to 500-fold excess. Cultures were set up in T cell media (Advanced RPMI, 10% Human AB Serum, GlutaMax, 50 uM b-mercaptoethanol) supplemented with 50 U/mL IL-2 (Cat. # 202-IL, R&D Systems) and 30 ng/mL anti-CD3 antibody (Cat. # 16-0037-85, ThermoFisher). Cells were fed with fresh IL-2 on days 2, 5 and 10, and expanded according to their growth rates.

Expansion is typically 1,000-fold during a first REP cycle. Expansion during REP1 varied highly (316 - 7,800-fold, data not shown). Inaccurate quantification of low input cell numbers may have contributed to this variability. Shown here in FIG. 23A is fold-expansion obtained from a second REP protocol following the first cycle (n = 20 experiments, 11 donors, 12 STIMs). Expansion ranged from about 200 up to about 2000 fold. However, there was no clear correlation between extent of expansion in REP1 and REP2 for a particular cell population in these experiments.

In FIG. 23B, the percentages of antigen-specific T cells in the REP populations are shown for the 20 experiments of the REP protocol. What was observed was that high percentages of antigen-specific T cells (% Ag+), typically ~90%, were maintained during at least two REP cycles. In contrast, Low %Ag+ after REP1 led to low %Ag+ after REP2.

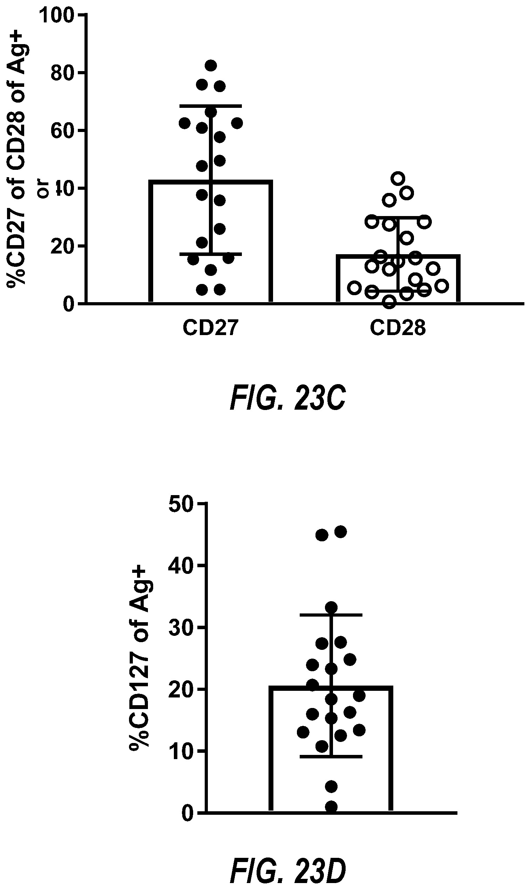

In FIG. 23C, the percentages of antigen-specific T cells also expressing co-stimulatory receptors CD27 and CD28, after the completion of REP2, are shown. In FIG. 23D, the percentages of antigen-specific T cells also expressing CD127, a marker for a central memory phenotype which can presage persistence in vivo, after the completion of REP2 is shown. While the distribution of expression of any of the markers was not tightly clustered, and some of the individual experiments showed low (e.g., a few percent) of cells that express the desired markers, the cellular products obtained in each of these experiments demonstrated sufficiently positive phenotype across all categories to render them candidates for in-vivo introduction. Some of the depressed values seen, such as expression of CD28, may be due to the extensive stimulation using CD28 ligands used during the activation cycles, leading to depressed expression of these surface markers.

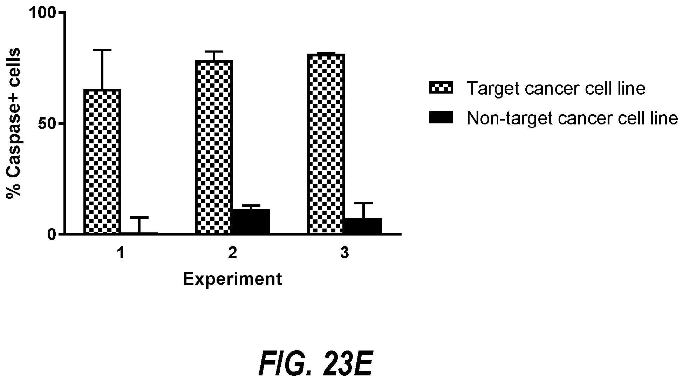

In FIG. 23E, the results of antigen-specific cytotoxicity assay for each of three individual cellular populations, after two rounds of REP, are shown. The assay was performed as described in Example 24, using Mel526 cells as the Target cancer cell line and A375 cells as the non-targeted cell line, wherein the antigen specific T cells were SLC45A2-specific T cells. In each experiment, more than 50% of the targeted Mel526 cells exhibited Caspase 3 triggered fluorescent signal, while none to a few percent of the A375 non-targeted cells exhibited apoptotic behavior as signaled by the fluorogenic cleavage product of the Caspase-3 substrate. Therefore, the activated T cells still exhibited antigen-specific cell killing behavior after all of the rounds of activation and expansion.

Therefore, the processes of activation via antigen presentation on a synthetic surface as described herein can provide well controlled, reproducible and characterizable cellular products suitable for use in immunotherapy. The antigen-presenting surfaces described herein provide lower cost of manufacture for these individualized therapies compared to currently available experimental processes.

### Example 26. Binding of Immunogenic and Non-Immunogenic Peptides to MHC Class I Complexes

### Experimental Procedure

To test binding of Immunogenic and Non-Immunogenic peptides to an MHC Class I complex, a peptide-HLA-A*02:01 complex with an initial peptide LMYAKRAFV (SEQ ID NO: 4) in the peptide binding groove was purchased. The initial peptide included a dinitrophenyl (DNP) moiety conjugated to the lysine at position 5. Two peptide antigens were then tested for their ability to bind to the MHC Class I complex: SLYSYFQKV (SEQ ID NO: 5) derived from SLC45A2 and SLLPIMWQLY (SEQ ID NO: 6) derived from TCL1. The peptides were resuspended in DMSO to 5 mg/mL. The peptides were then further diluted ten-fold in PBS. To set up 0.05 mL peptide switching reactions, 20 micromolar SLYSYFQKV (SEQ ID NO: 5) or SLLPIMWQLY (SEQ ID NO: 6) peptide, 1 micromolar HLA-A*02:01 (with initial peptide) and 1 mM Glycl-Methionine (exchange factor) were mixed in Assay Buffer (PBS without Magnesium and Calcium, supplemented with 2 mM EDTA and 0.1% BSA). In addition, a control peptide switching reaction using the peptide ELAGIGILTV (SEQ ID NO: 7) was set up; this peptide is known to bind with high affinity to HLA-A*02:01 and is used to determine "complete" peptide exchange. The peptide switching reactions proceeded at room temperature for 4 hours, then the switched complexes were stored at 4°C until further use.

Samples of unswitched peptide-MHC and switched peptide-MHC were then captured on Streptavidin-coated DynaBeads (ThermoFisher). About 10⁷ DynaBeads per switching reaction were washed once with 1 mL of Assay Buffer and then captured on a magnetic rack. The peptide-MHC complexes were diluted to 0.83 micorgrams/mL in Assay Buffer and used to resuspend the beads captured on the magnetic rack. The beads were mixed at 2,000 rpm for four minutes to capture the peptide-MHC complexes. The beads were again captured on a magnetic rack, washed once with 1 mL of Assay Buffer, and resuspended at about 10⁸ beads /mL. The peptide-MHC beads were then stored at 4°C until they were analyzed for peptide exchange.

To quantify peptide exchange, a FITC-conjugated anti-DNP antibody specific for the DNP-conjugated initial peptide was added to a sample of each captured peptide-MHC. About 2 x 10⁵ beads of either unswitched peptide-MHC, control switched peptide-MHC, or the test switched peptide-MHCs were diluted into 0.1 mL of Assay Buffer in 1.5 mL microcentrifuge tubes. One microliter of FITC-conjugated anti-DNP antibody and one microliter of an APC-conjugated, conformationally sensitive antibody which only recognizes pMHCs in the folded, complex conformation (Clone W6/32, Biolegend) was then added to each tube. The samples were stained for 30 minutes in the dark. The beads were captured on a magnetic rack, the staining solution was removed, and then the beads were washed with 1 mL of Assay Buffer. Each bead sample was resuspended in Assay Buffer and transferred to a 5 mL Polystrene tube. The staining for pre-assembled peptide and intact pHLA complexes were detected by Flow Cytometry on a FACSCelesta with High-Throughput Sampler (BD Biosciences). The beads were identified by Forward Scatter- and Side Scatter-Amplitudes. Approximately 5,000 bead events were recorded for each sample. The Median Fluorescence Intensity (MFI) in the APC and FITC channels of each sample was then recorded.

To quantify peptide switching, the MFI of the unswitched sample was set as zero switching, and the MFI of the ELAGIGILTV (SEQ ID NO: 7) switched sample was set as 100%. The MFI of the test peptides (as determined using the FITC channel and the FITC-conjugated anti-DNP antibody) was then used to determine the percent switching according to the following formula:$100 * \left[MFI\left(unswitched\right)-MFI\left(test peptide switching\right)]/[MFI\left(unswitched\right)-MFI\left(control switching\right)\right] .$ The MFI measurements determined using the APC channel and the APC-conjugated, conformationally sensitive antibody are not used in the formula (and, thus, are not required for the experimental measurement of peptide switching/binding). However, the APC signal can be useful in that it provides an indication that the MHC complexes on the beads remain properly folding following the peptide exchange reaction.

### Results

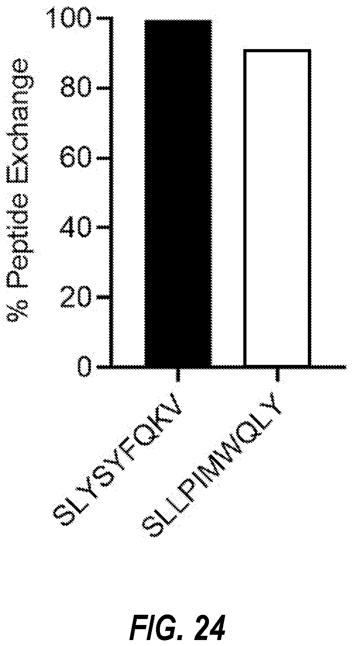

The quantitation of the peptide switching indicated that both the Immunogenic SLC45A2-derived and Non-Immunogenic TCL1-derived peptides were able to bind to HLA-A*02:01 (Fig. 24). SLC45A2-derived peptide switched nearly completely, relative to the control peptide (about 99% switching). The TCL1-derived peptide did not switch as efficiently, but still was able to generate about 90% peptide switching.

### Variations

The foregoing determination of peptide switching can be performed with any peptide antigen of interest, a different initial peptide (e.g., any initial peptide disclosed herein), and any of the exchange factors disclosed herein. Any form of initial peptide labeling could be employed, including direct conjugation with a fluorescent label; and use of the APC-conjugated, conformationally sensitive antibody (and the related MFI measurements) can be discarded. Furthermore, the experiment can be readily adapted to measurement of peptide switching on MHC Class II complexes.

### Example 27. Peptide Binding and Stability Under Culture Conditions

### Experimental Procedure

To assess the stability of the pMHC Class I-peptide antigen complexes under the conditions that are used to culture T Cells, pMHC Class I complexes were first bound to beads. Biotinylated HLA-A*02:01 complexes loaded with either SLYSYFQKV (SEQ ID NO: 5) or SLLPIMWQLY (SEQ ID NO: 6) peptides were diluted to 0.83 micrograms/mL in Assay Buffer (PBS with 2 mM EDTA and 0.1% Bovine Serum Albumin). To 0.6 mL of the pMHC solutions in microcentrifuge tubes, 10⁷ Streptavidin-coated DynaBeads (M-280, ThermoFisher) were added. The beads were mixed in the pMHC solution for 4 minutes at 2,000 rpm on a ThermoMixer (Eppendorf). The pMHC-beads were then captured on a magnetic rack, and the solution containing unbound pMHC removed by aspiration. 1 mL of Assay Buffer was added to the tubes, and then aspirated. The beads were then resuspended in 0.1 mL of Assay Buffer. The beads were then stored at 4°C until use.

To wells of a 96-well, round-bottom microplate, 0.2 mL of T Cell Culture Media (Advanced RPMI, 10% Human AB Serum, 1 mM GlutaMax) was added and equilibrated to 37°C in a standard tissue culture incubator. To three wells each, four microliters of each pHLA-beads was added to a well. The process of adding beads to wells was repeated at time intervals resulting in beads that were held in the media at 37°C for 48, 32, 24, 16, 8, 4, 2, and 1 hr. The plate was centrifuged at 400g for 5 minutes, and the media removed by flicking the plate. A sample of beads held at 4°C for the duration of the time course was then added to three wells to create the 0 hr time point.

An APC-conjugated, conformationally sensitive antibody which only recognizes pMHC molecules in the folded, complex conformation (Clone W6/32, Biolegend) was then added to each well. The antibody was diluted 50-fold from the manufacturer stock into Assay Buffer, and 0.05 mL of antibody mixture was added to each well. The samples were stained for 30 minutes at room temperature under foil. The plate was centrifuged, and the staining solution removed by flicking the plate. 0.2 mL of Assay Buffer was added to each well of the plate, which was again centrifuged. The plate was flicked to remove the Assay Buffer, and each well resuspend in 0.15 mL of FACS buffer.

Antibody binding to the beads was then detected on a FACSCelesta with High-Throughput Sampler (BD Biosciences). Beads were identified by Forward Scatter- and Side Scatter-Amplitudes. Approximately 25,000 bead events were recorded for each sample. The Median Fluorescence Intensity (MFI) in the APC channel for the pHLA-beads in each sample was then recorded. The MFIs were then plotted against the time spent at 37°C for each sample. The resulting decay curves were then fitted to an exponential decay curve using the curve_fit module in SciPy, a freely available Scientific Computing package for Python. The half-lives for the pHLA complexes were then calculated from the fitted decay constant.

### Results

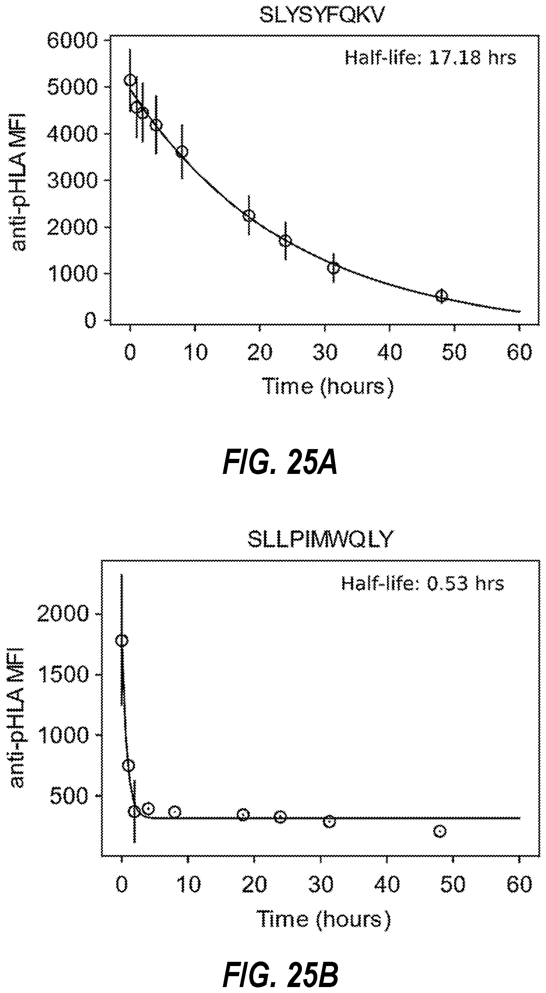

Results are shown in Figs. 25A-B for SLYSYFQKV (SEQ ID NO: 5) and SLLPIMWQLY (SEQ ID NO: 6). The half-life of the SLYSYFQKV (SEQ ID NO: 5)-HLA-A*02:01 complex was estimated to be about 17 hours. The half life of the SLLPIMWQLY (SEQ ID NO: 6)-HLA-A*02:01 complex was estimated to be about 0.5 hours.

### Variations

The foregoing determination of MHC Class I-peptide antigen complex stability was performed with MHC Class I complexes that were folded with their respective peptide antigens. However, the same experimental measurement of stability could be performed with MHC complexes that undergo a peptide exchange reaction of the type described herein (e.g., as described in Example 26). Furthermore, the determination of complex stability can be performed with any peptide antigen of interest, and the experiment can be readily adapted to measurement of MHC Class II-peptide antigen complex stability.

### Example 28. Preparation and Use of Antigen-Presenting Beads

### Experimental Procedure

Antigen-presenting beads presenting the SLC45A2-derived peptide SLYSYFQKV (SEQ ID NO: 5) were prepared by two procedures. In the first procedure, pre-assembled Biotinylated peptide-HLA-A*02:01 complexes bearing the SLYSYFQKV (SEQ ID NO: 5) peptide antigen were purchased from a manufacturer (Biolegend, custom order). In the second procedure, Biotinylated peptide-HLA-A*02:01 complexes bearing the SLYSYFQKV (SEQ ID NO: 5) peptide antigen were prepared by first incubating SLYSYFQKV (SEQ ID NO: 5) peptide with an exchange factor and HLA-A*02:01 complexes pre-assembled with an initial peptide. Lyophilized SLYSYFQKV (SEQ ID NO: 5) peptide (GenScript) was dissolved in DMSO to 5 mg/mL. The peptide antigen was then further diluted ten-fold in PBS. To setup a 0.05 mL peptide switching reaction, 20 micromolar peptide, 1 micromolar HLA-A*02:01 and 1 mM Glycl-Methionine (exchange factor) were mixed in Assay Buffer. The peptide switching reaction proceeded at room temperature for 4 hours, then the switched MHC complexes were stored at 4 C until further use.

The pre-assembled peptide-MHC and peptide switched peptide-MHC complexes were then used to prepare APBs. Samples of about 1.2 x 10⁷ Streptavidin-coated DynaBeads (ThermoFisher) were washed once with 1 mL of Assay Buffer, then resuspended in Assay Buffer with either pre-assembled peptide-MHC or switched peptide-MHC at 0.83 micrograms/mL. The beads were mixed at 2,000 rpm for four minutes to capture peptide-MHC. The beads were captured on a magnetic rack, and the peptide-MHC functionalization mixture was then removed by aspiration. A mixture of Biotinylated anti-CD28 (Biolegend) and anti-CD2 (Biolegend) was then added to the beads (1:1 anti-CD28:CD2 at 5 micrograms/mL total antibody). The beads were again mixed at 2,000 rpm for four minutes. Beads were captured on a magnetic rack, and the antibody functionalization mixture was removed by aspiration. The beads were washed once with Assay Buffer, resuspended at a density of about 1e8 beads per mL, and then stored at 4°C until use.

To expand antigen-specific T Cells with the APBs, CD8⁺ T Cells were isolated from PBMCs isolated from normal, healthy donors according to the manufacturer's recommended protocol (EasySep, StemCell Technologies). The CD8+ T Cells were split into two samples: one for the APBs prepared with pre-assembled peptide-MHC, and one for the APBs prepared with switched peptide-MHCs. The two types of APBs were mixed with the isolated CD8+T Cells at a ratio of 1 Cell:1 APB in T Cell Culture Media with 30 ng/mL IL-21. After mixing the cells and beads in culture media, 0.2 mL per well was distributed into the wells of a tissue culture-treated, 96-well, round-bottom microplate. The plates were then incubated in a standard 5% CO₂, 37°C incubator for two days. After two days in culture, IL-21 was diluted to 150 nanograms/mL in growth media. 50 microliters of IL-21 diluted in media is added to each well, and the plate was returned to the incubator additional culture.

After a total of seven days of culture, the cells were then restimulated with appropriate APBs. From each well of the plate, 0.05 mL of media were removed. IL-21 was diluted to 150 ng/mL in fresh media, and APBs were added to the IL-21/media mixture at a final density of 4 x 10⁶ APBs/mL. 50 microliters of this IL-21/APB/media mixture were added to each well, resulting in an additional 2 x 10⁵ APBs being added to each well. The plates were then returned to the incubator.

The next day, the plates were removed from the incubator, and 50 microliters of media again removed from each well. IL-2 (R&D Systems) was diluted into fresh media to 50 Units/mL. To this media containing IL-2, IL-7 (R&D Systems) was added at a final concentration of 12.5 ng/mL. 50 microliters of this IL-2/IL-7/media mixture was added to each well, and the well was returned to the incubator.

The next day, the plate was removed from the incubator, and 50 microliters of media again removed from each well. IL-21 was diluted into fresh media to 150 nanograms/mL. 50 microliters of this IL-21/media mixture was added to each well, and the well returned to the incubator.

After culturing the cells for an additional 5 days, the cells were analyzed for antigen-specific T Cell expansion and expression of memory precursor surface markers (CD45RO, CD28 and CD127).

### Results

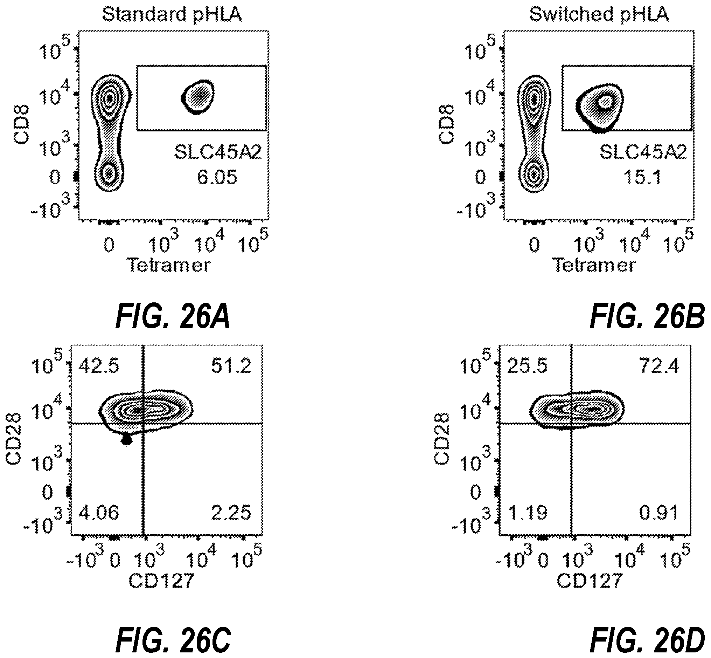

Analysis of the stimulated wells of CD8+ T Cells indicated that the APBs generated using the switched peptide-MHCs successfully generated Antigen Specific T Cell colonies (Figs. 26A-D). Analysis of the Antigen-Specific T Cells indicated that the cells expressed high levels of CD45RO, CD28 and CD127, which indicates the cells had taken on a Central Memory Precursor T Cell phenotype.

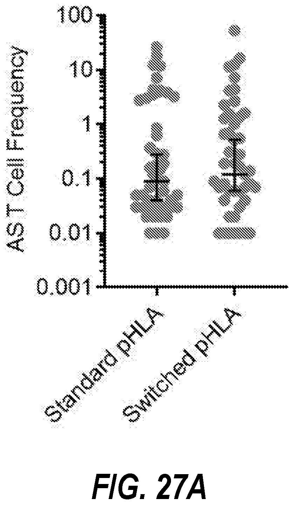

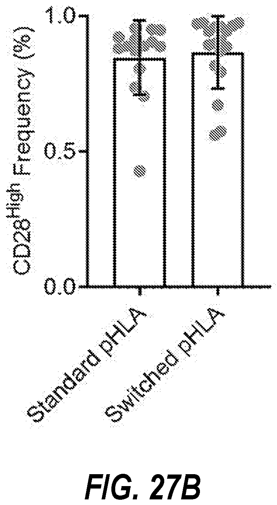

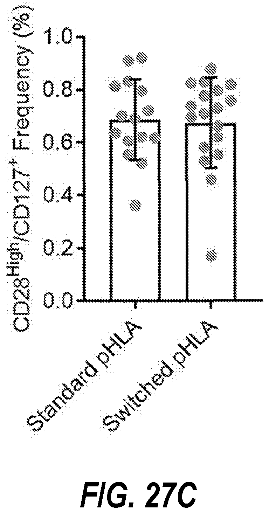

Comparing the APBs prepared from switched-peptide-MHCs vs conventional MHCs, we observed that the number of Antigen Specific (AS) T Cell colonies (wells in which the Antigen Specific T Cells expanded to greater than 0.5% of all cells in the well) (not shown) and the frequency of Antigen Specific T Cells in those colonies were similar using both types of APBs (Fig. 27A). In addition, the frequency of CD45RO+ Antigen Specific T Cells expressing high levels of CD28 was consistently high, indicating that the switched peptide APBs supported IL-21 mediated support of CD28 expression (Fig. 27B). In addition, the number of CD127+ Antigen Specific T Cells in the CD28High population was consistent between APB types (Fig. 28C).

This data indicates that peptide switching can be used to generate APBs that efficiently expand Antigen Specific T Cells with a Central Memory Precursor phenotype.
The invention is further described by the following numbered paragraphs (paras):
1. A kit for generating an antigen-presenting surface, the kit comprising:
   (a) a covalently functionalized synthetic surface;
   (b) a primary activating molecule that includes a major histocompatibility complex (MHC) molecule configured to bind to a T cell receptor (TCR), and a first reactive moiety configured to react with or bind to the covalently functionalized surface;
      and
   (c) an initial peptide bound to the MHC molecule, wherein the initial peptide is non-immunogenic.
2. The kit of para 2 further comprising one or more of:
   at least one co-activating molecule that includes a second reactive moiety configured to react with or bind to the covalently functionalized surface, wherein each co-activating molecule is selected from a TCR co-activating molecule and an adjunct TCR activating molecule;
   a surface-blocking molecule capable of covalently binding to the covalently functionalized synthetic surface;
   a buffer suitable for performing an exchange reaction; and
   instructions for performing an exchange reaction wherein a peptide antigen displaces the exchange factor.
3. The kit of para 1 further comprising an exchange factor, wherein the exchange factor is provided separately from the primary activating molecule.
4. A method of forming a proto-antigen-presenting surface, the method comprising:
   synthesizing a plurality of major histocompatibility complex (MHC) molecules in the presence of initial peptide, thereby forming a plurality of complexes each comprising an MHC molecule and an initial peptide;
   wherein: the initial peptide is non-immunogenic; and (i) a plurality of primary activating molecules comprise the MHC molecules and first reactive moieties, or (ii) a plurality of primary activating molecules is prepared by adding first reactive moieties to the MHC molecules, and
   the method further comprises reacting the first reactive moieties of the plurality of primary activating molecules with a first plurality of binding moieties disposed on a covalently functionalized synthetic surface, thereby forming the proto-antigen-presenting surface.
5. The method of para 4 further comprising reacting the plurality of MHC molecules synthesized in the presence of the initial peptide with exchange factor and a peptide antigen.
6. A method of analyzing stability of a complex comprising a major histocompatibility complex (MHC) molecule and a peptide antigen, wherein the MHC molecule is configured to bind to a T cell receptor (TCR), the method comprising:
   contacting a plurality of the MHC molecules with the peptide antigen and an exchange factor, thereby forming peptide antigen-bound MHC molecules, wherein an initial peptide is bound to the MHC molecules before contact with the peptide antigen and exchange factor;
   wherein
   (i) a plurality of primary activating molecules comprise the MHC molecules and first reactive moieties or (ii) a plurality of primary activating molecules is prepared by adding first reactive moieties to the MHC molecules, and the method further comprises reacting the first reactive moieties of the plurality of primary activating molecules with a first plurality of binding moieties disposed on a covalently functionalized synthetic surface; and
   measuring total binding and/or an extent of dissociation of the peptide antigen from the MHC molecule.
7. The method of para 6, wherein measuring total binding and/or the extent of dissociation comprises measuring binding of an agent to the MHC molecule, wherein the agent specifically binds to (i) the initial peptide, and/or (ii) a peptide-bound conformation of the MHC molecule.
8. The method of para 6, wherein the agent does not recognize a peptide-unbound conformation of the MHC molecule.
9. The method of para 6, wherein the method further comprises determining one or more kinetic parameters of the peptide antigen-bound MHC molecule.
10. A method of analyzing stability of a plurality of complexes each comprising a histocompatibility complex (MHC) molecule and a peptide antigen, comprising performing the method of any one of paras 6-9 with each of a plurality of different peptide antigens.
11. The kit of any one of paras 1-3 or the method of any one of paras 4-9, wherein the initial peptide comprises at least 4 or 5 amino acid residues; or has a length of about 4, 5, 6, 7, 8, 9, 10, 11, 12, 13, 14, 15, 16, 17, or amino acid residues (e.g., ranging from 8 to 10 amino acid residues, or 13 to 18 amino acid residues).
12. The kit of any one of paras 1-3 or the method of any one of paras 4-9,, wherein the initial peptide comprises a lysine as the fourth or fifth amino acid residue.
13. The kit of any one of paras 1-3 or the method of any one of paras 4-9, wherein the initial peptide comprises a label attached to the fourth or fifth amino acid residue (e.g., lysine).
14. The kit of any one of paras 1-3 or the method of any one of paras 4-9, wherein the initial peptide has a sequence comprising or consisting of a sequence from a naturally occurring (e.g., mammalian or human) polypeptide.
15. The method or kit of any one of paras 1-9, wherein the sequence of the initial peptide comprises or consists of sequence from a cytoskeletal polypeptide, e.g., an actin or tubulin polypeptide, or sequence from a ribosomal polypeptide, e.g., the RPSA, RPS2, RPL3, RPL4, RPL5, RPL6, RPL7A, or RPP0 polypeptides.
16. The method or kit of any one of paras 1-9, wherein the initial peptide binds the MHC molecule with a half-life of at least about 4 hours.
17. The method or kit of any one of paras 1-9, wherein the covalently functionalized synthetic surface presents a plurality of azido groups.
18. The method or kit of any one of paras 1-9, wherein the covalently functionalized synthetic surface presents a plurality of biotin-binding agents, and wherein the first reactive moieties are configured to specifically bind to the biotin-binding agent.
19. The method of any one of paras 4-9, wherein a plurality of co-activating molecular ligands, each including a TCR co-activating molecule or an adjunct TCR activating molecule, are present on the covalently functionalized synthetic surface or are added to the covalently functionalized synthetic surface by reacting a plurality of co-activating molecules, each including second reactive moiety and a TCR co-activating molecule or an adjunct TCR activating molecule, with a second plurality of binding moieties of the covalently functionalized synthetic surface configured for binding the second reactive moieties.
20. A proto-antigen-presenting surface, the surface comprising:
   a plurality of primary activating molecular ligands, wherein each primary activating molecular ligand includes a major histocompatibility complex (MHC) molecule configured to bind to a T cell receptor (TCR) of a T cell and wherein an exchange factor or an initial peptide is bound to the MHC molecules, wherein the initial peptide is non-immunogenic; and
   a plurality of co-activating molecular ligands each including a TCR co-activating molecule or an adjunct TCR activating molecule.
21. The proto-antigen-presenting surface of para 20, wherein each of the plurality of primary activating molecular ligands and the plurality of co-activating molecular ligands is specifically bound to the antigen presenting surface.
22. The kit of any one of paras 1-3, the method of any one of paras 4-9, or the surface of para 20 or 21, wherein the exchange factor comprises Leu, Phe, Val, Arg, Met, Lys, Ile, homoleucine, cyclohexylalanine, or Norleucine as its C-terminal amino acid residue.
23. The kit of any one of paras 1-3, the method of any one of paras 4-9, or the surface of para 20 or 21, wherein the exchange factor comprises Gly, Ala, Ser, or Cys as its penultimate C-terminal residue.
24. The kit of any one of paras 1-3, the method of any one of paras 4-9, or the surface of para 20 or 21, wherein the exchange factor is 2 amino acid residues in length.
25. The kit of any one of paras 1-3, the method of any one of paras 4-9, or the surface of para 20 or 21, wherein the covalently functionalized synthetic surface or the proto-antigen-presenting surface further comprises at least one plurality of surface-blocking molecular ligands covalently attached to the surface:
   (i) each of the plurality of surface-blocking molecular ligands includes a hydrophilic moiety, an amphiphilic moiety, a zwitterionic moiety, and/or a negatively charged moiety;
   (ii) each of the plurality of surface-blocking molecular ligands includes a linker and a terminal surface-blocking group, optionally wherein the linkers of the plurality of surface-blocking molecular ligands are of the same length or are of different lengths; or
   (iii) each of the plurality of surface-blocking molecular ligands includes a linker and a terminal surface-blocking group, wherein the terminal surface-blocking group comprises a hydrophilic moiety, amphiphilic moiety, zwitterionic moiety, and/or negatively charged moiety, optionally wherein the linkers of the plurality of surface-blocking molecular ligands are of the same length or are of different lengths;
   (iv) each of the plurality of surface-blocking molecular ligands is covalently bound to the covalently functionalized synthetic surface or the proto-antigen-presenting surface and/or
   (v) the plurality of the surface-blocking molecular ligands and may include 2, 3, or 4 different surface-blocking groups and/or 2, 3, 4, or more different lengths of linkers, chosen in any combination.
26. The kit of any one of paras 1-3, the method of any one of paras 4-9, or the surface of para 20 or 21, wherein the covalently functionalized synthetic surface or the proto-antigen-presenting surface is a wafer, an inner surface of a tube, an inner surface of a microfluidic device, or a bead.
27. The surface, kit, or method of para 26, wherein the inner surface of the microfluidic device is within a chamber of the microfluidic device.
28. The surface, kit, or method of para 27, wherein the chamber is a sequestration pen and the microfluidic device further comprises a flow region for containing a flow of a first fluidic medium; and the sequestration pen comprises an isolation region for containing a second fluidic medium, the isolation region having a single opening, wherein the isolation region of the sequestration pen is an unswept region of the microfluidic device; and a connection region fluidically connecting the isolation region to the flow region; optionally wherein the microfluidic device comprises a microfluidic channel comprising at least a portion of the flow region.
29. The surface, kit, or method of para 28, wherein the microfluidic device comprises a microfluidic channel comprising at least a portion of the flow region, and the connection region comprises a proximal opening into the microfluidic channel having a width W_{con} ranging from about 20 microns to about 100 microns and a distal opening into the isolation region, and wherein a length L_{con} of the connection region from the proximal opening to the distal opening is at least 1.0 times a width W_{con} of the proximal opening of the connection region.
30. The surface, kit, or method of para 29, wherein the length L_{con} of the connection region from the proximal opening to the distal opening is at least 1.5 times the width W_{con} of the proximal opening of the connection region.
31. The surface, kit, or method of para 29, wherein the length L_{con} of the connection region from the proximal opening to the distal opening is at least 2.0 times the width W_{con} of the proximal opening of the connection region.
32. The surface, kit, or method of any one of paras 29, wherein the width W_{con} of the proximal opening of the connection region ranges from about 20 microns to about 60 microns.
33. The surface, kit, or method of any one of paras 29, wherein the length L_{con} of the connection region from the proximal opening to the distal opening is between about 20 microns and about 500 microns.
34. The surface, kit, or method of any one of paras 29, wherein a width of the microfluidic channel at the proximal opening of the connection region is between about 50 microns and about 500 microns.
35. The surface, kit, or method of any one of paras 29, wherein a height of the microfluidic channel at the proximal opening of the connection region is between 20 microns and 100 microns.
36. The surface, kit, or method of any one of paras 28, wherein the volume of the isolation region ranges from about 2 x 10⁴ to about 2 x 10⁶ cubic microns.
37. The surface, kit, or method of any one of paras 28, wherein the proximal opening of the connection region is parallel to a direction of the flow of the first medium in the flow region.
38. The surface, kit, or method of any one of paras 28, wherein the microfluidic device comprises an enclosure comprising a base, a microfluidic circuit structure disposed on the base, and a cover which collectively define a microfluidic circuit, and the microfluidic circuit comprises the flow region, the microfluidic channel, and the sequestration pen.
39. The surface, kit, or method of para 27, wherein the covalently functionalized surface or the proto-antigen-presenting surface includes a portion configured to exclude biotin-binding agent or biotin functionalities which is disposed at at least one surface of a microfluidic channel of the microfluidic device.
40. The surface of para 20 or 21, wherein the plurality of co-activating molecular ligands comprises TCR co-activating molecules and adjunct TCR activating molecules and a ratio of the TCR co-activating molecules to the adjunct TCR activating molecules of the plurality of co-activating molecular ligands is about 100:1 to about 1:100.
41. The kit of any one of paras 1-3, the method of any one of paras 4-9, or the surface of para 20 or 21, wherein the MHC molecule comprises a human leukocyte antigen A (HLA-A) heavy chain.
42. The kit of para 2, or the surface of para 20 or 21, wherein the TCR co-activating molecule includes a CD28 binding protein or a fragment thereof which retains binding ability with CD28.
43. The kit of para 2, or the surface of para 20 or 21, wherein the adjunct TCR activating molecule is configured to provide adhesion stimulation and/or the adjunct TCR activating molecular ligand includes a CD2 binding protein, anti-CD2 antibody, or a fragment thereof, wherein the fragment of the CD2 binding protein or anti-CD2 antibody retains binding ability with CD2.
44. The proto-antigen-presenting surface of para 20 or 21, wherein a ratio of the primary activating molecular ligands to the co-activating molecular ligands present on the antigen-presenting surface is about 1:10 to about 2:1, about 1:5 to about 2:1, about 1:2 to about 2:1, about 1:10 to about 1:1, about 1:5 to about 1:1, about 1:1 to about 2:1, or about 1:2 to about 1:1.
45. The proto-antigen-presenting surface of para 20 or 21, further including a plurality of adhesion stimulatory molecular ligands, optionally wherein each adhesive molecular ligand includes a ligand for a cell adhesion receptor comprising an ICAM protein sequence.
46. The proto-antigen-presenting surface of para 20 or 21, further including a plurality of growth-stimulatory molecular ligands, wherein each of the growth-stimulatory molecular ligands includes a growth factor receptor ligand.
47. The kit of any one of embodiments 1-3, further comprising a plurality of surface-blocking molecules, wherein the covalently functionalized surface further comprises a first additional plurality of binding moieties configured for binding the surface-blocking molecule.
48. A method of preparing an antigen-presenting surface comprising a peptide antigen, the method comprising reacting the peptide antigen with a proto-antigen-presenting surface according to any para 20 or 21, wherein the exchange factor or initial peptide is substantially displaced and the peptide antigen becomes associated with the MHC molecules.
49. The method of para 48, wherein the peptide antigen comprises a tumor-associated antigen.
50. The method of para 48, further comprising contacting a T lymphocyte with the antigen-presenting surface comprising the peptide antigen.
51. The method of para 50, wherein the T lymphocyte is from a subject in need of treatment for cancer.
52. The method of para 50, wherein the T lymphocyte is CD8⁺.
53. A population of T cells comprising activated T cells produced by the method of para 50.
54. A microfluidic device or pharmaceutical composition comprising the population of para 53.
55. A method of screening a plurality of peptide antigens for T-cell activation, the method comprising:
   reacting a plurality of different peptide antigens with a plurality of proto-antigen-presenting surfaces according to para 20 or 21, thereby substantially displacing the exchange factors or initial peptides and forming a plurality of antigen-presenting surfaces;
   contacting a plurality of T cells with the antigen-presenting surfaces; and
   monitoring the T cells for activation, wherein activation of a T cell indicates that a peptide antigen associated with the surface with which the T cell was contacted is able to contribute to T cell activation.
56. The method of para 55, wherein the proto-antigen-presenting surfaces are reacted separately with the plurality of different peptide antigens, thereby generating a plurality of different antigen-presenting surfaces, or wherein the proto-antigen-presenting surfaces are reacted separately with pools of members of the plurality of different peptide antigens, thereby generating a plurality of different antigen-presenting surfaces.

## Claims

1. A proto-antigen-presenting surface, the surface comprising:
a plurality of primary activating molecular ligands, wherein each primary activating molecular ligand includes a major histocompatibility complex (MHC) molecule configured to bind to a T cell receptor (TCR) of a T cell and wherein an exchange factor or an initial peptide is bound to the MHC molecules; and
a plurality of co-activating molecular ligands each including a TCR co-activating molecule or an adjunct TCR activating molecule.

2. The proto-antigen-presenting surface according to claim 1, wherein each of the plurality of primary activating molecular ligands and the plurality of co-activating molecular ligands is specifically bound to the antigen presenting surface.

3. The proto-antigen-presenting surface according to any preceding claim, wherein the exchange factor comprises: Leu, Phe, Val, Arg, Met, Lys, Ile, homoleucine, cyclohexylalanine, or Norleucine as its C-terminal amino acid residue; and/or a free N-terminal amine; and/or Gly, Ala, Ser, or Cys as its penultimate C-terminal residue; optionally wherein the exchange factor is 2, 3, 4, or 5 amino acid residues in length, preferably wherein the exchange factor is 2 amino acid residues in length.

4. The proto-antigen-presenting surface according to any preceding claim, wherein the initial peptide is non-immunogenic in the organism from which the MHC molecule is originated, optionally wherein the initial peptide comprises at least 4 or 5 amino acid residues, preferably wherein the initial peptide has a length of 4, 5, 6, 7, 8, 9, 10, 11, 12, 13, 14, 15, 16, 17, or 18 amino acid residues, more preferably the initial peptide has a length that ranges from 8 to 10 amino acid residues or 13 to 15 amino acid residues; comprises a lysine as the fourth or fifth amino acid residue; and/or binds the MHC molecule with a half-life of at least about 4, 6, 8, 10, 12, 14, 16, 18, 20, 24, 28, 32, 36, or 48 hours.

5. The proto-antigen-presenting surface according to any preceding claim, wherein the proto-antigen-presenting surface further comprises at least one plurality of surface-blocking molecular ligands covalently attached to the surface, optionally wherein:
(i) each of the plurality of surface-blocking molecular ligands includes a hydrophilic moiety, an amphiphilic moiety, a zwitterionic moiety, and/or a negatively charged moiety;
(ii) each of the plurality of surface-blocking molecular ligands includes a linker and a terminal surface-blocking group, optionally wherein the linkers of the plurality of surface-blocking molecular ligands are of the same length or are of different lengths;
(iii) each of the plurality of surface-blocking molecular ligands includes a linker and a terminal surface-blocking group, wherein the terminal surface-blocking group comprises a hydrophilic moiety, amphiphilic moiety, zwitterionic moiety, and/or negatively charged moiety, optionally wherein the linkers of the plurality of surface-blocking molecular ligands are of the same length or are of different lengths;
(iv) each of the plurality of surface-blocking molecular ligands is covalently bound to the proto-antigen-presenting surface; and/or
(v) the plurality of the surface-blocking molecular ligands includes 2, 3, or 4 different surface-blocking groups and/or 2, 3, 4, or more different lengths of linkers, chosen in any combination.

6. The proto-antigen-presenting surface according to any preceding claim, wherein the proto-antigen-presenting surface is a wafer, an inner surface of a tube, an inner surface of a microfluidic device, or a bead.

7. The proto-antigen-presenting surface according to any preceding claim, wherein the proto-antigen-presenting surface is at least one inner surface of a microfluidic device, preferably wherein the inner surface of the microfluidic device is within a chamber of the microfluidic device, more preferably wherein the chamber is a sequestration pen, optionally wherein the microfluidic device further comprises a flow region for containing a flow of a first fluidic medium; and the sequestration pen comprises an isolation region for containing a second fluidic medium, the isolation region having a single opening, wherein the isolation region of the sequestration pen is an unswept region of the microfluidic device; and a connection region fluidically connecting the isolation region to the flow region.

8. The proto-antigen-presenting surface according to any preceding claim, wherein the plurality of co-activating molecular ligands comprises TCR co-activating molecules and adjunct TCR activating molecules, optionally wherein a ratio of the TCR co-activating molecules to the adjunct TCR activating molecules of the plurality of co-activating molecular ligands is about 100:1 to about 1:100, about 10:1 to about 1:20, or about 10:1 to about 1:10.

9. The proto-antigen-presenting surface according to any preceding claim, wherein the MHC molecule includes an MHC protein sequence and a beta microglobulin, optionally wherein the MHC molecule comprises a human leukocyte antigen A (HLA-A) heavy chain, a human leukocyte antigen B (HLA-B) heavy chain, or a human leukocyte antigen C (HLA-C) heavy chain.

10. The proto-antigen-presenting surface according to any preceding claim, wherein the TCR co-activating molecule comprises a CD28 binding protein or a fragment thereof which retains binding ability with CD28; or an anti-CD28 antibody or fragment thereof, wherein the fragment retains binding activity with CD28.

11. The proto-antigen-presenting surface according to any preceding claim, wherein the adjunct TCR activating molecule is configured to provide adhesion stimulation and/or the adjunct TCR activating molecule comprises a CD2 binding protein or a fragment thereof, wherein the fragment retains binding ability with CD2; an anti-CD2 antibody or a fragment thereof, wherein the fragment retains binding activity with CD2; a CD2 agonist; a CD27 agonist; or a CD137 agonist.

12. The proto-antigen-presenting surface according to any preceding claim, wherein a ratio of the primary activating molecular ligands to the co-activating molecular ligands present on the antigen-presenting surface is about 1:10 to about 2:1, about 1:5 to about 2:1, about 1:2 to about 2:1, about 1:10 to about 1:1, about 1:5 to about 1:1, about 1:1 to about 2:1, or about 1:2 to about 1:1.

13. The proto-antigen-presenting surface according to any preceding claim, further including a plurality of adhesion stimulatory molecular ligands, optionally wherein each adhesive molecular ligand includes a ligand for a cell adhesion receptor comprising an ICAM protein sequence.

14. The proto-antigen-presenting surface according to any preceding claim, further including a plurality of growth-stimulatory molecular ligands, wherein each of the growth-stimulatory molecular ligands includes a growth factor receptor ligand.

15. A method of screening a plurality of peptide antigens for T-cell activation, the method comprising:
(i) reacting a plurality of different peptide antigens with a plurality of proto-antigen-presenting surfaces according to any preceding claim, thereby substantially displacing the exchange factors or initial peptides and forming a plurality of antigen-presenting surfaces;
(ii) contacting a plurality of T cells with the antigen-presenting surfaces; and
(iii) monitoring the T cells for activation, wherein activation of a T cell indicates that a peptide antigen associated with the surface with which the T cell was contacted is able to contribute to T cell activation.
